(19) Europäisches Patentamt
European Patent Office
Office européen des brevets

(11) EP 3 670 662 A1

(12) **EUROPEAN PATENT APPLICATION**

(43) Date of publication:
**24.06.2020 Bulletin 2020/26**

(21) Application number: **18214455.0**

(22) Date of filing: **20.12.2018**

(51) Int Cl.:
***C12P 19/14*** *(2006.01)* ***C12P 19/18*** *(2006.01)*
***C12P 19/26*** *(2006.01)* ***C12N 9/24*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BASF SE**
**67056 Ludwigshafen am Rhein (DE)**

(72) Inventors:
- **Baldenius, Kai-Uwe**
**68159 Mannheim (DE)**
- **Breuer, Michael**
**67056 Ludwigshafen (DE)**
- **Ruffer, Corinna**
**67056 Ludwigshafen (DE)**
- **Weingarten, Melanie**
**67056 Ludwigshafen (DE)**
- **Zschoche, Reinhard**
**67056 Ludwigshafen (DE)**
- **Seemayer, Stefan**
**67056 Ludwigshafen (DE)**
- **Nidetzky, Bernd**
**8010 Graz (AT)**
- **Schmoelzer, Katharina**
**8010 Graz (AT)**
- **Puhl, Michael**
**67056 Ludwigshafen (DE)**

(74) Representative: **BASF IP Association**
**BASF SE**
**G-FLP-C6**
**67056 Ludwigshafen (DE)**

(54) **ENZYMATIC HEXOSAMINIDATION OF LACTOSE**

(57) The present invention relates to methods for producing human milk oligosaccharide (HMO) core structures using glycosidases from family GH20 hexosaminidases. In particular, the present invention provides methods for producing lacto-*N*-triose II (LNT II) and/or lacto-*N*-tetraose (LNT) by reacting glucosamine-oxazoline and/or lacto-*N*-biose-oxazoline with lactose catalysed by an enzyme of the glycoside hydrolase family 20 (GH20) according to the classification of the Carbohydrate-Active-Enzymes (CAZy) database. Specific optimized enzymes are identified to catalyse the reactions.



Printed by Jouve, 75001 PARIS (FR)

## Description

The present invention relates to methods for producing human milk oligosaccharide (HMO) core structures using glycosidases from family GH20 hexosaminidases. In particular, the present invention provides methods for producing lacto-*N*-triose II (LNT II) and/or lacto-*N*-tetraose (LNT) by reacting glucosamine-oxazoline and/or lacto-*N*-biose-oxazoline with lactose catalysed by an enzyme of the glycoside hydrolase family 20 (GH20) according to the classification of the Carbohydrate-Active-Enzymes (CAZy) database. Specific optimized enzymes are identified to catalyse the reactions. Furthermore, the present invention also relates to the use of enzymes of the glycoside hydrolase family 20 (GH20) for producing lacto-*N*-triose II or lacto-*N*-tetraose.

Different *endo*-type glycosidases (e.g. Endo M, Endo A, chitinases) were applied for synthesis of the *N*-linked oligosaccharide core trisaccharide Man(β1-4)-GlcNAc(β1-4)-GlcNAc and derivatives thereof, using the corresponding disaccharide oxazoline as a donor substrate.[1,2] The *N*-linked oligosaccharide core trisaccharide Man(β1-4)-GlcNAc(β1-4)-GlcNAc was obtained only in 32% yield (6.4 mM) from disaccharide-oxazoline in the chitinase-catalyzed reaction, although a large excess of the acceptor substrate (32-fold) and 20% of acetone (v/v) were used.[2] Artificial chitin and other glycosaminoglycan derivatives have been synthesized by polymerization of modified disaccharide oxazolines using family GH18 chitinase and GH56 hyaluronidase as the catalyst.[3] The polymerization of disaccharide oxazoline with Endo A was also described.[4]

Hexosaminidases are hydrolases with often very low trans-glycosylation activity. In order to solve the hydrolysis problem and provide attractive yields, enzymes from different glycosid hydrolase families have been mutated to enhance trans-glycosylation activity and decrease or suppress hydrolysis. When optimizing the specific activity of glycoside hydrolases, however, also suitable donor-substrates and reaction conditions for the trans-glycosylation need to be identified to achieve high yields.

The hexosaminidases Bbhl (β-*N*-acetythexosaminidase) and LnbB (lacto-*N*-biosidase) from *B. bifidum* JCM1254 are members of the glycoside hydrolase family 20 (GH20) according to the classification of the Carbohydrate-Active-Enzymes (CAZy) database. The Carbohydrate-Active-Enzymes database is available at http://www.cazy.org in the version updated on 20 November 2018 (Lombard, V.; Golaconda Ramulu, H.; Drula, E.; Coutinho, P. M.; Henrissat, B., The carbohydrate-active enzymes database (CAZy) in 2013, Nucleic Acids Res., 2014, 42 (D1), D490-D495.). The database for automated carbohydrate-active enzyme annotation (dbCAN) provides annotation data, which is generated based on the family classification from CAZy database (http://csbl.bmb.uga.edu/dbCAN/index.php, Yin Y, Mao X, Yang JC, Chen X, Mao F and Xu Y, dbCAN: a web resource for automated carbohydrate-active enzyme annotation, Nucleic Acids Res. 2012 Jul; 40(Web Server issue):W445-51).The glycosylations to produce LNT II and LNT are shown in Scheme 1. Recently, Xiao and co-workers demonstrated biocatalytic synthesis of LNT II at moderate yield (37%) by trans-glycosylation, using wild-type Bbhl.[5] However, despite intensive reaction optimization (substrate concentration, organic solvent concentration, pH, temperature), the maximum yield was strongly compromised due to low donor to acceptor ratios and due to secondary hydrolysis of the LNT II. LnbB also showed trans-glycosylation activity, but LNT was obtained only in very low yield (<4%).[6] Other researchers have tried to improve the transglycosylation of lactose with N-Ac-glucosamine from N,N-diacetylchitobiose by introduction of certain mutations into GH20 hexosaminidases, but only managed to increase the LNT II yield from 0.5% to 5% while LNT II remained contaminated with at least two isomeric trisaccharides.[7]

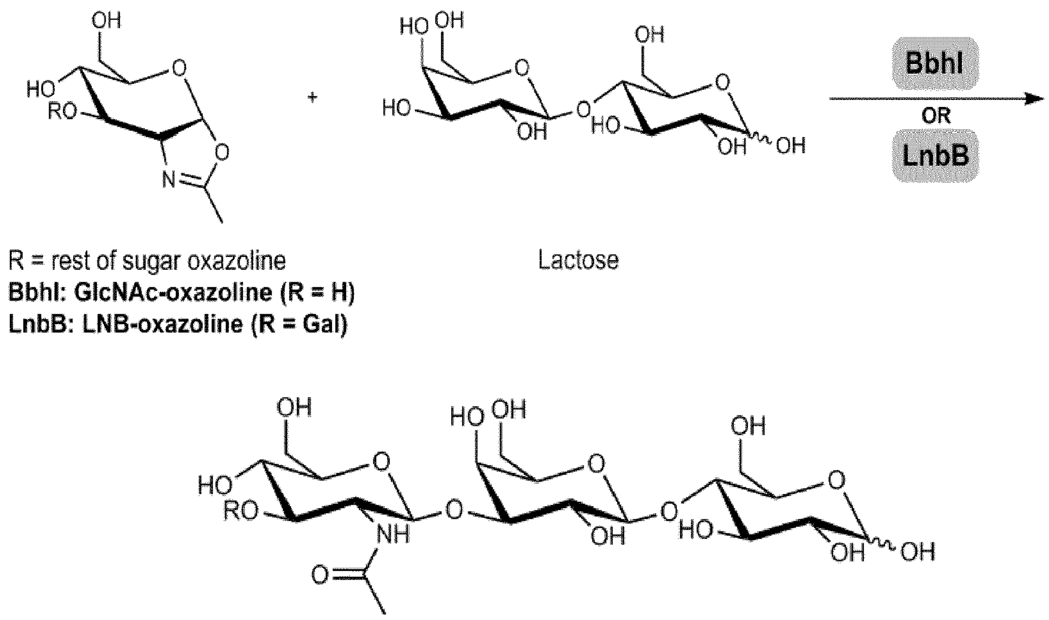


R = rest of sugar oxazoline
**BbhI: GlcNAc-oxazoline (R = H)**
**LnbB: LNB-oxazoline (R = Gal)**

Lactose

**Lacto-*N*-triose II (R = H)**
**Lacto-*N*-tetraose (R = Gal)**

**Scheme 1: *T*ransglycosylation reactions to produce lacto-*N*-triose II (LNT II) and lacto-*N*-tetraose (LNT).** Trans-glycosylation reaction from sugar oxazolines as a donor and lactose as an acceptor is shown. Wild-type and mutant enzymes of family GH20 hexosaminidases from *Bifidobacterium bifidum* JCM1254 were used. LNT II was synthesized from GlcNAc-oxazoline by the β-*N*-acetylhexosamindase BbhI, and LNT from lacto-*N*-biose-oxazoline (LNB-oxazoline) by the lacto-*N*-biosidase LnbB.

It was an objective of the present invention to provide techniques to improve the commercial usability of the trans-glycosylation of lactose to LNT II and LNT by BbhI and LnbB through optimizing the specific activity of the enzymes as well as identifying suitable donor-substrates and reaction conditions. In particular, it was an objective of the present invention to increase the yield, the final product concentration and the space-time-yield of the transglycosylation compared to the methods described in the prior art.

The objective is met by a method for producing lacto-*N*-triose II and/or lacto-*N*-tetraose comprising the step:

(i) reacting glucosamine-oxazoline and/or lacto-*N*-biose-oxazoline with lactose catalysed by an enzyme of the glycoside hydrolase family 20 (GH20) according to the classification of the Carbohydrate-Active-Enzymes (CAZy) database (http://www.cazy.org, updated on 20 November 2018) to obtain lacto-*N*-triose II and/or lacto-*N*-tetraose.

Preferably, step (i) is performed under conditions suitable for the production of lacto-*N*-triose II or lacto-*N*-tetraose or both.

In a preferred embodiment of the method according to the present invention, glucosamine-oxazoline and/or lacto-*N*-biose-oxazoline is contacted with lactose and at least one enzyme of the glycoside hydrolase family 20 (GH20) according to the classification of the Carbohydrate-Active-Enzymes (CAZy) database (http://www.cazy.org, updated on 20 November 2018) under conditions suitable for the formation of lacto-*N*-triose II or lacto-*N*-tetraose or both.

It was found in the context of the present invention, that the reaction of an enzyme of the glycoside hydrolase family 20 (GH20) according to the classification of the Carbohydrate-Active-Enzymes (CAZy) database with lactose and an oxazoline donor-substrate provides a promising way to obtain HMO core structures in suitable yields.

Glycoside hydrolase family 20 (GH20) enzymes are enzymes of the EC 3.2.1.- class according to the EC classification of enzymes (www.brenda-enyzmes.org, Schomburg et al., The BRENDA enzyme information system - From a database to an expert system, J Biotechnol, 2017, 261, 194-206; www.expasy.org, Artimo et al., ExPASy: SIB bioinformatics resource portal, Nucleic Acids Res. 40(W1): W597-W603, 2012). In the context of the present invention, an enzyme of the glycoside hydrolase family 20 (GH20) is an enzyme that is capable to perform a trans-glycosylation reaction. Preferably an enzyme of the glycoside hydrolase family 20 (GH20) is an enzyme of the EC class 3.2.1.52 or EC class 3.2.1.140, and more preferably a β-*N*-acetylhexosaminidase or a lacto-*N*-biosidase, even more preferably a β-*N*-acetylhexosaminidase.

The catalytic activity in this family of enzymes is assigned to the GH20 domain (PFAM code PF00728) which typically folds into a $(\beta/\alpha)_8$-barrel topology. Except for dispersin B from *A. actinomycetemcomitans* that presents only a single GH20 domain, the rest of structures show the catalytic domain accompanied by several domains with quite diverse functionalities: a non-catalytic domain, commonly named as GH20b, which is conserved in most GH20 enzymes although with unknown function, several lectin domains, carbohydrate binding domains, and other domains of unknown function.[34]

In a preferred embodiment of the various aspects of the present invention, an enzyme of the glycoside hydrolase family 20 (GH20) is an enzyme, which comprises a domain having the PFAM code PF00728 according to the Pfam database (https://pfam.xfam.org, Pfam 32.0, September 2018).

Sugar oxazolines are attractive donor substrates for trans-glycosylation by retaining glycosidases, which follow a substrate-assisted reaction mechanism. They represent a highly active substrate species mimicking the transition state. Shoda and co-workers demonstrated their practical one-step synthesis in good yield (>80%) from unprotected *N*-acetyl-2-amino sugars in water by using 2-chloro-1,3-dimethyl-1*H*-benzimidazol-3-ium chloride (CDMBI) as a dehydrative agent and $Na_3PO_4$ as a base.[8] Practical preparation of lacto-*N*-biose (LNB) in bulk quantities from sucrose and *N*-Acetylglucosamine (GlcNAc), using a one-pot four-enzyme reaction with lacto-*N*-biose phosphorylase (LNBP) from *Bifidobacterium bifidum* as a key enzyme, was reported by Kitaoka and co-workers.[9,10] However, so far, oxazoline donor substrates have not been successfully employed for transglycosylation with a GH20 enzyme. In Slámová et al., it is explained that the oxazoline ring is very unstable depending on the pH and it is described that no transglycosylation products were observed in the reaction of β-*N*-acetylhexosaminidases with oxazoline donor substrates.[11]

In a preferred embodiment of the present invention, in the method described above, step (i) is performed in an aqueous solution containing at least 50 g/L lactose, preferably at least 100 g/L lactose, particularly preferably at least 150 g/L lactose, most preferably at least 190 g/L lactose.

Surprisingly, it was found in the context of the present invention, that a high lactose concentration does not negatively influence the product yield despite the increasing viscosity. At the same time, the high concentrations allow an easier scale-up because of the smaller volumes that need to be handled.

Particularly preferred is a method as described above, which is performed under conditions that are free or essentially free of organic solvents.

"Essentially free" in this context preferably means that at any given point in time, in the reaction mixture of step (i) and, in particular in the purified and not purified product obtained by the method described above, there is 10 g/L or less, more preferably 1 g/L or less of an organic solvent present. The use of organic solvents such as DMSO or Ethanol is undesirable in any product intended for human consumption, especially in baby food, which represents a main application for the present invention. Advantageously, the method according to the present invention allows production of HMO core structures under reaction conditions that are free or essentially free of organic solvents.

In a further preferred embodiment of the present invention, in the method described above, the glucosamine-oxazoline and/or lacto-*N*-biose-oxazoline, respectively, is added to the lactose and the enzyme of the glycoside hydrolase family 20 (GH20) over a period of at least 20 minutes, preferably at least 60 minutes.

The glucosamine-oxazoline and/or lacto-*N*-biose-oxazoline may be added in batches over the respective period of time or, preferably, are added continuously over the respective period of time.

In one preferred embodiment of the method for producing lacto-*N*-triose II according to the invention, the enzyme of the glycoside hydrolase family 20 (GH20) is a β-*N*-acetylhexosaminidase, preferably β-*N*-acetylhexosaminidase of *Bifidobacterium bifidum* JCM1254 having an amino acid sequence of SEQ ID NO: 1 (Bbhl, GenBank accession number: AB504521.1) or an enzyme having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 1 and having β-*N*-acetylhexosaminidase activity, or a β-*N*-acetylhexosaminidase enzyme comprising an amino acid sequence of SEQ ID NO: 2, or a β-*N*-acetylhexosaminidase enzyme comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 2.

In one preferred embodiment of the method for producing lacto-*N*-tetraose according to the invention, the enzyme of the glycoside hydrolase family 20 (GH20) is a lacto-*N*-biosidase, preferably lacto-*N*-biosidase of *Bifidobacterium bifidum* JCM1254 having an amino acid sequence of SEQ ID NO: 3 (LnbB, GenBank accession number: EU281545.1), or an enzyme having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 and having lacto-*N*-biosidase activity, or a lacto-*N*-biosidase enzyme comprising an amino acid sequence of SEQ ID NO: 4, or a lacto-*N*-biosidase enzyme comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 4.

"Identity" in relation to comparison of two amino acid sequences herein is calculated by dividing the number of identical residues by the length of the alignment region, which is showing the shorter sequence over its complete length. This value multiplied by 100 gives "%-identity". To determine the %-identity between two amino acid sequences (i.e. pairwise sequence alignment), two sequences have to be aligned over their complete length (i.e. global alignment) in a first step. For producing a global alignment of two sequences, any suitable computer program, like program "NEEDLE" (The European Molecular Biology Open Software Suite (EMBOSS)), program "MATGAT" (Campanella, J.J, Bitincka, L. and Smalley, J. (2003), BMC Bioinformatics, 4:29), program "CLUSTAL" (Higgins, D:G: and Sharp, P.M. (1988), Gene, 73, 237-244), program "MegAlign Pro" (DNASTAR) or similar programs may be used. In lack of any program, sequences may also be aligned manually. After aligning two sequences, in a second step, an identity value shall be determined from the alignment. Depending on the applied method for %-identity calculation, different %-identity values can be calculated from a given alignment. Consequently, computer programs which create a sequences alignment, and in addition calculate %-identity values from the alignment, may also report different %-identity values from a given alignment, depending which calculation method is used by the program. Therefore, the following calculation of %-identity according to the invention applies: %-identity = (identical residues / length of the alignment region which is showing the shorter sequence over its complete length) *100. The calculation of %-identity according to the invention is exemplified as follows (the sole purpose of SEQ ID NO: 26 and SEQ ID NO 27 is to demonstrate calculation according to the invention; besides this purpose, said sequences are not inventive or functionally meaningful):

SEQ ID NO 26: TTTTTTAAAAAAAACCCCHHHCCCCAAARVHHHHHTTTTTTTT- length: 43 amino acids

SEQ ID NO 27: TTAAAAAAAACCCCHHCCCCAAADLSSHHHHHTTTT - length: 36 amino acids

Hence, the shorter sequence is sequence 2.

Producing a pairwise global alignment which is showing both sequences over their complete lengths results in

```
TTTTTTAAAAAAAACCCCHHHCCCCAAARV----HHHHHTTTTTTTT
      ||||||||||||| |  || ||||||||  ;    |||||||||
-------TTAAAAAAAACCCC--HHCCCCAAADLSSHHHHHTTTT-------
```

Producing a pairwise alignment which is showing the shorter sequence over its complete length according the invention consequently results in:

```
TTAAAAAAAACCCCHHHCCCCAAARV----HHHHHTTTT
||||||||| |||| ||  | ||| |||||  ;    ||| ||||||
TTAAAAAAAACCCC--HHCCCCAAADLSSHHHHHTTTT
```

The number of identical residues is 32, the alignment length showing the shorter sequence over its complete length is 37 (one gap is present which is factored in the alignment length of the shorter sequence). Therefore, %-identity according to the invention is: (32 / 37) * 100 = 86%.

The enzymes used in the context of the present invention may have 1, 2, 3, 4, 5, 6, 7, 8, 9 or 10 conservative amino acid substitutions.

Preferably, the enzymes used in the context of the present invention have non-naturally occurring amino acid sequences and represent artificial constructs, which have been taken out of their natural biological context and have

been specifically designed for the purpose as described herein. They may therefore be truncated, but functional versions of naturally occurring proteins and may carry certain modifications, for example those that simplify their biotechnological production or purification, e.g. a His-tag.

The invention also provides nucleotide sequences, in particular DNA sequences, and methods as described above making use of such nucleotide sequences, wherein the nucleotide sequences hybridize under stringent conditions with a DNA or RNA sequence, which codes for an enzyme of the glycoside hydrolase family GH20 as described above, in particular a β-*N*-acetylhexosaminidase or a lacto-*N*-biosidase according to SEQ ID NO: 1 or 3 or a truncated functional version of the respective enzymes according to SEQ ID NO: 2 or 4.

The term "hybridisation" as defined herein is a process wherein substantially complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

This formation or melting of hybrids is dependent on various parameters, for example the temperature. An increase in temperature favours melting, while a decrease in temperature favours hybridisation. However, this hybrid forming process is not following an applied change in temperature in a linear fashion: the hybridisation process is dynamic, and already formed nucleotide pairs are supporting the pairing of adjacent nucleotides as well. So, with good approximation, hybridisation is a yes-or-no process, and there is a temperature, which basically defines the border between hybridisation and no hybridisation. This temperature is the melting temperature (Tm). Tm is the temperature in degrees Celsius, at which 50% of all molecules of a given nucleotide sequence are hybridised into a double strand, and 50% are present as single strands.

The melting temperature (Tm) is dependent from the physical properties of the analysed nucleic acid sequence and hence can indicate the relationship between two distinct sequences. However, the melting temperature (Tm) is also influenced by various other parameters, which are not directly related with the sequences, and the applied conditions of the hybridization experiment must be taken into account. For example, an increase of salts (e.g. monovalent cations) is resulting in a higher Tm.

Tm for a given hybridisation condition can be determined by doing a physical hybridisation experiment, but Tm can also be estimated *in silico* for a given pair of DNA sequences. In this embodiment, the equation of Meinkoth and Wahl (Anal. Biochem., 138:267-284, 1984) is used for stretches having a length of 50 or more bases:

$$Tm = 81.5°C + 16.6 (\log M) + 0.41 (\% GC) - 0.61 (\% form) - 500/L$$

M is the molarity of monovalent cations, % GC is the percentage of guanosine and cytosine nucleotides in the DNA stretch, % form is the percentage of formamide in the hybridisation solution, and L is the length of the hybrid in base pairs. The equation is for salt ranges of 0.01 to 0.4 M and % GC in ranges of 30% to 75%.

While above Tm is the temperature for a perfectly matched probe, Tm is reduced by about 1°C for each 1% of mismatching (Bonner et al., J. Mol. Biol. 81: 123-135, 1973):

$$Tm = [\ 81.5°C + 16.6(\log M) + 0.41 (\%GC) - 0.61 (\%formamide) - 500/L\ ] - \%non\text{-}identity$$

This equation is useful for probes having 35 or more nucleotides and is widely referenced in scientific method literature (e.g. in: "Recombinant DNA Principles and Methodologies", James Greene, Chapter "Biochemistry of Nucleic acids", Paul S. Miller, page 55; 1998, CRC Press), in many patent applications (e.g. in: US 7026149), and also in data sheets of commercial companies (e.g. "Equations for Calculating Tm" from www.genomics.agilent.com).

The enzymes of the glycoside hydrolase family GH20 may be used in the method according to the present invention in free or immobilized form. They may e.g. be attached to a suitable carrier or beads or similar structures.

The hexosaminidases BbhI (β-*N*-acetylhexosaminidase) and LnbB (lacto-*N*-biosidase) from *B. bifidum* JCM1254 were identified as promising candidates for the enzymatic synthesis of human milk oligosaccharide (HMO) core structures. β-*N*-acetylhexosaminidase BbhI belongs to the enzyme class EC 3.2.1.52 and lacto-*N*-biosidase LnbB to the enzyme class EC 3.2.1.140. The sequences of the wild type enzymes from *Bifidobacterium bifidum* JCM1254 can be found in the GenBank database under the accession number AB504521.1 (published on March 31, 2010), which corresponds to SEQ ID NO: 1, and EU281545.1 (published on July 1, 2008), which corresponds to SEQ ID NO: 3.

Exemplary truncated constructs of the enzymes, which are fully functional, are represented by SEQ ID NO: 2 (truncated BbhI, aa 33-1599) and SEQ ID NO: 4 (truncated LnbB, aa 35-1064).

BbhI was previously described by Chen, X., et al. in "Efficient and regioselective synthesis of β-GalNAc/GlcNAc-lactose by a bifunctional transglycosylating β-N-acetylhexosaminidase from Bifidobacterium bifidum" Appl. Environ. Microbiol. 82, 5642-5652 (2016).[5] The Enzyme was obtained from genomic DNA of *B. bifidum* JCM 1254. Furthermore, Miwa, M., et al. (Cooperation of β-galactosidase and β-N-acetylhexosaminidase from bifidobacteria in assimilation of human milk oligosaccharides with type 2 structure. Glycobiology 20, 1402-1409 (2010)) used Bbh1 amplified by PCR using genomic DNA from *B. bifidum* JCM1254 as template.[12] The bifidobacterial strain was obtained from the Japan Collection of Microorganisms (JCM), RIKEN Bioresource Center, Japan.

LnbB was previously described in Wada, J., et al. "Bifidobacterium bifidum lacto-N-biosidase, a critical enzyme for the degradation of human milk oligosaccharides with a type 1 structure", Appl. Environ. Microbiol. 74, 3996-4004 (2008).[6] LnbB was amplified by PCR using genomic DNA from *B. bifidum* JCM1254 as a template. The bacterial strains were obtained from the Japan Collection of Microorganisms (JCM), RIKEN Bioresource Center, Japan.

In order to increase the trans-glycosylation activity with respect to the hydrolase activity, different mutations were tested. It was found that replacement in SEQ ID NO: 1 of $Asp^{746}$ by Ala, Glu or Gln gave very noticeable improvement with respect to secondary hydrolysis and little or no loss in productive activity. Purified preparations of wild-type and variant enzymes were obtained from *Escherichia coli* overexpression cultures, with a C-terminal $His_6$-tag for purification by metal chelate chromatography.

In a preferred embodiment, in the method for producing lacto-*N*-triose II described above, the β-*N*-acetylhexosaminidase has an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 1 (BbhI, GenBank accession number: AB504521.1) and carries a mutation at a position counted as position 746 of SEQ ID NO: 1 and/or at a position counted as position 827 of SEQ ID NO: 1, preferably carries a glutamic acid or an alanine or a glutamine at a position counted as position 746 of SEQ ID NO: 1 and/or carries a phenylalanine at a position counted as position 827 of SEQ ID NO: 1, or the β-*N*-acetylhexosaminidase comprises an amino acid sequence of any of SEQ ID NOs: 5 to 8 or comprises an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 5 to 8.

SEQ ID NOs: 5 to 8 represent the truncated versions of BbhI carrying the above described mutations. The mutations at position 746 and 827 of SEQ ID NO: 1 are not to be understood as absolute, but corresponding mutations at equivalent positions of enzymes belonging to the GH20 family or to the EC class EC 3.2.1.52 and having a glycosidase activity, in particular β-*N*-acetylhexosaminidases, e.g. homolog enzymes from other organisms, also provide the functionality according to the invention. Equivalent positions can be identified by a global sequence alignment as shown in Figure 16.

Preferably, the BbhI used in the context of the present invention has a non-naturally occurring amino acid sequence and represents an artificial construct, which has been taken out of its natural biological context and has been specifically designed for the purpose as described herein. It may therefore be a truncated, but functional version of the naturally occurring protein and may carry certain modifications, for example those that simplify its biotechnological production or purification, e.g. a His-tag.

The invention also provides nucleotide sequences, in particular DNA sequences, and methods as described above making use of such nucleotide sequences, wherein the nucleotide sequences hybridize under stringent conditions with a DNA or RNA sequence, which codes for an enzyme of the glycoside hydrolase family GH20 as described above, in particular a β-*N*-acetylhexosaminidase carrying a mutation at a position counted as position 746 of SEQ ID NO: 1 and/or at a position counted as position 827 of SEQ ID NO: 1, preferably carrying a glutamic acid or an alanine or a glutamine at a position counted as position 746 of SEQ ID NO: 1 and/or carrying a phenylalanine at a position counted as position 827 of SEQ ID NO: 1 or a truncated functional version of the enyzme according to any of SEQ ID NO: 5 to 8.

The trans-glycosylation activities of the BbhI enzymes in the LNT II synthesis reaction from GlcNAc-oxazoline or GlcNAc-β-*p*NP onto lactose were assayed. In a first step, a 10- or 20-fold excess of the acceptor substrate was used to promote product formation. Fig. 1 (GlcNAc-oxazoline) and the Fig. 2 (GlcNAc-β-*p*NP) compare reaction time courses for the BbhI variants to that of the wild-type enzyme. All product concentrations were observed analytically by HPLC analysis with UV detection at 195 nm (Fig. 3). The elution of the *N*-acetyl-2-amino sugars (LNT II, GlcNAc) could be monitored by measuring the absorbance of the N-acetyl-group. Table 1 summarizes the specific activities (trans-glycosylation, secondary hydrolysis) and selectivity parameters ($R_{TH}$ value) of the BbhI enzymes. In order to quantify the change in reaction selectivity under "synthesis conditions" in the presence of acceptor substrate, the parameter $R_{TH}$

(ratio of the specific trans-glycosylation activity of GlcNAc-to-lactose transfer to the specific activity of non-productive donor substrate hydrolysis (primary hydrolysis) in the presence of acceptor substrate) was used. Using wild-type BbhI and GlcNAc-oxazoline as a donor (Fig. 1a), LNT II was obtained in a maximum yield of about 52% (34 mM). The D746E mutant showed a significantly enhanced trans-glycosylation activity. The maximum LNT II yield was 87% (53 mM) (Fig. 1b). The Y827F mutant (Fig. 1c) showed a similar behavior as the D746E variant, giving a maximum yield of 80%. The Asp to Ala mutation at residue 746 completely abolished the hydrolysis activity (Fig. 1 d) of BbhI, but the trans-glycosylation activity was also reduced compared to the wild-type. Replacement of the $Asp^{746}$ by Gln resulted in almost complete abolishment of both secondary hydrolysis and trans-glycosylation activities (Fig. 1 e). The enzymes' inherent hydrolase activity caused also partly non-productive utilization of the GlcNAc-oxazoline substrate, thus restricting the LNT II yield under the conditions used. The D746E mutant exhibited a significantly enhanced trans-glycosylation activity over the primary hydrolysis, with a $R_{TH}$ value of 50 (Fig. 1f, Tab. 1). Comparable results to the LNT II synthesis from GlcNAc-oxazoline were obtained with GlcNAc-β-*p*NP as a donor substrate (Tab. 1, Fig. 2). In all cases, formation of LNT II was confirmed by TLC analyses (Figs. 2f, 4). In general, higher trans-glycosylation activities were observed with GlcNAc-oxazoline. Note, non-enzymatic hydrolysis of GlcNAc-oxazoline (ring opening of the oxazoline moiety) can also occur, depending on the pH.[4, 13] It was known from literature that the pH value strongly affected product formation by wild-type BbhI using GlcNAc-β-*p*NP.[5] The optimum pH in terms of product yield was at pH 5.8, with a sharp decrease at pH 9. Although sugar oxazolines have a higher stability at relatively high pH (>8, data not shown), pH 7.5 represented a good compromise between enzyme activity and donor substrate stability. Maximum yields were obtained within 10 min - 120 min. Note, using a 3.2-fold excess of GlcNAc-oxazoline over lactose, to counteract enzymatic and non-enzymatic GlcNAc-oxazoline hydrolysis, caused poor LNT II yields: The maximum yield was only 32% with the wild-type and 54% with the D746E variant (Fig. 5).

**Table 1:** Activity and selectivity parameters of wild-type and site-directed variants of BbhI

| BbhI | GlcNAc-oxazoline[a] | | | GlcNAc-β-*p*NP[b] | | |
|---|---|---|---|---|---|---|
| | Trans-glycosylation (U $mg^{-1}$) | $R_{T/H}$[c] | Secondary hydrolysis (U $mg^{-1}$) | Trans-glycosylation (U $mg^{-1}$) | $R_{T/H}$[c] | Secondary hydrolysis (U $mg^{-1}$) |
| WT | 38 | 3 | 1.9 | 12 | 3 | 1.1 |
| D746E | ≥3.7[d] | 50 | $6.0\times10^{-2}$ | $4.4\times10^{-1}$ | 40 | $2.7\times10^{-2}$ |
| D746A | $2.2\times10^{-1}$ | 8 | n.d. | $1.0\times10^{-2}$ | 6 | n.d. |
| D746Q | $2.6\times10^{-2}$ | ≥1 | n.d. | $1.3\times10^{-3}$ | 2 | n.d. |
| Y827F | 2.4 | 8 | $6.3\times10^{-2}$ | $2.6\times10^{-1}$ | 5 | $1.9\times10^{-2}$ |

n.d., not detectable.
[a]60 mM GlcNAc-oxazoline, 600 mM lactose, 37°C, pH 7.5.
[b]20 mM GlcNAc-β-*p*NP, 400 mM lactose, 20% DMSO, 55°C, pH 5.8.
[c]$R_{TH}$ is the ratio of the specific trans-glycosylation activity of GlcNAc-to-lactose transfer to the specific activity of non-productive donor substrate hydrolysis (GlcNAc-oxazoline or GlcNAc-β-*p*NP; primary hydrolysis) under synthesis conditions'.
[d]First data point was most probably outside the linear range.

Overall, Tab. 1 and Fig. 1f clearly show that the D746E variant of the β-*N*-acetylhexosaminidase BbhI was the best candidate for further optimization of LNT II synthesis. With GlcNAc-oxazoline as a donor substrate, this mutant had a specific trans-glycosylation activity of ≥3.7 U $mg^{-1}$ and showed strong preference for trans-glycosylation, as compared to primary and secondary hydrolysis. Compared to the reaction with GlcNAc-β-*p*NP, the trans-glycosylation activity was ~10-fold increased and no toxic *p*-nitrophenol was released as a byproduct. LNT II synthesis from GlcNAc-β-*p*NP by wild-type BbhI could be reproduced.[5] Mutants of chitinases from family GH18 were previously shown to have enhanced trans-glycosylation activity for chitooligomer synthesis, whereas the hydrolysis activity for the product was diminished.[14, 15] So far, only two engineered GH20 hexosaminidases with improved trans-glycosylation activities are known.[11, 7] Among them, a fungal chitinase with the Tyr470 replaced by Phe did not accept GlcNAc-oxazoline as a donor substrate in trans-glycosylation reaction.[11] LNT II synthesis from *N*,*N'*-diacetylchitobiose as a donor substrate by loop mutants of the β-*N*-acetylhexosaminidase HEX1 from metagenomic origin suffered from low yields (≤30%).[7] Furthermore, four regioisomers were formed and LNT II (4.7% yield) was not the main product. The present invention provides for the first time a GH20 glycosidase-like enzyme, which is as a useful catalyst for small molecule synthesis from sugar oxazoline.

The D746E mutant of BbhI shows remarkable trans-glycosylation activity with only marginal or no product hydrolysis activity. The combined use of this glycosidase mutated enyzme with a highly activated donor substrate, the sugar oxazoline, resulted in a 60-fold enhanced LNT II concentration when compared to the reference reaction with wild-type BbhI and GlcNAc-β-*p*NP.[5] The yield was doubled (based on donor and acceptor substrate) and the *TTN*mass value (388 $g_{LNT\ II\ gBbhI-1}$) was 1.6-fold improved at a 170-fold increased STY (562 g $L^{-1}$ $h^{-1}$). Further intensification of LNT II synthesis from GlcNAc-oxazoline could be realized by changing from batch to a continuous process mode. Benchmarked against the best LNT II syntheses using *N*-acetylglucosaminyltransferases, either *in vitro* or *in vivo,* the LNT II concentration of 515 mM represents an improvement by one to two orders of magnitude.[16, 17, 18, 19, 20, 21, 22, 23] The *TTN*mass was significantly enhanced (2- to 26-fold) compared to the *in vitro* syntheses of LNT II, using one-pot multienzyme systems for monosaccharide activation and the transfer process.[19, 21] So far, large scale production of LNT II could only be achieved with glycosyltransferase-based *in vivo* systems (whole-cell or fermentation-based).[17, 20, 24] However, much larger reaction volumes (20- to 100-fold) are required to obtain the product in quantities comparable to the transglycosylation process presented herein.[17, 20, 21]

For the first time, a readily scalable transglycosylation process is provided by the present invention, allowing the production of a small molecule, the HMO core structure LNT II, from a sugar oxazoline in bulk quantities. So far, there is only one report of a GH18 chitinase, showing the potential of a glycosidase for the synthesis of small molecules from a sugar oxazoline.[25] But the synthesis of the disaccharide *N*,*N'*-diacetylchitobiose (172 mM) from GlcNAc (3-fold excess) and GlcNAc-oxazoline by a chitinase from *Bacillus* sp. suffered from low yield (43%). Synthesis of tri- and tetrasaccharides from disaccharide oxazolines was also described.[26] However, significantly lower yields (50 - 70%, based on donor) and product concentrations (~10-fold) were obtained, compared to the transglycosylase process presented herein.[26] Typically, polyaddition of sugar oxazoline derivatives or step-wise synthesis of oligosaccharide-containing macromonomers was reported for chitinases.[26, 46]

In a preferred embodiment, in the method for producing lacto-*N*-tetraose described above, the lacto-*N*-biosidase has an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 (LnbB, GenBank accession number: EU281545.1) and carries a mutation at a position counted as position 320 of SEQ ID NO: 3 and/or at a position counted as position 419 of SEQ ID NO: 3, preferably carries a glutamic acid or an alanine at a position counted as position 320 of SEQ ID NO: 3 and/or carries a phenylalanine at a position counted as position 419 of SEQ ID NO: 3, or the lacto-*N*-biosidase comprises an amino acid sequence of any of SEQ ID NOs: 9 to 11 or comprises an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 9 to 11.

SEQ ID NOs: 9 to 11 represent the truncated versions of LnbB carrying the above described mutations. The mutations at position 320 and 419 of SEQ ID NO: 3 are not to be understood as absolute, but corresponding mutations at equivalent positions of enzymes belonging to the GH20 family or to the EC class EC 3.2.1.140 and having a glycosidase activity, in particular lacto-*N*-biosidases, e.g. homolog enzymes from other organisms, also provide the functionality according to the invention. Equivalent positions can be identified by a global sequence alignment as shown in Figure 16.

Preferably, the LnbB used in the context of the present invention has a non-naturally occurring amino acid sequence and represents an artificial construct, which has been taken out of its natural biological context and has been specifically designed for the purpose as described herein. It may therefore be a truncated, but functional version of the naturally occurring protein and may carry certain modifications, for example those that simplify its biotechnological production or purification, e.g. a His-tag.

The invention also provides nucleotide sequences, in particular DNA sequences, and methods as described above making use of such nucleotide sequences, wherein the nucleotide sequences hybridize under stringent conditions with a DNA or RNA sequence, which codes for an enzyme of the glycoside hydrolase family GH20 as described above, in particular a lacto-*N*-biosidase carrying a mutation at a position counted as position 320 of SEQ ID NO: 3 and/or at a position counted as position 419 of SEQ ID NO: 3, preferably carrying a glutamic acid or an alanine at a position counted as position 320 of SEQ ID NO: 3 and/or carrying a phenylalanine at a position counted as position 419 of SEQ ID NO: 3 or a truncated functional version of the enzyme according to any of SEQ ID NO: 9 to 11.

The trans-glycosylation activities of wild-type LnbB and the variants thereof were assayed under the same reaction conditions as described above for the BbhI. Full reaction time courses of LNT synthesis from LNB-oxazoline or LNB-*β*-*p*NP onto lactose (30 - 50-fold excess) are depicted in Fig. 6 (LNB-oxazoline) and the Fig. 7 (LNB-β-*p*NP). HPLC analysis was used for quantification (Fig. 8). Further confirmation of product identity was obtained by TLC analysis (Fig. 9). Table 2 compares the specific activities of the LnbB variants to that of the wild-type enzyme for both donor substrates.

Similar results were obtained with LNB-oxazoline and LNB-β-*p*NP, but LNB-oxazoline was the preferred substrate. As observed for BbhI, replacement in SEQ ID NO: 3 of the corresponding $Asp^{320}$ by Glu in LnbB was the most beneficial mutation. In contrast to wild-type LnbB, which showed strong product hydrolysis, secondary hydrolysis was completely abolished by this mutation. LNT was obtained from LNB-oxazoline in a yield of 30% (3.5 mM), and no product hydrolysis was detected within 22 h. Incubation with a 5-fold increased amount of D320E gave the same product yield correspondingly faster (Fig. 10).

**Table 2:** Activity and selectivity parameters of wild-type and site-directed variants of LnbB

| LnbB | LNB-oxazoline[a] | | LNB-β-*p*NP[b] | |
|---|---|---|---|---|
| | Trans-glycosylation (U $mg^{-1}$) | Secondary hydrolysis (U $mg^{-1}$) | Trans-glycosylation (U $mg^{-1}$) | Secondary hydrolysis (U $mg^{-1}$) |
| WT | 25 | $9.5\times10^{-1}$ | 4.0 | 1.0 |
| D320E | $2.2\times10^{-1}$ | n.d. | $2.1\times10^{-1}$ | $2.3\times10^{-4}$ |
| D320A | $8.9\times10^{-4}$ | n.d. | $8.0\times10^{-4}$ | n.d. |
| Y419F | $7.5\times10^{-2}$ | $4.8\times10^{-3}$ | $3.3\times10^{-2}$ | $3.8\times10^{-2}$ |

n.d., not detectable.
[a]12 mM LNB-oxazoline, 600 mM lactose, 37°C, pH 7.5.
[b]20 mM LNB-β-*p*NP, 600 mM lactose, 15% DMSO, 37°C, pH 5.8.

So far, the LnbB was identified as critical enzyme for the degradation of HMOs, liberating LNB from their non-reducing end.[6, 28, 29] But the wild-type enzyme was not a useful catalyst for the reverse reaction, the trans-glycosylation.[6] Efficient and practical enzymatic synthesis of LNT has not been achieved. A *Bacillus circulans* β-galactosidase gave LNT in 19% yield, together with an undesired LNT regioisomer.[16] Conversion of lactose into LNT by lacto-*N*-biosidase from *Aureobacterium* sp. L-101 was very inefficient (yield <4%).[16] LNT benzyl glycoside was produced from LNT II benzyl glycoside (synthesized from lactose benzyl glycoside and UDP-GlcNAc by an *N*-acetylglucosaminyltransferase) and UDP-Gal in 74% yield (~10 mM), using a GST-tagged *E. coli* (β1,3-galactosyltransferase fusion protein.[30] However, expensive nucleotide-activated sugars were needed. Large-scale production of LNT (173 g, 12.7 g $L^{-1}$) was only achieved by fed-batch cultivation of metabolically engineered *E. coli,* overexpressing tailored glycosyltransferases.[17] However, a one to one mixture of LNT and LNT II was obtained.[17]

In the context of the present invention, the transglycosylase mutant D320E was created. Synthesis of LNT from LNB-oxazoline without any detectable hydrolysis of the product was demonstrated.

In one preferred embodiment, the methods described above further comprise the step:

(ii) deactivating the enzyme of the glycoside hydrolase family 20 (GH20).

Deactivation of the enzyme of the glycoside hydrolase family 20 (GH20) allows further processing of the reaction product(s) obtained in the method according to the invention, without risking unwanted side reactions. For example, it is possible to further react lacto-*N*-triose II to lacto-*N*-tetraose or lacto-*N*-neotetraose. For example, β-galactosidases[16, 31] or *Leloir* glycosyltransferases[19, 20, 21] can be used, giving lacto-*N*-neotetraose (LNnT) or lacto-*N*-tetraose (LNT).

In a further preferred embodiment, the method for producing lacto-*N*-triose II described above therefore further comprises the step:

(iii) adding a β-galactosidase or a galactosyl transferase and UDP-galactose, to the mixture of step (i) or, preferably to the mixture of step (ii), and optionally adding further lactose to obtain lacto-*N*-tetraose or lacto-*N*-neotetraose.

Further reaction of lacto-*N*-triose II with a β-galactosidase provides LNT or LNnT by regioselective galactosyl-transfer.[16, 31] Likewise, reaction of lacto-*N*-triose II with a galactosyl transferase and UDP-galactose as a donor substrate provides LNT or LNnT. [19, 20, 21] Alternatively, UDP-glucose can be combined with an epimerase to provide the UDP-galactose donor substrate *in situ.*

The present invention also relates to the use of an enzyme of the glycoside hydrolase family 20 (GH20) according to the classification of the Carbohydrate-Active-Enzymes (CAZy) database (http://www.cazy.org, updated on 20 November 2018) for producing lacto-*N*-triose II or lacto-*N*-tetraose from lactose.

Preferred is the use of an enzyme of the glycoside hydrolase family 20 (GH20) for producing lacto-*N*-triose II as described above, wherein the enzyme of the glycoside hydrolase family 20 (GH20) is β-*N*-acetylhexosaminidase, preferably β-*N*-acetylhexosaminidase of *Bifidobacterium bifidum* JCM1254 having an amino acid sequence of SEQ ID NO: 1 (BbhI, GenBank accession number: AB504521.1) or an enzyme having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least

87%, at least 88%, at least 89%, at least 90%, at least 91%, at least 92%, at least 93%, at least 94%, at least 95%, at least 96%, at least 97%, at least 98%, at least 99% to SEQ ID NO: 1 and having β-*N*-acetylhexosaminidase activity, or a β-*N*-acetylhexosaminidase enzyme comprising an amino acid sequence of SEQ ID NO: 2, or a β-*N*-acetylhexosaminidase enzyme comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 2.

Particularly preferred is the use of an enzyme of the glycoside hydrolase family 20 (GH20) for producing lacto-*N*-triose II as described above, wherein the β-*N*-acetylhexosaminidase has an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 1 (Bbhl, GenBank accession number: AB504521.1) and carries a mutation at a position counted as position 746 of SEQ ID NO: 1 and/or at a position counted as position 827 of SEQ ID NO: 1, preferably carries a glutamic acid or an alanine or a glutamine at a position counted as position 746 of SEQ ID NO: 1 and/or carries a phenylalanine at a position counted as position 827 of SEQ ID NO: 1, or wherein the β-*N*-acetylhexosaminidase comprises an amino acid sequence of any of SEQ ID NOs: 5 to 8 or comprises an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 5 to 8.

Preferably, the Bbhl used in the context of the present invention has a non-naturally occurring amino acid sequence and represents artificial constructs, which has been taken out of its natural biological context and has been specifically designed for the purpose as described herein. It may therefore be a truncated, but functional version of the naturally occurring protein and may carry certain modifications, for example those that simplify its biotechnological production or purification, e.g. a His-tag.

Further preferred is the use of an enzyme of the glycoside hydrolase family 20 (GH20) for producing lacto-*N*-tetraose as described above, wherein the enzyme of the glycoside hydrolase family 20 (GH20) is lacto-*N*-biosidase, preferably lacto-*N*-biosidase of *Bifidobacterium bifidum* JCM1254 having an amino acid sequence of SEQ ID NO: 3 (LnbB, GenBank accession number: EU281545.1), or an enzyme having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 and having lacto-*N*-biosidase activity, or a lacto-*N*-biosidase enzyme comprising an amino acid sequence of SEQ ID NO: 4, or a lacto-*N*-biosidase enzyme comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 4.

Particularly preferred is the use of an enzyme of the glycoside hydrolase family 20 (GH20) for producing lacto-*N*-tetraose as described above, wherein the lacto-*N*-biosidase has an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 (LnbB, GenBank accession number: EU281545.1) and carries a mutation at a position counted as position 320 of SEQ ID NO: 3 and/or at a position counted as position 419 of SEQ ID NO: 3, preferably carries a glutamic acid or an alanine at a position counted as position 320 of SEQ ID NO: 3 and/or carries a phenylalanine at a position counted as position 419 of SEQ ID NO: 3, or wherein the lacto-*N*-biosidase comprises an amino acid sequence of any of SEQ ID NOs: 9 to 11 or comprises an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 9 to 11.

Preferably, the LnbB used in the context of the present invention has a non-naturally occurring amino acid sequence and represents an artificial construct, which has been taken out of its natural biological context and has been specifically designed for the purpose as described herein. It may therefore be a truncated, but functional version of the

naturally occurring protein and may carry certain modifications, for example those that simplify its biotechnological production or purification, e.g. a His-tag.

According to a further preferred embodiment of the use as described above, the enzyme of the glycoside hydrolase family 20 (GH20) is used in a method according to any of the embodiments described above.

The present invention describes a highly productive transglycosylation process, which is fit for process scale-up to enable production at demonstration scale. The current LNT II synthesis is performance-wise without precedent in preparation of HMO core-structures by enzymatic glycosylation. Significant intensification of biocatalysis compared to β-*N*-acetylhexosaminidase- and glycosyltransferase-catalyzed reactions, respectively, was achieved using the enzymes described above.[5, 16, 17, 18, 19, 20, 21, 22, 23] The simple recovery of LNT II in a purity of about 80% with mostly lactose as impurity is clearly beneficial for the scalability of the established process. A transglycosylation process allowing actual production in bulk quantities is novel and broadly relevant in the field. LNT II is one of the major building blocks of HMOs. [32]The simple and sophisticated LNT II synthesis described herein offers the entry to HMO structures. In addition, LNT and LNnT can be obtained using the enyzmes described above.

In the context if the present invention, certain mutants of BbhI and LnbB have been identified, which are highly useful in the production of HMO core structures.

The present invention therefore also relates to β-*N*-acetylhexosaminidase having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 1 (BbhI, GenBank accession number: AB504521.1) and carrying a mutation at a position counted as position 746 of SEQ ID NO: 1 and/or at a position counted as position 827 of SEQ ID NO: 1, preferably carrying a glutamic acid or an alanine or a glutamine at a position counted as position 746 of SEQ ID NO: 1 and/or carrying a phenylalanine at a position counted as position 827 of SEQ ID NO: 1, or β-*N*-acetylhexosaminidase comprising an amino acid sequence of any of SEQ ID NOs: 5 to 8 or comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 5 to 8.

Preferably, the BbhI according to the present invention has a non-naturally occurring amino acid sequence and represents an artificial construct, which has been taken out of its natural biological context and has been specifically designed for the purpose as described herein. It may therefore be a truncated, but functional version of the naturally occurring protein and may carry certain modifications, for example those that simplify its biotechnological production or purification, e.g. a His-tag.

Furthermore, the present invention also relates to lacto-*N*-biosidase having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 (LnbB, GenBank accession number: EU281545.1) and carrying a mutation at a position counted as position 320 of SEQ ID NO: 3 and/or at a position counted as position 419 of SEQ ID NO: 3, preferably carrying a glutamic acid or an alanine at a position counted as position 320 of SEQ ID NO: 3 and/or carrying a phenylalanine at a position counted as position 419 of SEQ ID NO: 3, or lacto-*N*-biosidase comprising an amino acid sequence of any of SEQ ID NOs: 9 to 11 or comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 9 to 11.

Preferably, the LnbB according to the present invention has a non-naturally occurring amino acid sequence and represents artificial constructs, which has been taken out of its natural biological context and has been specifically designed for the purpose as described herein. It may therefore be a truncated, but functional version of the naturally occurring protein and may carry certain modifications, for example those that simplify its biotechnological production or purification, e.g. a His-tag.

The invention also provides nucleotide sequences, in particular DNA sequences, wherein the nucleotide sequences hybridize under stringent conditions with a DNA or RNA sequence, which codes for an enzyme of the glycoside hydrolase family GH20 as described above, in particular a β-*N*-acetylhexosaminidase carrying a mutation at a position counted as position 746 of SEQ ID NO: 1 and/or at a position counted as position 827 of SEQ ID NO: 1, preferably carrying a glutamic acid or an alanine or a glutamine at a position counted as position 746 of SEQ ID NO: 1 and/or carrying a phenylalanine at a position counted as position 827 of SEQ ID NO: 1 or a lacto-*N*-biosidase carrying a mutation

at a position counted as position 320 of SEQ ID NO: 3 and/or at a position counted as position 419 of SEQ ID NO: 3, preferably carrying a glutamic acid or an alanine at a position counted as position 320 of SEQ ID NO: 3 and/or carrying a phenylalanine at a position counted as position 419 of SEQ ID NO: 3 or comprising an amino acid sequence according to any of SEQ ID NO: 5 to 11.

The present invention also provides an aqueous solution comprising an enzyme of the glycoside hydrolase family 20 (GH20) as defined above, lactose, preferably at least 50 g/L lactose, more preferably at least 100 g/L lactose, particularly preferably at least 150 g/L lactose, most preferably at least 190 g/L lactose and

a) glucosamine-oxazoline and/or lacto-*N*-biose-oxazoline; and/or

b) lacto-*N*-triose II and/or lacto-*N*-tetraose.

Preferably, the aqueous solution is free or essentially free of organic solvents.

"Essentially free" in this context preferably means that there is 10 g/L or less, more preferably 1 g/L or less of an organic solvent present in the solution.

In a preferred embodiment, the enzyme of the glycoside hydrolase family 20 (GH20) is a β-*N*-acetylhexosaminidase, preferably β-*N*-acetylhexosaminidase of *Bifidobacterium bifidum* JCM1254 having an amino acid sequence of SEQ ID NO: 1 (Bbhl, GenBank accession number: AB504521.1) or an enzyme having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 1 and having β-*N*-acetylhexosaminidase activity, or a β-*N*-acetylhexosaminidase enzyme comprising an amino acid sequence of SEQ ID NO: 2, or a β-*N*-acetylhexosaminidase enzyme comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 2.

In a further preferred embodiment, the enzyme of the glycoside hydrolase family 20 (GH20) is lacto-*N*-biosidase, preferably lacto-*N*-biosidase of *Bifidobacterium bifidum* JCM1254 having an amino acid sequence of SEQ ID NO: 3 (LnbB, GenBank accession number: EU281545.1), or an enzyme having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 and having lacto-*N*-biosidase activity, or a lacto-*N*-biosidase enzyme comprising an amino acid sequence of SEQ ID NO: 4, or a lacto-*N*-biosidase enzyme comprising an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 4.

Preferably, the enzyme(s) present in the aqueous solution have a non-naturally occurring amino acid sequence and represent artificial constructs, which have been taken out of their natural biological context and have been specifically designed for the purpose as described herein. They may therefore be truncated, but functional versions of the naturally occurring proteins and may carry certain modifications, for example those that simplify their biotechnological production or purification, e.g. a His-tag.

Particularly preferred is an aqueous solution as described above, wherein the β-*N*-acetylhexosaminidase has an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 1 (Bbhl, GenBank accession number: AB504521.1) and carries a mutation at a position counted as position 746 of SEQ ID NO: 1 and/or at a position counted as position 827 of SEQ ID NO: 1, preferably carries a glutamic acid or an alanine or a glutamine at a position counted as position 746 of SEQ ID NO: 1 and/or carries a phenylalanine at a position counted as position 827 of SEQ ID NO: 1, or wherein the β-*N*-acetylhexosaminidase comprises an amino acid sequence of any of SEQ ID NOs: 5 to 8 or comprises an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%,

at least 97%, at least 98%, at least 99% to any of SEQ ID NOs: 5 to 8.

Further particularly preferred is an aqueous solution as described above, wherein the lacto-*N*-biosidase has an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to SEQ ID NO: 3 (LnbB, GenBank accession number: EU281545.1) and carries a mutation at a position counted as position 320 o SEQ ID NO: 3 and/or at a position counted as position 419 of SEQ ID NO: 3, preferably carries a glutamic acid or an alanine at a position counted as position 320 of SEQ ID NO: 3 and/or carries a phenylalanine at a position counted as position 419 of SEQ ID NO: 3, or wherein the lacto-*N*-biosidase comprises an amino acid sequence of any of SEQ ID NOs: 9 to 11 or comprises an amino acid sequence having an amino acid sequence identity of at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% to any of SEQ ID NOs: 9 to 11.

**Short description of the Figures:**

**Figure 1: LNT II synthesis by wild-type BbhI and mutants thereof.** Time courses show synthesis from 60 mM GlcNAc-oxazoline using a 10-fold excess of lactose. (a) Wild-type, 0.23 μM; (b) D746E, 8.4 μM; (c) Y827F, 4 μM; (d) D746A, 18 μM; (e) D746Q, 9 μM. LNT II, filled circles; yield, open circles. (f) Trans-glycosylation *versus* primary hydrolysis. $R_{TH}$ is the ratio of the specific trans-glycosylation activity of GlcNAc-to-lactose transfer to the specific activity of non-productive GlcNAc-oxazoline hydrolysis (primary hydrolysis) under 'synthesis conditions'. For the wild type and each mutant three bars are shown, the left bar indicates the specific trans-glycosylation activity; the middle bar indicates the maximum LNT II yield; the right bar indicates the $R_{TH}$ value.

**Figure 2: LNT II synthesis from GlcNAc-β-*p*NP by wild-type BbhI and mutants thereof.** Time courses show synthesis from 20 mM GlcNAc-β-*p*NP using a 20-fold excess of lactose in the presence of 20% DMSO. (a) Wild-type, 0.12 μM; (b) D746E, 8.4 μM; (c) D746A, 24 μM; (d) D746Q, 8.6 μM; (e) Y827F, 8 μM. LNT II, filled circles; yield, open circles; pNP released, open triangles. (f) TLC-analysis of LNT II synthesis using BbhI enzymes. Reaction mixtures were 2-fold diluted. Carbohydrates were visualized by thymol-sulfuric acid reagent. *p*NP was detected by UV (254 nm). Lane 1, pNP; lane 2, lactose; lane 3, LNT II; lane 4, wild-type reaction after 1.5 h; lane 5, D746E reaction after 20 min; lane 6, Y975F reaction after 1.5 h; lane 7, D746A reaction after 8 h; lane 8, D746Q reaction after 6 h.

**Figure 3: HPLC analysis of LNT II synthesis from GlcNAc-oxazoline by wild-type BbhI and mutants thereof.** Overlay of HPLC-chromatograms showing maximum LNT II formation obtained by various enzyme variants of BbhI. Reaction mixtures contained 60 mM GlcNAc-oxazoline, a 10-fold excess of lactose and 0.23 - 18 μM of the enzymes. Samples were 10-fold (D746Q) and 25-fold diluted (all other enzymes), respectively. UV-detection at 195 nm was used.

**Figure 4: TLC analysis of LNT II synthesis from GlcNAc-oxazoline by wild-type BbhI and mutants thereof.** 130 mM (D746E) or 60 mM (all other enzymes) GlcNAc-oxazoline, 600 mM lactose and 0.23 - 18 μM of the enzymes were used. Carbohydrates were visualized by thymol-sulfuric acid reagent. GlcNAc was also detected by UV (254 nm) (a) Lane 1, GlcNAc; lane 2, lactose; lane 3, LNT II; lane 4, wild-type reaction after 1 h, 1:10; lane 5, D746E reaction after 50 min, 1:50; lane 6, D746A reaction after 5 h, 1:25; lane 7, galactose; lane 8, glucose. (b) Lane 1, GlcNAc; lane 2, lactose; lane 3, LNT II; lane 4, Y827F reaction after 50 min, 1:9.

**Figure 5: LNT II synthesis from GlcNAc-oxazoline by wild-type BbhI and the D746E mutant.** Time courses show synthesis from 64 mM GlcNAc-oxazoline and 20 mM lactose. (a) Wild-type, 0.23 μM; (b) D746E, 8.4 μM. LNT II, filled circles; yield, open circles.

**Figure 6: LNT synthesis by wild-type LnbB and mutants thereof.** Time courses show synthesis from 12 mM LNB-oxazoline using a 50-fold excess of lactose. (a) Wild-type, 0.5 μM; (b) D320E, 4 μM; (c) D320A, 20 μM; (d) Y419F, 4 μM. LNT , filled circles; yield, open circles; LNT II, filled triangles.

**Figure 7: LNT synthesis from LNB-β-*p*NP by wild-type LnbB and mutants thereof.** Time courses show synthesis from 20 mM LNB-β-*p*NP in the presence of 15% DMSO, using a 30-fold excess of lactose. (a) Wild-type, 0.5 μM; (b) D320E, 10 μM; (c) D320A, 20 μM; (d) Y419F, 4 μM. LNT, filled circles; yield, open circles; pNP released, open triangles. (e) TLC-analysis of LNT synthesis using LnbB enzymes. Reaction mixtures were 5-fold diluted. Carbohydrates were visualized by thymol-sulfuric acid reagent. Lane 1, LNB; lane 2, lactose; lane 3, LNT; lane 4, wild-type reaction after 10 min; lane 5, D320E reaction after 20 min; lane 6, Y419F reaction after 2.5 h; lane 7, D320A reaction after 21 h.

**Figure 8: HPLC analysis of LNT synthesis from LNB-oxazoline by wild-type LnbB and mutants thereof.** Overlay of HPLC-chromatograms showing maximum LNT formation obtained by various enzyme variants of LnbB. Reaction mixtures contained 12 mM LNB-oxazoline, a 50-fold excess of lactose and 0.5 - 20 μM of the enzymes. Samples were 5-fold diluted. UV-detection at 195 nm was used.

**Figure 9: TLC analysis of LNT synthesis from LNB-oxazoline by wild-type LnbB and mutants thereof.** 12 mM LNB-oxazoline, a 50-fold excess of lactose and 0.5 - 20 μM of the enzymes were used. Reaction mixtures were 5-fold diluted. Carbohydrates were visualized by thymol-sulfuric acid reagent. (a) Lane 1, GlcNAc; lane 2, LNB; lane 3, lactose; lane 4, LNT II; lane 5, LNT; lane 6, wild-type reaction after 10 min; lane 7, D320E reaction after 20 min; lane 8, Y419F reaction after 2.5 h; lane 9, D320A reaction after 21 h. (b) Lane 1, GlcNAc; lane 2, LNB; lane 3, LNT II; lane 4, LNT; lane 5, wild-type reaction after 0 min; lane 6, wild-type reaction after 10 min; lane 7, D320E reaction after 0 h; lane 8, D320E reaction after 1 h; lane 9, lactose.

**Figure 10: Synthesis of LNT by the LnbB D320E mutant.** Reaction mixture contained 12 mM LNB-oxazoline, a 50-fold excess of lactose and 20 μM D320E. LNT , filled circles; yield, open circles; LNT II, filled triangles.

**Figure 11: Bulk synthesis of LNT II by the Bbh**I **D746E mutant.** (a) Time-course of LNT II synthesis by the D746E variant (4 μM) using equimolar amounts of GlcNAc-oxazoline and lactose (600 mM). (b) Comparison of wild-type BbhI (triangles) and D746E mutant (circles) for LNT2 synthesis, using a 2.4 fold excess of lactose over GlcNAc-oxazoline (255 mM) and 0.4 μM of enzyme. LNT II, filled symbols; yield, open symbols. Overlay of HPLC-UV traces (c) and HPLC-RI traces (d) used to evaluate the purity of LNT II produced on gram-scale. Note, the first peak in the HPLC-RI traces is the injection peak.

**Figure 12: LNT II synthesis with increasing GlcNAc-oxazoline concentration by the D746E mutant of Bbh**I. Time courses show synthesis using varying concentrations of GlcNAc-oxazoline, 600 mM lactose and 4.2 μM D746E. GlcNAc-oxazoline concentration was: (a) 130 mM, (b) 260 mM, (c) 500 mM. LNT II, filled circles; yield, open circles. (d) TLC-analysis of LNT II using 500 mM of GlcNAc-oxazoline. Reaction mixtures were 50-fold diluted. Lane 1, GlcNAc; lane 2, lactose; lane 3, LNT II; lane 4, 0 h; lane 5, 2.5 min; lane 6, 30 min; lane 7, 5 h; lane 8, 23.5 h. (e) TLC-analysis of LNT II using 600 mM of GlcNAc-oxazoline. Reaction mixtures were 200-fold diluted. Lane 1, GlcNAc; lane 2, lactose; lane 3, LNT II; lane 4, 0 h; lane 5, 2.5 min; lane 6, 5 min; lane 7, 30 min; lane 8, 7 h; lane 9, 23.5 h. Carbohydrates were visualized by thymol-sulfuric acid reagent. GlcNAc was also detected by UV (254 nm).

**Figure 13: LNT II synthesis from GlcNAc-β-*p*NP by wild-type Bbh**I **and the D746E mutant.** Time courses show synthesis from 100 mM GlcNAc-β-*p*NP and 600 mM lactose in the presence of 20% DMSO. (a) D746E, 8.4 μM; (b) wild-type, 0.12 μM. LNT II, filled circles; yield, open circles; *p*NP released, open triangles.

**Figure 14: $^{1}$H NMR spectrum of isolated LNT II.** LNT II was dissolved in $D_2O$. Spectrum is in accordance with previously published data.[4]

**Figure 15: $^{13}$C NMR spectrum of isolated LNT II.** LNT II was dissolved in $D_2O$. Full spectrum and partial spectrum (inset) showing that LNT II (GlcNAc-β1,3-Gal-β1,4-Glc) was the only regioisomer formed (82 ppm). No other regioisomers could be detected. Spectrum is in accordance with previously published data.[4]

**Figure 16: Global sequence alignment of the wildtype and mutant constructs of Bbh**I **and LnbB (SEQ ID NOs: 12-20) and corresponding sequences from other organisms (SEQ ID NOs 21-24).** The alignment was performed with the program "MegAlign Pro" Version: 12.2.0 (82) Copyright © 2012-2015, DNASTAR, Inc.. The used algorithm was "MUSCLE" (Multiple Sequenz Comparison by Log-Expectation). The aligned sequences are the following: BbhI, wild type (wt), truncated construct including a His-tag (SEQ ID NO: 12), BbhI, D746E mutant, truncated construct including a His-tag (SEQ ID NO: 13), BbhI, D746A mutant, truncated construct including a His-tag (SEQ ID NO: 14), BbhI, D746Q mutant, truncated construct including a His-tag (SEQ ID NO: 15), BbhI, Y827F

mutant, truncated construct including a His-tag (SEQ ID NO: 16), LnbB, wild type (wt), truncated construct including a His-tag (SEQ ID NO: 17), LnbB, D320E mutant, truncated construct including a His-tag (SEQ ID NO: 18), LnbB, D320A mutant, truncated construct including a His-tag (SEQ ID NO: 19), LnbB, Y419F mutant, truncated construct including a His-tag (SEQ ID NO: 20); Hex 1, from Actinomycetales bacterium, GenBank AKC34128.1 (SEQ ID NO: 21), Hex 2, from Bacteroidetes bacterium, GenBank AKC34129.1 (SEQ ID NO: 22), Chb, from Serratia marcescens, GenBank AAB03808.1 (SEQ ID NO: 23), SpHex, from Streptomyces plicatus, GenBank AAC38798.3 (SEQ ID NO: 24). The consensus sequence of the alignment is also shown (SEQ ID NO: 25).

## Examples:

### Materials

Media components and chemicals were of reagent grade from Sigma Aldrich/Fluka (Austria/Germany), Roth (Karlsruhe, Germany) or Merck (Vienna, Austria). HisTrap FF 5 mL column was from GE Healthcare (Vienna, Austria). Minisart® NML syringe membrane filter (0.45 μm) and Vivaspin® Turbo 15 centrifugal concentrators (30 kDa, 50 kDa) were from Sartorius (Goettingen, Germany). GlcNAc, 2-hydroxybenzimidazole (purity 97%), dimethyl sulfone (purity 99.96%) and succinonitrile were from Sigma Aldrich (Austria/Germany). 4-nitrophenyl 2-acetamido-2-deoxy-β-D-glucopyranoside (GlcNAc-β-*p*NP), 4-nitrophenyl 2-acetamido-2-deoxy-3-*O*-(β-D-galactopyranosyl)-β-D-glucopyranoside (LNB-β-*p*NP), lacto-*N*-triose II (LNT II), lacto-*N*-tetraose (LNT, purity ≥90%), lacto-*N*-biose (LNB) and α-D-gα-lactose-1-phosphate dipotassium salt hydrate (Gal 1-P) were from Carbosynth (Compton, Berkshire, UK). Chromabond Flash FM 70/10C C18 ac adsorbent was von Macherey Nagel (Schoonebeek, Netherlands). Acetonitrile (HPLC gradient grade) was from Chem-Lab NV (Zedelgem, Belgium).

### Example 1: Enzyme preparation

Production of the enzymes (without signal peptide and transmembrane region/membrane anchor) and their purification were done according to protocols from literature.[5, 6, 29] Briefly, synthetic BbhI genes (wild-type *N*-acetylhexosaminidase from *B. bifidum* JCM1254 (GenBank: AB504521.1, aa 33-1599)[5] and D746E, D746A, D746Q, Y827F variants) and synthetic LnbB genes (wild-type lacto-*N*-biosidase from *B. bifidum* JCM1254 (GenBank: EU281545.1, aa 35-1064)[6] and D320E, D320A, Y419F variants) codon-optimized for *E. coli* expression were ligated into *Nde*I-*Xho*I-cut pET21b(+) and pET24b(+) plasmids, respectively (BioCat GmbH, Heidelberg, Germany). Residue numbering of full length enzymes is used. All inserts were confirmed by DNA sequencing. BbhI enzymes were expressed in *E. coli* BL21(DE3) at 25°C for 20 h by induction with 0.5 mM isopropyl-β-D-thiogalactopyranoside (IPTG), using LB-medium supplemented with 115 mg L-1 ampicillin. LnbB enzymes were expressed in *E. coli* BL21(DE3) following an auto-induction protocol[33] in LB medium with 50 μg $mL^{-1}$, kanamycin, 25 mM $Na_2HPO_4$, 25 mM $K_2HPO_4$, 50 mM $NH_4Cl$, 5 mM $Na_2SO_4$, 2 mM $MgSO_4$, 0.5% glycerol, 0.05% glucose and 0.2% lactose at 110 rpm and 30°C for 20 h.[34] Each enzyme was produced as C-terminal $His_6$-tag fusion protein (SEQ ID NOs: 12 to 20) Enzyme purification was done by single-step $His_6$-tag affinity chromatography. The enzyme preparations used were (almost) pure by the criterion of migration as single protein band in SDS PAGE.

### Example 2: Protein purification by $His_6$-tag affinity chromatography

For protein purification, cell pellet from 1 L cell culture was resuspended in 25 - 30 mL binding buffer (20 mM sodium phosphate, 150 mM NaCl, 15 mM imidazol, pH 7.4) and frozen at -20°C overnight. 35 mL aliquots of thawed cell suspension were ultrasonicated on ice bath at 60% amplitude for 6 min (2 s pulse on and 4 s pulse off) using a Sonic Dismembrator (Ultrasonic Processor FB-505; Fisher Scientific, Austria) equipped with a 1.27 cm probe for cell disruption. Cell lysates were centrifuged at 4°C and 21,130 g for 1 h (Eppendorf centrifuge 5424R) and filtered *via* 0.45 μm cellulose-acetate syringe filters. Target proteins were purified from the cell-free extract *via* their C-terminal $His_6$-tag using an ÄktaPrime plus system (GE Healthcare, Germany) at 4°C. The cleared cell lysate was loaded onto a HisTrap FF 5 mL column (GE Healthcare, Austria) at a flow rate of 2 mL $min^{-1}$. The column had been equilibrated with binding buffer. After a washing step of 15 column volumes (CVs), the enzyme was eluted with 300 mM imidazol within 6 CVs at a flow rate of 4 mL $min^{-1}$. Target protein containing fractions were pooled. Eluted enzyme was concentrated and buffer exchanged to 20 mM sodium phosphate, 150 mM NaCl, pH 7.4 using Vivaspin® Turbo 15 centrifugal concentrators (30 kDa or 50 kDa, 3645 g, 4°C). SDS PAGE was used to confirm purity of enzyme preparations. Protein concentrations were measured with a DeNovix SA-11+ spectrophotometer (DeNovix Inc, US) at 280 nm. -40 mg of BbhI and -120 mg of LnbB enzymes, respectively, were typically obtained per liter of culture medium. Purified enzymes were aliquoted and stored at -70°C.

**Example 3: Preparation of 2-chloro-1,3-dimethyl-1*H*-benzimidazol-3-ium chloride (CDMBI)**

CDMBI was prepared as described previously,[8] with the following modifications. CDMBI was prepared by chemical synthesis in 2 steps. For preparation of 1,3-dimethylbenzimidazolone (DMBI), the N-atoms of 2-hydroxybenzimidazole were methylated by the action of MeI in the presence of KOH. The DMBI yield could be increased from 70%[8] to 92% by using KOH instead of NaOH, which was previously described in literature.[8] The reference experiment with NaOH as a base yielded 75% of DMBI.

*Step 1: Preparation of 1,3-dimethylbenzimidazolone (DMBI).* To a mixture of 50 g 2-hydroxybenzimidazole (1 equiv., 97% purity) and 216.55 g toluene (6.5 equiv.), 5.83 g $Bu_4NBr$ (0.05 equiv.) and 202.86 g KOH (40% w/w, 4 equiv.) were added. The reaction mixture was heated to 60°C and 118.04 g MeI (2.3 equiv.) were added dropwise within 60 min at high stirring rate. The mixture was stirred for 4 days at 60°C. Note, the reaction time can be reduced to 21 h without any change in yield. After cooling the mixture to 45°C, the phases were separated. The organic layer was washed at 45°C 3 times with 75 mL 1 N HCl, once with 75 mL saturated $NaHCO_3$ and dried over $Na_2SO_4$. After phase separation, the solvent was removed at 50°C and 150 - 5 mbar. 58.5 g of crude DMBI were obtained. The residue was recrystallized as follows: crude DMBI was taken up in 90 g acetone/n-heptane (3:1 v/v) at 65°C. The mixture was allowed to cool to room temperature for 16 h, then cooled to 5°C and stirred for further 3 h. After filtration, the crystalline DMBI was washed once with 40 mL ice-cold n-heptane/acetone (3:1 v/v) and dried under nitrogen to give 53.7 g of DMBI as a white solid (92%) $^1$H-NMR (700 MHz, $CDCl_3$): $\delta$ = 7.10 (m, 2H), 6.97 (m, 2H), 3.42 (s, 6H). $^{13}$C-NMR (175 MHz, $CDCl_3$): $\delta$ = 27.14, 107.29, 121.17, 129.99, 154.63.

DMBI was converted to CDMBI in a yield of 54% by using oxalyl chloride. When compared to literature, an additional 1.1 equiv. of total oxalyl chloride was used, the reaction temperature was decreased from 80°C to 70°C, and the reaction times were prolonged.

*Step 2: Preparation of 2-chloro-1,3-dimethyl-1H-benzimidazol-3-ium chloride (CDMBI).* To a solution of 34.4 g DMBI (1 equiv.) in 271 g toluene (13.9 equiv.), 80 g oxalyl chloride (3 equiv.) were added at 40 °C. The mixture was heated to 70°C. After 5 d at 70°C no precipitate was formed. Then, 40 g of oxalyl chloride (1.5 equiv.) were added, and the mixture was stirred at 70°C overnight. The suspension was cooled to 0 - 5°C within 3 h and stirred for further 3 h at this temperature. After filtration, the filter cake was washed with 70 mL of ice-cold toluene and dried in vacuo to give 24.8 g of CDMBI (54%). $^1$H-NMR (500 MHz, $D_2O$): $\delta$ = 7.85 (m, 2H), 7.72 (m, 2H), 4.08 (s, 3H). $^{13}$C-NMR (125 MHz, $D_2O$): $\delta$ = 35.28, 115.51, 130.08, 134.22, 143.45. In comparison to the protocol reported in literature (49% yield),[8] an additional 1.1 equiv. of total oxalyl chloride was used, the reaction temperature was decreased from 80°C to 70°C and the reaction times were prolonged.

Then, CDMBI and $Na_3PO_4$ were used for oxazoline formation from *N*-Acetylgucosamin (GlcNAc) or lacto-*N*-biose (LNB).[8]

Overall, the CDMBI synthesis was significantly improved. The CDMBI yield over 2 steps could be increased from 34%[8] to 50% by the modifications just described above.

**Example 4: Preparation of lacto-*N*-biose (LNB)**

LNB was synthesized from Gal 1-P and GlcNAc by lacto-*N*-biose phosphorylase (LNBP) from *Bifidobacterium longum* JCM 1217, previously described by Kitaoka and co-workers.[9, 10] *LNBP production.* Production of the LNBP and purification were done according to protocols from literature.[9] Briefly, synthetic LNBP gene (GenBank: AB181926.1, aa 20-2275) not codon-optimized for *E. coli* expression was ligated into *N*deI-*Xho*I-cut pET30a(+) plasmid (GenScript, Piscataway, USA). Insert was confirmed by DNA sequencing. LNBP was expressed in *E. coli* BL21(DE3) at 30°C for 20 h by induction with 0.5 mM isopropyl-$\beta$-D-thiogalactopyranoside (IPTG), using LB-medium supplemented with 50 mg $L^{-1}$ kanamycin. LNBP was produced as C-terminal $His_6$-tag fusion protein. Enzyme purification was done by single-step $His_6$-tag affinity chromatography (see above). The following buffers were used: binding buffer (20 mM MOPS, 500 mM NaCl, 15 mM imidazol, pH 7.4), elution buffer (20 mM MOPS, 500 mM NaCl, 300 mM imidazol, pH 7.4), storage buffer (20 mM MOPS, 150 mM NaCl, pH 7.5). -50 mg of LNBP was typically obtained per liter of culture medium. The enzyme preparation used was (almost) pure by the criterion of migration as single protein band in SDS PAGE.

*Enzymatic synthesis of LNB.* Reaction was performed in a total volume of 40 mL using 5.4 mmol Gal 1-P (1.82 g) and 1.8 mmol GlcNAc (0.40 g) dissolved in water. The pH was adjusted to 6.8 with 4 M HCl and the reaction was started by adding 0.05 mg $mL^{-1}$ (0.6 $\mu$M) LNBP. The conversion was performed in a 50 mL Sarstedt tube (diameter 2.8 cm, height 11.5 cm) under magnetic stirring (stir bar: 18 x 5 mm; 500 rpm) at 37°C. For temperature control, the Sarstedt tube was placed in a water bath. The pH was constantly monitored and manually controlled by adding 4 M HCI (within first 1.5 h). Incubation was for 3.5 h. Samples were taken at certain times and analyzed by HPLC. The reaction yield was 92% (42 mM, 16 g $L^{-1}$).

*Downstream processing (DSP).* Major task of the DSP was the removal of Gal 1-P (93 mM) from the LNB (42 mM). Only a small amount of GlcNAc (3 mM) was present. Gal 1-P was removed from the mixture by anion-exchange

chromatography (AEC) after enzyme-removal by ultra-filtration (Vivaspin concentrators 30 kDa, 4000 rpm, 20°C). AEC was performed at pH 7.5. To allow efficient removal of Gal 1-P by binding to the anion-exchange column, the filtrate was 8-fold diluted to an ionic strength of -3.6 mS $cm^{-1}$ with ultra-pure water. LNB and remaining GlcNAc are not ionized at pH 7.5 and elute in the flow-through. AEC was performed on an ÄktaPrime plus system (GE Healthcare, Germany) at room temperature. A self-packed Proteus 20 mL FliQ column (100 x 16.0 mm, Generon, UK) containing about 15 mL of Toyopearl SuperQ-650M was applied. Ultra-pure water (mobile phase A) and 1 M potassium chloride in ultra-pure water (mobile phase B) were used for binding and elution, respectively. Column was equilibrated with mobile phase A at 4 mL $min^{-1}$ (5 CVs). 40 mL sample were loaded at a flow rate of 2 mL $min^{-1}$ using mobile phase A. LNB eluted together with GlcNAc within 5 CVs. Gal 1-P was eluted with mobile phase B at 4 mL $min^{-1}$ (5 CVs). Detection was by conductivity. Complete removal of Gal 1-P from LNB was verified by TLC analysis. LNB containing fractions were pooled. Sample was concentrated under reduced pressure at 40°C, frozen in liquid nitrogen under rotary motion before freeze-drying overnight (Christ Alpha 1-4, B. Braun Biotech International, Melsungen, Germany). The final product (80% isolated yield) was analyzed by HPLC and its chemical identity confirmed by $^1H$ NMR. 5% (w/w) of GlcNAc were detected in the final product.

## Example 5: Preparation of sugar oxazolines

GlcNAc- and LNB-oxazoline were prepared as described previously.[8] Briefly, CDMBI (3 equiv.) was used as dehydrative condensing agent and $Na_3PO_4$ (7.5 equiv.) as a base for oxazoline formation from GlcNAc (1 equiv.) or LNB (1 equiv.). *N*-acetyl-2-amino sugars were added and the resulting solution was cooled to 0 - 3°C. CDMBI (3 equiv.) was added to the solution in portions within 15 min, and the mixture was stirred for 1 h at the same temperature.

GlcNAc is easily available at low cost. Practical preparation of LNB in bulk quantities from sucrose and GlcNAc, using a one-pot four-enzyme reaction with lacto-*N*-biose phosphorylase (LNBP) from *Bifidobacterium bifidum* as a key enzyme, was reported by Kitaoka and co-workers.[9, 10] Analogously, LNB was synthesized from Gal1P and GlcNAc by LNBP. Anion-exchange chromatography (AEC) and freeze-drying was used for preliminary downstream processing (DSP) (80% isolated yield, purity ≥90%). Initial downstream processing (DSP) of the sugar oxazolines included filtration (Chromabond Flash FM 70/10C C18 ac adsorbent (10 g per g of *N*-acetyl-2-amino sugar used)) and freeze-drying (lyophilized crude product). Product identities were unequivocally confirmed by $^1H$ NMR spectroscopy. NMR spectra were in accordance with published data.[8] The content of sugar oxazolines was determined by quantitative $^1H$ NMR spectroscopy with dimethyl sulfone or succinonitrile as internal standards. No DMBI was detectable, but the excess of salt remained in the lyophilized crude product. GlcNAc-oxazoline was obtained on 30 mmol scale in a yield of ~ 60%. LNB-oxazoline was obtained on 0.5 mmol scale in a yield of 79%.

In order to ensure efficient trans-glycosylation from the sugar oxazolines in the next step, the lyophilized crude products were desalted. Desalting was established by extraction with acetonitrile (9.5 g per g lyophilized crude product, 1h stirring at room temperature), which is scalable. After filtration, washing (2 * with acetonitrile (3 g per g lyophilized crude product)), concentration and drying in vacuo, GlcNAc-oxazoline was obtained on 3.5 mmol scale in a yield of -60%. LNB-oxazoline was obtained on 0.082 mmol scale in a yield of <10%. The yields were determined by HPLC analysis of the *N*-acetyl-2-amino sugars released (GlcNAc, LNB) after complete hydrolysis of the oxazoline ring.

## Example 6: BbhI and LnbB activity assays

BbhI and LnbB activities were assayed in a total volume of 600 μL and 400 μL, respectively, using 50 mM sodium phosphate buffer, pH 7.5 (with oxazoline donor substrates) or pH 5.8 (with pNP-labelled donor substrates). Reaction mixture with sugar oxazoline donor substrate contained 60 mM GlcNAc-oxazoline or 12 mM LNB-oxazoline, 600 mM lactose and 0.23 - 18 μM enzyme. Reaction mixture with pNP-labelled donor substrate contained 20 mM GlcNAc-β-*p*NP or LNB-β-*p*NP, 15 - 20% DMSO, 400 - 600 mM lactose and 0.12 - 24 μM enzyme. Enzymatic conversion was carried out at 37°C or 55°C (as indicated in the text; see Table 1) and agitation rate of 650 rpm using a Thermomixer comfort (Eppendorf, Germany).

Reactions were stopped at certain times by heating for 10 min at 99°C. Precipitated protein was removed by centrifugation at 13,200 rpm for 10 min. Samples were analyzed by hydrophilic interaction liquid chromatography (HILIC-HPLC) and thin layer chromatography (TLC).

Specific activities were calculated from initial rates of product formation (trans-glycosylation) and product hydrolysis (secondary hydrolysis), respectively, obtained at 400 - 600 mM of lactose ('synthesis conditions'). One unit (1 U) of trans-glycosylation activity was defined as the amount of enzyme that could transfer 1 μmol of *N*-acetyl-2-amino sugar (BbhI: GlcNAc; LnbB: LNB) per min to lactose under the conditions described above. One unit (1 U) of secondary hydrolysis activity was defined as the amount of enzyme that could release 1 μmol of *N*-acetyl-2-amino sugar (BbhI: GlcNAc; LnbB: LNB) per min from the product formed under the conditions described above. For $R_{TH}$ analysis, specific activities for total (productive and non-productive) GlcNAc-β-*p*NP or LNB-β-*p*NP hydrolysis (based on pNP released)

and trans-glycosylation were calculated from initial rate data obtained at 400 - 600 of lactose. The difference gave the specific activity for non-productive donor substrate hydrolysis (primary hydrolysis). For the oxazoline substrates, the total donor hydrolysis could only be calculated based on initial rate data of release of *N*-acetyl-2-amino sugar (BbhI: GlcNAc; LnbB: LNB) for the variants of BbhI and LnbB having their catalytic Asp replaced by Glu. In all other cases, the $R_{TH}$ values were estimated based on endpoint measurements at maximum LNT II or LNT yield.

**Example 7: Preparative-scale synthesis of LNT II**

In order to allow bulk synthesis of LNT II, its synthesis from GlcNAc-oxazoline by the D746E glycosidase mutant of BbhI had to be optimized with respect to the donor-to-acceptor ratio applied. Increasing concentrations of GlcNac-oxazoline (130 - 600 mM) were applied at a constant lactose concentration of 600 mM and the conversions compared at 37°C (Fig. 11a, Fig. 12). The adjustment of the donor-to-acceptor ratio to 1 had no negative impact on the final yield (85 - 90%). Fig. 11a shows synthesis of ~1 g of LNT II in a batch volume of only 3.6 mL under the optimized conditions. The initial LNT II production rate was 2190 g $L^{-1}$ $h^{-1}$. LNT II was obtained in excellent yield (85%) and concentration (281 g $L^{-1}$, 515 mM) within 30 min of reaction. The *STY* of the biotransformation overall was 562 g $L^{-1}$ $h^{-1}$. The mass-based turnover number (g product formed per g enzyme added; *TTN*mass) reached a value of 388. Only marginal product hydrolysis was observed under the reaction conditions applied. The ratio of trans-glycosylation over secondary hydrolysis reached a value of -1800. When the D746E variant and the wild-type enzyme were assayed under exactly the same conditions (using one-tenth of the enzyme concentration compared to the bulk synthesis described above), one sees clearly the benefits of the new glycosidase, namely no secondary hydrolysis and doubling of the LNT II yield (Fig. 11b). The initial LNT II production rates of the two enzymes were comparable, but the conversion with the wild-type drastically slowed down already after 5 min while it remained constant over ~0.5 h with the mutant. Note, 600 mM of each substrate was the upper concentration limit used in the reaction, allowing their full solubility. LNT II was obtained in 80% yield without any detectable hydrolysis of the product (Fig. 13a). Under these conditions, the wild-type yielded ~50% of LNT II and showed also an improved trans-glycosylation to secondary hydrolysis ratio of ~40 (Fig. 13b).

For DSP of LNT II, the reaction was stopped by heating when no further increase in product concentration was detected. The sample contained only 85 mM of GlcNAc and lactose next to 515 mM of LNT II. Therefore, a simple DSP, including centrifugation for enzyme removal and freeze-drying for water removal, was sufficient to isolate LNT II in a purity of -80% (based on LNT II content of the final product, Figs. 11c,d). The main residual impurities were 5% (w/w) GlcNAc and 10% (w/w) lactose. If a higher product purity is required, nanofiltration could be used for removal of GlcNAc and lactose from the LNT II.[35, 36] About 1 g of LNT II was obtained as a white powder in ≥85% yield. LNT II was thus prepared from GlcNAc-oxazoline in 73% overall yield. Product identity was unequivocally confirmed by $^{1}H$ and $^{13}C$ NMR spectroscopy (Figs. 14 and 15). NMR spectra were in accordance with published data.[5] LNT II was the only regioisomer detected (Fig. 15). Overlay of NMR spectra ($^{13}C$, HSQC) of isolated LNT II (Fig. 15) and commercial standard showed exact match of the signal at 82 ppm, characteristic for β-GlcNAc linked to the C-3 position of the β-galactosyl residue of lactose.

The enzymatic conversion was carried out at pH 7.5 and 37°C in a total volume of 3.6 mL, using equimolar amounts of GlcNAc-oxazoline and lactose (600 mM). Reaction was started by adding 0.73 mg $mL^{-1}$ (4 μM) of BbhI D746E variant. The conversion was performed in a 50 mL Sarstedt tube (diameter 2.8 cm, height 11.5 cm) under magnetic stirring (stir bar: 18 x 5 mm; 250 rpm). For temperature control, the Sarstedt tube was placed in a water bath. Samples were taken at certain times and analyzed by HPLC.

For downstream processing (DSP) of LNT II, enzyme was precipitated after 45 min reaction time by heating for 15 min at 99°C. Precipitated protein was removed by centrifugation at 13,200 rpm for 15 min. Sample was frozen at -70°C and freeze-dried overnight (Christ Alpha 1-4, B. Braun Biotech International, Melsungen, Germany). The final product was analyzed by HPLC and its chemical identity confirmed by NMR.

**Example 8: Synthesis of LNT II by fed-batch addition of GlcNAc-oxazoline**

1.8 mmol GlcNAc-oxazoline was dissolved in 1.3 mL ice-cold water and added continuously over a period of 65 min to the reaction solution containing 2.1 mmol of lactose and 0.3 mg (0.4 μM) of BbhI D746E in 2.3 mL of phosphate buffer pH 7.5. The enzymatic conversion was carried out at 37°C under magnetic stirring. For temperature control, the reaction tube was placed in a water bath. Samples were taken at certain times and analyzed by HPLC. Formation of the product was demonstrated. The reaction was terminated after 4 h by heat deactivation of the enzyme.

**Analytics**

LNT II, LNT, GlcNAc, LNB, *p*NP and lactose were analyzed by HILIC-HPLC using a Luna® $NH_2$ column (3 μm, 100 Å, 250 x 4.6 mm; Phenomenex, Germany). HPLC analysis was performed at 30°C with a mobile phase of 75%

acetonitrile and 25% water at an isocratic flow rate of 1 mL $min^{-1}$. UV-detection at 195 nm was used for quantification of LNT II, LNT, GlcNAc, LNB and pNP. For preparative synthesis of LNT II, lactose was monitored by refractive index (RI) detection.

TLC was performed on silica gel 60 F254 aluminum sheet (Merck, Germany). The plate was developed in a solvent system of 1-butanol - acetic acid - water (2/1/1 by volume). TLC plates were analyzed under UV light (254 nm). Then carbohydrates were visualized by heating the plate after spraying it with thymol - sulfuric acid reagent.

LNT II, LNT, GlcNAc, LNB, *p*NP and lactose were used as authentic standards.

Varian (Agilent) INOVA 500-MHz NMR spectrometer (Agilent Technologies, Santa Clara, California, USA) and the VNMRJ 2.2D software were used for all NMR measurements. Dimethyl sulfone and succinonitrile were used as internal standards for quantitative $^{1}$H NMR measurements. 19.08 mg of GlcNAc-oxazoline (lyophilized crude product) and 11.28 mg dimethyl sulfone were dissolved in $D_2O$. 11.41 mg of LNB-oxazoline (lyophilized crude product) and 12.85 mg succinonitrile were dissolved in $D_2O$. -200 mg of isolated LNT II were dissolved in 600 μL $D_2O$. Commercial LNT II standard (from Carbosynth; 65 mM) was dissolved in $D_2O$ - $H_2O$ (11.5:1 v/v). $^{1}$H NMR spectra (499.98 MHz) were measured on a 5 mm indirect detection PFG-probe, while a 5 mm dual direct detection probe with z-gradients was used for $^{13}$C NMR spectra (125.71 MHz). Standard pre-saturation sequence was used: relaxation delay 2 s; 90° proton pulse; 2.048 s acquisition time; spectral width 8 kHz; number of points 32 k. $^{13}$C NMR spectra were recorded with the following pulse sequence: standard $^{13}$C pulse sequence with 45° carbon pulse, relaxation delay 2 s, Waltz decoupling during acquisition, 2 s acquisition time. The HSQC spectrum was measured with 128 scans per increment and adiabatic carbon 180° pulses. Mnova 9.0 was used for evaluation of spectra.

References:

1. Fujita, M., Shoda, S.-i., Haneda, K., Inazu, T., Takegawa, K., Yamamoto, K. A novel disaccharide substrate having 1,2-oxazoline moiety for detection of transglycosylating activity of endoglycosidases. Biochim. Biophys. Acta Gen. Subj. 1528, 9-14 (2001).

2. Kobayashi, A., Kuwata, H., Kohri, M., Izumi, R., Watanabe, T., Shoda, S. i. A bacterial chitinase acts as catalyst for synthesis of the N-linked oligosaccharide core trisaccharide by employing a sugar oxazoline substrate. J. Carbohyd. Chem. 25, 533-541 (2006).

3. Wang, L.-X., Huang, W. Enzymatic transglycosylation for glycoconjugate synthesis. Curr. Opin. Chem. Biol. 13, 592-600 (2009).

4. Ochiai, H., Huang, W., Wang, L.-X. Endo-β-N-acetyigtucosaminidase-catatyzed polymerization of β-Glcp-(1→4)-GlcpNAc oxazoline: a revisit to enzymatic transglycosylation. Carbohydr. Res. 344, 592-598 (2009).

5. Chen, X., et al. Efficient and regioselective synthesis of β-GalNAc/GlcNAc-lactose by a bifunctional transglycosylating β-N-acetylhexosaminidase from Bifidobacterium bifidum. Appl. Environ. Microbiol. 82, 5642-5652 (2016).

6. Wada, J., et al. Bifidobacterium bifidum lacto-N-biosidase, a critical enzyme for the degradation of human milk oligosaccharides with a type 1 structure. Appl. Environ. Microbiol. 74, 3996-4004 (2008).

7. Jamek, S. B., et al. Loop protein engineering for improved transglycosylation activity of a β-N-acetylhexosaminidase. ChemBioChem 19, 1858-1865 (2018).

8. Noguchi, M., Fujieda, T., Huang, W. C., Ishihara, M., Kobayashi, A., Shoda, S.-i. A practical one-step synthesis of 1,2-oxazoline derivatives from unprotected sugars and its application to chemoenzymatic β-N-acetylglucosaminidation of disialo-oligosaccharide. Helv. Chim. Acta 95, 1928-1936 (2012).

9. Kitaoka, M. & Nishimoto, M. Method for producing lacto-*N*-biose I and galacto-*N*-biose. US Patent US8173399B2 (2012).

10. Nishimoto, M., Kitaoka, M. Practical preparation of lacto-N-biose I, a candidate for the bifidus factor in human milk. Biosci. Biotechnol. Biochem. 71, 2101-2104 (2007).

11. Slámová, K., et al. Synthesis of derivatized chitooligomers using transglycosidases engineered from the fungal

GH20 β-N-acetylhexosaminidase. Adv. Synth. Catal. 357, 1941-1950 (2015).

12. Miwa, M., et al. Cooperation of β-galactosidase and β-N-acetythexosaminidase from bifidobacteria in assimilation of human milk oligosaccharides with type 2 structure. Glycobiology 20, 1402-1409 (2010).

13. Andre-Miral, C., et al. De novo design of a trans-β-N-acetylglucosaminidase activity from a GH1 β-glycosidase by mechanism engineering. Glycobiology 25, 394-402 (2015).

14. Martinez, E. A., et al. Engineering chitinases for the synthesis of chitin oligosaccharides: catalytic amino acid mutations convert the GH-18 family glycoside hydrolases into transglycosylases. J. Mol. Catal. B: Enzym. 74, 89-96 (2012).

15. Shoda, S.-i., et al. Efficient method for the elongation of the N-acetylglucosamine unit by combined use of chitinase and β-galactosidase. Helv. Chim. Acta 85, 3919-3936 (2002).

16. Murata, T., Inukai, T., Suzuki, M., Yamagishi, M., Usui, T. Facile enzymatic conversion of lactose into lacto-N-tetraose and lacto-N-neotetraose. Glycoconj. J. 16, 189-195 (1999).

17. Baumgärtner, F., Sprenger, G. A., Albermann, C. Galactose-limited fed-batch cultivation of Escherichia coli for the production of lacto-N-tetraose. Enzyme Microb. Technol. 75-76, 37-43 (2015).

18. Blixt, O., van Die, I., Norberg, T., van den Eijnden, D. H. High-level expression of the Neisseria meningitidis IgtA gene in Escherichia coli and characterization of the encoded N-acetylglucosaminyltransferase as a useful catalyst in the synthesis of Glc-NAcβ1→3Gal and GalNAcβ1→3Gal linkages. Glycobiology 9, 1061-1071 (1999).

19. Chen, C., et al. Sequential one-pot multienzyme (OPME) synthesis of lacto-N-neotetraose and its sialyl and fucosyl derivatives. Chem. Commun. 51, 7689-7692 (2015).

20. Johnson, K. F. Synthesis of oligosaccharides by bacterial enzymes. Glycoconj. J. 16, 141-146 (1999).

21. Yu, H., et al. Synthetic disialyl hexasaccharides protect neonatal rats from necrotizing enterocolitis. Angew. Chem. Int. Edit. 53, 6687-6691 (2014).

22. Priem, B., Gilbert, M., Wakarchuk, W. W., Heyraud, A., Samain, E. A new fermentation process allows large-scale production of human milk oligosaccharides by metabolically engineered bacteria. Glycobiology 12, 235-240 (2002).

23. Prieto, P. A., Kleman-Leyer, K. M. Process for synthesizing oligosaccharides. US patent 5945314A (1999).

24. Baumgärtner, F., Conrad, J., Sprenger, G. A., Albermann, C. Synthesis of the human milk oligosaccharide lacto-N-tetraose in metabolically engineered, plasmid-free E. coli. ChemBioChem 15, 1896-1900 (2014).

25. Kobayashi, S., Kiyosada, T., Shoda, S.-i. A novel method for synthesis of chitobiose via enzymatic glycosylation using a sugar oxazoline as glycosyl donor. Tetrahedron Lett. 38, 2111-2112 (1997).

26. Ochiai, H., Ohmae, M., Kobayashi, S. Enzymatic glycosidation of sugar oxazolines having a carboxylate group catalyzed by chitinase. Carbohydr. Res. 339, 2769-2788 (2004).

27. Kohri, M., Kobayashi, A., Shoda, S.-i. Design and utilization of chitinases with low hydrolytic activities. Trends Glycosci. Glycotechnol. 19, 165-180 (2007).

28. Hattie, M., et al. Gaining insight into the catalysis by GH20 lacto-N-biosidase using small molecule inhibitors and structural analysis. Chem. Commun. 51, 15008-15011 (2015).

29. Ito, T., et al. Crystal structures of a glycoside hydrolase family 20 lacto-N-biosidase from Bifidobacterium bifidum. J. Biol. Chem. 288, 11795-11806 (2013).

30. Liu, X.-w., et al. Characterization and synthetic application of a novel β1,3-galactosyltransferase from Escherichia

coli O55:H7. Bioorg. Med. Chem. 17, 4910-4915 (2009).

31. Zeuner, B., Nyffenegger, C., Mikkelsen, J. D., Meyer, A. S. Thermostable β-galactosidases for the synthesis of human milk oligosaccharides. New Biotechnol. 33, 355-360 (2016).

32. Bode, L. Human milk oligosaccharides: Every baby needs a sugar mama. Glycobiology 22, 1147-1162 (2012).

33. Studier, F. W. Protein production by auto-induction in high-density shaking cultures. Protein Expr. Purif. 41, 207-234 (2005).

34. Val-Cid, C., Biarnés, X., Faijes, M., Planas, A. Structural-functional analysis reveals a specific domain organization in family GH20 hexosaminidases. PLOS ONE 10, e0128075 (2015).

35. Goulas, A. K., Kapasakalidis, P. G., Sinclair, H. R., Rastall, R. A., Grandison, A. S. Purification of oligosaccharides by nanofiltration. J. Membrane Sci. 209, 321-335 (2002).

36. Nordvang, R. T., et al. Separation of 3'-sialyllactose and lactose by nanofiltration: a trade-off between charge repulsion and pore swelling induced by high pH. Sep. Purif. Technol. 138, 77-83 (2014).

## Claims

1. Method for producing lacto-*N*-triose II and/or lacto-*N*-tetraose comprising the step:

   (i) reacting glucosamine-oxazoline and/or lacto-*N*-biose-oxazoline with lactose catalysed by an enzyme of the glycoside hydrolase family 20 (GH20) according to the classification of the Carbohydrate-Active-Enzymes (CAZy) database (http://www.cazy.org, updated on 20 November 2018) to obtain lacto-*N*-triose II and/or lacto-*N*-tetraose.

2. Method according to claim 1, wherein step (i) is performed in an aqueous solution containing at least 50 g/L lactose, preferably at least 100 g/L lactose, particularly preferably at least 150 g/L lactose, most preferably at least 190 g/L lactose.

3. Method according to claim 1 or claim 2, wherein the method is performed under conditions that are free or essentially free of organic solvents.

4. Method according to any one of claims 1 to 3, wherein the glucosamine-oxazoline and/or lacto-*N*-biose-oxazoline, respectively, is added to the lactose and the enzyme of the glycoside hydrolase family 20 (GH20) over a period of at least 20 minutes, preferably at least 60 minutes.

5. Method for producing lacto-*N*-triose II according to any one of claims 1 to 4, wherein the enzyme of the glycoside hydrolase family 20 (GH20) is a β-*N*-acetylhexosaminidase, preferably β-*N*-acetylhexosaminidase of *Bifidobacterium bifidum* JCM1254 having an amino acid sequence of SEQ ID NO: 1 (Bbhl, GenBank accession number: AB504521.1) or an enzyme having an amino acid sequence identity of at least 70% to SEQ ID NO: 1 and having β-*N*-acetylhexosaminidase activity, or a β-*N*-acetylhexosaminidase enzyme comprising an amino acid sequence of SEQ ID NO: 2, or a β-*N*-acetylhexosaminidase enzyme comprising an amino acid sequence having an amino acid sequence identity of at least 70% to SEQ ID NO: 2.

6. Method for producing lacto-*N*-tetraose according to any one of claims 1 to 4, wherein the enzyme of the glycoside hydrolase family 20 (GH20) is a lacto-*N*-biosidase, preferably lacto-*N*-biosidase of *Bifidobacterium bifidum* JCM1254 having an amino acid sequence of SEQ ID NO: 3 (LnbB, GenBank accession number: EU281545.1), or an enzyme having an amino acid sequence identity of at least 70% to SEQ ID NO: 3 and having lacto-*N*-biosidase activity, or a lacto-*N*-biosidase enzyme comprising an amino acid sequence of SEQ ID NO: 4, or a lacto-*N*-biosidase enzyme comprising an amino acid sequence having an amino acid sequence identity of at least 70% to SEQ ID NO: 4.

7. Method according to any of the preceding claims, wherein the method further comprises the step:
   (ii) deactivating the enzyme of the glycoside hydrolase family 20 (GH20).

8. Method for producing lacto-*N*-triose II according to any of claims 1 to 5 or 7 further comprising the step:
(iii) adding a β-galactosidase or a galactosyl transferase and UDP-galactose, to the mixture of step (i) or, preferably to the mixture of step (ii), and optionally adding further lactose to obtain lacto-*N*-tetraose or lacto-*N*-neotetraose.

9. Use of an enzyme of the glycoside hydrolase family 20 (GH20) according to the classification of the Carbohydrate-Active-Enzymes (CAZy) database (http://www.cazy.org, updated on 20 November 2018) for producing lacto-*N*-triose II or lacto-*N*-tetraose from lactose, preferably use of an enzyme as defined in claim 5 for producing lacto-*N*-triose II or use of an enzyme as defined in claim 6 for producing lacto-*N*-tetraose.

10. Use according to claim 9, wherein the enzyme of the glycoside hydrolase family 20 (GH20) is used in a method according to any of claims 1 to 8.

11. β-*N*-acetylhexosaminidase having an amino acid sequence identity of at least 70% to SEQ ID NO: 1 (BbhI, GenBank accession number: AB504521.1) and carrying a mutation at a position counted as position 746 of SEQ ID NO: 1 and/or at a position counted as position 827 of SEQ ID NO: 1, preferably carrying a glutamic acid or an alanine or a glutamine at a position counted as position 746 of SEQ ID NO: 1 and/or carrying a phenylalanine at a position counted as position 827 of SEQ ID NO: 1, or β-*N*-acetylhexosaminidase comprising an amino acid sequence of any of SEQ ID NOs: 5 to 8 or comprising an amino acid sequence having an amino acid sequence identity of at least 70% to any of SEQ ID NOs: 5 to 8.

12. Lacto-*N*-biosidase having an amino acid sequence identity of at least 70% to SEQ ID NO: 3 (LnbB, GenBank accession number: EU281545.1) and carrying a mutation at a position counted as position 320 of SEQ ID NO: 3 and/or at a position counted as position 419 of SEQ ID NO: 3, preferably carrying a glutamic acid or an alanine at a position counted as position 320 of SEQ ID NO: 3 and/or carrying a phenylalanine at a position counted as position 419 of SEQ ID NO: 3, or lacto-*N*-biosidase comprising an amino acid sequence of any of SEQ ID NOs: 9 to 11 or comprising an amino acid sequence having an amino acid sequence identity of at least 70% to any of SEQ ID NOs: 9 to 11.

13. Aqueous solution comprising an enzyme of the glycoside hydrolase family 20 (GH20) as defined in any of the preceding claims, lactose, preferably at least 50 g/L lactose, more preferably at least 100 g/L lactose, particularly preferably at least 150 g/L lactose, most preferably at least 190 g/L lactose and

a) glucosamine-oxazoline and/or lacto-*N*-biose-oxazoline; and/or
b) lacto-*N*-triose II and/or lacto-*N*-tetraose.

**Fig. 1**

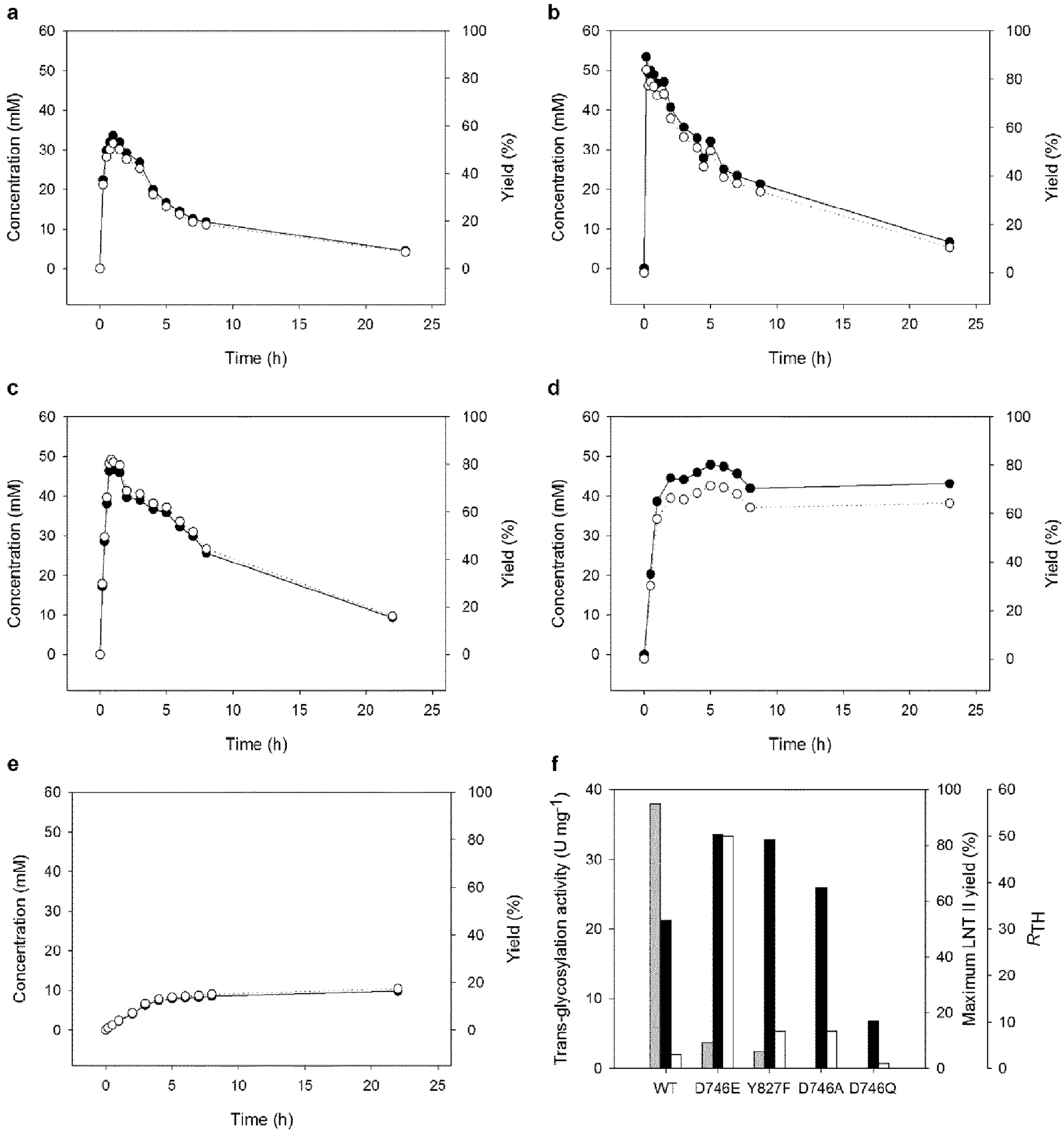
a
b
c
d
e
f
Concentration (mM)
Yield (%)
Time (h)
Trans-glycosylation activity (U mg$^{-1}$)
Maximum LNT II yield (%)
$R_{TH}$
WT
D746E
Y827F
D746A
D746Q

Fig. 2

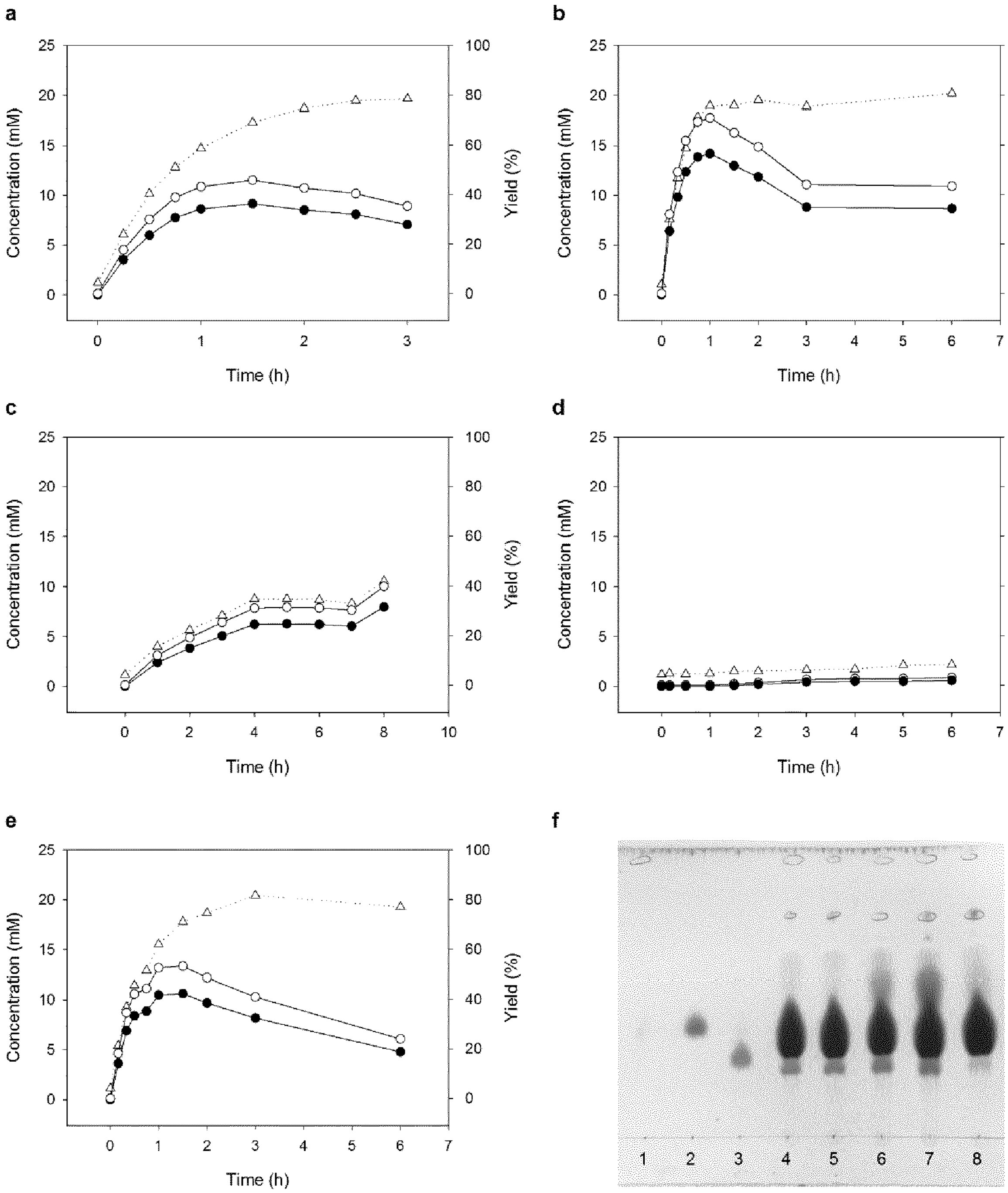

Fig. 3

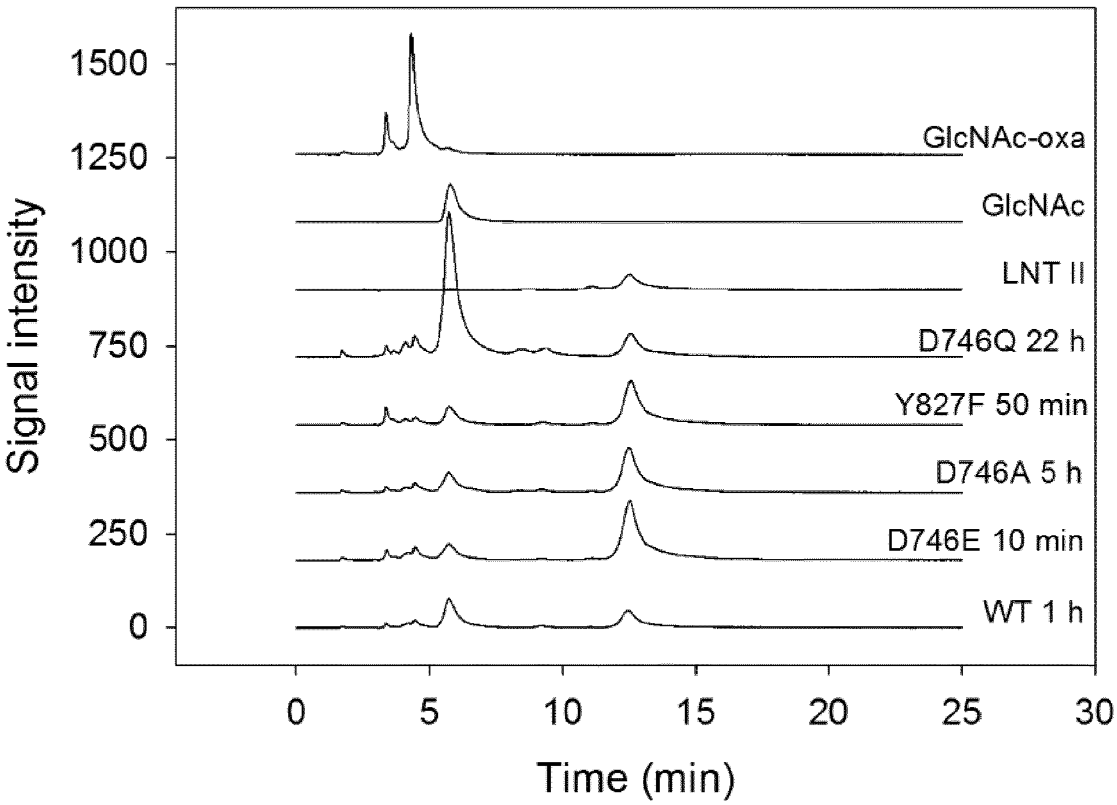
Signal intensity
1500
1250
1000
750
500
250
0
GlcNAc-oxa
GlcNAc
LNT II
D746Q 22 h
Y827F 50 min
D746A 5 h
D746E 10 min
WT 1 h
0
5
10
15
20
25
30
Time (min)

Fig. 4

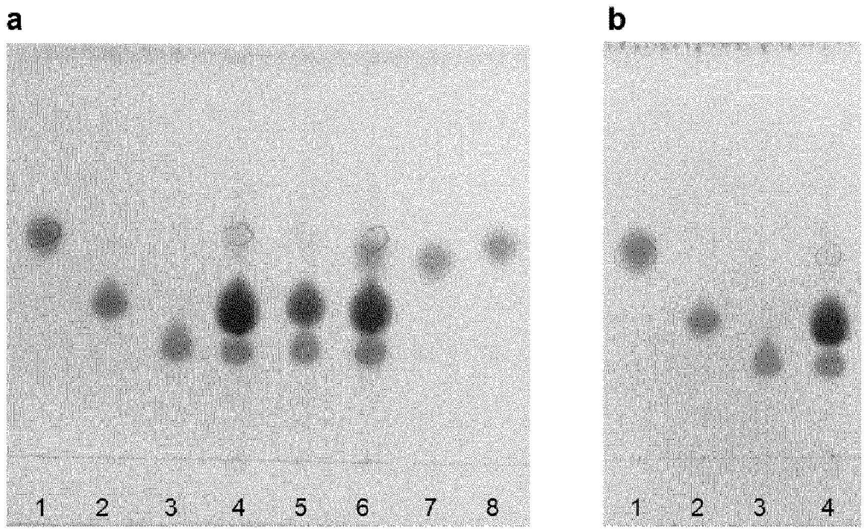
a
1 2 3 4 5 6 7 8
b
1 2 3 4

Fig. 5

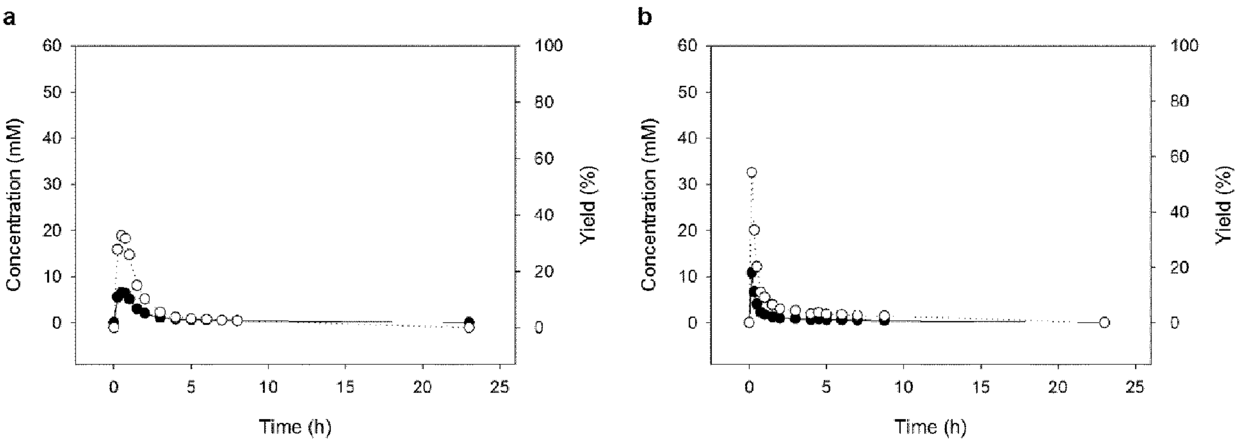
a
Concentration (mM)
Yield (%)
Time (h)
b
Concentration (mM)
Yield (%)
Time (h)

**Fig. 6**

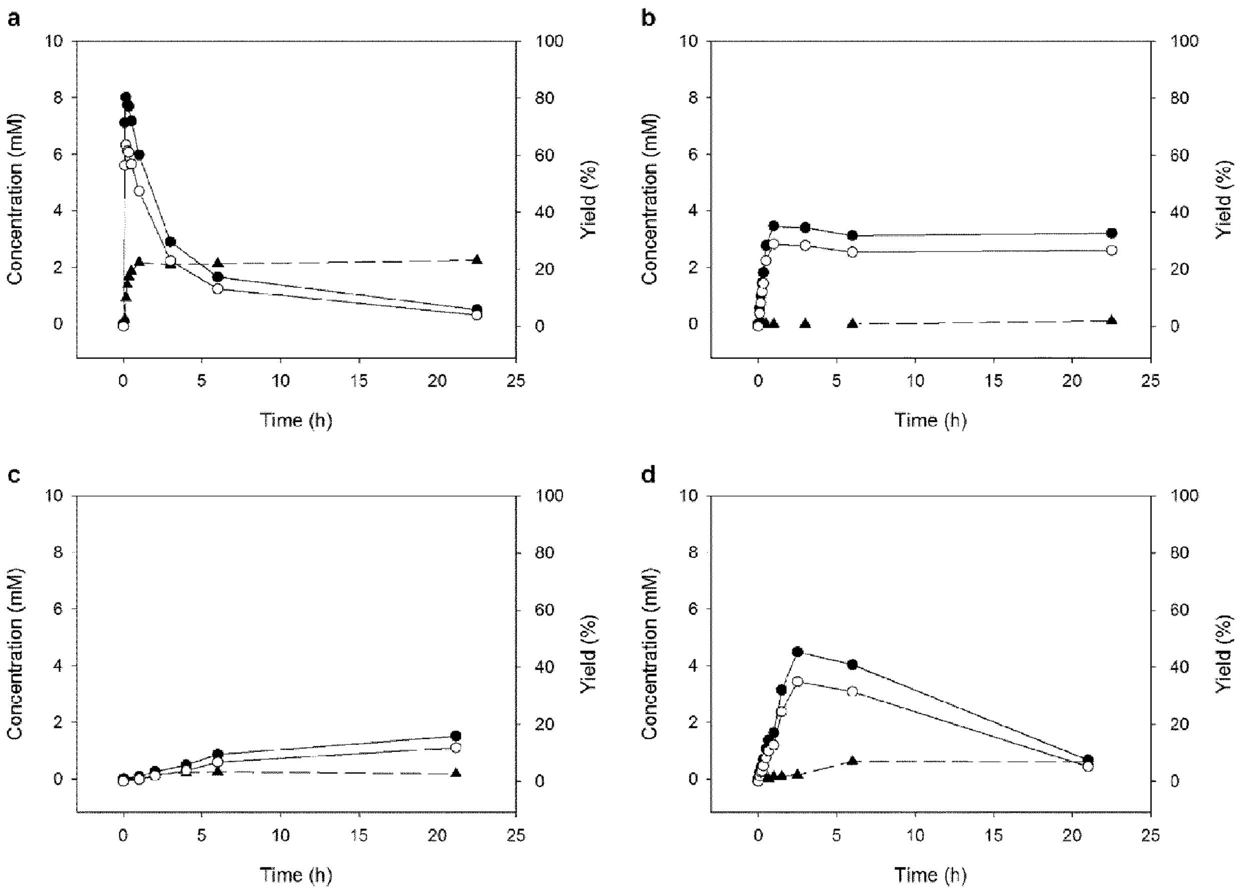
a
b
c
d
Concentration (mM)
Yield (%)
Time (h)
0
2
4
6
8
10
20
40
60
80
100
5
15
25

Fig. 7

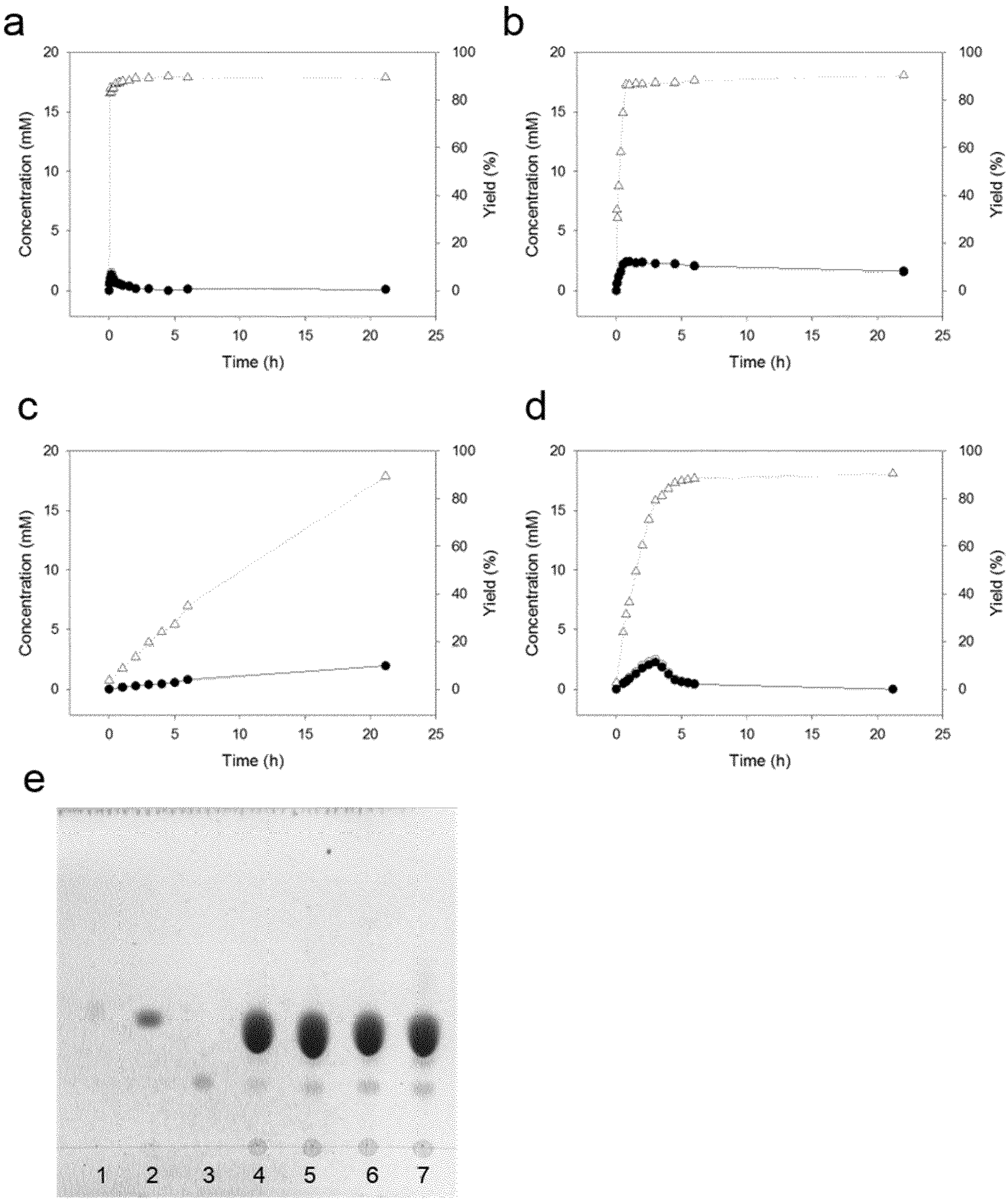

Fig 8

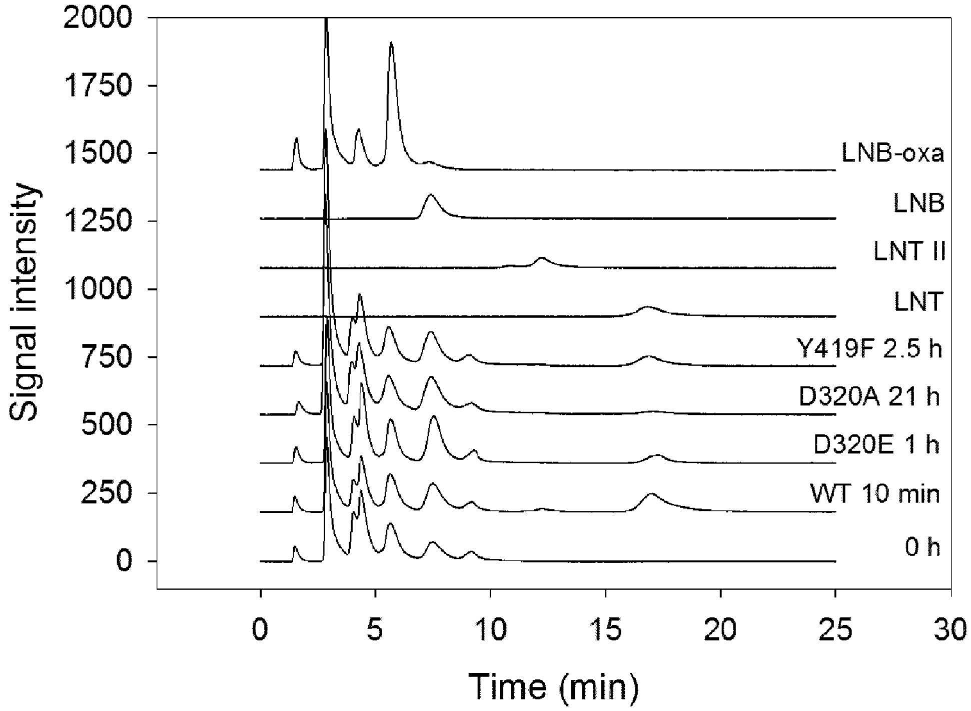
Signal intensity
2000
1750
1500
1250
1000
750
500
250
0
LNB-oxa
LNB
LNT II
LNT
Y419F 2.5 h
D320A 21 h
D320E 1 h
WT 10 min
0 h
0
5
10
15
20
25
30
Time (min)

Fig. 9

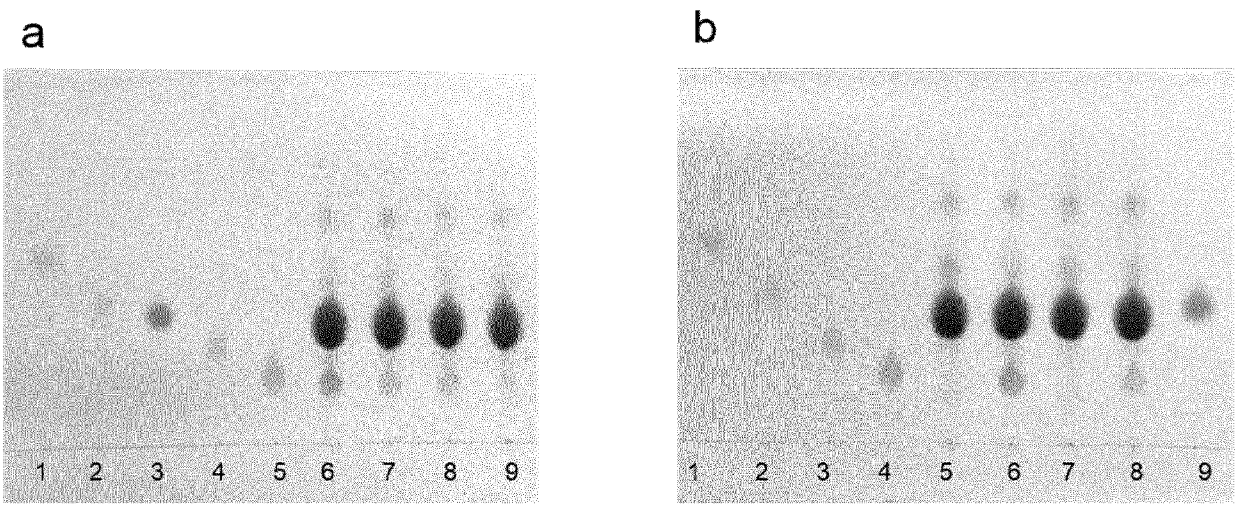
a
1 2 3 4 5 6 7 8 9
b
1 2 3 4 5 6 7 8 9

Fig. 10

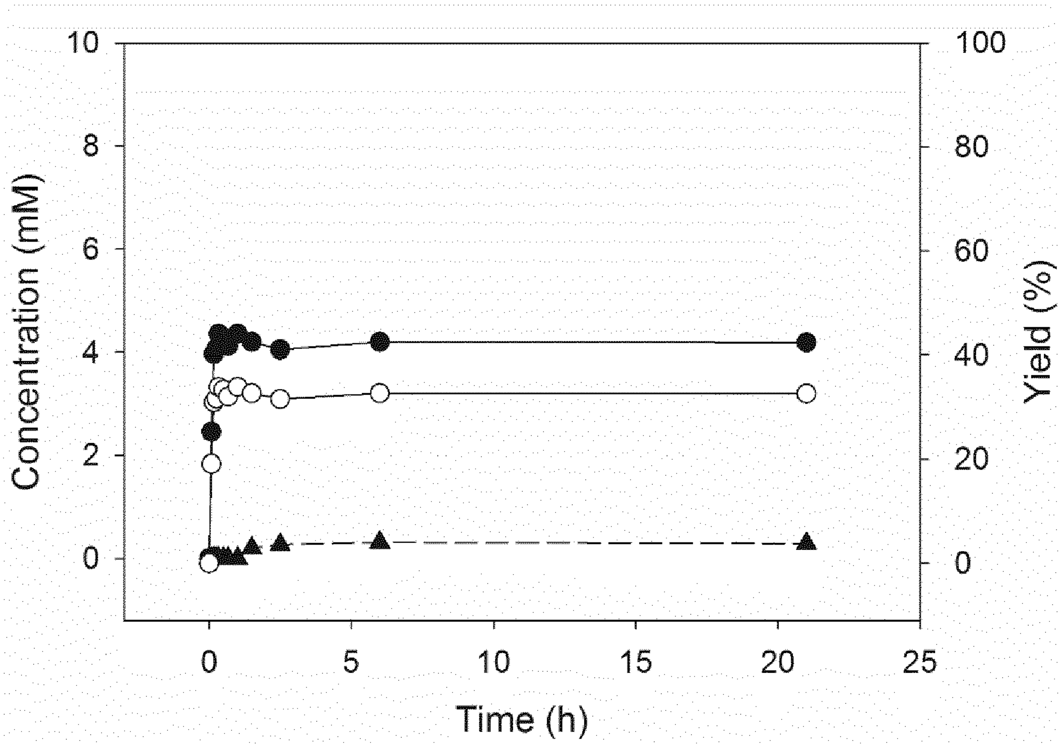

10
8
6
4
2
0
Concentration (mM)
100
80
60
40
20
0
Yield (%)
0
5
10
15
20
25
Time (h)

Fig. 11

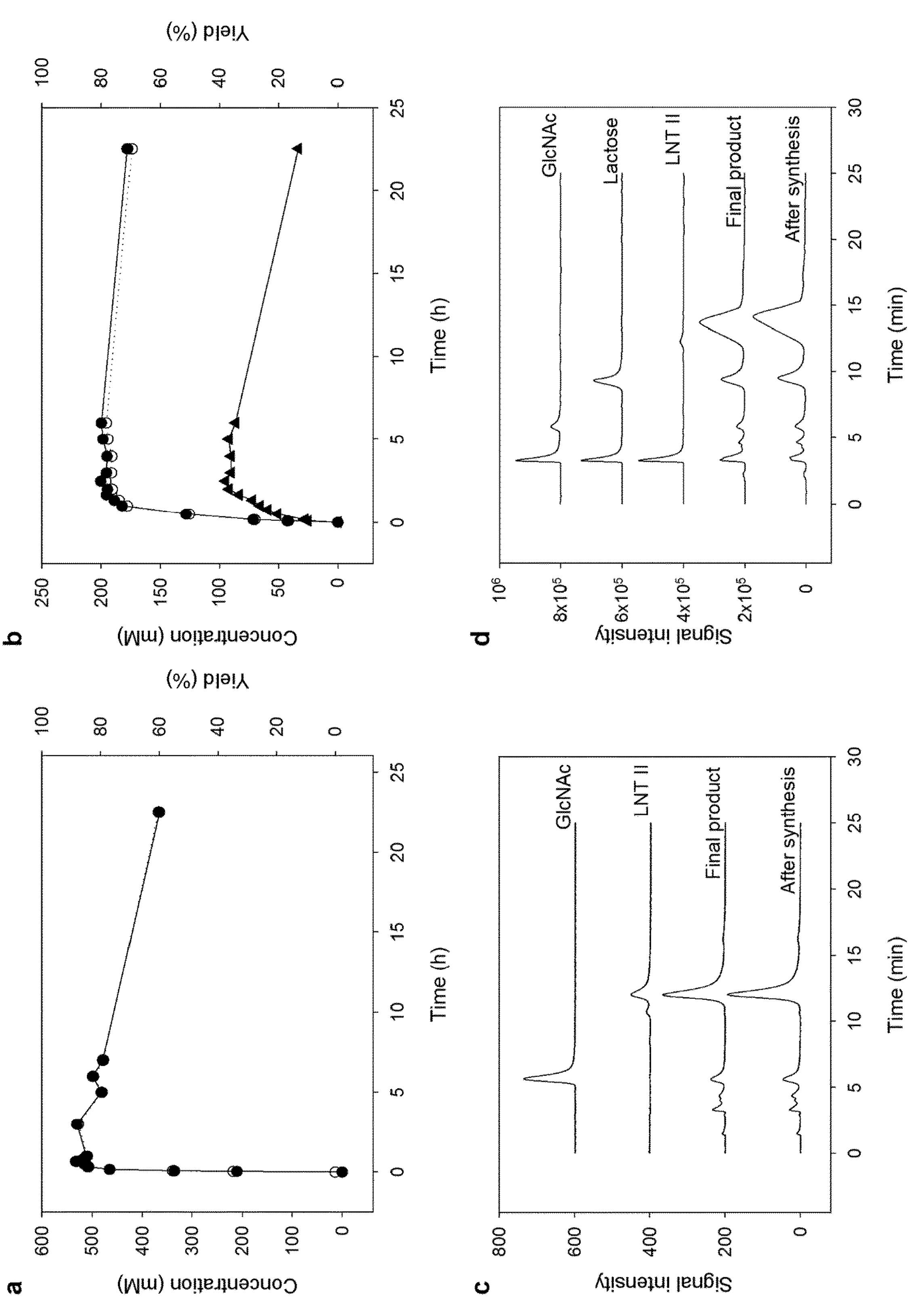
a
Concentration (mM)
Yield (%)
Time (h)
b
Concentration (mM)
Yield (%)
Time (h)
c
Signal intensity
GlcNAc
LNT II
Final product
After synthesis
Time (min)
d
Signal intensity
GlcNAc
Lactose
LNT II
Final product
After synthesis
Time (min)

Fig. 12

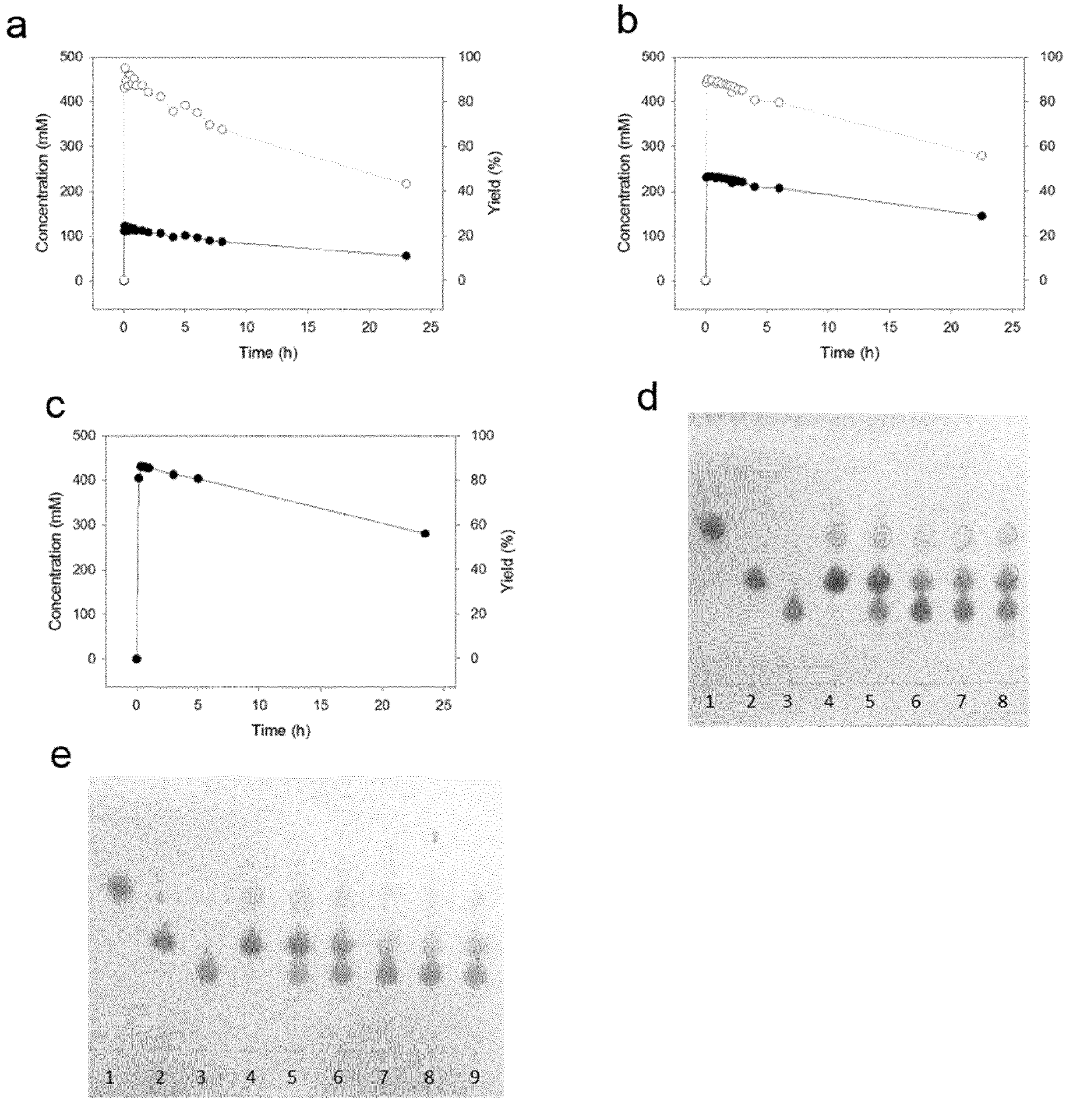
a
Concentration (mM)
Yield (%)
Time (h)
b
Concentration (mM)
Yield (%)
Time (h)
c
Concentration (mM)
Yield (%)
Time (h)
d
1 2 3 4 5 6 7 8
e
1 2 3 4 5 6 7 8 9

Fig. 13

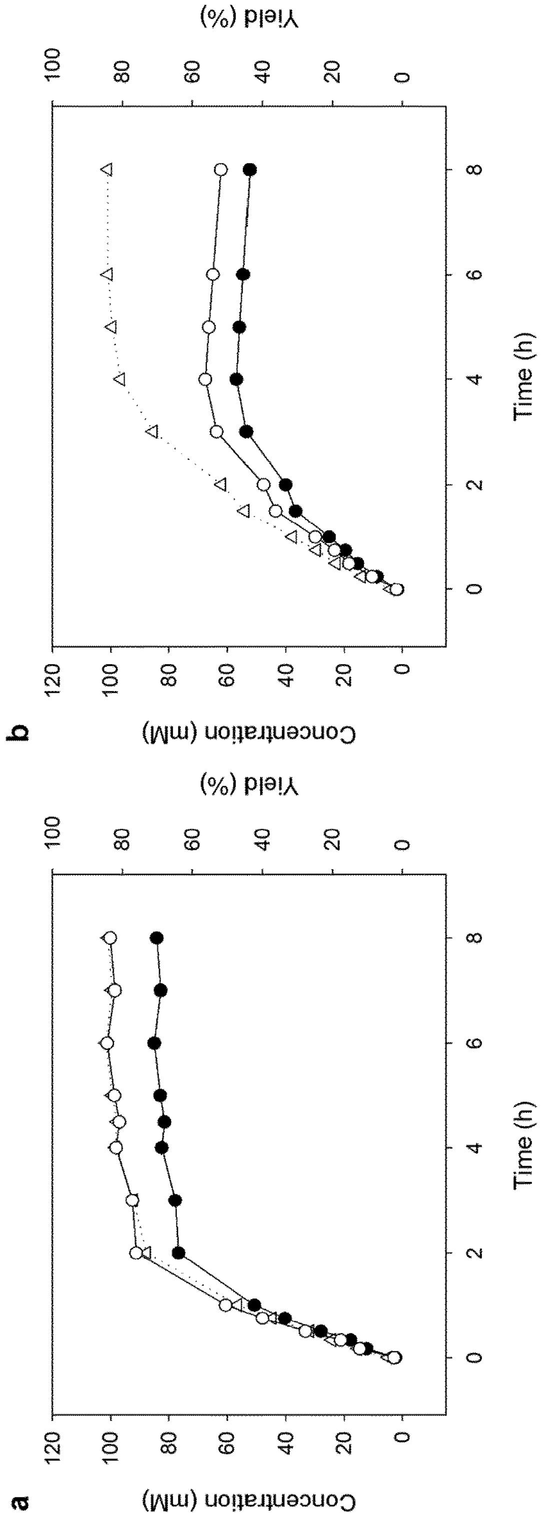

Fig. 14

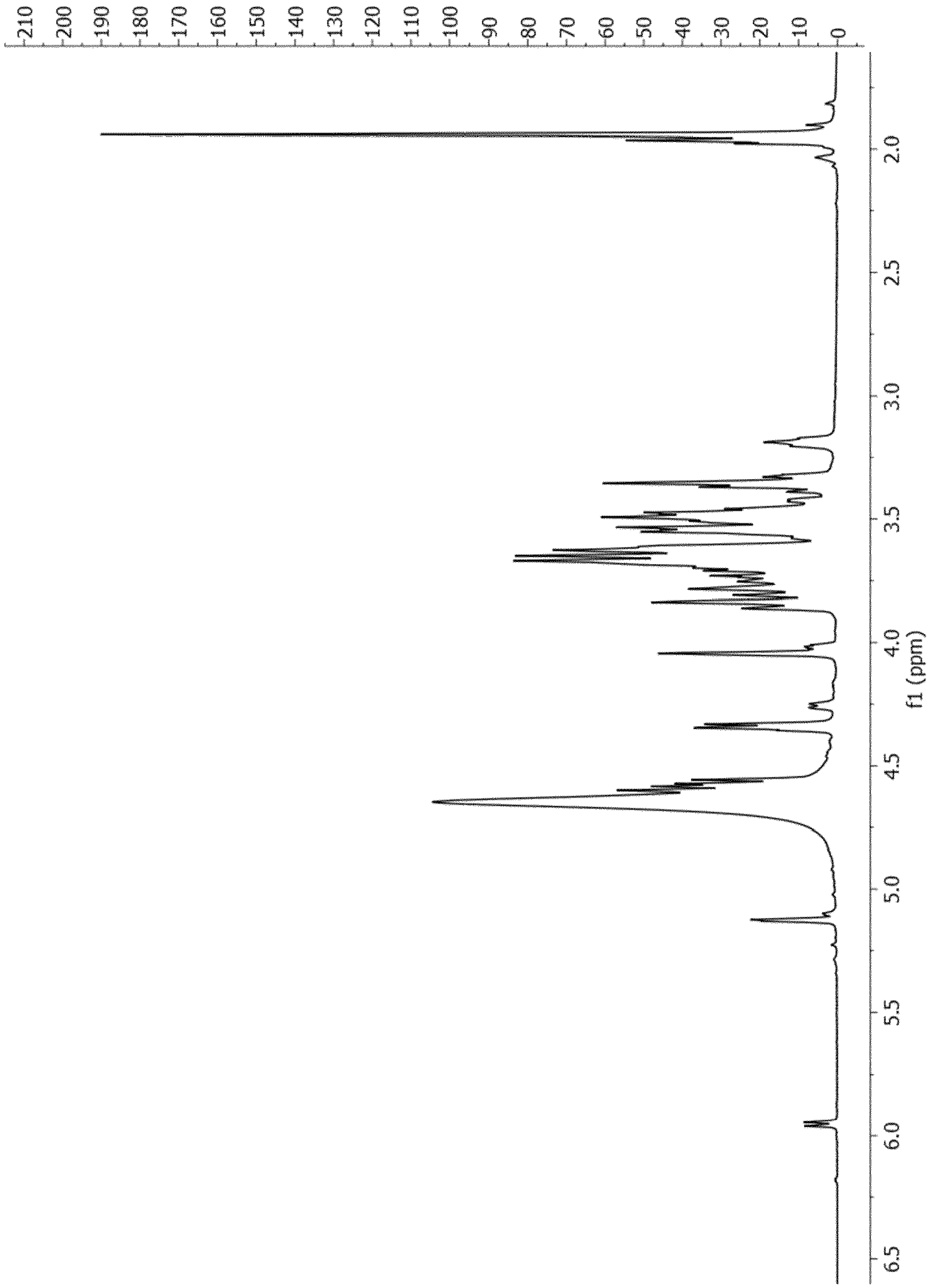

Fig. 15

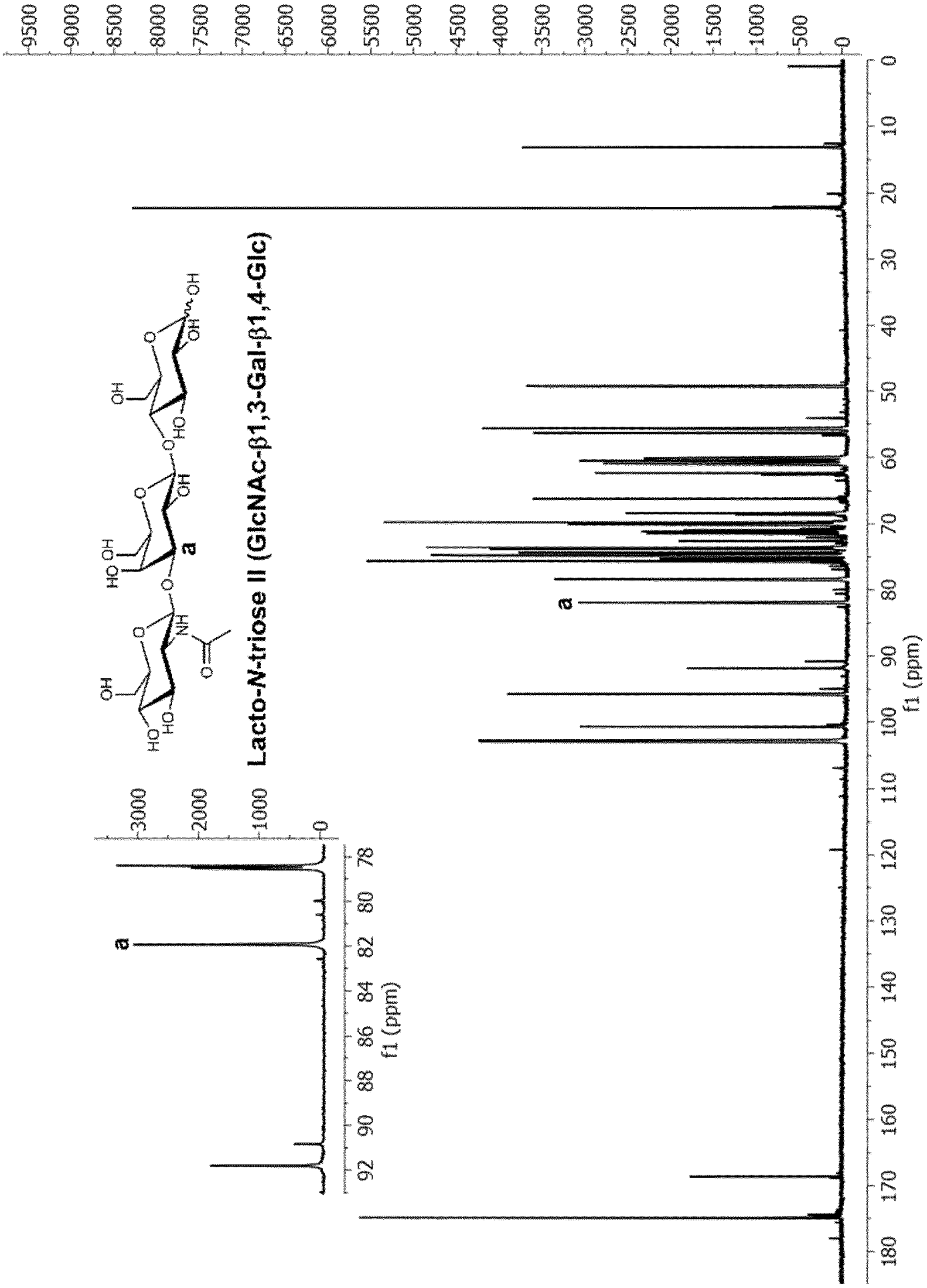

Fig. 16

```
Ruler 1                          1       10        20        30        40        50
Consensus                        --------MAsmTg-----xgqmxxxP---------------------------------
SEQ ID NO 12: Bbhl_WT            --------MASMTG-----GQQMGRDPNSSSVDKLSDDNLALNQTVTASSYEVATTAPEK
SEQ ID NO 13: Bbhl_D746E         --------MASMTG-----GQQMGRDPNSSSVDKLSDDNLALNQTVTASSYEVATTAPEK
SEQ ID NO 14: Bbhl_D746A         --------MASMTG-----GQQMGRDPNSSSVDKLSDDNLALNQTVTASSYEVATTAPEK
SEQ ID NO 15: Bbhl_D746Q         --------MASMTG-----GQQMGRDPNSSSVDKLSDDNLALNQTVTASSYEVATTAPEK
SEQ ID NO 16: Bbhl_Y827F         --------MASMTG-----GQQMGRDPNSSSVDKLSDDNLALNQTVTASSYEVATTAPEK
SEQ ID NO 17: LnbB_WT            --------MADDSA-----AGYSATAP---------------------------------
SEQ ID NO 18: LnbB_D320E         --------MADDSA-----AGYSATAP---------------------------------
SEQ ID NO 19: LnbB_D320A         --------MADDSA-----AGYSATAP---------------------------------
SEQ ID NO 20: LnbB_Y419F         --------MADDSA-----AGYSATAP---------------------------------
SEQ ID NO 21: HEX1 (AKC34128.1)  --------MSPVMN----------------------------------------------
SEQ ID NO 22: HEX2 (AKC34129.1)  -----MKTIASLLI-----AGSLATSA---------------------------------
SEQ ID NO 23: Chb (AAB03808.1)   MNAFKLSALARLTATMGFLGGMGSAMADQQLVDQLSQLKLNVK-----------------
SEQ ID NO 24: SpHex (AAC38798.3) --------MT--TG-----AAPDRKAP---------------------------------

Ruler 1                                   70        80        90        100       110
Consensus                        ------------------------------------------------------------
SEQ ID NO 12: Bbhl_WT            AVDGDLGTRWGTAQNKAANEWIEVGLGGTKTVKQINIDFERKDADQNITSFKVELKQGDT
SEQ ID NO 13: Bbhl_D746E         AVDGDLGTRWGTAQNKAANEWIEVGLGGTKTVKQINIDFERKDADQNITSFKVELKQGDT
SEQ ID NO 14: Bbhl_D746A         AVDGDLGTRWGTAQNKAANEWIEVGLGGTKTVKQINIDFERKDADQNITSFKVELKQGDT
SEQ ID NO 15: Bbhl_D746Q         AVDGDLGTRWGTAQNKAANEWIEVGLGGTKTVKQINIDFERKDADQNITSFKVELKQGDT
SEQ ID NO 16: Bbhl_Y827F         AVDGDLGTRWGTAQNKAANEWIEVGLGGTKTVKQINIDFERKDADQNITSFKVELKQGDT
SEQ ID NO 17: LnbB_WT            ------------------------------------------------------------
SEQ ID NO 18: LnbB_D320E         ------------------------------------------------------------
SEQ ID NO 19: LnbB_D320A         ------------------------------------------------------------
SEQ ID NO 20: LnbB_Y419F         ------------------------------------------------------------
SEQ ID NO 21: HEX1 (AKC34128.1)  ------------------------------------------------------------
SEQ ID NO 22: HEX2 (AKC34129.1)  ------------------------------------------------------------
SEQ ID NO 23: Chb (AAB03808.1)   ------------------------------------------------------------
SEQ ID NO 24: SpHex (AAC38798.3) ------------------------------------------------------------
```

Fig. 16 continued

```
Ruler 1                                         130       140       150       160       170
Consensus                          -----------------------------------------------------------V
SEQ ID NO 12: BbhI_WT              YTKVYQKDTRAKQQEIILLDQAQQASAVKVTVLSADGGTMN-----------------WV
SEQ ID NO 13: BbhI_D746E           YTKVYQKDTRAKQQEIILLDQAQQASAVKVTVLSADGGTMN-----------------WV
SEQ ID NO 14: BbhI_D746A           YTKVYQKDTRAKQQEIILLDQAQQASAVKVTVLSADGGTMN-----------------WV
SEQ ID NO 15: BbhI_D746Q           YTKVYQKDTRAKQQEIILLDQAQQASAVKVTVLSADGGTMN-----------------WV
SEQ ID NO 16: BbhI_Y827F           YTKVYQKDTRAKQQEIILLDQAQQASAVKVTVLSADGGTMN-----------------WV
SEQ ID NO 17: LnbB_WT              -----------------------------------------------------------V
SEQ ID NO 18: LnbB_D320E           -----------------------------------------------------------V
SEQ ID NO 19: LnbB_D320A           -----------------------------------------------------------V
SEQ ID NO 20: LnbB_Y419F           -----------------------------------------------------------V
SEQ ID NO 21: HEX1 (AKC34128.1)    ------------------------------------------------------------
SEQ ID NO 22: HEX2 (AKC34129.1)    ------------------------------------------------------------
SEQ ID NO 23: Chb (AAB03808.1)     -----------------MLDNRAGENGVDCAALGADWASCNRVLFTLSNDGQAIDGKDWV
SEQ ID NO 24: SpHex (AAC38798.3)   ------------------------------------------------------------

Ruler 1                                         190       200       210       220       230
Consensus                          Nv----------------------------------------------------------
SEQ ID NO 12: BbhI_WT              NVGINEISVYSAPKETVLDTADTNHMLGATMTASSNETATLTPDKAIDQNRTGRNNRWAS
SEQ ID NO 13: BbhI_D746E           NVGINEISVYSAPKETVLDTADTNHMLGATMTASSNETATLTPDKAIDQNRTGRNNRWAS
SEQ ID NO 14: BbhI_D746A           NVGINEISVYSAPKETVLDTADTNHMLGATMTASSNETATLTPDKAIDQNRTGRNNRWAS
SEQ ID NO 15: BbhI_D746Q           NVGINEISVYSAPKETVLDTADTNHMLGATMTASSNETATLTPDKAIDQNRTGRNNRWAS
SEQ ID NO 16: BbhI_Y827F           NVGINEISVYSAPKETVLDTADTNHMLGATMTASSNETATLTPDKAIDQNRTGRNNRWAS
SEQ ID NO 17: LnbB_WT              NL----------------------------------------------------------
SEQ ID NO 18: LnbB_D320E           NL----------------------------------------------------------
SEQ ID NO 19: LnbB_D320A           NL----------------------------------------------------------
SEQ ID NO 20: LnbB_Y419F           NL----------------------------------------------------------
SEQ ID NO 21: HEX1 (AKC34128.1)    ------------------------------------------------------------
SEQ ID NO 22: HEX2 (AKC34129.1)    ------------------------------------------------------------
SEQ ID NO 23: Chb (AAB03808.1)     ------------------------------------------------------------
SEQ ID NO 24: SpHex (AAC38798.3)   -------VRPTPLDRVI-------------------------------------------
```

Fig. 16 continued

```
Ruler 1                              250       260       270       280       290
Consensus                            ---------------------------------PrPxxVq----SFD-s-----------
SEQ ID NO 12: BbhI_WT                GYETPSNIWLKAEFPRLTAVKDIRIYFFERDVNPKPTNVQ----SFDLSYTDSEGTEHTL
SEQ ID NO 13: BbhI_D746E             GYETPSNIWLKAEFPRLTAVKDIRIYFFERDVNPKPTNVQ----SFDLSYTDSEGTEHTL
SEQ ID NO 14: BbhI_D746A             GYETPSNIWLKAEFPRLTAVKDIRIYFFERDVNPKPTNVQ----SFDLSYTDSEGTEHTL
SEQ ID NO 15: BbhI_D746Q             GYETPSNIWLKAEFPRLTAVKDIRIYFFERDVNPKPTNVQ----SFDLSYTDSEGTEHTL
SEQ ID NO 16: BbhI_Y827F             GYETPSNIWLKAEFPRLTAVKDIRIYFFERDVNPKPTNVQ----SFDLSYTDSEGTEHTL
SEQ ID NO 17: LnbB_WT                ---------------------------------TRPATVP----SMD-------------
SEQ ID NO 18: LnbB_D320E             ---------------------------------TRPATVP----SMD-------------
SEQ ID NO 19: LnbB_D320A             ---------------------------------TRPATVP----SMD-------------
SEQ ID NO 20: LnbB_Y419F             ---------------------------------TRPATVP----SMD-------------
SEQ ID NO 21: HEX1 (AKC34128.1)      ------------------------------TVIPRPSSVL--------------------
SEQ ID NO 22: HEX2 (AKC34129.1)      -----------AEPPALI---------------PQPVSVQPGEGSFKFS-----------
SEQ ID NO 23: Chb (AAB03808.1)       ------------------------IYFHS----PRQ-TLRVDNDQFKIAHLTGDLYKLEP
SEQ ID NO 24: SpHex (AAC38798.3)     ---------------------------------PAPASVD--------------------

Ruler 1                              310       320       330       340       350
Consensus                            --Gya---sGxGy-----------------------------------eW----------
SEQ ID NO 12: BbhI_WT                KSGYAMTASGAGYVADVVIQLDQAVNARSLKLSNFAIKSSEYNNVSVAEWEAYSNDQAEP
SEQ ID NO 13: BbhI_D746E             KSGYAMTASGAGYVADVVIQLDQAVNARSLKLSNFAIKSSEYNNVSVAEWEAYSNDQAEP
SEQ ID NO 14: BbhI_D746A             KSGYAMTASGAGYVADVVIQLDQAVNARSLKLSNFAIKSSEYNNVSVAEWEAYSNDQAEP
SEQ ID NO 15: BbhI_D746Q             KSGYAMTASGAGYVADVVIQLDQAVNARSLKLSNFAIKSSEYNNVSVAEWEAYSNDQAEP
SEQ ID NO 16: BbhI_Y827F             KSGYAMTASGAGYVADVVIQLDQAVNARSLKLSNFAIKSSEYNNVSVAEWEAYSNDQAEP
SEQ ID NO 17: LnbB_WT                --GWT---DGTG------------------------------------AW----------
SEQ ID NO 18: LnbB_D320E             --GWT---DGTG------------------------------------AW----------
SEQ ID NO 19: LnbB_D320A             --GWT---DGTG------------------------------------AW----------
SEQ ID NO 20: LnbB_Y419F             --GWT---DGTG------------------------------------AW----------
SEQ ID NO 21: HEX1 (AKC34128.1)      ---------GTG------------------------------------------------
SEQ ID NO 22: HEX2 (AKC34129.1)      --------AGT-------------------------------------------------
SEQ ID NO 23: Chb (AAB03808.1)       TAKFSGFPAGKAVEIPVVAEY----------------------------WQLFRND----
SEQ ID NO 24: SpHex (AAC38798.3)     -------PGGAPY-----------------------------------------------
```

Fig. 16 continued

| Ruler 1 | 370 · 380 · 390 · 400 · 410 |
|---|---|
| Consensus | ----------------------------TxPxGyx------------------------- |
| SEQ ID NO 12: BbhI_WT | GATLDSVVSDLESNHLTIETDTDTLALPTVPDGYTVKFNGADYEQLIAADGTVNHPLVDK |
| SEQ ID NO 13: BbhI_D746E | GATLDSVVSDLESNHLTIETDTDTLALPTVPDGYTVKFNGADYEQLIAADGTVNHPLVDK |
| SEQ ID NO 14: BbhI_D746A | GATLDSVVSDLESNHLTIETDTDTLALPTVPDGYTVKFNGADYEQLIAADGTVNHPLVDK |
| SEQ ID NO 15: BbhI_D746Q | GATLDSVVSDLESNHLTIETDTDTLALPTVPDGYTVKFNGADYEQLIAADGTVNHPLVDK |
| SEQ ID NO 16: BbhI_Y827F | GATLDSVVSDLESNHLTIETDTDTLALPTVPDGYTVKFNGADYEQLIAADGTVNHPLVDK |
| SEQ ID NO 17: LnbB_WT | ----------------------------TLGEGTR------------------------- |
| SEQ ID NO 18: LnbB_D320E | ----------------------------TLGEGTR------------------------- |
| SEQ ID NO 19: LnbB_D320A | ----------------------------TLGEGTR------------------------- |
| SEQ ID NO 20: LnbB_Y419F | ----------------------------TLGEGTR------------------------- |
| SEQ ID NO 21: HEX1 (AKC34128.1) | -------------RSFELPADARILVQPNAPE---------------------------- |
| SEQ ID NO 22: HEX2 (AKC34129.1) | --------------------------------GIR------------------------- |
| SEQ ID NO 23: Chb (AAB03808.1) | ----------------------------FLPRWYA------------------------- |
| SEQ ID NO 24: SpHex (AAC38798.3) | ----------------------------RITRGTHIRV---------------------- |

| Ruler 1 | 430 · 440 · 450 · 460 · 470 |
|---|---|
| Consensus | ------VV---------------------------------------------------- |
| SEQ ID NO 12: BbhI_WT | TVQVAYVVTDTATGNTKTTSDIPYVVKGTNQQQEGNNAKPTIIPEIAEWHSTSAAKLAAS |
| SEQ ID NO 13: BbhI_D746E | TVQVAYVVTDTATGNTKTTSDIPYVVKGTNQQQEGNNAKPTIIPEIAEWHSTSAAKLAAS |
| SEQ ID NO 14: BbhI_D746A | TVQVAYVVTDTATGNTKTTSDIPYVVKGTNQQQEGNNAKPTIIPEIAEWHSTSAAKLAAS |
| SEQ ID NO 15: BbhI_D746Q | TVQVAYVVTDTATGNTKTTSDIPYVVKGTNQQQEGNNAKPTIIPEIAEWHSTSAAKLAAS |
| SEQ ID NO 16: BbhI_Y827F | TVQVAYVVTDTATGNTKTTSDIPYVVKGTNQQQEGNNAKPTIIPEIAEWHSTSAAKLAAS |
| SEQ ID NO 17: LnbB_WT | ------VV---------------------------------------------------- |
| SEQ ID NO 18: LnbB_D320E | ------VV---------------------------------------------------- |
| SEQ ID NO 19: LnbB_D320A | ------VV---------------------------------------------------- |
| SEQ ID NO 20: LnbB_Y419F | ------VV---------------------------------------------------- |
| SEQ ID NO 21: HEX1 (AKC34128.1) | ------VA---------------------------------------------------- |
| SEQ ID NO 22: HEX2 (AKC34129.1) | ------------------------------------------------------------ |
| SEQ ID NO 23: Chb (AAB03808.1) | ---------------------------------TSGDAKPKMLANT-------------- |
| SEQ ID NO 24: SpHex (AAC38798.3) | ------------------------------------------------------------ |

Fig. 16 continued

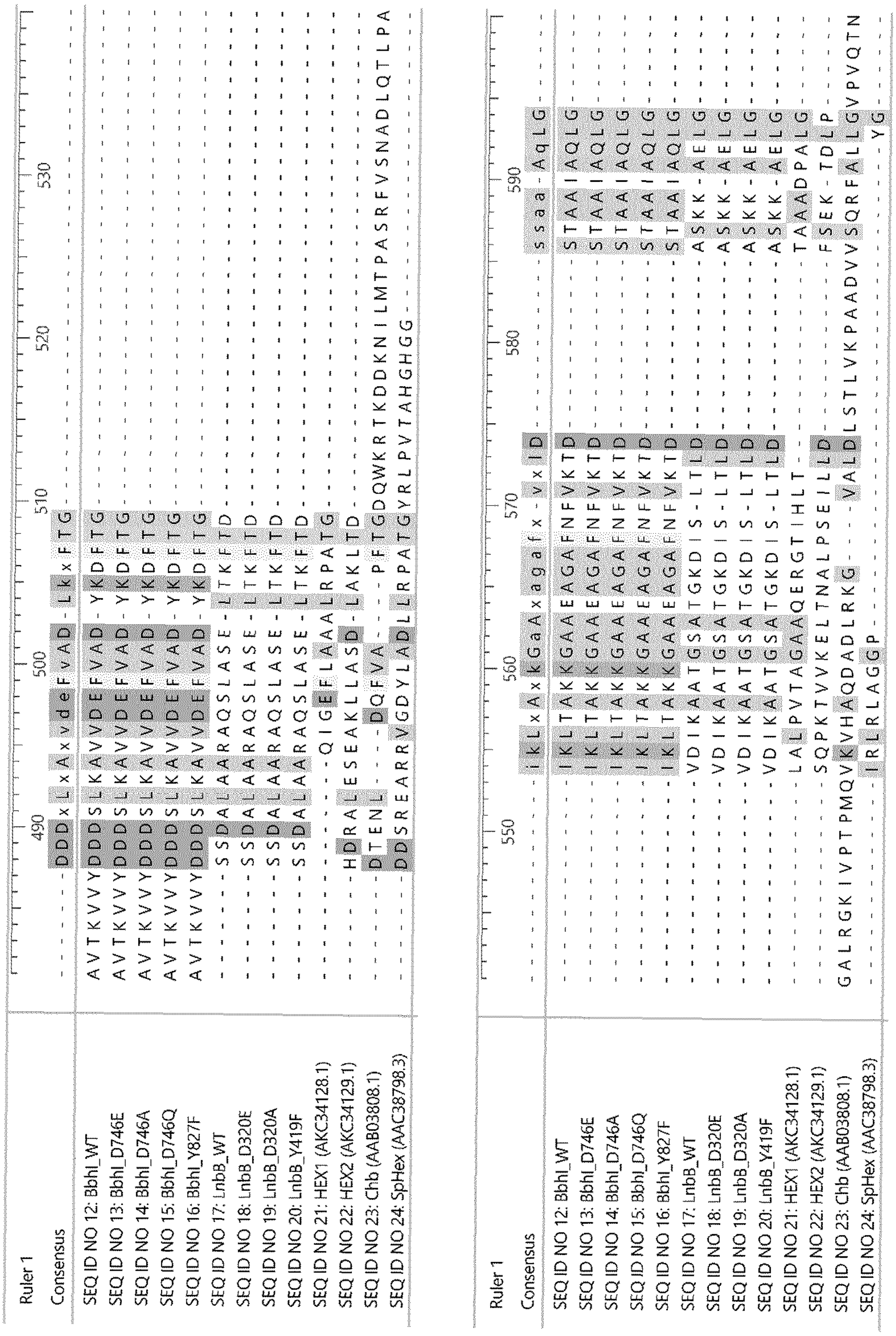

Fig. 16 continued

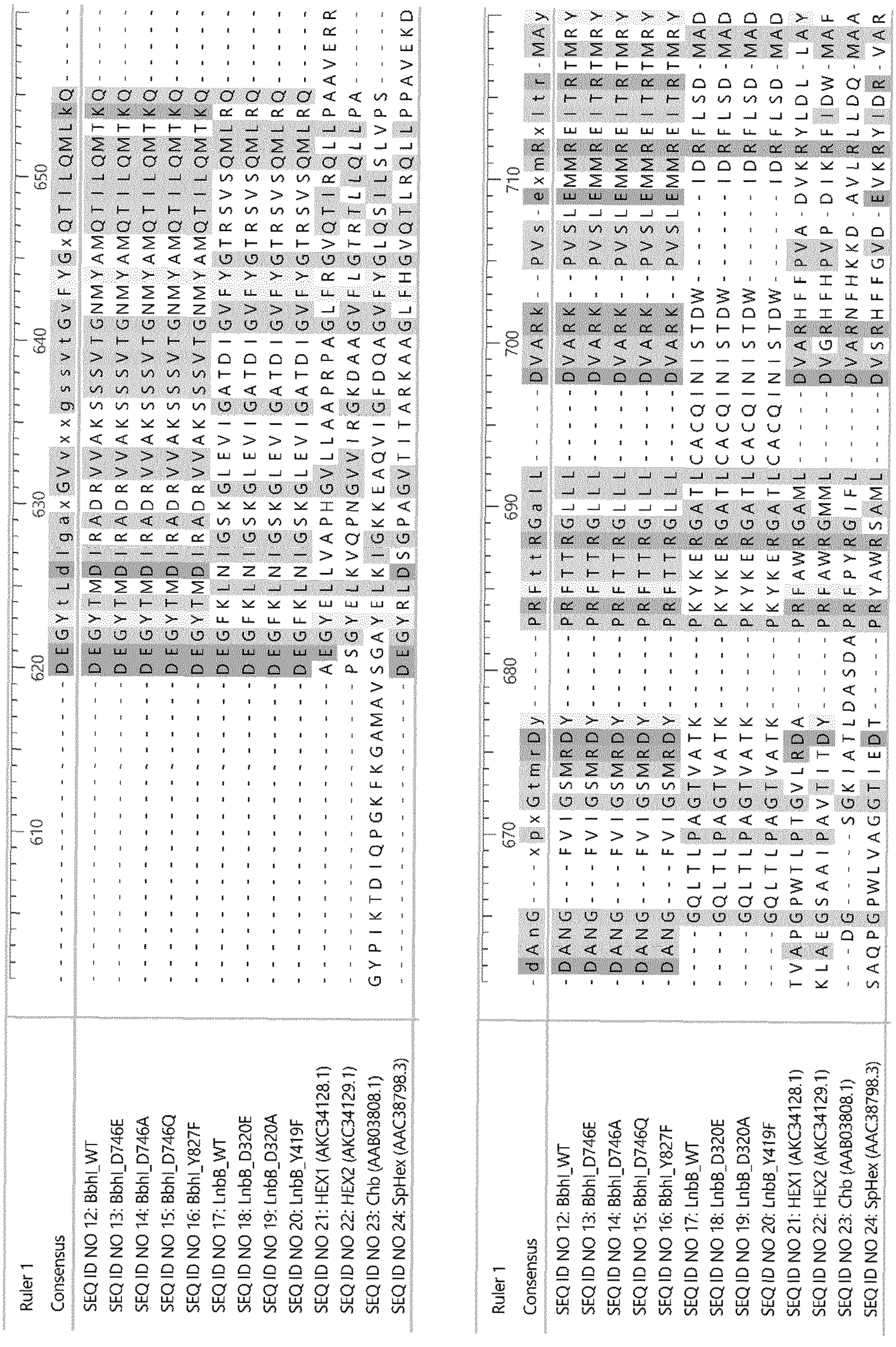

Fig. 16 continued

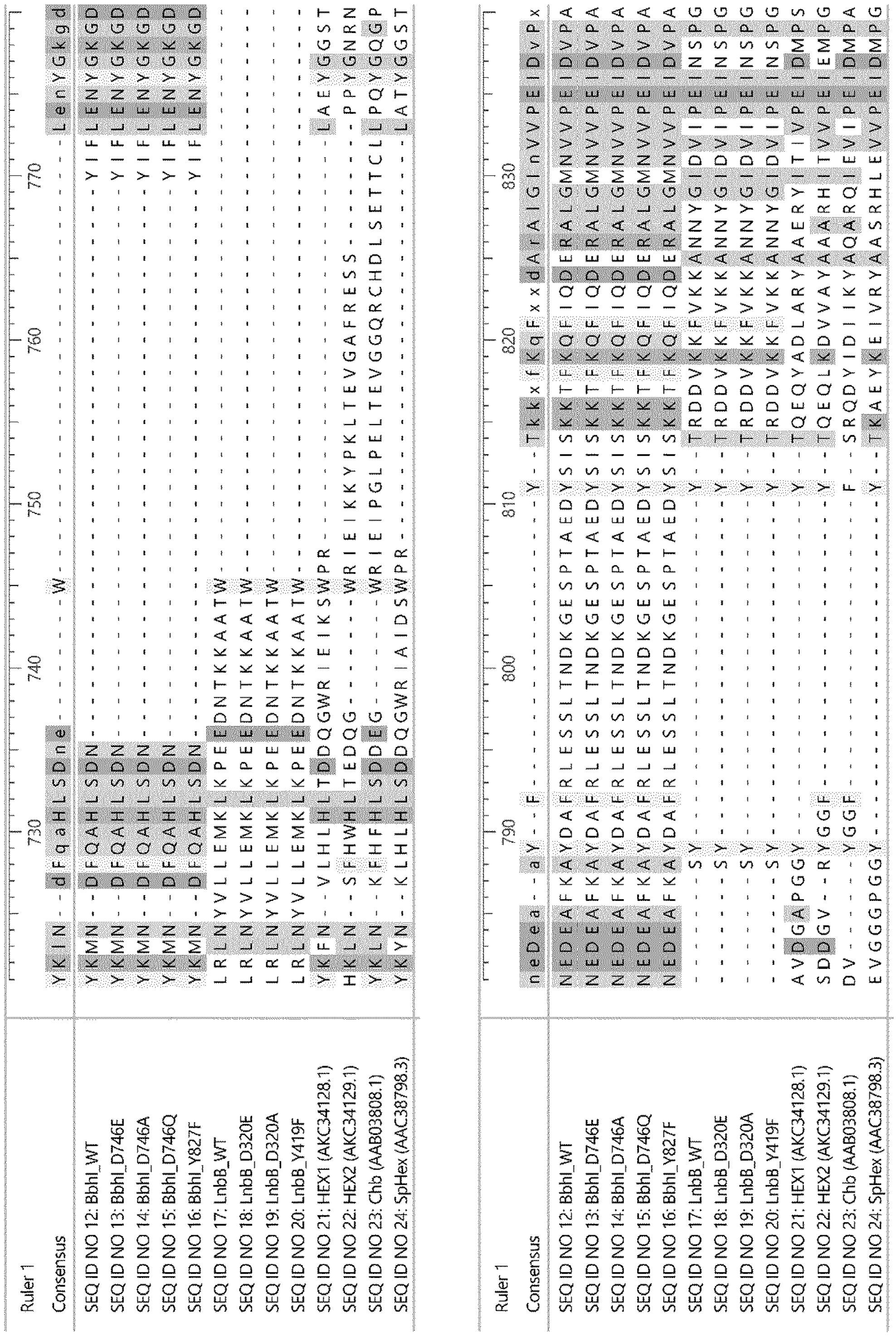

Fig. 16 continued

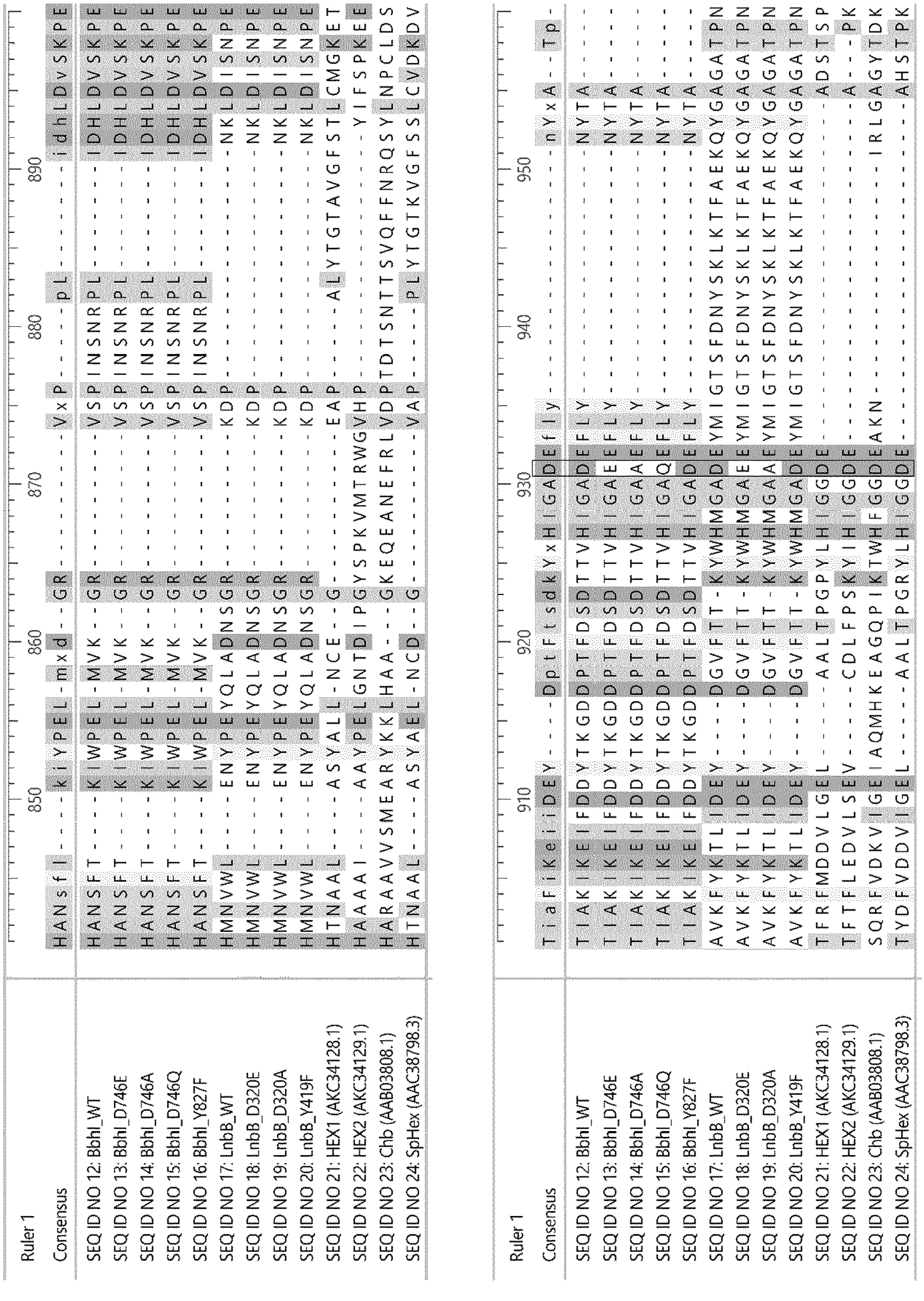

Fig. 16 continued

| Ruler 1 (970–1.010) | |
|---|---|
| Consensus | d-----------------yrk----------------------FINelvpYVKxxG-vrm |
| SEQ ID NO 12: BbhI_WT | ------------------YRK----------------------FINEIVPYIKDTNTVRM |
| SEQ ID NO 13: BbhI_D746E | ------------------YRK----------------------FINEIVPYIKDTNTVRM |
| SEQ ID NO 14: BbhI_D746A | ------------------YRK----------------------FINEIVPYIKDTNTVRM |
| SEQ ID NO 15: BbhI_D746Q | ------------------YRK----------------------FINEIVPYIKDTNTVRM |
| SEQ ID NO 16: BbhI_Y827F | ------------------YRK----------------------FINEIVPYIKDTNTVRM |
| SEQ ID NO 17: LnbB_WT | DA----------------FTG----------------------FINDIDKYVKAKG-KQL |
| SEQ ID NO 18: LnbB_D320E | DA----------------FTG----------------------FINDIDKYVKAKG-KQL |
| SEQ ID NO 19: LnbB_D320A | DA----------------FTG----------------------FINDIDKYVKAKG-KQL |
| SEQ ID NO 20: LnbB_Y419F | DA----------------FTG----------------------FINDIDKYVKAKG-KQL |
| SEQ ID NO 21: HEX1 (AKC34128.1) | ED----------------YQT----------------------FMRQVQAIVQSHG---- |
| SEQ ID NO 22: HEX2 (AKC34129.1) | DQ----------------WKQSKFAQEVIKREGLKNEEELQSWFIRRIGRFLESKN---- |
| SEQ ID NO 23: Chb (AAB03808.1) | AKPEPGKGIIDQSNEDKPWAKSQVCQTMIKEGKVADMEHLPSYFGQEVSKLVKAHGIDRM |
| SEQ ID NO 24: SpHex (AAC38798.3) | AD----------------FVA----------------------FMKRVQPIVAKYG---- |

| Ruler 1 (1.030–1.070) | |
|---|---|
| Consensus | ---------------------vi--WgGltwind---EltxDgienvemxl---WS---- |
| SEQ ID NO 12: BbhI_WT | -------------------------WGGLTWINDHKTEITKDAIENVEMNL---WS---- |
| SEQ ID NO 13: BbhI_D746E | -------------------------WGGLTWINDHKTEITKDAIENVEMNL---WS---- |
| SEQ ID NO 14: BbhI_D746A | -------------------------WGGLTWINDHKTEITKDAIENVEMNL---WS---- |
| SEQ ID NO 15: BbhI_D746Q | -------------------------WGGLTWINDHKTEITKDAIENVEMNL---WS---- |
| SEQ ID NO 16: BbhI_Y827F | -------------------------WGGLTWINDHKTEITKDAIENVEMNL---WS---- |
| SEQ ID NO 17: LnbB_WT | RIWNDGIVNTKNVSLNK--DIVIEYWYGAGRKPQ---ELVQDGYTLMNATQALYWSRSAQ |
| SEQ ID NO 18: LnbB_D320E | RIWNDGIVNTKNVSLNK--DIVIEYWYGAGRKPQ---ELVQDGYTLMNATQALYWSRSAQ |
| SEQ ID NO 19: LnbB_D320A | RIWNDGIVNTKNVSLNK--DIVIEYWYGAGRKPQ---ELVQDGYTLMNATQALYWSRSAQ |
| SEQ ID NO 20: LnbB_Y419F | RIWNDGIVNTKNVSLNK--DIVIEYWYGAGRKPQ---ELVQDGYTLMNATQALFWSRSAQ |
| SEQ ID NO 21: HEX1 (AKC34128.1) | -------------------KTVVG-WG----------EIAQAPLTP--GTMVQHWR---- |
| SEQ ID NO 22: HEX2 (AKC34129.1) | -------------------RNLIG-WD----------EIQEGGLPKTATMMV--WR---- |
| SEQ ID NO 23: Chb (AAB03808.1) | QAWQDGLKDAESSKAFATSRVGVNFWD------------------------TLYWG---- |
| SEQ ID NO 24: SpHex (AAC38798.3) | -------------------KTVVG-WHQLA------------GAEPVEGALVQYWG---- |

**Fig. 16 continued**

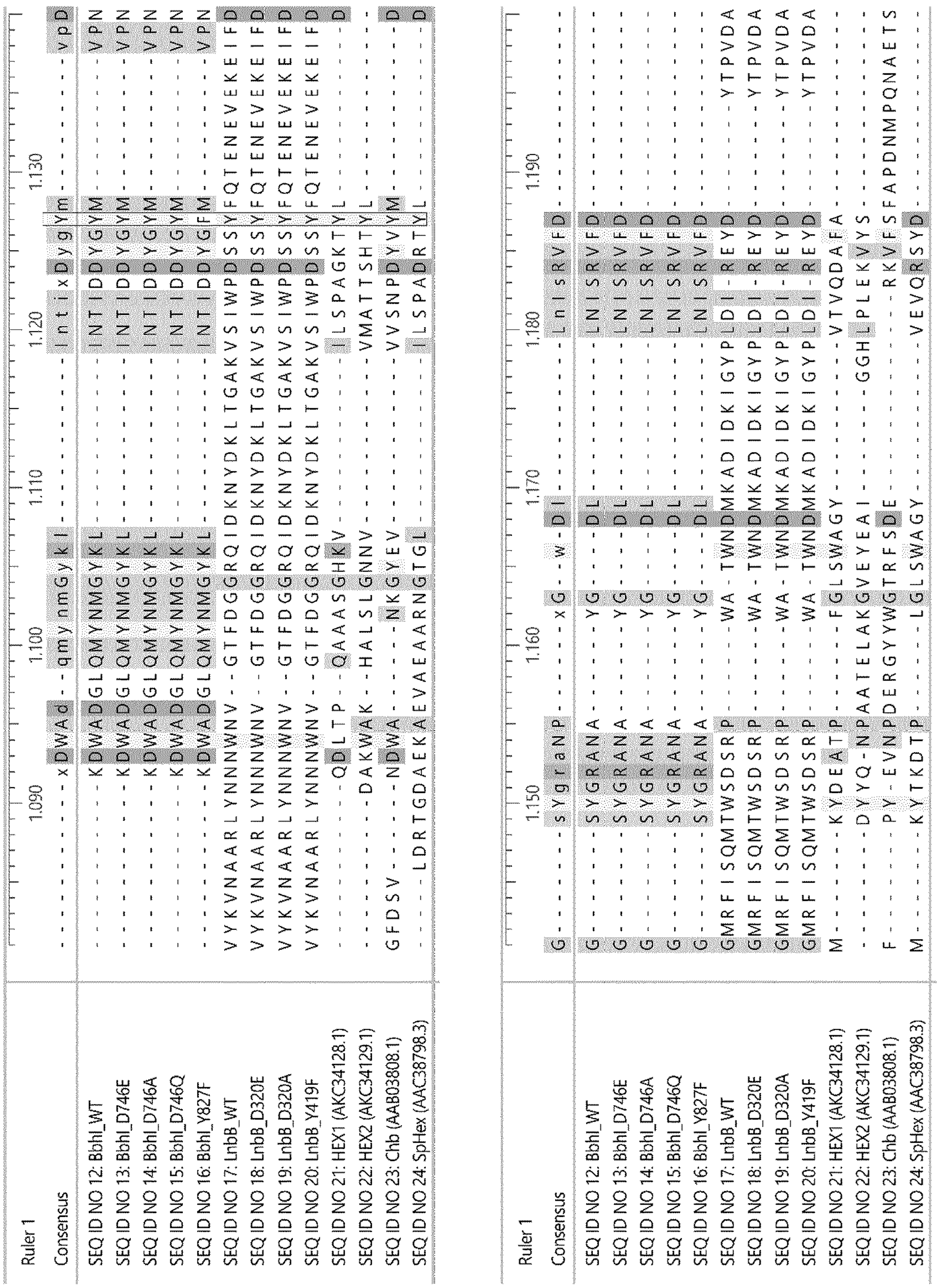

**Fig. 16 continued**

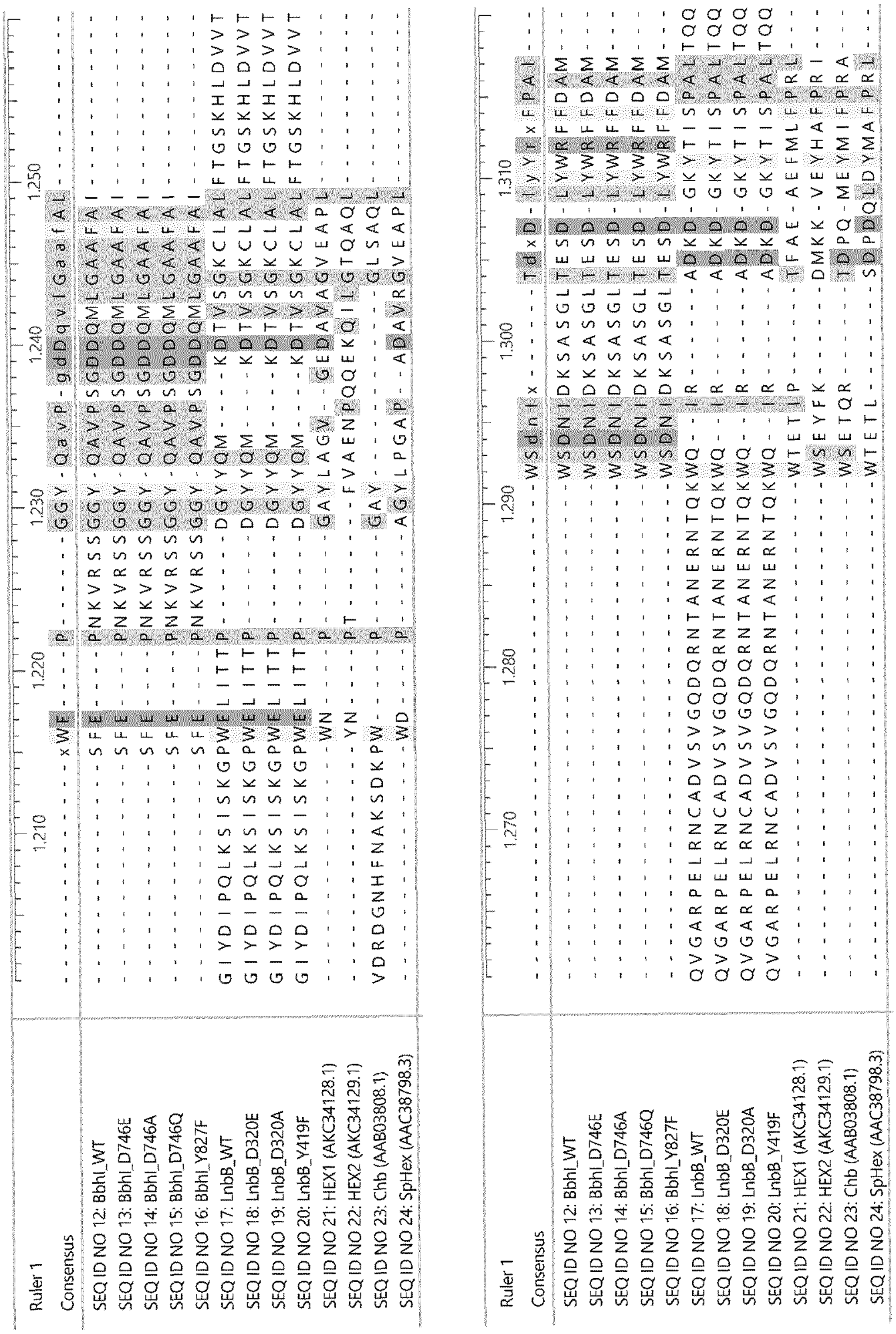

Fig. 16 continued

| Ruler 1 | 1.330 1.340 1.350 1.360 1.370 |
|---|---|
| Consensus | ------------------------------------------------------------ |
| SEQ ID NO 12: Bbhl_WT | ------------------------------------------------------------ |
| SEQ ID NO 13: Bbhl_D746E | ------------------------------------------------------------ |
| SEQ ID NO 14: Bbhl_D746A | ------------------------------------------------------------ |
| SEQ ID NO 15: Bbhl_D746Q | ------------------------------------------------------------ |
| SEQ ID NO 16: Bbhl_Y827F | ------------------------------------------------------------ |
| SEQ ID NO 17: LnbB_WT | RLAIATGNEQNIDLETHRPAAGTVAQFPADLVSDNALFTLTGHMGMSATVDSKTVNPASP |
| SEQ ID NO 18: LnbB_D320E | RLAIATGNEQNIDLETHRPAAGTVAQFPADLVSDNALFTLTGHMGMSATVDSKTVNPASP |
| SEQ ID NO 19: LnbB_D320A | RLAIATGNEQNIDLETHRPAAGTVAQFPADLVSDNALFTLTGHMGMSATVDSKTVNPASP |
| SEQ ID NO 20: LnbB_Y419F | RLAIATGNEQNIDLETHRPAAGTVAQFPADLVSDNALFTLTGHMGMSATVDSKTVNPASP |
| SEQ ID NO 21: HEX1 (AKC34128.1) | ------------------------------------------------------------ |
| SEQ ID NO 22: HEX2 (AKC34129.1) | ------------------------------------------------------------ |
| SEQ ID NO 23: Chb (AAB03808.1) | ------------------------------------------------------------ |
| SEQ ID NO 24: SpHex (AAC38798.3) | ------------------------------------------------------------ |

| Ruler 1 | 1.390 1.400 1.410 1.420 1.430 |
|---|---|
| Consensus | ---------------------PfVAEkgW------------------------------- |
| SEQ ID NO 12: Bbhl_WT | ---------------------PFYAEKTWAATGKEKGTAAKLTALAAKQGTGPRTNPYYQ |
| SEQ ID NO 13: Bbhl_D746E | ---------------------PFYAEKTWAATGKEKGTAAKLTALAAKQGTGPRTNPYYQ |
| SEQ ID NO 14: Bbhl_D746A | ---------------------PFYAEKTWAATGKEKGTAAKLTALAAKQGTGPRTNPYYQ |
| SEQ ID NO 15: Bbhl_D746Q | ---------------------PFYAEKTWAATGKEKGTAAKLTALAAKQGTGPRTNPYYQ |
| SEQ ID NO 16: Bbhl_Y827F | ---------------------PFYAEKTWAATGKEKGTAAKLTALAAKQGTGPRTNPYYQ |
| SEQ ID NO 17: LnbB_WT | SKITVKVRAASNANTGDVTVTPVVPE-GW------------------------------- |
| SEQ ID NO 18: LnbB_D320E | SKITVKVRAASNANTGDVTVTPVVPE-GW------------------------------- |
| SEQ ID NO 19: LnbB_D320A | SKITVKVRAASNANTGDVTVTPVVPE-GW------------------------------- |
| SEQ ID NO 20: LnbB_Y419F | SKITVKVRAASNANTGDVTVTPVVPE-GW------------------------------- |
| SEQ ID NO 21: HEX1 (AKC34128.1) | ---------------------LAVAELGW------------------------------- |
| SEQ ID NO 22: HEX2 (AKC34129.1) | ---------------------AALAEVAW------------------------------- |
| SEQ ID NO 23: Chb (AAB03808.1) | ---------------------LSVAERSW------------------------------- |
| SEQ ID NO 24: SpHex (AAC38798.3) | ---------------------PGVAELGW------------------------------- |

Fig. 16 continued

| | |
|---|---|
| Ruler 1 | 1.450 1.460 1.470 1.480 1.490 |
| Consensus | ------------------------------------------------------------ |
| SEQ ID NO 12: BbhI_WT | ATSKNSVYESYDFNDGLADASGNGRDLTIGDGSKAAVKDQSLKLAGGSSYATSKLDKLGN |
| SEQ ID NO 13: BbhI_D746E | ATSKNSVYESYDFNDGLADASGNGRDLTIGDGSKAAVKDQSLKLAGGSSYATSKLDKLGN |
| SEQ ID NO 14: BbhI_D746A | ATSKNSVYESYDFNDGLADASGNGRDLTIGDGSKAAVKDQSLKLAGGSSYATSKLDKLGN |
| SEQ ID NO 15: BbhI_D746Q | ATSKNSVYESYDFNDGLADASGNGRDLTIGDGSKAAVKDQSLKLAGGSSYATSKLDKLGN |
| SEQ ID NO 16: BbhI_Y827F | ATSKNSVYESYDFNDGLADASGNGRDLTIGDGSKAAVKDQSLKLAGGSSYATSKLDKLGN |
| SEQ ID NO 17: LnbB_WT | ------------------------------------------------------------ |
| SEQ ID NO 18: LnbB_D320E | ------------------------------------------------------------ |
| SEQ ID NO 19: LnbB_D320A | ------------------------------------------------------------ |
| SEQ ID NO 20: LnbB_Y419F | ------------------------------------------------------------ |
| SEQ ID NO 21: HEX1 (AKC34128.1) | ------------------------------------------------------------ |
| SEQ ID NO 22: HEX2 (AKC34129.1) | ------------------------------------------------------------ |
| SEQ ID NO 23: Chb (AAB03808.1) | ------------------------------------------------------------ |
| SEQ ID NO 24: SpHex (AAC38798.3) | ------------------------------------------------------------ |

| | |
|---|---|
| Ruler 1 | 1.510 1.520 1.530 1.540 1.550 |
| Consensus | ------------aaKPGdilfea-aPaGthdIr----------------YFd-------- |
| SEQ ID NO 12: BbhI_WT | GNELTFDVTLQQAAKPGDILFEADAPYGTHDIRVMENGKLGFTRELYNYYFDYELPVGKT |
| SEQ ID NO 13: BbhI_D746E | GNELTFDVTLQQAAKPGDILFEADAPYGTHDIRVMENGKLGFTRELYNYYFDYELPVGKT |
| SEQ ID NO 14: BbhI_D746A | GNELTFDVTLQQAAKPGDILFEADAPYGTHDIRVMENGKLGFTRELYNYYFDYELPVGKT |
| SEQ ID NO 15: BbhI_D746Q | GNELTFDVTLQQAAKPGDILFEADAPYGTHDIRVMENGKLGFTRELYNYYFDYELPVGKT |
| SEQ ID NO 16: BbhI_Y827F | GNELTFDVTLQQAAKPGDILFEADAPYGTHDIRVMENGKLGFTRELYNYYFDYELPVGKT |
| SEQ ID NO 17: LnbB_WT | ------------EIKPGSVSLKS-IPAGKAAIA----------------YFN-------- |
| SEQ ID NO 18: LnbB_D320E | ------------EIKPGSVSLKS-IPAGKAAIA----------------YFN-------- |
| SEQ ID NO 19: LnbB_D320A | ------------EIKPGSVSLKS-IPAGKAAIA----------------YFN-------- |
| SEQ ID NO 20: LnbB_Y419F | ------------EIKPGSVSLKS-IPAGKAAIA----------------YFN-------- |
| SEQ ID NO 21: HEX1 (AKC34128.1) | ------------------------SPAAGRT----------------------------- |
| SEQ ID NO 22: HEX2 (AKC34129.1) | ------------------------TPLALKN----------------------------- |
| SEQ ID NO 23: Chb (AAB03808.1) | ------------------------------------------------------------ |
| SEQ ID NO 24: SpHex (AAC38798.3) | ------------------------SPASTHD----------------------------- |

## Fig. 16 continued

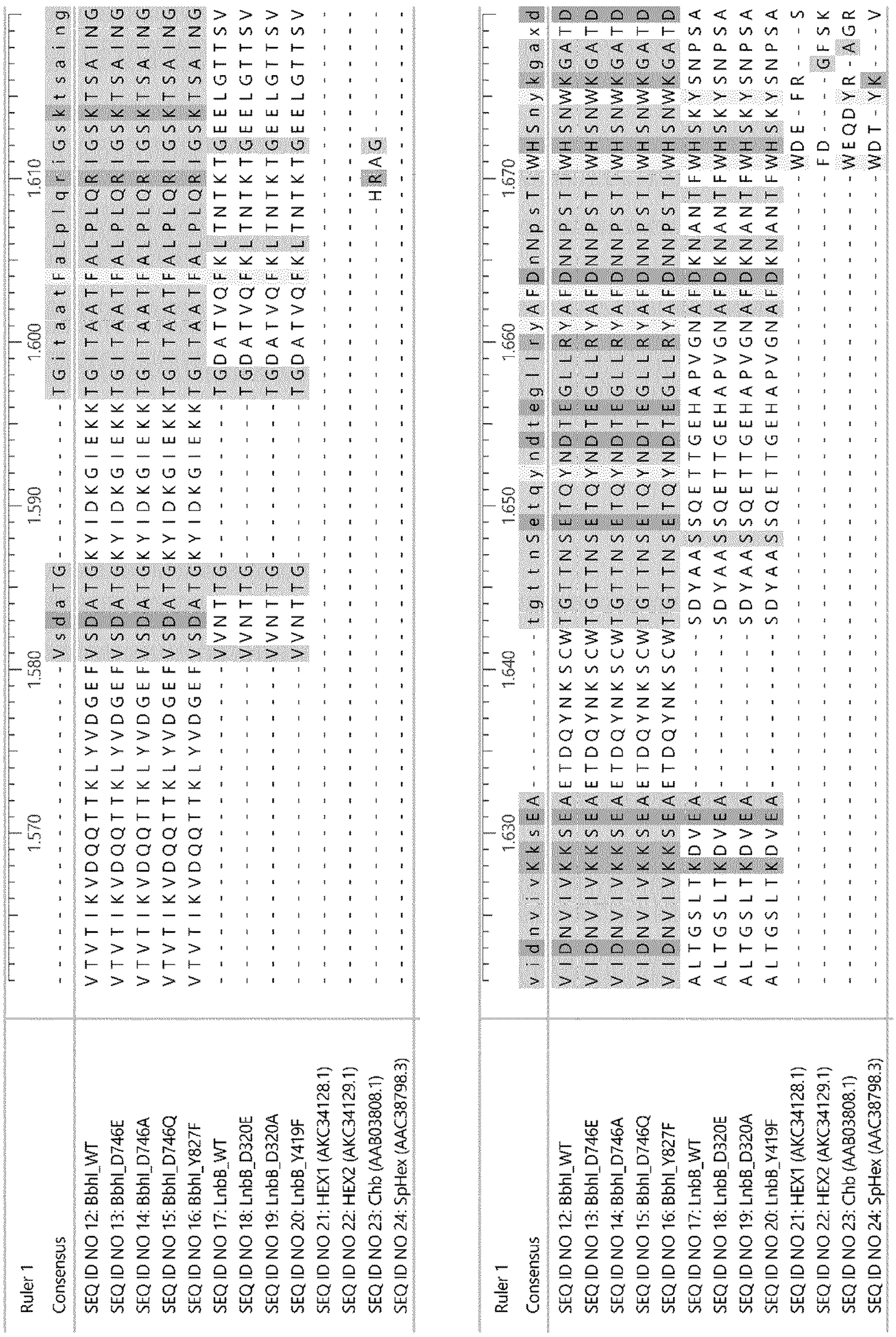

Fig. 16 continued

| Ruler 1 | 1.690 … 1.700 … 1.710 … 1.720 … 1.730 |
|---|---|
| Consensus | kLtGsnxHy--------ytin--------------------------------------- |
| SEQ ID NO 12: BbhI_WT | KLTGSNSFYAEIDMCQKYTIN--------------------------------------- |
| SEQ ID NO 13: BbhI_D746E | KLTGSNSFYAEIDMCQKYTIN--------------------------------------- |
| SEQ ID NO 14: BbhI_D746A | KLTGSNSFYAEIDMCQKYTIN--------------------------------------- |
| SEQ ID NO 15: BbhI_D746Q | KLTGSNSFYAEIDMCQKYTIN--------------------------------------- |
| SEQ ID NO 16: BbhI_Y827F | KLTGSNSFYAEIDMCQKYTIN--------------------------------------- |
| SEQ ID NO 17: LnbB_WT | NL----PHW--------LAFKASPGEGNKIAAITHLYRQDKLNGPAKNVAVYVVAASDAN |
| SEQ ID NO 18: LnbB_D320E | NL----PHW--------LAFKASPGEGNKIAAITHLYRQDKLNGPAKNVAVYVVAASDAN |
| SEQ ID NO 19: LnbB_D320A | NL----PHW--------LAFKASPGEGNKIAAITHLYRQDKLNGPAKNVAVYVVAASDAN |
| SEQ ID NO 20: LnbB_Y419F | NL----PHW--------LAFKASPGEGNKIAAITHLYRQDKLNGPAKNVAVYVVAASDAN |
| SEQ ID NO 21: HEX1 (AKC34128.1) | RL----AHH--------------------------------------------------- |
| SEQ ID NO 22: HEX2 (AKC34129.1) | RLEGIMQHY--------------------------------------------------- |
| SEQ ID NO 23: Chb (AAB03808.1) | EYKGGETHFVDTQALEK------------------------------------------- |
| SEQ ID NO 24: SpHex (AAC38798.3) | RLAAQAPYW--------------------------------------------------- |

| Ruler 1 | 1.750 … 1.760 … 1.770 … 1.780 … 1.790 |
|---|---|
| Consensus | --------------qFsfTp--rTsqdsgqvtkaDlYVKanaNDeWkqvaT-------- |
| SEQ ID NO 12: BbhI_WT | --------------QFSFTP--RTSQDSGQVTKADLYVKANANDEWKQVATDQVFEASR |
| SEQ ID NO 13: BbhI_D746E | --------------QFSFTP--RTSQDSGQVTKADLYVKANANDEWKQVATDQVFEASR |
| SEQ ID NO 14: BbhI_D746A | --------------QFSFTP--RTSQDSGQVTKADLYVKANANDEWKQVATDQVFEASR |
| SEQ ID NO 15: BbhI_D746Q | --------------QFSFTP--RTSQDSGQVTKADLYVKANANDEWKQVATDQVFEASR |
| SEQ ID NO 16: BbhI_Y827F | --------------QFSFTP--RTSQDSGQVTKADLYVKANANDEWKQVATDQVFEASR |
| SEQ ID NO 17: LnbB_WT | SVADVTNWGEPVATAEFPYTKELQTIALPNTIPSGDVYVKFQINDAWGLTET------- |
| SEQ ID NO 18: LnbB_D320E | SVADVTNWGEPVATAEFPYTKELQTIALPNTIPSGDVYVKFQINDAWGLTET------- |
| SEQ ID NO 19: LnbB_D320A | SVADVTNWGEPVATAEFPYTKELQTIALPNTIPSGDVYVKFQINDAWGLTET------- |
| SEQ ID NO 20: LnbB_Y419F | SVADVTNWGEPVATAEFPYTKELQTIALPNTIPSGDVYVKFQINDAWGLTET------- |
| SEQ ID NO 21: HEX1 (AKC34128.1) | ----------------------------------------------------------- |
| SEQ ID NO 22: HEX2 (AKC34129.1) | ----------------------------------------------------------- |
| SEQ ID NO 23: Chb (AAB03808.1) | ----------------------------------------------------------- |
| SEQ ID NO 24: SpHex (AAC38798.3) | ----------------------------------------------------------- |

Fig. 16 continued

| Ruler 1 | 1.810 · 1.820 · 1.830 · 1.840 · 1.850 |
|---|---|
| Consensus | --------------------SsndgWvAVx------ExxvanKpss-------------- |
| SEQ ID NO 12: BbhI_WT | AKKTFMFDEQEVRYVKFVAKSSNDGWVAVS------EFGVANKPSSTVRVFVAADPAEGG |
| SEQ ID NO 13: BbhI_D746E | AKKTFMFDEQEVRYVKFVAKSSNDGWVAVS------EFGVANKPSSTVRVFVAADPAEGG |
| SEQ ID NO 14: BbhI_D746A | AKKTFMFDEQEVRYVKFVAKSSNDGWVAVS------EFGVANKPSSTVRVFVAADPAEGG |
| SEQ ID NO 15: BbhI_D746Q | AKKTFMFDEQEVRYVKFVAKSSNDGWVAVS------EFGVANKPSSTVRVFVAADPAEGG |
| SEQ ID NO 16: BbhI_Y827F | AKKTFMFDEQEVRYVKFVAKSSNDGWVAVS------EFGVANKPSSTVRVFVAADPAEGG |
| SEQ ID NO 17: LnbB_WT | --------------------SAGVTWAAVA------ELAATAKATP-------------- |
| SEQ ID NO 18: LnbB_D320E | --------------------SAGVTWAAVA------ELAATAKATP-------------- |
| SEQ ID NO 19: LnbB_D320A | --------------------SAGVTWAAVA------ELAATAKATP-------------- |
| SEQ ID NO 20: LnbB_Y419F | --------------------SAGVTWAAVA------ELAATAKATP-------------- |
| SEQ ID NO 21: HEX1 (AKC34128.1) | ------------------------------------------------------------ |
| SEQ ID NO 22: HEX2 (AKC34129.1) | ------------------------------------------------------------ |
| SEQ ID NO 23: Chb (AAB03808.1) | ------------------------DWLRFANILGQRELAKLDKGGVAYRL---------- |
| SEQ ID NO 24: SpHex (AAC38798.3) | ------------------------------------------------------------ |

| Ruler 1 | 1.870 · 1.880 · 1.890 · 1.900 · 1.910 |
|---|---|
| Consensus | ------------------------------------------------------------ |
| SEQ ID NO 12: BbhI_WT | TVSVAAEGETGTDTAVDVASGASVTAKAVAADGYRFSGWFTTASETAVSTDATYTFAADG |
| SEQ ID NO 13: BbhI_D746E | TVSVAAEGETGTDTAVDVASGASVTAKAVAADGYRFSGWFTTASETAVSTDATYTFAADG |
| SEQ ID NO 14: BbhI_D746A | TVSVAAEGETGTDTAVDVASGASVTAKAVAADGYRFSGWFTTASETAVSTDATYTFAADG |
| SEQ ID NO 15: BbhI_D746Q | TVSVAAEGETGTDTAVDVASGASVTAKAVAADGYRFSGWFTTASETAVSTDATYTFAADG |
| SEQ ID NO 16: BbhI_Y827F | TVSVAAEGETGTDTAVDVASGASVTAKAVAADGYRFSGWFTTASETAVSTDATYTFAADG |
| SEQ ID NO 17: LnbB_WT | ------------------------------------------------------------ |
| SEQ ID NO 18: LnbB_D320E | ------------------------------------------------------------ |
| SEQ ID NO 19: LnbB_D320A | ------------------------------------------------------------ |
| SEQ ID NO 20: LnbB_Y419F | ------------------------------------------------------------ |
| SEQ ID NO 21: HEX1 (AKC34128.1) | ------------------------------------------------------------ |
| SEQ ID NO 22: HEX2 (AKC34129.1) | ------------------------------------------------------------ |
| SEQ ID NO 23: Chb (AAB03808.1) | ------------------------------------------------------------ |
| SEQ ID NO 24: SpHex (AAC38798.3) | ------------------------------------------------------------ |

**Fig. 16 continued**

| Ruler 1 | 1.930 · 1.940 · 1.950 · 1.960 · 1.970 |
|---|---|
| Consensus | -----akfTkdstPdpGPk------avtkPexT----GxTFxaxGIAvtA-tmsdGsTkt |
| SEQ ID NO 12: BbhI_WT | NTALTAKFTKDSTPDPGPKPTISSIAVTKPTVTDYKVGDTFDATGLAVTA-TMSDGSTKT |
| SEQ ID NO 13: BbhI_D746E | NTALTAKFTKDSTPDPGPKPTISSIAVTKPTVTDYKVGDTFDATGLAVTA-TMSDGSTKT |
| SEQ ID NO 14: BbhI_D746A | NTALTAKFTKDSTPDPGPKPTISSIAVTKPTVTDYKVGDTFDATGLAVTA-TMSDGSTKT |
| SEQ ID NO 15: BbhI_D746Q | NTALTAKFTKDSTPDPGPKPTISSIAVTKPTVTDYKVGDTFDATGLAVTA-TMSDGSTKT |
| SEQ ID NO 16: BbhI_Y827F | NTALTAKFTKDSTPDPGPKPTISSIAVTKPTVTDYKVGDTFDATGLAVTA-TMSDGSTKT |
| SEQ ID NO 17: LnbB_WT | -----VELTEPEQPKDNPE------VTETPEAT----GVTVSGDGVANGALSLKKGTTAQ |
| SEQ ID NO 18: LnbB_D320E | -----VELTEPEQPKDNPE------VTETPEAT----GVTVSGDGVANGALSLKKGTTAQ |
| SEQ ID NO 19: LnbB_D320A | -----VELTEPEQPKDNPE------VTETPEAT----GVTVSGDGVANGALSLKKGTTAQ |
| SEQ ID NO 20: LnbB_Y419F | -----VELTEPEQPKDNPE------VTETPEAT----GVTVSGDGVANGALSLKKGTTAQ |
| SEQ ID NO 21: HEX1 (AKC34128.1) | ----------------GPR----------LEGL----GVNF------------------- |
| SEQ ID NO 22: HEX2 (AKC34129.1) | ------------------------------------------------------------ |
| SEQ ID NO 23: Chb (AAB03808.1) | -------------PVPGARVAAGKL---EANIALPGLGIEYSTDG--------------- |
| SEQ ID NO 24: SpHex (AAC38798.3) | ------------------------------EAA----GIDF------------------- |

| Ruler 1 | 1.990 · 2.000 · 2.010 · 2.020 · 2.030 |
|---|---|
| Consensus | LTAge----ApqD----------------------------------------------- |
| SEQ ID NO 12: BbhI_WT | LTAGEYTLSATQDGAAVALDKAFAKAGKVTVTVTANGKTATFDVTVTAKDPDPEPATLKS |
| SEQ ID NO 13: BbhI_D746E | LTAGEYTLSATQDGAAVALDKAFAKAGKVTVTVTANGKTATFDVTVTAKDPDPEPATLKS |
| SEQ ID NO 14: BbhI_D746A | LTAGEYTLSATQDGAAVALDKAFAKAGKVTVTVTANGKTATFDVTVTAKDPDPEPATLKS |
| SEQ ID NO 15: BbhI_D746Q | LTAGEYTLSATQDGAAVALDKAFAKAGKVTVTVTANGKTATFDVTVTAKDPDPEPATLKS |
| SEQ ID NO 16: BbhI_Y827F | LTAGEYTLSATQDGAAVALDKAFAKAGKVTVTVTANGKTATFDVTVTAKDPDPEPATLKS |
| SEQ ID NO 17: LnbB_WT | LTAKV----APDD----------------------------------------------- |
| SEQ ID NO 18: LnbB_D320E | LTAKV----APDD----------------------------------------------- |
| SEQ ID NO 19: LnbB_D320A | LTAKV----APDD----------------------------------------------- |
| SEQ ID NO 20: LnbB_Y419F | LTAKV----APDD----------------------------------------------- |
| SEQ ID NO 21: HEX1 (AKC34128.1) | -------FRAPEI----------------------------------------------- |
| SEQ ID NO 22: HEX2 (AKC34129.1) | ------------------------------------------------------------ |
| SEQ ID NO 23: Chb (AAB03808.1) | ------------------------------------------------------------ |
| SEQ ID NO 24: SpHex (AAC38798.3) | -------YRSPQV----------------------------------------------- |

Fig. 16 continued

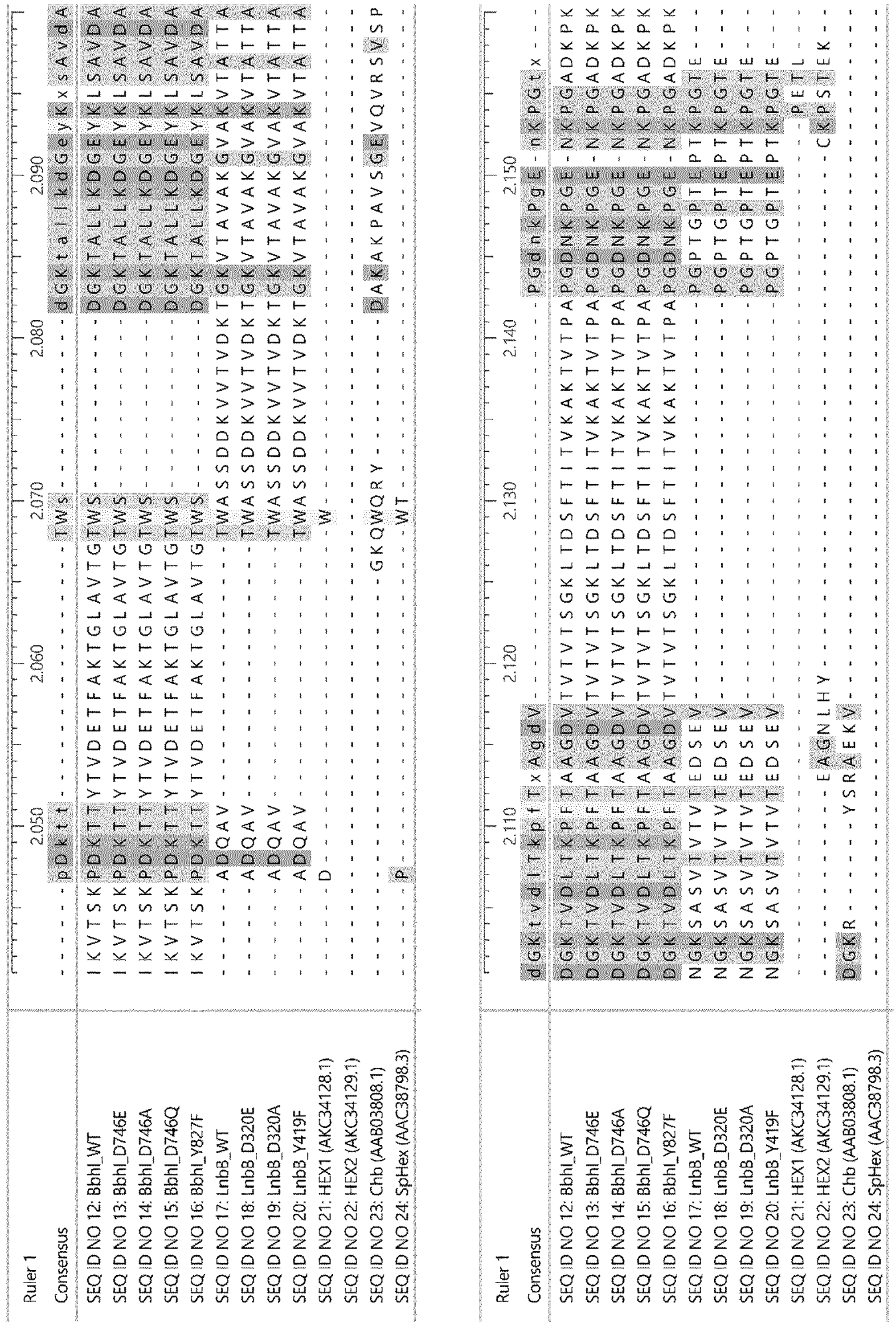

Fig. 16 continued

| | |
|---|---|
| Ruler 1 | 2.170 |
| Consensus | - - - - - - - - - L E H H H H H H |
| SEQ ID NO 12: BbhI_WT | P N T P D E V A K L E H H H H H H |
| SEQ ID NO 13: BbhI_D746E | P N T P D E V A K L E H H H H H H |
| SEQ ID NO 14: BbhI_D746A | P N T P D E V A K L E H H H H H H |
| SEQ ID NO 15: BbhI_D746Q | P N T P D E V A K L E H H H H H H |
| SEQ ID NO 16: BbhI_Y827F | P N T P D E V A K L E H H H H H H |
| SEQ ID NO 17: LnbB_WT | - - - - - - - - - L E H H H H H H |
| SEQ ID NO 18: LnbB_D320E | - - - - - - - - - L E H H H H H H |
| SEQ ID NO 19: LnbB_D320A | - - - - - - - - - L E H H H H H H |
| SEQ ID NO 20: LnbB_Y419F | - - - - - - - - - L E H H H H H H |
| SEQ ID NO 21: HEX1 (AKC34128.1) | - - - - - - - - - - - - - - - - - |
| SEQ ID NO 22: HEX2 (AKC34129.1) | - - - - - - - - - - - - - - - - - |
| SEQ ID NO 23: Chb (AAB03808.1) | - - - - - - - - - - - - - - - - - |
| SEQ ID NO 24: SpHex (AAC38798.3) | - - - - - - - - - - - - - - - - - |

Europäisches Patentamt
European Patent Office
Office européen des brevets

# EUROPEAN SEARCH REPORT

Application Number
EP 18 21 4455

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAODI CHEN ET AL: "ABSTRACT", APPLIED AND ENVIRONMENTAL MICROBIOLOGY, vol. 82, no. 18, 15 September 2016 (2016-09-15), pages 5642-5652, XP055595935, US ISSN: 0099-2240, DOI: 10.1128/AEM.01325-16 * figures 2-4; table 2 * ----- | 9 | INV. C12P19/14 C12P19/18 C12P19/26 C12N9/24 |
| X | WADA J ET AL: "Bifidobacterium bifidum lacto-N-biosidase, a critical enzyme for the degradation of human milk oligosaccharides with a type 1 structure", APPLIED AND ENVIRONMENTAL MICROBIOLOGY, AMERICAN SOCIETY FOR MICROBIOLOGY, US, vol. 74, no. 13, 1 July 2008 (2008-07-01), pages 3996-4004, XP002640863, ISSN: 0099-2240, DOI: 10.1128/AEM.00149-08 * page 3998, column 1, paragraph 5; figure 5 * ----- | 9 | |
| A | EP 2 722 394 A1 (HERO AG [CH]) 23 April 2014 (2014-04-23) * figure 1 * ----- | 1-13 | TECHNICAL FIELDS SEARCHED (IPC) C12P C12N |
| A | MURATA T ET AL: "FACILE ENZYMATIC CONVERSION OF LACTOSE INTO LACTO-N-TETRAOSE AND LACTO-N-NEOTETRAOSE", GLYCOCONJUGATE JOU, CHAPMAN & HALL, BOSTON, vol. 16, no. 3, 1 March 1999 (1999-03-01), pages 189-195, XP008029474, ISSN: 0282-0080, DOI: 10.1023/A:1007020219275 * page 190, column 2, paragraph 2 - page 191, column 2, paragraph 1 * ----- -/-- | 1-13 | |

The present search report has been drawn up for all claims

3

| Place of search | Date of completion of the search | Examiner |
|---|---|---|
| Berlin | 14 June 2019 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 21 4455

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| T | DATABASE MEDLINE [Online] US NATIONAL LIBRARY OF MEDICINE (NLM), BETHESDA, MD, US; 16 May 2019 (2019-05-16), SCHMÖLZER KATHARINA ET AL: "Lacto-N-tetraose synthesis by wild-type and glycosynthase variants of the [beta]-N-hexosaminidase from Bifidobacterium bifidum.", XP055595096, Database accession no. NLM31094393 * abstract * & ORGANIC & BIOMOLECULAR CHEMISTRY 16 MAY 2019, 16 May 2019 (2019-05-16), ISSN: 1477-0539 ----- | 1-13 | |
| T | DATABASE MEDLINE [Online] US NATIONAL LIBRARY OF MEDICINE (NLM), BETHESDA, MD, US; 4 June 2019 (2019-06-04), SCHMÖLZER KATHARINA ET AL: "Correction: Lacto-N-tetraose synthesis by wild-type and glycosynthase variants of the [beta]-N-hexosaminidase from Bifidobacterium bifidum.", XP055595888, Database accession no. NLM31161187 * abstract * & ORGANIC & BIOMOLECULAR CHEMISTRY 04 JUN 2019, 4 June 2019 (2019-06-04), ISSN: 1477-0539 ----- | 1-13 | TECHNICAL FIELDS SEARCHED (IPC) |

The present search report has been drawn up for all claims

3

| Place of search | Date of completion of the search | Examiner |
|---|---|---|
| Berlin | 14 June 2019 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 21 4455

This annex lists the patent family members relating to the patent documents cited in the above-mentioned European search report. The members are as contained in the European Patent Office EDP file on
The European Patent Office is in no way liable for these particulars which are merely given for the purpose of information.

14-06-2019

| Patent document cited in search report | Publication date | Patent family member(s) | Publication date |
|---|---|---|---|
| EP 2722394 A1 | 23-04-2014 | CN 103764835 A | 30-04-2014 |
| | | DK 2722394 T3 | 18-06-2018 |
| | | EP 2722394 A1 | 23-04-2014 |
| | | ES 2671555 T3 | 07-06-2018 |
| | | PL 2722394 T3 | 28-09-2018 |
| | | WO 2012168495 A1 | 13-12-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7026149 B
- US 8173399 B2
- US 5945314 A

### Non-patent literature cited in the description


- **LOMBARD, V. ; GOLACONDA RAMULU, H. ; DRULA, E. ; COUTINHO, P. M. ; HENRISSAT, B.** The carbohydrate-active enzymes database (CAZy). *Nucleic Acids Res.,* 2013, vol. 42 (D1), D490-D495
- *Nucleic Acids Res.,* July 2012
- **SCHOMBURG et al.** The BRENDA enzyme information system - From a database to an expert system. *J Biotechnol,* 2017, vol. 261, 194-206
- **ARTIMO et al.** ExPASy: SIB bioinformatics resource portal. *Nucleic Acids Res.,* 2012, vol. 40 (W1), W597-W603
- *Pfam 32.0,* September 2018, https://pfam.xfam.org
- **CAMPANELLA, J.J ; BITINCKA, L. ; SMALLEY, J.** *BMC Bioinformatics,* 2003, vol. 4, 29
- **HIGGINS, D:G ; SHARP, P.M.** *Gene,* 1988, vol. 73, 237-244
- **MEINKOTH ; WAHL.** *Anal. Biochem.,* 1984, vol. 138, 267-284
- **BONNER et al.** *J. Mol. Biol.,* 1973, vol. 81, 123-135
- Recombinant DNA Principles and Methodologies. **JAMES GREENE ; PAUL S. MILLER.** Biochemistry of Nucleic acids. CRC Press, 1998, 55
- **CHEN, X. et al.** Efficient and regioselective synthesis of β-GalNAc/GlcNAc-lactose by a bifunctional transglycosylating β-N-acetylhexosaminidase from Bifidobacterium bifidum. *Appl. Environ. Microbiol.,* 2016, vol. 82, 5642-5652
- **MIWA, M. et al.** Cooperation of β-galactosidase and β-N-acetylhexosaminidase from bifidobacteria in assimilation of human milk oligosaccharides with type 2 structure. *Glycobiology,* 2010, vol. 20, 1402-1409
- **WADA, J. et al.** Bifidobacterium bifidum lacto-N-biosidase, a critical enzyme for the degradation of human milk oligosaccharides with a type 1 structure. *Appl. Environ. Microbiol.,* 2008, vol. 74, 3996-4004
- **FUJITA, M. ; SHODA, S.-I. ; HANEDA, K. ; INAZU, T. ; TAKEGAWA, K. ; YAMAMOTO, K.** A novel disaccharide substrate having 1,2-oxazoline moiety for detection of transglycosylating activity of endoglycosidases. *Biochim. Biophys. Acta Gen. Subj.,* 2001, vol. 1528, 9-14
- **KOBAYASHI, A. ; KUWATA, H. ; KOHRI, M. ; IZUMI, R. ; WATANABE, T. ; SHODA, S. I.** A bacterial chitinase acts as catalyst for synthesis of the N-linked oligosaccharide core trisaccharide by employing a sugar oxazoline substrate. *J. Carbohyd. Chem.,* 2006, vol. 25, 533-541
- **WANG, L.-X. ; HUANG, W.** Enzymatic transglycosylation for glycoconjugate synthesis. *Curr. Opin. Chem. Biol.,* 2009, vol. 13, 592-600
- **OCHIAI, H. ; HUANG, W. ; WANG, L.-X.** Endo-β-N-acetyigtucosaminidase-catatyzed polymerization of β-Glcp-(1→4)-GlcpNAc oxazoline: a revisit to enzymatic transglycosylation. *Carbohydr. Res.,* 2009, vol. 344, 592-598
- **JAMEK, S. B. et al.** Loop protein engineering for improved transglycosylation activity of a β-N-acetylhexosaminidase. *ChemBioChem,* 2018, vol. 19, 1858-1865
- **NOGUCHI, M. ; FUJIEDA, T. ; HUANG, W. C. ; ISHIHARA, M. ; KOBAYASHI, A. ; SHODA, S.-I.** A practical one-step synthesis of 1,2-oxazoline derivatives from unprotected sugars and its application to chemoenzymatic β-N-acetylglucosaminidation of disialo-oligosaccharide. *Helv. Chim. Acta,* 2012, vol. 95, 1928-1936
- **NISHIMOTO, M. ; KITAOKA, M.** Practical preparation of lacto-N-biose I, a candidate for the bifidus factor in human milk. *Biosci. Biotechnol. Biochem.,* 2007, vol. 71, 2101-2104
- **SLÁMOVÁ, K. et al.** Synthesis of derivatized chitooligomers using transglycosidases engineered from the fungal GH20 β-N-acetylhexosaminidase. *Adv. Synth. Catal.,* 2015, vol. 357, 1941-1950

- **MIWA, M. et al.** Cooperation of β-galactosidase and β-N-acetythexosaminidase from bifidobacteria in assimilation of human milk oligosaccharides with type 2 structure. *Glycobiology,* 2010, vol. 20, 1402-1409
- **ANDRE-MIRAL, C. et al.** De novo design of a trans-β-N-acetylglucosaminidase activity from a GH1 β-glycosidase by mechanism engineering. *Glycobiology,* 2015, vol. 25, 394-402
- **MARTINEZ, E. A. et al.** Engineering chitinases for the synthesis of chitin oligosaccharides: catalytic amino acid mutations convert the GH-18 family glycoside hydrolases into transglycosylases. *J. Mol. Catal. B: Enzym.,* 2012, vol. 74, 89-96
- **SHODA, S.-I. et al.** Efficient method for the elongation of the N-acetylglucosamine unit by combined use of chitinase and β-galactosidase. *Helv. Chim. Acta,* 2002, vol. 85, 3919-3936
- **MURATA, T. ; INUKAI, T. ; SUZUKI, M. ; YAMAGISHI, M. ; USUI, T.** Facile enzymatic conversion of lactose into lacto-N-tetraose and lacto-N-neotetraose. *Glycoconj. J.,* 1999, vol. 16, 189-195
- **BAUMGÄRTNER, F. ; SPRENGER, G. A. ; ALBERMANN, C.** Galactose-limited fed-batch cultivation of Escherichia coli for the production of lacto-N-tetraose. *Enzyme Microb. Technol.,* 2015, vol. 75-76, 37-43
- **BLIXT, O. ; VAN DIE, I. ; NORBERG, T. ; VAN DEN EIJNDEN, D. H.** High-level expression of the Neisseria meningitidis IgtA gene in Escherichia coli and characterization of the encoded N-acetylglucosaminyltransferase as a useful catalyst in the synthesis of Glc-NAcβ1→3Gal and GalNAcβ1→3Gal linkages. *Glycobiology,* 1999, vol. 9, 1061-1071
- **CHEN, C. et al.** Sequential one-pot multienzyme (OPME) synthesis of lacto-N-neotetraose and its sialyl and fucosyl derivatives. *Chem. Commun.,* 2015, vol. 51, 7689-7692
- **JOHNSON, K. F.** Synthesis of oligosaccharides by bacterial enzymes. *Glycoconj. J.,* 1999, vol. 16, 141-146
- **YU, H. et al.** Synthetic disialyl hexasaccharides protect neonatal rats from necrotizing enterocolitis. *Angew. Chem. Int. Edit.,* 2014, vol. 53, 6687-6691
- **PRIEM, B. ; GILBERT, M. ; WAKARCHUK, W. W. ; HEYRAUD, A. ; SAMAIN, E.** A new fermentation process allows large-scale production of human milk oligosaccharides by metabolically engineered bacteria. *Glycobiology,* 2002, vol. 12, 235-240
- **BAUMGÄRTNER, F. ; CONRAD, J. ; SPRENGER, G. A. ; ALBERMANN, C.** Synthesis of the human milk oligosaccharide lacto-N-tetraose in metabolically engineered, plasmid-free E. coli. *ChemBioChem,* 2014, vol. 15, 1896-1900
- **KOBAYASHI, S. ; KIYOSADA, T. ; SHODA, S.-I.** A novel method for synthesis of chitobiose via enzymatic glycosylation using a sugar oxazoline as glycosyl donor. *Tetrahedron Lett.,* 1997, vol. 38, 2111-2112
- **OCHIAI, H. ; OHMAE, M. ; KOBAYASHI, S.** Enzymatic glycosidation of sugar oxazolines having a carboxylate group catalyzed by chitinase. *Carbohydr. Res.,* 2004, vol. 339, 2769-2788
- **KOHRI, M. ; KOBAYASHI, A. ; SHODA, S.-I.** Design and utilization of chitinases with low hydrolytic activities. *Trends Glycosci. Glycotechnol.,* 2007, vol. 19, 165-180
- **HATTIE, M. et al.** Gaining insight into the catalysis by GH20 lacto-N-biosidase using small molecule inhibitors and structural analysis. *Chem. Commun.,* 2015, vol. 51, 15008-15011
- **ITO, T. et al.** Crystal structures of a glycoside hydrolase family 20 lacto-N-biosidase from Bifidobacterium bifidum. *J. Biol. Chem.,* 2013, vol. 288, 11795-11806
- **LIU, X.-W. et al.** Characterization and synthetic application of a novel β1,3-galactosyltransferase from Escherichia coli O55:H7. *Bioorg. Med. Chem.,* 2009, vol. 17, 4910-4915
- **ZEUNER, B. ; NYFFENEGGER, C. ; MIKKELSEN, J. D. ; MEYER, A. S.** Thermostable β-galactosidases for the synthesis of human milk oligosaccharides. *New Biotechnol,* 2016, vol. 33, 355-360
- **BODE, L.** Human milk oligosaccharides: Every baby needs a sugar mama. *Glycobiology,* 2012, vol. 22, 1147-1162
- **STUDIER, F. W.** Protein production by auto-induction in high-density shaking cultures. *Protein Expr. Purif.,* 2005, vol. 41, 207-234
- **VAL-CID, C. ; BIARNÉS, X. ; FAIJES, M. ; PLANAS, A.** Structural-functional analysis reveals a specific domain organization in family GH20 hexosaminidases. *PLOS ONE,* 2015, vol. 10, e0128075
- **GOULAS, A. K. ; KAPASAKALIDIS, P. G. ; SINCLAIR, H. R. ; RASTALL, R. A. ; GRANDISON, A. S.** Purification of oligosaccharides by nanofiltration. *J. Membrane Sci.,* 2002, vol. 209, 321-335
- **NORDVANG, R. T. et al.** Separation of 3'-sialyllactose and lactose by nanofiltration: a trade-off between charge repulsion and pore swelling induced by high pH. *Sep. Purif. Technol.,* 2014, vol. 138, 77-83

SEQUENCE LISTING

<110> BASF SE

<120> Enzymatic hexosaminidation of lactose

<130> 181557

<160> 27

<170> PatentIn version 3.5

<210> 1
<211> 1627
<212> PRT
<213> Bifidobacterium bifidum

<400> 1

```
Met Arg Leu Arg Arg Val Lys Ala Ala Ile Gly Ser Val Leu Ala Ala
1               5                   10                  15

Val Thr Leu Leu Ser Met Ser Leu Thr Gly Val Thr Ala Ala Gln Ala
            20                  25                  30

Ser Asp Asp Asn Leu Ala Leu Asn Gln Thr Val Thr Ala Ser Ser Tyr
        35                  40                  45

Glu Val Ala Thr Thr Ala Pro Glu Lys Ala Val Asp Gly Asp Leu Gly
    50                  55                  60

Thr Arg Trp Gly Thr Ala Gln Asn Lys Ala Ala Asn Glu Trp Ile Glu
65                  70                  75                  80

Val Gly Leu Gly Gly Thr Lys Thr Val Lys Gln Ile Asn Ile Asp Phe
                85                  90                  95

Glu Arg Lys Asp Ala Asp Gln Asn Ile Thr Ser Phe Lys Val Glu Leu
            100                 105                 110

Lys Gln Gly Asp Thr Tyr Thr Lys Val Tyr Gln Lys Asp Thr Arg Ala
        115                 120                 125

Lys Gln Gln Glu Ile Ile Leu Leu Asp Gln Ala Gln Gln Ala Ser Ala
    130                 135                 140

Val Lys Val Thr Val Leu Ser Ala Asp Gly Gly Thr Met Asn Trp Val
145                 150                 155                 160

Asn Val Gly Ile Asn Glu Ile Ser Val Tyr Ser Ala Pro Lys Glu Thr
                165                 170                 175
```

```
Val Leu Asp Thr Ala Asp Thr Asn His Met Leu Gly Ala Thr Met Thr
            180                 185                 190

Ala Ser Ser Asn Glu Thr Ala Thr Leu Thr Pro Asp Lys Ala Ile Asp
        195                 200                 205

Gln Asn Arg Thr Gly Arg Asn Asn Arg Trp Ala Ser Gly Tyr Glu Thr
    210                 215                 220

Pro Ser Asn Ile Trp Leu Lys Ala Glu Phe Pro Arg Leu Thr Ala Val
225                 230                 235                 240

Lys Asp Ile Arg Ile Tyr Phe Phe Glu Arg Asp Val Asn Pro Lys Pro
                245                 250                 255

Thr Asn Val Gln Ser Phe Asp Leu Ser Tyr Thr Asp Ser Glu Gly Thr
            260                 265                 270

Glu His Thr Leu Lys Ser Gly Tyr Ala Met Thr Ala Ser Gly Ala Gly
        275                 280                 285

Tyr Val Ala Asp Val Val Ile Gln Leu Asp Gln Ala Val Asn Ala Arg
    290                 295                 300

Ser Leu Lys Leu Ser Asn Phe Ala Ile Lys Ser Ser Glu Tyr Asn Asn
305                 310                 315                 320

Val Ser Val Ala Glu Trp Glu Ala Tyr Ser Asn Asp Gln Ala Glu Pro
                325                 330                 335

Gly Ala Thr Leu Asp Ser Val Val Ser Asp Leu Glu Ser Asn His Leu
            340                 345                 350

Thr Ile Glu Thr Asp Thr Asp Thr Leu Ala Leu Pro Thr Val Pro Asp
        355                 360                 365

Gly Tyr Thr Val Lys Phe Asn Gly Ala Asp Tyr Glu Gln Leu Ile Ala
    370                 375                 380

Ala Asp Gly Thr Val Asn His Pro Leu Val Asp Lys Thr Val Gln Val
385                 390                 395                 400

Ala Tyr Val Val Thr Asp Thr Ala Thr Gly Asn Thr Lys Thr Thr Ser
                405                 410                 415

Asp Ile Pro Tyr Val Val Lys Gly Thr Asn Gln Gln Gln Glu Gly Asn
            420                 425                 430
```

```
Asn Ala Lys Pro Thr Ile Ile Pro Glu Ile Ala Glu Trp His Ser Thr
        435                 440                 445

Ser Ala Ala Lys Leu Ala Ala Ser Ala Val Thr Lys Val Val Tyr Asp
    450                 455                 460

Asp Asp Ser Leu Lys Ala Val Val Asp Glu Phe Val Ala Asp Tyr Lys
465                 470                 475                 480

Asp Phe Thr Gly Ile Lys Leu Thr Ala Lys Lys Gly Ala Ala Glu Ala
                485                 490                 495

Gly Ala Phe Asn Phe Val Lys Thr Asp Ser Thr Ala Ala Ile Ala Gln
            500                 505                 510

Leu Gly Asp Glu Gly Tyr Thr Met Asp Ile Arg Ala Asp Arg Val Val
        515                 520                 525

Ala Lys Ser Ser Ser Val Thr Gly Asn Met Tyr Ala Met Gln Thr Ile
    530                 535                 540

Leu Gln Met Thr Lys Gln Asp Ala Asn Gly Phe Val Ile Gly Ser Met
545                 550                 555                 560

Arg Asp Tyr Pro Arg Phe Thr Thr Arg Gly Leu Leu Leu Asp Val Ala
                565                 570                 575

Arg Lys Pro Val Ser Leu Glu Met Met Arg Glu Ile Thr Arg Thr Met
            580                 585                 590

Arg Tyr Tyr Lys Met Asn Asp Phe Gln Ala His Leu Ser Asp Asn Tyr
        595                 600                 605

Ile Phe Leu Glu Asn Tyr Gly Lys Gly Asp Asn Glu Asp Glu Ala Phe
    610                 615                 620

Lys Ala Tyr Asp Ala Phe Arg Leu Glu Ser Ser Leu Thr Asn Asp Lys
625                 630                 635                 640

Gly Glu Ser Pro Thr Ala Glu Asp Tyr Ser Ile Ser Lys Lys Thr Phe
                645                 650                 655

Lys Gln Phe Ile Gln Asp Glu Arg Ala Leu Gly Met Asn Val Val Pro
            660                 665                 670

Glu Ile Asp Val Pro Ala His Ala Asn Ser Phe Thr Lys Ile Trp Pro
        675                 680                 685
```

Glu Leu Met Val Lys Gly Arg Val Ser Pro Ile Asn Ser Asn Arg Pro
    690                 695                 700

Leu Ile Asp His Leu Asp Val Ser Lys Pro Glu Thr Ile Ala Lys Ile
705                 710                 715                 720

Lys Glu Ile Phe Asp Asp Tyr Thr Lys Gly Asp Asp Pro Thr Phe Asp
                725                 730                 735

Ser Asp Thr Thr Val His Ile Gly Ala Asp Glu Phe Leu Tyr Asn Tyr
            740                 745                 750

Thr Ala Tyr Arg Lys Phe Ile Asn Glu Ile Val Pro Tyr Ile Lys Asp
        755                 760                 765

Thr Asn Thr Val Arg Met Trp Gly Gly Leu Thr Trp Ile Asn Asp His
    770                 775                 780

Lys Thr Glu Ile Thr Lys Asp Ala Ile Glu Asn Val Glu Met Asn Leu
785                 790                 795                 800

Trp Ser Lys Asp Trp Ala Asp Gly Leu Gln Met Tyr Asn Met Gly Tyr
                805                 810                 815

Lys Leu Ile Asn Thr Ile Asp Asp Tyr Gly Tyr Met Val Pro Asn Gly
            820                 825                 830

Ser Tyr Gly Arg Ala Asn Ala Tyr Gly Asp Leu Leu Asn Ile Ser Arg
        835                 840                 845

Val Phe Asp Ser Phe Glu Pro Asn Lys Val Arg Ser Ser Gly Gly Tyr
    850                 855                 860

Gln Ala Val Pro Ser Gly Asp Asp Gln Met Leu Gly Ala Ala Phe Ala
865                 870                 875                 880

Ile Trp Ser Asp Asn Ile Asp Lys Ser Ala Ser Gly Leu Thr Glu Ser
                885                 890                 895

Asp Leu Tyr Trp Arg Phe Phe Asp Ala Met Pro Phe Tyr Ala Glu Lys
            900                 905                 910

Thr Trp Ala Ala Thr Gly Lys Glu Lys Gly Thr Ala Ala Lys Leu Thr
        915                 920                 925

Ala Leu Ala Ala Lys Gln Gly Thr Gly Pro Arg Thr Asn Pro Tyr Tyr

```
    930                 935                 940

Gln Ala Thr Ser Lys Asn Ser Val Tyr Glu Ser Tyr Asp Phe Asn Asp
945                 950                 955                 960

Gly Leu Ala Asp Ala Ser Gly Asn Gly Arg Asp Leu Thr Ile Gly Asp
                965                 970                 975

Gly Ser Lys Ala Ala Val Lys Asp Gln Ser Leu Lys Leu Ala Gly Gly
            980                 985                 990

Ser Ser Tyr Ala Thr Ser Lys Leu  Asp Lys Leu Gly Asn  Gly Asn Glu
        995                 1000                 1005

Leu Thr  Phe Asp Val Thr Leu  Gln Gln Ala Ala Lys  Pro Gly Asp
    1010                 1015                 1020

Ile Leu  Phe Glu Ala Asp Ala  Pro Tyr Gly Thr His  Asp Ile Arg
    1025                 1030                 1035

Val Met  Glu Asn Gly Lys Leu  Gly Phe Thr Arg Glu  Leu Tyr Asn
    1040                 1045                 1050

Tyr Tyr  Phe Asp Tyr Glu Leu  Pro Val Gly Lys Thr  Val Thr Val
    1055                 1060                 1065

Thr Ile  Lys Val Asp Gln Gln  Thr Thr Lys Leu Tyr  Val Asp Gly
    1070                 1075                 1080

Glu Phe  Val Ser Asp Ala Thr  Gly Lys Tyr Ile Asp  Lys Gly Ile
    1085                 1090                 1095

Glu Lys  Lys Thr Gly Ile Thr  Ala Ala Thr Phe Ala  Leu Pro Leu
    1100                 1105                 1110

Gln Arg  Ile Gly Ser Lys Thr  Ser Ala Ile Asn Gly  Val Ile Asp
    1115                 1120                 1125

Asn Val  Ile Val Lys Lys Ser  Glu Ala Glu Thr Asp  Gln Tyr Asn
    1130                 1135                 1140

Lys Ser  Cys Trp Thr Gly Thr  Thr Asn Ser Glu Thr  Gln Tyr Asn
    1145                 1150                 1155

Asp Thr  Glu Gly Leu Leu Arg  Tyr Ala Phe Asp Asn  Asn Pro Ser
    1160                 1165                 1170
```

Thr Ile Trp His Ser Asn Trp Lys Gly Ala Thr Asp Lys Leu Thr
1175 1180 1185

Gly Ser Asn Ser Phe Tyr Ala Glu Ile Asp Met Cys Gln Lys Tyr
1190 1195 1200

Thr Ile Asn Gln Phe Ser Phe Thr Pro Arg Thr Ser Gln Asp Ser
1205 1210 1215

Gly Gln Val Thr Lys Ala Asp Leu Tyr Val Lys Ala Asn Ala Asn
1220 1225 1230

Asp Glu Trp Lys Gln Val Ala Thr Asp Gln Val Phe Glu Ala Ser
1235 1240 1245

Arg Ala Lys Lys Thr Phe Met Phe Asp Glu Gln Glu Val Arg Tyr
1250 1255 1260

Val Lys Phe Val Ala Lys Ser Ser Asn Asp Gly Trp Val Ala Val
1265 1270 1275

Ser Glu Phe Gly Val Ala Asn Lys Pro Ser Ser Thr Val Arg Val
1280 1285 1290

Phe Val Ala Ala Asp Pro Ala Glu Gly Gly Thr Val Ser Val Ala
1295 1300 1305

Ala Glu Gly Glu Thr Gly Thr Asp Thr Ala Val Asp Val Ala Ser
1310 1315 1320

Gly Ala Ser Val Thr Ala Lys Ala Val Ala Ala Asp Gly Tyr Arg
1325 1330 1335

Phe Ser Gly Trp Phe Thr Thr Ala Ser Glu Thr Ala Val Ser Thr
1340 1345 1350

Asp Ala Thr Tyr Thr Phe Ala Ala Asp Gly Asn Thr Ala Leu Thr
1355 1360 1365

Ala Lys Phe Thr Lys Asp Ser Thr Pro Asp Pro Gly Pro Lys Pro
1370 1375 1380

Thr Ile Ser Ser Ile Ala Val Thr Lys Pro Thr Val Thr Asp Tyr
1385 1390 1395

Lys Val Gly Asp Thr Phe Asp Ala Thr Gly Leu Ala Val Thr Ala
1400 1405 1410

```
Thr Met  Ser Asp Gly Ser Thr  Lys Thr Leu Thr Ala  Gly Glu Tyr
    1415                 1420                 1425

Thr Leu  Ser Ala Thr Gln Asp  Gly Ala Ala Val Ala  Leu Asp Lys
    1430                 1435                 1440

Ala Phe  Ala Lys Ala Gly Lys  Val Thr Val Thr Val  Thr Ala Asn
    1445                 1450                 1455

Gly Lys  Thr Ala Thr Phe Asp  Val Thr Val Thr Ala  Lys Asp Pro
    1460                 1465                 1470

Asp Pro  Glu Pro Ala Thr Leu  Lys Ser Ile Lys Val  Thr Ser Lys
    1475                 1480                 1485

Pro Asp  Lys Thr Thr Tyr Thr  Val Asp Glu Thr Phe  Ala Lys Thr
    1490                 1495                 1500

Gly Leu  Ala Val Thr Gly Thr  Trp Ser Asp Gly Lys  Thr Ala Leu
    1505                 1510                 1515

Leu Lys  Asp Gly Glu Tyr Lys  Leu Ser Ala Val Asp  Ala Asp Gly
    1520                 1525                 1530

Lys Thr  Val Asp Leu Thr Lys  Pro Phe Thr Ala Ala  Gly Asp Val
    1535                 1540                 1545

Thr Val  Thr Val Thr Ser Gly  Lys Leu Thr Asp Ser  Phe Thr Ile
    1550                 1555                 1560

Thr Val  Lys Ala Lys Thr Val  Thr Pro Ala Pro Gly  Asp Asn Lys
    1565                 1570                 1575

Pro Gly  Glu Asn Lys Pro Gly  Ala Asp Lys Pro Lys  Pro Asn Thr
    1580                 1585                 1590

Pro Asp  Glu Val Ala Lys Thr  Gly Ala Ser Val Thr  Ala Val Val
    1595                 1600                 1605

Phe Ser  Ala Leu Leu Leu Leu  Ser Ala Gly Tyr Leu  Leu Val Arg
    1610                 1615                 1620

Lys Arg  Arg Ile
    1625

<210>  2
<211>  1567
```

<212> PRT
<213> Bifidobacterium bifidum

<400> 2

```
Ser Asp Asp Asn Leu Ala Leu Asn Gln Thr Val Thr Ala Ser Ser Tyr
1               5                   10                  15

Glu Val Ala Thr Thr Ala Pro Glu Lys Ala Val Asp Gly Asp Leu Gly
            20                  25                  30

Thr Arg Trp Gly Thr Ala Gln Asn Lys Ala Ala Asn Glu Trp Ile Glu
        35                  40                  45

Val Gly Leu Gly Gly Thr Lys Thr Val Lys Gln Ile Asn Ile Asp Phe
    50                  55                  60

Glu Arg Lys Asp Ala Asp Gln Asn Ile Thr Ser Phe Lys Val Glu Leu
65                  70                  75                  80

Lys Gln Gly Asp Thr Tyr Thr Lys Val Tyr Gln Lys Asp Thr Arg Ala
                85                  90                  95

Lys Gln Gln Glu Ile Ile Leu Leu Asp Gln Ala Gln Gln Ala Ser Ala
            100                 105                 110

Val Lys Val Thr Val Leu Ser Ala Asp Gly Gly Thr Met Asn Trp Val
        115                 120                 125

Asn Val Gly Ile Asn Glu Ile Ser Val Tyr Ser Ala Pro Lys Glu Thr
    130                 135                 140

Val Leu Asp Thr Ala Asp Thr Asn His Met Leu Gly Ala Thr Met Thr
145                 150                 155                 160

Ala Ser Ser Asn Glu Thr Ala Thr Leu Thr Pro Asp Lys Ala Ile Asp
                165                 170                 175

Gln Asn Arg Thr Gly Arg Asn Asn Arg Trp Ala Ser Gly Tyr Glu Thr
            180                 185                 190

Pro Ser Asn Ile Trp Leu Lys Ala Glu Phe Pro Arg Leu Thr Ala Val
        195                 200                 205

Lys Asp Ile Arg Ile Tyr Phe Phe Glu Arg Asp Val Asn Pro Lys Pro
    210                 215                 220

Thr Asn Val Gln Ser Phe Asp Leu Ser Tyr Thr Asp Ser Glu Gly Thr
225                 230                 235                 240
```

Glu His Thr Leu Lys Ser Gly Tyr Ala Met Thr Ala Ser Gly Ala Gly
245 250 255

Tyr Val Ala Asp Val Val Ile Gln Leu Asp Gln Ala Val Asn Ala Arg
260 265 270

Ser Leu Lys Leu Ser Asn Phe Ala Ile Lys Ser Ser Glu Tyr Asn Asn
275 280 285

Val Ser Val Ala Glu Trp Glu Ala Tyr Ser Asn Asp Gln Ala Glu Pro
290 295 300

Gly Ala Thr Leu Asp Ser Val Val Ser Asp Leu Glu Ser Asn His Leu
305 310 315 320

Thr Ile Glu Thr Asp Thr Asp Thr Leu Ala Leu Pro Thr Val Pro Asp
325 330 335

Gly Tyr Thr Val Lys Phe Asn Gly Ala Asp Tyr Glu Gln Leu Ile Ala
340 345 350

Ala Asp Gly Thr Val Asn His Pro Leu Val Asp Lys Thr Val Gln Val
355 360 365

Ala Tyr Val Val Thr Asp Thr Ala Thr Gly Asn Thr Lys Thr Thr Ser
370 375 380

Asp Ile Pro Tyr Val Val Lys Gly Thr Asn Gln Gln Gln Glu Gly Asn
385 390 395 400

Asn Ala Lys Pro Thr Ile Ile Pro Glu Ile Ala Glu Trp His Ser Thr
405 410 415

Ser Ala Ala Lys Leu Ala Ala Ser Ala Val Thr Lys Val Val Tyr Asp
420 425 430

Asp Asp Ser Leu Lys Ala Val Val Asp Glu Phe Val Ala Asp Tyr Lys
435 440 445

Asp Phe Thr Gly Ile Lys Leu Thr Ala Lys Lys Gly Ala Ala Glu Ala
450 455 460

Gly Ala Phe Asn Phe Val Lys Thr Asp Ser Thr Ala Ala Ile Ala Gln
465 470 475 480

Leu Gly Asp Glu Gly Tyr Thr Met Asp Ile Arg Ala Asp Arg Val Val

485 490 495

Ala Lys Ser Ser Ser Val Thr Gly Asn Met Tyr Ala Met Gln Thr Ile
500 505 510

Leu Gln Met Thr Lys Gln Asp Ala Asn Gly Phe Val Ile Gly Ser Met
515 520 525

Arg Asp Tyr Pro Arg Phe Thr Thr Arg Gly Leu Leu Leu Asp Val Ala
530 535 540

Arg Lys Pro Val Ser Leu Glu Met Met Arg Glu Ile Thr Arg Thr Met
545 550 555 560

Arg Tyr Tyr Lys Met Asn Asp Phe Gln Ala His Leu Ser Asp Asn Tyr
565 570 575

Ile Phe Leu Glu Asn Tyr Gly Lys Gly Asp Asn Glu Asp Glu Ala Phe
580 585 590

Lys Ala Tyr Asp Ala Phe Arg Leu Glu Ser Ser Leu Thr Asn Asp Lys
595 600 605

Gly Glu Ser Pro Thr Ala Glu Asp Tyr Ser Ile Ser Lys Lys Thr Phe
610 615 620

Lys Gln Phe Ile Gln Asp Glu Arg Ala Leu Gly Met Asn Val Val Pro
625 630 635 640

Glu Ile Asp Val Pro Ala His Ala Asn Ser Phe Thr Lys Ile Trp Pro
645 650 655

Glu Leu Met Val Lys Gly Arg Val Ser Pro Ile Asn Ser Asn Arg Pro
660 665 670

Leu Ile Asp His Leu Asp Val Ser Lys Pro Glu Thr Ile Ala Lys Ile
675 680 685

Lys Glu Ile Phe Asp Asp Tyr Thr Lys Gly Asp Asp Pro Thr Phe Asp
690 695 700

Ser Asp Thr Thr Val His Ile Gly Ala Asp Glu Phe Leu Tyr Asn Tyr
705 710 715 720

Thr Ala Tyr Arg Lys Phe Ile Asn Glu Ile Val Pro Tyr Ile Lys Asp
725 730 735

```
Thr Asn Thr Val Arg Met Trp Gly Gly Leu Thr Trp Ile Asn Asp His
            740                 745                 750

Lys Thr Glu Ile Thr Lys Asp Ala Ile Glu Asn Val Glu Met Asn Leu
        755                 760                 765

Trp Ser Lys Asp Trp Ala Asp Gly Leu Gln Met Tyr Asn Met Gly Tyr
    770                 775                 780

Lys Leu Ile Asn Thr Ile Asp Asp Tyr Gly Tyr Met Val Pro Asn Gly
785                 790                 795                 800

Ser Tyr Gly Arg Ala Asn Ala Tyr Gly Asp Leu Leu Asn Ile Ser Arg
                805                 810                 815

Val Phe Asp Ser Phe Glu Pro Asn Lys Val Arg Ser Ser Gly Gly Tyr
            820                 825                 830

Gln Ala Val Pro Ser Gly Asp Asp Gln Met Leu Gly Ala Ala Phe Ala
        835                 840                 845

Ile Trp Ser Asp Asn Ile Asp Lys Ser Ala Ser Gly Leu Thr Glu Ser
    850                 855                 860

Asp Leu Tyr Trp Arg Phe Phe Asp Ala Met Pro Phe Tyr Ala Glu Lys
865                 870                 875                 880

Thr Trp Ala Ala Thr Gly Lys Glu Lys Gly Thr Ala Ala Lys Leu Thr
                885                 890                 895

Ala Leu Ala Ala Lys Gln Gly Thr Gly Pro Arg Thr Asn Pro Tyr Tyr
            900                 905                 910

Gln Ala Thr Ser Lys Asn Ser Val Tyr Glu Ser Tyr Asp Phe Asn Asp
        915                 920                 925

Gly Leu Ala Asp Ala Ser Gly Asn Gly Arg Asp Leu Thr Ile Gly Asp
    930                 935                 940

Gly Ser Lys Ala Ala Val Lys Asp Gln Ser Leu Lys Leu Ala Gly Gly
945                 950                 955                 960

Ser Ser Tyr Ala Thr Ser Lys Leu Asp Lys Leu Gly Asn Gly Asn Glu
                965                 970                 975

Leu Thr Phe Asp Val Thr Leu Gln Gln Ala Ala Lys Pro Gly Asp Ile
            980                 985                 990
```

Leu Phe Glu Ala Asp Ala Pro Tyr Gly Thr His Asp Ile Arg Val Met
995 1000 1005

Glu Asn Gly Lys Leu Gly Phe Thr Arg Glu Leu Tyr Asn Tyr Tyr
1010 1015 1020

Phe Asp Tyr Glu Leu Pro Val Gly Lys Thr Val Thr Val Thr Ile
1025 1030 1035

Lys Val Asp Gln Gln Thr Thr Lys Leu Tyr Val Asp Gly Glu Phe
1040 1045 1050

Val Ser Asp Ala Thr Gly Lys Tyr Ile Asp Lys Gly Ile Glu Lys
1055 1060 1065

Lys Thr Gly Ile Thr Ala Ala Thr Phe Ala Leu Pro Leu Gln Arg
1070 1075 1080

Ile Gly Ser Lys Thr Ser Ala Ile Asn Gly Val Ile Asp Asn Val
1085 1090 1095

Ile Val Lys Lys Ser Glu Ala Glu Thr Asp Gln Tyr Asn Lys Ser
1100 1105 1110

Cys Trp Thr Gly Thr Thr Asn Ser Glu Thr Gln Tyr Asn Asp Thr
1115 1120 1125

Glu Gly Leu Leu Arg Tyr Ala Phe Asp Asn Asn Pro Ser Thr Ile
1130 1135 1140

Trp His Ser Asn Trp Lys Gly Ala Thr Asp Lys Leu Thr Gly Ser
1145 1150 1155

Asn Ser Phe Tyr Ala Glu Ile Asp Met Cys Gln Lys Tyr Thr Ile
1160 1165 1170

Asn Gln Phe Ser Phe Thr Pro Arg Thr Ser Gln Asp Ser Gly Gln
1175 1180 1185

Val Thr Lys Ala Asp Leu Tyr Val Lys Ala Asn Ala Asn Asp Glu
1190 1195 1200

Trp Lys Gln Val Ala Thr Asp Gln Val Phe Glu Ala Ser Arg Ala
1205 1210 1215

Lys Lys Thr Phe Met Phe Asp Glu Gln Glu Val Arg Tyr Val Lys
1220 1225 1230

Phe Val Ala Lys Ser Ser Asn Asp Gly Trp Val Ala Val Ser Glu
1235 1240 1245

Phe Gly Val Ala Asn Lys Pro Ser Ser Thr Val Arg Val Phe Val
1250 1255 1260

Ala Ala Asp Pro Ala Glu Gly Gly Thr Val Ser Val Ala Ala Glu
1265 1270 1275

Gly Glu Thr Gly Thr Asp Thr Ala Val Asp Val Ala Ser Gly Ala
1280 1285 1290

Ser Val Thr Ala Lys Ala Val Ala Ala Asp Gly Tyr Arg Phe Ser
1295 1300 1305

Gly Trp Phe Thr Thr Ala Ser Glu Thr Ala Val Ser Thr Asp Ala
1310 1315 1320

Thr Tyr Thr Phe Ala Ala Asp Gly Asn Thr Ala Leu Thr Ala Lys
1325 1330 1335

Phe Thr Lys Asp Ser Thr Pro Asp Pro Gly Pro Lys Pro Thr Ile
1340 1345 1350

Ser Ser Ile Ala Val Thr Lys Pro Thr Val Thr Asp Tyr Lys Val
1355 1360 1365

Gly Asp Thr Phe Asp Ala Thr Gly Leu Ala Val Thr Ala Thr Met
1370 1375 1380

Ser Asp Gly Ser Thr Lys Thr Leu Thr Ala Gly Glu Tyr Thr Leu
1385 1390 1395

Ser Ala Thr Gln Asp Gly Ala Ala Val Ala Leu Asp Lys Ala Phe
1400 1405 1410

Ala Lys Ala Gly Lys Val Thr Val Thr Val Thr Ala Asn Gly Lys
1415 1420 1425

Thr Ala Thr Phe Asp Val Thr Val Thr Ala Lys Asp Pro Asp Pro
1430 1435 1440

Glu Pro Ala Thr Leu Lys Ser Ile Lys Val Thr Ser Lys Pro Asp
1445 1450 1455

Lys Thr Thr Tyr Thr Val Asp Glu Thr Phe Ala Lys Thr Gly Leu

1460 1465 1470

Ala Val Thr Gly Thr Trp Ser Asp Gly Lys Thr Ala Leu Leu Lys
1475 1480 1485

Asp Gly Glu Tyr Lys Leu Ser Ala Val Asp Ala Asp Gly Lys Thr
1490 1495 1500

Val Asp Leu Thr Lys Pro Phe Thr Ala Ala Gly Asp Val Thr Val
1505 1510 1515

Thr Val Thr Ser Gly Lys Leu Thr Asp Ser Phe Thr Ile Thr Val
1520 1525 1530

Lys Ala Lys Thr Val Thr Pro Ala Pro Gly Asp Asn Lys Pro Gly
1535 1540 1545

Glu Asn Lys Pro Gly Ala Asp Lys Pro Lys Pro Asn Thr Pro Asp
1550 1555 1560

Glu Val Ala Lys
1565

<210> 3
<211> 1112
<212> PRT
<213> Bifidobacterium bifidum

<400> 3

Met Glu Lys Ser Ser Asn Arg Arg Phe Gly Val Arg Thr Val Ala Ala
1 5 10 15

Ile Val Ala Gly Leu Met Val Gly Gly Met Cys Thr Ala Met Thr Ala
20 25 30

Ser Ala Ala Asp Asp Ser Ala Ala Gly Tyr Ser Ala Thr Ala Pro Val
35 40 45

Asn Leu Thr Arg Pro Ala Thr Val Pro Ser Met Asp Gly Trp Thr Asp
50 55 60

Gly Thr Gly Ala Trp Thr Leu Gly Glu Gly Thr Arg Val Val Ser Ser
65 70 75 80

Asp Ala Leu Ala Ala Arg Ala Gln Ser Leu Ala Ser Glu Leu Thr Lys
85 90 95

Phe Thr Asp Val Asp Ile Lys Ala Ala Thr Gly Ser Ala Thr Gly Lys

100 105 110

Asp Ile Ser Leu Thr Leu Asp Ala Ser Lys Lys Ala Glu Leu Gly Asp
115 120 125

Glu Gly Phe Lys Leu Asn Ile Gly Ser Lys Gly Leu Glu Val Ile Gly
130 135 140

Ala Thr Asp Ile Gly Val Phe Tyr Gly Thr Arg Ser Val Ser Gln Met
145 150 155 160

Leu Arg Gln Gly Gln Leu Thr Leu Pro Ala Gly Thr Val Ala Thr Lys
165 170 175

Pro Lys Tyr Lys Glu Arg Gly Ala Thr Leu Cys Ala Cys Gln Ile Asn
180 185 190

Ile Ser Thr Asp Trp Ile Asp Arg Phe Leu Ser Asp Met Ala Asp Leu
195 200 205

Arg Leu Asn Tyr Val Leu Leu Glu Met Lys Leu Lys Pro Glu Glu Asp
210 215 220

Asn Thr Lys Lys Ala Ala Thr Trp Ser Tyr Tyr Thr Arg Asp Asp Val
225 230 235 240

Lys Lys Phe Val Lys Lys Ala Asn Asn Tyr Gly Ile Asp Val Ile Pro
245 250 255

Glu Ile Asn Ser Pro Gly His Met Asn Val Trp Leu Glu Asn Tyr Pro
260 265 270

Glu Tyr Gln Leu Ala Asp Asn Ser Gly Arg Lys Asp Pro Asn Lys Leu
275 280 285

Asp Ile Ser Asn Pro Glu Ala Val Lys Phe Tyr Lys Thr Leu Ile Asp
290 295 300

Glu Tyr Asp Gly Val Phe Thr Thr Lys Tyr Trp His Met Gly Ala Asp
305 310 315 320

Glu Tyr Met Ile Gly Thr Ser Phe Asp Asn Tyr Ser Lys Leu Lys Thr
325 330 335

Phe Ala Glu Lys Gln Tyr Gly Ala Gly Ala Thr Pro Asn Asp Ala Phe
340 345 350

```
Thr Gly Phe Ile Asn Asp Ile Asp Lys Tyr Val Lys Ala Lys Gly Lys
        355                 360                 365

Gln Leu Arg Ile Trp Asn Asp Gly Ile Val Asn Thr Lys Asn Val Ser
    370                 375                 380

Leu Asn Lys Asp Ile Val Ile Glu Tyr Trp Tyr Gly Ala Gly Arg Lys
385                 390                 395                 400

Pro Gln Glu Leu Val Gln Asp Gly Tyr Thr Leu Met Asn Ala Thr Gln
                405                 410                 415

Ala Leu Tyr Trp Ser Arg Ser Ala Gln Val Tyr Lys Val Asn Ala Ala
            420                 425                 430

Arg Leu Tyr Asn Asn Asn Trp Asn Val Gly Thr Phe Asp Gly Gly Arg
        435                 440                 445

Gln Ile Asp Lys Asn Tyr Asp Lys Leu Thr Gly Ala Lys Val Ser Ile
    450                 455                 460

Trp Pro Asp Ser Ser Tyr Phe Gln Thr Glu Asn Glu Val Glu Lys Glu
465                 470                 475                 480

Ile Phe Asp Gly Met Arg Phe Ile Ser Gln Met Thr Trp Ser Asp Ser
                485                 490                 495

Arg Pro Trp Ala Thr Trp Asn Asp Met Lys Ala Asp Ile Asp Lys Ile
            500                 505                 510

Gly Tyr Pro Leu Asp Ile Arg Glu Tyr Asp Tyr Thr Pro Val Asp Ala
        515                 520                 525

Gly Ile Tyr Asp Ile Pro Gln Leu Lys Ser Ile Ser Lys Gly Pro Trp
    530                 535                 540

Glu Leu Ile Thr Thr Pro Asp Gly Tyr Tyr Gln Met Lys Asp Thr Val
545                 550                 555                 560

Ser Gly Lys Cys Leu Ala Leu Phe Thr Gly Ser Lys His Leu Asp Val
                565                 570                 575

Val Thr Gln Val Gly Ala Arg Pro Glu Leu Arg Asn Cys Ala Asp Val
            580                 585                 590

Ser Val Gly Gln Asp Gln Arg Asn Thr Ala Asn Glu Arg Asn Thr Gln
        595                 600                 605
```

```
Lys Trp Gln Ile Arg Ala Asp Lys Asp Gly Lys Tyr Thr Ile Ser Pro
    610                 615                 620

Ala Leu Thr Gln Gln Arg Leu Ala Ile Ala Thr Gly Asn Glu Gln Asn
625                 630                 635                 640

Ile Asp Leu Glu Thr His Arg Pro Ala Ala Gly Thr Val Ala Gln Phe
                645                 650                 655

Pro Ala Asp Leu Val Ser Asp Asn Ala Leu Phe Thr Leu Thr Gly His
            660                 665                 670

Met Gly Met Ser Ala Thr Val Asp Ser Lys Thr Val Asn Pro Ala Ser
        675                 680                 685

Pro Ser Lys Ile Thr Val Lys Val Arg Ala Ala Ser Asn Ala Asn Thr
    690                 695                 700

Gly Asp Val Thr Val Thr Pro Val Val Pro Glu Gly Trp Glu Ile Lys
705                 710                 715                 720

Pro Gly Ser Val Ser Leu Lys Ser Ile Pro Ala Gly Lys Ala Ala Ile
                725                 730                 735

Ala Tyr Phe Asn Val Val Asn Thr Thr Gly Thr Gly Asp Ala Thr Val
            740                 745                 750

Gln Phe Lys Leu Thr Asn Thr Lys Thr Gly Glu Glu Leu Gly Thr Thr
        755                 760                 765

Ser Val Ala Leu Thr Gly Ser Leu Thr Lys Asp Val Glu Ala Ser Asp
    770                 775                 780

Tyr Ala Ala Ser Ser Gln Glu Thr Thr Gly Glu His Ala Pro Val Gly
785                 790                 795                 800

Asn Ala Phe Asp Lys Asn Ala Asn Thr Phe Trp His Ser Lys Tyr Ser
                805                 810                 815

Asn Pro Ser Ala Asn Leu Pro His Trp Leu Ala Phe Lys Ala Ser Pro
            820                 825                 830

Gly Glu Gly Asn Lys Ile Ala Ala Ile Thr His Leu Tyr Arg Gln Asp
        835                 840                 845

Lys Leu Asn Gly Pro Ala Lys Asn Val Ala Val Tyr Val Val Ala Ala
    850                 855                 860
```

Ser Asp Ala Asn Ser Val Ala Asp Val Thr Asn Trp Gly Glu Pro Val
865 870 875 880

Ala Thr Ala Glu Phe Pro Tyr Thr Lys Glu Leu Gln Thr Ile Ala Leu
885 890 895

Pro Asn Thr Ile Pro Ser Gly Asp Val Tyr Val Lys Phe Gln Ile Asn
900 905 910

Asp Ala Trp Gly Leu Thr Glu Thr Ser Ala Gly Val Thr Trp Ala Ala
915 920 925

Val Ala Glu Leu Ala Ala Thr Ala Lys Ala Thr Pro Val Glu Leu Thr
930 935 940

Glu Pro Glu Gln Pro Lys Asp Asn Pro Glu Val Thr Glu Thr Pro Glu
945 950 955 960

Ala Thr Gly Val Thr Val Ser Gly Asp Gly Val Ala Asn Gly Ala Leu
965 970 975

Ser Leu Lys Lys Gly Thr Thr Ala Gln Leu Thr Ala Lys Val Ala Pro
980 985 990

Asp Asp Ala Asp Gln Ala Val Thr Trp Ala Ser Ser Asp Asp Lys Val
995 1000 1005

Val Thr Val Asp Lys Thr Gly Lys Val Thr Ala Val Ala Lys Gly
1010 1015 1020

Val Ala Lys Val Thr Ala Thr Thr Ala Asn Gly Lys Ser Ala Ser
1025 1030 1035

Val Thr Val Thr Val Thr Glu Asp Ser Glu Val Pro Gly Pro Thr
1040 1045 1050

Gly Pro Thr Glu Pro Thr Lys Pro Gly Thr Glu Lys Pro Thr Thr
1055 1060 1065

Lys Pro Thr Thr Lys Pro Asn Asp Gly Lys Leu Ser Ala Thr Gly
1070 1075 1080

Ala Asp Thr Ala Val Leu Ala Thr Ile Ala Ala Leu Phe Ala Leu
1085 1090 1095

Ala Gly Gly Ala Val Val Ala Val Arg Arg Arg Ser Val Arg

1100 1105 1110

<210> 4
<211> 1030
<212> PRT
<213> Bifidobacterium bifidum

<400> 4

Ala Asp Asp Ser Ala Ala Gly Tyr Ser Ala Thr Ala Pro Val Asn Leu
1 5 10 15

Thr Arg Pro Ala Thr Val Pro Ser Met Asp Gly Trp Thr Asp Gly Thr
20 25 30

Gly Ala Trp Thr Leu Gly Glu Gly Thr Arg Val Val Ser Ser Asp Ala
35 40 45

Leu Ala Ala Arg Ala Gln Ser Leu Ala Ser Glu Leu Thr Lys Phe Thr
50 55 60

Asp Val Asp Ile Lys Ala Ala Thr Gly Ser Ala Thr Gly Lys Asp Ile
65 70 75 80

Ser Leu Thr Leu Asp Ala Ser Lys Lys Ala Glu Leu Gly Asp Glu Gly
85 90 95

Phe Lys Leu Asn Ile Gly Ser Lys Gly Leu Glu Val Ile Gly Ala Thr
100 105 110

Asp Ile Gly Val Phe Tyr Gly Thr Arg Ser Val Ser Gln Met Leu Arg
115 120 125

Gln Gly Gln Leu Thr Leu Pro Ala Gly Thr Val Ala Thr Lys Pro Lys
130 135 140

Tyr Lys Glu Arg Gly Ala Thr Leu Cys Ala Cys Gln Ile Asn Ile Ser
145 150 155 160

Thr Asp Trp Ile Asp Arg Phe Leu Ser Asp Met Ala Asp Leu Arg Leu
165 170 175

Asn Tyr Val Leu Leu Glu Met Lys Leu Lys Pro Glu Glu Asp Asn Thr
180 185 190

Lys Lys Ala Ala Thr Trp Ser Tyr Tyr Thr Arg Asp Asp Val Lys Lys
195 200 205

Phe Val Lys Lys Ala Asn Asn Tyr Gly Ile Asp Val Ile Pro Glu Ile

210 215 220

Asn Ser Pro Gly His Met Asn Val Trp Leu Glu Asn Tyr Pro Glu Tyr
225 230 235 240

Gln Leu Ala Asp Asn Ser Gly Arg Lys Asp Pro Asn Lys Leu Asp Ile
245 250 255

Ser Asn Pro Glu Ala Val Lys Phe Tyr Lys Thr Leu Ile Asp Glu Tyr
260 265 270

Asp Gly Val Phe Thr Thr Lys Tyr Trp His Met Gly Ala Asp Glu Tyr
275 280 285

Met Ile Gly Thr Ser Phe Asp Asn Tyr Ser Lys Leu Lys Thr Phe Ala
290 295 300

Glu Lys Gln Tyr Gly Ala Gly Ala Thr Pro Asn Asp Ala Phe Thr Gly
305 310 315 320

Phe Ile Asn Asp Ile Asp Lys Tyr Val Lys Ala Lys Gly Lys Gln Leu
325 330 335

Arg Ile Trp Asn Asp Gly Ile Val Asn Thr Lys Asn Val Ser Leu Asn
340 345 350

Lys Asp Ile Val Ile Glu Tyr Trp Tyr Gly Ala Gly Arg Lys Pro Gln
355 360 365

Glu Leu Val Gln Asp Gly Tyr Thr Leu Met Asn Ala Thr Gln Ala Leu
370 375 380

Tyr Trp Ser Arg Ser Ala Gln Val Tyr Lys Val Asn Ala Ala Arg Leu
385 390 395 400

Tyr Asn Asn Asn Trp Asn Val Gly Thr Phe Asp Gly Gly Arg Gln Ile
405 410 415

Asp Lys Asn Tyr Asp Lys Leu Thr Gly Ala Lys Val Ser Ile Trp Pro
420 425 430

Asp Ser Ser Tyr Phe Gln Thr Glu Asn Glu Val Glu Lys Glu Ile Phe
435 440 445

Asp Gly Met Arg Phe Ile Ser Gln Met Thr Trp Ser Asp Ser Arg Pro
450 455 460

Trp Ala Thr Trp Asn Asp Met Lys Ala Asp Ile Asp Lys Ile Gly Tyr
465 470 475 480

Pro Leu Asp Ile Arg Glu Tyr Asp Tyr Thr Pro Val Asp Ala Gly Ile
485 490 495

Tyr Asp Ile Pro Gln Leu Lys Ser Ile Ser Lys Gly Pro Trp Glu Leu
500 505 510

Ile Thr Thr Pro Asp Gly Tyr Tyr Gln Met Lys Asp Thr Val Ser Gly
515 520 525

Lys Cys Leu Ala Leu Phe Thr Gly Ser Lys His Leu Asp Val Val Thr
530 535 540

Gln Val Gly Ala Arg Pro Glu Leu Arg Asn Cys Ala Asp Val Ser Val
545 550 555 560

Gly Gln Asp Gln Arg Asn Thr Ala Asn Glu Arg Asn Thr Gln Lys Trp
565 570 575

Gln Ile Arg Ala Asp Lys Asp Gly Lys Tyr Thr Ile Ser Pro Ala Leu
580 585 590

Thr Gln Gln Arg Leu Ala Ile Ala Thr Gly Asn Glu Gln Asn Ile Asp
595 600 605

Leu Glu Thr His Arg Pro Ala Ala Gly Thr Val Ala Gln Phe Pro Ala
610 615 620

Asp Leu Val Ser Asp Asn Ala Leu Phe Thr Leu Thr Gly His Met Gly
625 630 635 640

Met Ser Ala Thr Val Asp Ser Lys Thr Val Asn Pro Ala Ser Pro Ser
645 650 655

Lys Ile Thr Val Lys Val Arg Ala Ala Ser Asn Ala Asn Thr Gly Asp
660 665 670

Val Thr Val Thr Pro Val Val Pro Glu Gly Trp Glu Ile Lys Pro Gly
675 680 685

Ser Val Ser Leu Lys Ser Ile Pro Ala Gly Lys Ala Ala Ile Ala Tyr
690 695 700

Phe Asn Val Val Asn Thr Thr Gly Thr Gly Asp Ala Thr Val Gln Phe
705 710 715 720

```
Lys Leu Thr Asn Thr Lys Thr Gly Glu Glu Leu Gly Thr Thr Ser Val
                725                 730                 735

Ala Leu Thr Gly Ser Leu Thr Lys Asp Val Glu Ala Ser Asp Tyr Ala
            740                 745                 750

Ala Ser Ser Gln Glu Thr Thr Gly Glu His Ala Pro Val Gly Asn Ala
        755                 760                 765

Phe Asp Lys Asn Ala Asn Thr Phe Trp His Ser Lys Tyr Ser Asn Pro
    770                 775                 780

Ser Ala Asn Leu Pro His Trp Leu Ala Phe Lys Ala Ser Pro Gly Glu
785                 790                 795                 800

Gly Asn Lys Ile Ala Ala Ile Thr His Leu Tyr Arg Gln Asp Lys Leu
                805                 810                 815

Asn Gly Pro Ala Lys Asn Val Ala Val Tyr Val Val Ala Ala Ser Asp
            820                 825                 830

Ala Asn Ser Val Ala Asp Val Thr Asn Trp Gly Glu Pro Val Ala Thr
        835                 840                 845

Ala Glu Phe Pro Tyr Thr Lys Glu Leu Gln Thr Ile Ala Leu Pro Asn
    850                 855                 860

Thr Ile Pro Ser Gly Asp Val Tyr Val Lys Phe Gln Ile Asn Asp Ala
865                 870                 875                 880

Trp Gly Leu Thr Glu Thr Ser Ala Gly Val Thr Trp Ala Ala Val Ala
                885                 890                 895

Glu Leu Ala Ala Thr Ala Lys Ala Thr Pro Val Glu Leu Thr Glu Pro
            900                 905                 910

Glu Gln Pro Lys Asp Asn Pro Glu Val Thr Glu Thr Pro Glu Ala Thr
        915                 920                 925

Gly Val Thr Val Ser Gly Asp Gly Val Ala Asn Gly Ala Leu Ser Leu
    930                 935                 940

Lys Lys Gly Thr Thr Ala Gln Leu Thr Ala Lys Val Ala Pro Asp Asp
945                 950                 955                 960

Ala Asp Gln Ala Val Thr Trp Ala Ser Ser Asp Asp Lys Val Val Thr
                965                 970                 975
```

Val Asp Lys Thr Gly Lys Val Thr Ala Val Ala Lys Gly Val Ala Lys
980 985 990

Val Thr Ala Thr Thr Ala Asn Gly Lys Ser Ala Ser Val Thr Val Thr
995 1000 1005

Val Thr Glu Asp Ser Glu Val Pro Gly Pro Thr Gly Pro Thr Glu
1010 1015 1020

Pro Thr Lys Pro Gly Thr Glu
1025 1030

<210> 5
<211> 1567
<212> PRT
<213> Artificial Sequence

<220>
<223> Artificial sequence

<400> 5

Ser Asp Asp Asn Leu Ala Leu Asn Gln Thr Val Thr Ala Ser Ser Tyr
1 5 10 15

Glu Val Ala Thr Thr Ala Pro Glu Lys Ala Val Asp Gly Asp Leu Gly
20 25 30

Thr Arg Trp Gly Thr Ala Gln Asn Lys Ala Ala Asn Glu Trp Ile Glu
35 40 45

Val Gly Leu Gly Gly Thr Lys Thr Val Lys Gln Ile Asn Ile Asp Phe
50 55 60

Glu Arg Lys Asp Ala Asp Gln Asn Ile Thr Ser Phe Lys Val Glu Leu
65 70 75 80

Lys Gln Gly Asp Thr Tyr Thr Lys Val Tyr Gln Lys Asp Thr Arg Ala
85 90 95

Lys Gln Gln Glu Ile Ile Leu Leu Asp Gln Ala Gln Gln Ala Ser Ala
100 105 110

Val Lys Val Thr Val Leu Ser Ala Asp Gly Gly Thr Met Asn Trp Val
115 120 125

Asn Val Gly Ile Asn Glu Ile Ser Val Tyr Ser Ala Pro Lys Glu Thr
130 135 140

Val Leu Asp Thr Ala Asp Thr Asn His Met Leu Gly Ala Thr Met Thr
145 150 155 160

Ala Ser Ser Asn Glu Thr Ala Thr Leu Thr Pro Asp Lys Ala Ile Asp
165 170 175

Gln Asn Arg Thr Gly Arg Asn Asn Arg Trp Ala Ser Gly Tyr Glu Thr
180 185 190

Pro Ser Asn Ile Trp Leu Lys Ala Glu Phe Pro Arg Leu Thr Ala Val
195 200 205

Lys Asp Ile Arg Ile Tyr Phe Phe Glu Arg Asp Val Asn Pro Lys Pro
210 215 220

Thr Asn Val Gln Ser Phe Asp Leu Ser Tyr Thr Asp Ser Glu Gly Thr
225 230 235 240

Glu His Thr Leu Lys Ser Gly Tyr Ala Met Thr Ala Ser Gly Ala Gly
245 250 255

Tyr Val Ala Asp Val Val Ile Gln Leu Asp Gln Ala Val Asn Ala Arg
260 265 270

Ser Leu Lys Leu Ser Asn Phe Ala Ile Lys Ser Ser Glu Tyr Asn Asn
275 280 285

Val Ser Val Ala Glu Trp Glu Ala Tyr Ser Asn Asp Gln Ala Glu Pro
290 295 300

Gly Ala Thr Leu Asp Ser Val Val Ser Asp Leu Glu Ser Asn His Leu
305 310 315 320

Thr Ile Glu Thr Asp Thr Asp Thr Leu Ala Leu Pro Thr Val Pro Asp
325 330 335

Gly Tyr Thr Val Lys Phe Asn Gly Ala Asp Tyr Glu Gln Leu Ile Ala
340 345 350

Ala Asp Gly Thr Val Asn His Pro Leu Val Asp Lys Thr Val Gln Val
355 360 365

Ala Tyr Val Val Thr Asp Thr Ala Thr Gly Asn Thr Lys Thr Thr Ser
370 375 380

Asp Ile Pro Tyr Val Val Lys Gly Thr Asn Gln Gln Gln Glu Gly Asn
385 390 395 400

Asn Ala Lys Pro Thr Ile Ile Pro Glu Ile Ala Glu Trp His Ser Thr
405 410 415

Ser Ala Ala Lys Leu Ala Ala Ser Ala Val Thr Lys Val Val Tyr Asp
420 425 430

Asp Asp Ser Leu Lys Ala Val Val Asp Glu Phe Val Ala Asp Tyr Lys
435 440 445

Asp Phe Thr Gly Ile Lys Leu Thr Ala Lys Lys Gly Ala Ala Glu Ala
450 455 460

Gly Ala Phe Asn Phe Val Lys Thr Asp Ser Thr Ala Ala Ile Ala Gln
465 470 475 480

Leu Gly Asp Glu Gly Tyr Thr Met Asp Ile Arg Ala Asp Arg Val Val
485 490 495

Ala Lys Ser Ser Ser Val Thr Gly Asn Met Tyr Ala Met Gln Thr Ile
500 505 510

Leu Gln Met Thr Lys Gln Asp Ala Asn Gly Phe Val Ile Gly Ser Met
515 520 525

Arg Asp Tyr Pro Arg Phe Thr Thr Arg Gly Leu Leu Leu Asp Val Ala
530 535 540

Arg Lys Pro Val Ser Leu Glu Met Met Arg Glu Ile Thr Arg Thr Met
545 550 555 560

Arg Tyr Tyr Lys Met Asn Asp Phe Gln Ala His Leu Ser Asp Asn Tyr
565 570 575

Ile Phe Leu Glu Asn Tyr Gly Lys Gly Asp Asn Glu Asp Glu Ala Phe
580 585 590

Lys Ala Tyr Asp Ala Phe Arg Leu Glu Ser Ser Leu Thr Asn Asp Lys
595 600 605

Gly Glu Ser Pro Thr Ala Glu Asp Tyr Ser Ile Ser Lys Lys Thr Phe
610 615 620

Lys Gln Phe Ile Gln Asp Glu Arg Ala Leu Gly Met Asn Val Val Pro
625 630 635 640

Glu Ile Asp Val Pro Ala His Ala Asn Ser Phe Thr Lys Ile Trp Pro

645 650 655

Glu Leu Met Val Lys Gly Arg Val Ser Pro Ile Asn Ser Asn Arg Pro
660 665 670

Leu Ile Asp His Leu Asp Val Ser Lys Pro Glu Thr Ile Ala Lys Ile
675 680 685

Lys Glu Ile Phe Asp Asp Tyr Thr Lys Gly Asp Asp Pro Thr Phe Asp
690 695 700

Ser Asp Thr Thr Val His Ile Gly Ala Glu Glu Phe Leu Tyr Asn Tyr
705 710 715 720

Thr Ala Tyr Arg Lys Phe Ile Asn Glu Ile Val Pro Tyr Ile Lys Asp
725 730 735

Thr Asn Thr Val Arg Met Trp Gly Gly Leu Thr Trp Ile Asn Asp His
740 745 750

Lys Thr Glu Ile Thr Lys Asp Ala Ile Glu Asn Val Glu Met Asn Leu
755 760 765

Trp Ser Lys Asp Trp Ala Asp Gly Leu Gln Met Tyr Asn Met Gly Tyr
770 775 780

Lys Leu Ile Asn Thr Ile Asp Asp Tyr Gly Tyr Met Val Pro Asn Gly
785 790 795 800

Ser Tyr Gly Arg Ala Asn Ala Tyr Gly Asp Leu Leu Asn Ile Ser Arg
805 810 815

Val Phe Asp Ser Phe Glu Pro Asn Lys Val Arg Ser Ser Gly Gly Tyr
820 825 830

Gln Ala Val Pro Ser Gly Asp Asp Gln Met Leu Gly Ala Ala Phe Ala
835 840 845

Ile Trp Ser Asp Asn Ile Asp Lys Ser Ala Ser Gly Leu Thr Glu Ser
850 855 860

Asp Leu Tyr Trp Arg Phe Phe Asp Ala Met Pro Phe Tyr Ala Glu Lys
865 870 875 880

Thr Trp Ala Ala Thr Gly Lys Glu Lys Gly Thr Ala Ala Lys Leu Thr
885 890 895

```
Ala Leu Ala Ala Lys Gln Gly Thr Gly Pro Arg Thr Asn Pro Tyr Tyr
            900                 905                 910

Gln Ala Thr Ser Lys Asn Ser Val Tyr Glu Ser Tyr Asp Phe Asn Asp
        915                 920                 925

Gly Leu Ala Asp Ala Ser Gly Asn Gly Arg Asp Leu Thr Ile Gly Asp
    930                 935                 940

Gly Ser Lys Ala Ala Val Lys Asp Gln Ser Leu Lys Leu Ala Gly Gly
945                 950                 955                 960

Ser Ser Tyr Ala Thr Ser Lys Leu Asp Lys Leu Gly Asn Gly Asn Glu
                965                 970                 975

Leu Thr Phe Asp Val Thr Leu Gln Gln Ala Ala Lys Pro Gly Asp Ile
            980                 985                 990

Leu Phe Glu Ala Asp Ala Pro Tyr  Gly Thr His Asp Ile  Arg Val Met
        995                 1000                1005

Glu Asn  Gly Lys Leu Gly Phe  Thr Arg Glu Leu Tyr  Asn Tyr Tyr
    1010                1015                1020

Phe Asp  Tyr Glu Leu Pro Val  Gly Lys Thr Val Thr  Val Thr Ile
    1025                1030                1035

Lys Val  Asp Gln Gln Thr Thr  Lys Leu Tyr Val Asp  Gly Glu Phe
    1040                1045                1050

Val Ser  Asp Ala Thr Gly Lys  Tyr Ile Asp Lys Gly  Ile Glu Lys
    1055                1060                1065

Lys Thr  Gly Ile Thr Ala Ala  Thr Phe Ala Leu Pro  Leu Gln Arg
    1070                1075                1080

Ile Gly  Ser Lys Thr Ser Ala  Ile Asn Gly Val Ile  Asp Asn Val
    1085                1090                1095

Ile Val  Lys Lys Ser Glu Ala  Glu Thr Asp Gln Tyr  Asn Lys Ser
    1100                1105                1110

Cys Trp  Thr Gly Thr Thr Asn  Ser Glu Thr Gln Tyr  Asn Asp Thr
    1115                1120                1125

Glu Gly  Leu Leu Arg Tyr Ala  Phe Asp Asn Asn Pro  Ser Thr Ile
    1130                1135                1140
```

```
Trp His  Ser Asn Trp Lys Gly  Ala Thr Asp Lys Leu  Thr Gly Ser
    1145                 1150                 1155

Asn Ser  Phe Tyr Ala Glu Ile  Asp Met Cys Gln Lys  Tyr Thr Ile
    1160                 1165                 1170

Asn Gln  Phe Ser Phe Thr Pro  Arg Thr Ser Gln Asp  Ser Gly Gln
    1175                 1180                 1185

Val Thr  Lys Ala Asp Leu Tyr  Val Lys Ala Asn Ala  Asn Asp Glu
    1190                 1195                 1200

Trp Lys  Gln Val Ala Thr Asp  Gln Val Phe Glu Ala  Ser Arg Ala
    1205                 1210                 1215

Lys Lys  Thr Phe Met Phe Asp  Glu Gln Glu Val Arg  Tyr Val Lys
    1220                 1225                 1230

Phe Val  Ala Lys Ser Ser Asn  Asp Gly Trp Val Ala  Val Ser Glu
    1235                 1240                 1245

Phe Gly  Val Ala Asn Lys Pro  Ser Ser Thr Val Arg  Val Phe Val
    1250                 1255                 1260

Ala Ala  Asp Pro Ala Glu Gly  Gly Thr Val Ser Val  Ala Ala Glu
    1265                 1270                 1275

Gly Glu  Thr Gly Thr Asp Thr  Ala Val Asp Val Ala  Ser Gly Ala
    1280                 1285                 1290

Ser Val  Thr Ala Lys Ala Val  Ala Ala Asp Gly Tyr  Arg Phe Ser
    1295                 1300                 1305

Gly Trp  Phe Thr Thr Ala Ser  Glu Thr Ala Val Ser  Thr Asp Ala
    1310                 1315                 1320

Thr Tyr  Thr Phe Ala Ala Asp  Gly Asn Thr Ala Leu  Thr Ala Lys
    1325                 1330                 1335

Phe Thr  Lys Asp Ser Thr Pro  Asp Pro Gly Pro Lys  Pro Thr Ile
    1340                 1345                 1350

Ser Ser  Ile Ala Val Thr Lys  Pro Thr Val Thr Asp  Tyr Lys Val
    1355                 1360                 1365

Gly Asp  Thr Phe Asp Ala Thr  Gly Leu Ala Val Thr  Ala Thr Met
    1370                 1375                 1380
```

Ser Asp Gly Ser Thr Lys Thr Leu Thr Ala Gly Glu Tyr Thr Leu
1385 1390 1395

Ser Ala Thr Gln Asp Gly Ala Ala Val Ala Leu Asp Lys Ala Phe
1400 1405 1410

Ala Lys Ala Gly Lys Val Thr Val Thr Val Thr Ala Asn Gly Lys
1415 1420 1425

Thr Ala Thr Phe Asp Val Thr Val Thr Ala Lys Asp Pro Asp Pro
1430 1435 1440

Glu Pro Ala Thr Leu Lys Ser Ile Lys Val Thr Ser Lys Pro Asp
1445 1450 1455

Lys Thr Thr Tyr Thr Val Asp Glu Thr Phe Ala Lys Thr Gly Leu
1460 1465 1470

Ala Val Thr Gly Thr Trp Ser Asp Gly Lys Thr Ala Leu Leu Lys
1475 1480 1485

Asp Gly Glu Tyr Lys Leu Ser Ala Val Asp Ala Asp Gly Lys Thr
1490 1495 1500

Val Asp Leu Thr Lys Pro Phe Thr Ala Ala Gly Asp Val Thr Val
1505 1510 1515

Thr Val Thr Ser Gly Lys Leu Thr Asp Ser Phe Thr Ile Thr Val
1520 1525 1530

Lys Ala Lys Thr Val Thr Pro Ala Pro Gly Asp Asn Lys Pro Gly
1535 1540 1545

Glu Asn Lys Pro Gly Ala Asp Lys Pro Lys Pro Asn Thr Pro Asp
1550 1555 1560

Glu Val Ala Lys
1565

<210> 6
<211> 1567
<212> PRT
<213> Artificial Sequence

<220>
<223> Artificial sequence

<400> 6

Ser Asp Asp Asn Leu Ala Leu Asn Gln Thr Val Thr Ala Ser Ser Tyr
1               5                   10                  15

Glu Val Ala Thr Thr Ala Pro Glu Lys Ala Val Asp Gly Asp Leu Gly
            20                  25                  30

Thr Arg Trp Gly Thr Ala Gln Asn Lys Ala Ala Asn Glu Trp Ile Glu
        35                  40                  45

Val Gly Leu Gly Gly Thr Lys Thr Val Lys Gln Ile Asn Ile Asp Phe
    50                  55                  60

Glu Arg Lys Asp Ala Asp Gln Asn Ile Thr Ser Phe Lys Val Glu Leu
65                  70                  75                  80

Lys Gln Gly Asp Thr Tyr Thr Lys Val Tyr Gln Lys Asp Thr Arg Ala
                85                  90                  95

Lys Gln Gln Glu Ile Ile Leu Leu Asp Gln Ala Gln Gln Ala Ser Ala
            100                 105                 110

Val Lys Val Thr Val Leu Ser Ala Asp Gly Gly Thr Met Asn Trp Val
        115                 120                 125

Asn Val Gly Ile Asn Glu Ile Ser Val Tyr Ser Ala Pro Lys Glu Thr
    130                 135                 140

Val Leu Asp Thr Ala Asp Thr Asn His Met Leu Gly Ala Thr Met Thr
145                 150                 155                 160

Ala Ser Ser Asn Glu Thr Ala Thr Leu Thr Pro Asp Lys Ala Ile Asp
                165                 170                 175

Gln Asn Arg Thr Gly Arg Asn Asn Arg Trp Ala Ser Gly Tyr Glu Thr
            180                 185                 190

Pro Ser Asn Ile Trp Leu Lys Ala Glu Phe Pro Arg Leu Thr Ala Val
        195                 200                 205

Lys Asp Ile Arg Ile Tyr Phe Phe Glu Arg Asp Val Asn Pro Lys Pro
    210                 215                 220

Thr Asn Val Gln Ser Phe Asp Leu Ser Tyr Thr Asp Ser Glu Gly Thr
225                 230                 235                 240

Glu His Thr Leu Lys Ser Gly Tyr Ala Met Thr Ala Ser Gly Ala Gly
                245                 250                 255

```
Tyr Val Ala Asp Val Val Ile Gln Leu Asp Gln Ala Val Asn Ala Arg
            260                 265                 270

Ser Leu Lys Leu Ser Asn Phe Ala Ile Lys Ser Ser Glu Tyr Asn Asn
        275                 280                 285

Val Ser Val Ala Glu Trp Glu Ala Tyr Ser Asn Asp Gln Ala Glu Pro
    290                 295                 300

Gly Ala Thr Leu Asp Ser Val Val Ser Asp Leu Glu Ser Asn His Leu
305                 310                 315                 320

Thr Ile Glu Thr Asp Thr Asp Thr Leu Ala Leu Pro Thr Val Pro Asp
                325                 330                 335

Gly Tyr Thr Val Lys Phe Asn Gly Ala Asp Tyr Glu Gln Leu Ile Ala
            340                 345                 350

Ala Asp Gly Thr Val Asn His Pro Leu Val Asp Lys Thr Val Gln Val
        355                 360                 365

Ala Tyr Val Val Thr Asp Thr Ala Thr Gly Asn Thr Lys Thr Thr Ser
    370                 375                 380

Asp Ile Pro Tyr Val Val Lys Gly Thr Asn Gln Gln Gln Glu Gly Asn
385                 390                 395                 400

Asn Ala Lys Pro Thr Ile Ile Pro Glu Ile Ala Glu Trp His Ser Thr
                405                 410                 415

Ser Ala Ala Lys Leu Ala Ala Ser Ala Val Thr Lys Val Val Tyr Asp
            420                 425                 430

Asp Asp Ser Leu Lys Ala Val Val Asp Glu Phe Val Ala Asp Tyr Lys
        435                 440                 445

Asp Phe Thr Gly Ile Lys Leu Thr Ala Lys Lys Gly Ala Ala Glu Ala
    450                 455                 460

Gly Ala Phe Asn Phe Val Lys Thr Asp Ser Thr Ala Ala Ile Ala Gln
465                 470                 475                 480

Leu Gly Asp Glu Gly Tyr Thr Met Asp Ile Arg Ala Asp Arg Val Val
                485                 490                 495

Ala Lys Ser Ser Ser Val Thr Gly Asn Met Tyr Ala Met Gln Thr Ile
```

```
            500                 505                 510

Leu Gln Met Thr Lys Gln Asp Ala Asn Gly Phe Val Ile Gly Ser Met
        515                 520                 525

Arg Asp Tyr Pro Arg Phe Thr Thr Arg Gly Leu Leu Leu Asp Val Ala
    530                 535                 540

Arg Lys Pro Val Ser Leu Glu Met Met Arg Glu Ile Thr Arg Thr Met
545                 550                 555                 560

Arg Tyr Tyr Lys Met Asn Asp Phe Gln Ala His Leu Ser Asp Asn Tyr
                565                 570                 575

Ile Phe Leu Glu Asn Tyr Gly Lys Gly Asp Asn Glu Asp Glu Ala Phe
            580                 585                 590

Lys Ala Tyr Asp Ala Phe Arg Leu Glu Ser Ser Leu Thr Asn Asp Lys
        595                 600                 605

Gly Glu Ser Pro Thr Ala Glu Asp Tyr Ser Ile Ser Lys Lys Thr Phe
    610                 615                 620

Lys Gln Phe Ile Gln Asp Glu Arg Ala Leu Gly Met Asn Val Val Pro
625                 630                 635                 640

Glu Ile Asp Val Pro Ala His Ala Asn Ser Phe Thr Lys Ile Trp Pro
                645                 650                 655

Glu Leu Met Val Lys Gly Arg Val Ser Pro Ile Asn Ser Asn Arg Pro
            660                 665                 670

Leu Ile Asp His Leu Asp Val Ser Lys Pro Glu Thr Ile Ala Lys Ile
        675                 680                 685

Lys Glu Ile Phe Asp Asp Tyr Thr Lys Gly Asp Asp Pro Thr Phe Asp
    690                 695                 700

Ser Asp Thr Thr Val His Ile Gly Ala Ala Glu Phe Leu Tyr Asn Tyr
705                 710                 715                 720

Thr Ala Tyr Arg Lys Phe Ile Asn Glu Ile Val Pro Tyr Ile Lys Asp
                725                 730                 735

Thr Asn Thr Val Arg Met Trp Gly Gly Leu Thr Trp Ile Asn Asp His
            740                 745                 750
```

Lys Thr Glu Ile Thr Lys Asp Ala Ile Glu Asn Val Glu Met Asn Leu
755 760 765

Trp Ser Lys Asp Trp Ala Asp Gly Leu Gln Met Tyr Asn Met Gly Tyr
770 775 780

Lys Leu Ile Asn Thr Ile Asp Asp Tyr Gly Tyr Met Val Pro Asn Gly
785 790 795 800

Ser Tyr Gly Arg Ala Asn Ala Tyr Gly Asp Leu Leu Asn Ile Ser Arg
805 810 815

Val Phe Asp Ser Phe Glu Pro Asn Lys Val Arg Ser Ser Gly Gly Tyr
820 825 830

Gln Ala Val Pro Ser Gly Asp Asp Gln Met Leu Gly Ala Ala Phe Ala
835 840 845

Ile Trp Ser Asp Asn Ile Asp Lys Ser Ala Ser Gly Leu Thr Glu Ser
850 855 860

Asp Leu Tyr Trp Arg Phe Phe Asp Ala Met Pro Phe Tyr Ala Glu Lys
865 870 875 880

Thr Trp Ala Ala Thr Gly Lys Glu Lys Gly Thr Ala Ala Lys Leu Thr
885 890 895

Ala Leu Ala Ala Lys Gln Gly Thr Gly Pro Arg Thr Asn Pro Tyr Tyr
900 905 910

Gln Ala Thr Ser Lys Asn Ser Val Tyr Glu Ser Tyr Asp Phe Asn Asp
915 920 925

Gly Leu Ala Asp Ala Ser Gly Asn Gly Arg Asp Leu Thr Ile Gly Asp
930 935 940

Gly Ser Lys Ala Ala Val Lys Asp Gln Ser Leu Lys Leu Ala Gly Gly
945 950 955 960

Ser Ser Tyr Ala Thr Ser Lys Leu Asp Lys Leu Gly Asn Gly Asn Glu
965 970 975

Leu Thr Phe Asp Val Thr Leu Gln Gln Ala Ala Lys Pro Gly Asp Ile
980 985 990

Leu Phe Glu Ala Asp Ala Pro Tyr Gly Thr His Asp Ile Arg Val Met
995 1000 1005

Glu Asn Gly Lys Leu Gly Phe Thr Arg Glu Leu Tyr Asn Tyr Tyr
1010 1015 1020

Phe Asp Tyr Glu Leu Pro Val Gly Lys Thr Val Thr Val Thr Ile
1025 1030 1035

Lys Val Asp Gln Gln Thr Thr Lys Leu Tyr Val Asp Gly Glu Phe
1040 1045 1050

Val Ser Asp Ala Thr Gly Lys Tyr Ile Asp Lys Gly Ile Glu Lys
1055 1060 1065

Lys Thr Gly Ile Thr Ala Ala Thr Phe Ala Leu Pro Leu Gln Arg
1070 1075 1080

Ile Gly Ser Lys Thr Ser Ala Ile Asn Gly Val Ile Asp Asn Val
1085 1090 1095

Ile Val Lys Lys Ser Glu Ala Glu Thr Asp Gln Tyr Asn Lys Ser
1100 1105 1110

Cys Trp Thr Gly Thr Thr Asn Ser Glu Thr Gln Tyr Asn Asp Thr
1115 1120 1125

Glu Gly Leu Leu Arg Tyr Ala Phe Asp Asn Asn Pro Ser Thr Ile
1130 1135 1140

Trp His Ser Asn Trp Lys Gly Ala Thr Asp Lys Leu Thr Gly Ser
1145 1150 1155

Asn Ser Phe Tyr Ala Glu Ile Asp Met Cys Gln Lys Tyr Thr Ile
1160 1165 1170

Asn Gln Phe Ser Phe Thr Pro Arg Thr Ser Gln Asp Ser Gly Gln
1175 1180 1185

Val Thr Lys Ala Asp Leu Tyr Val Lys Ala Asn Ala Asn Asp Glu
1190 1195 1200

Trp Lys Gln Val Ala Thr Asp Gln Val Phe Glu Ala Ser Arg Ala
1205 1210 1215

Lys Lys Thr Phe Met Phe Asp Glu Gln Glu Val Arg Tyr Val Lys
1220 1225 1230

Phe Val Ala Lys Ser Ser Asn Asp Gly Trp Val Ala Val Ser Glu
1235 1240 1245

```
Phe Gly  Val Ala Asn Lys Pro  Ser Ser Thr Val Arg  Val Phe Val
    1250                 1255                 1260

Ala Ala  Asp Pro Ala Glu Gly  Gly Thr Val Ser Val  Ala Ala Glu
    1265                 1270                 1275

Gly Glu  Thr Gly Thr Asp Thr  Ala Val Asp Val Ala  Ser Gly Ala
    1280                 1285                 1290

Ser Val  Thr Ala Lys Ala Val  Ala Ala Asp Gly Tyr  Arg Phe Ser
    1295                 1300                 1305

Gly Trp  Phe Thr Thr Ala Ser  Glu Thr Ala Val Ser  Thr Asp Ala
    1310                 1315                 1320

Thr Tyr  Thr Phe Ala Ala Asp  Gly Asn Thr Ala Leu  Thr Ala Lys
    1325                 1330                 1335

Phe Thr  Lys Asp Ser Thr Pro  Asp Pro Gly Pro Lys  Pro Thr Ile
    1340                 1345                 1350

Ser Ser  Ile Ala Val Thr Lys  Pro Thr Val Thr Asp  Tyr Lys Val
    1355                 1360                 1365

Gly Asp  Thr Phe Asp Ala Thr  Gly Leu Ala Val Thr  Ala Thr Met
    1370                 1375                 1380

Ser Asp  Gly Ser Thr Lys Thr  Leu Thr Ala Gly Glu  Tyr Thr Leu
    1385                 1390                 1395

Ser Ala  Thr Gln Asp Gly Ala  Ala Val Ala Leu Asp  Lys Ala Phe
    1400                 1405                 1410

Ala Lys  Ala Gly Lys Val Thr  Val Thr Val Thr Ala  Asn Gly Lys
    1415                 1420                 1425

Thr Ala  Thr Phe Asp Val Thr  Val Thr Ala Lys Asp  Pro Asp Pro
    1430                 1435                 1440

Glu Pro  Ala Thr Leu Lys Ser  Ile Lys Val Thr Ser  Lys Pro Asp
    1445                 1450                 1455

Lys Thr  Thr Tyr Thr Val Asp  Glu Thr Phe Ala Lys  Thr Gly Leu
    1460                 1465                 1470

Ala Val  Thr Gly Thr Trp Ser  Asp Gly Lys Thr Ala  Leu Leu Lys
```

1475 1480 1485

Asp Gly Glu Tyr Lys Leu Ser Ala Val Asp Ala Asp Gly Lys Thr
1490 1495 1500

Val Asp Leu Thr Lys Pro Phe Thr Ala Ala Gly Asp Val Thr Val
1505 1510 1515

Thr Val Thr Ser Gly Lys Leu Thr Asp Ser Phe Thr Ile Thr Val
1520 1525 1530

Lys Ala Lys Thr Val Thr Pro Ala Pro Gly Asp Asn Lys Pro Gly
1535 1540 1545

Glu Asn Lys Pro Gly Ala Asp Lys Pro Lys Pro Asn Thr Pro Asp
1550 1555 1560

Glu Val Ala Lys
1565

<210> 7
<211> 1567
<212> PRT
<213> Artificial Sequence

<220>
<223> Artificial sequence

<400> 7

Ser Asp Asp Asn Leu Ala Leu Asn Gln Thr Val Thr Ala Ser Ser Tyr
1 5 10 15

Glu Val Ala Thr Thr Ala Pro Glu Lys Ala Val Asp Gly Asp Leu Gly
20 25 30

Thr Arg Trp Gly Thr Ala Gln Asn Lys Ala Ala Asn Glu Trp Ile Glu
35 40 45

Val Gly Leu Gly Gly Thr Lys Thr Val Lys Gln Ile Asn Ile Asp Phe
50 55 60

Glu Arg Lys Asp Ala Asp Gln Asn Ile Thr Ser Phe Lys Val Glu Leu
65 70 75 80

Lys Gln Gly Asp Thr Tyr Thr Lys Val Tyr Gln Lys Asp Thr Arg Ala
85 90 95

Lys Gln Gln Glu Ile Ile Leu Leu Asp Gln Ala Gln Gln Ala Ser Ala
100 105 110

Val Lys Val Thr Val Leu Ser Ala Asp Gly Gly Thr Met Asn Trp Val
115 120 125

Asn Val Gly Ile Asn Glu Ile Ser Val Tyr Ser Ala Pro Lys Glu Thr
130 135 140

Val Leu Asp Thr Ala Asp Thr Asn His Met Leu Gly Ala Thr Met Thr
145 150 155 160

Ala Ser Ser Asn Glu Thr Ala Thr Leu Thr Pro Asp Lys Ala Ile Asp
165 170 175

Gln Asn Arg Thr Gly Arg Asn Asn Arg Trp Ala Ser Gly Tyr Glu Thr
180 185 190

Pro Ser Asn Ile Trp Leu Lys Ala Glu Phe Pro Arg Leu Thr Ala Val
195 200 205

Lys Asp Ile Arg Ile Tyr Phe Phe Glu Arg Asp Val Asn Pro Lys Pro
210 215 220

Thr Asn Val Gln Ser Phe Asp Leu Ser Tyr Thr Asp Ser Glu Gly Thr
225 230 235 240

Glu His Thr Leu Lys Ser Gly Tyr Ala Met Thr Ala Ser Gly Ala Gly
245 250 255

Tyr Val Ala Asp Val Val Ile Gln Leu Asp Gln Ala Val Asn Ala Arg
260 265 270

Ser Leu Lys Leu Ser Asn Phe Ala Ile Lys Ser Ser Glu Tyr Asn Asn
275 280 285

Val Ser Val Ala Glu Trp Glu Ala Tyr Ser Asn Asp Gln Ala Glu Pro
290 295 300

Gly Ala Thr Leu Asp Ser Val Val Ser Asp Leu Glu Ser Asn His Leu
305 310 315 320

Thr Ile Glu Thr Asp Thr Asp Thr Leu Ala Leu Pro Thr Val Pro Asp
325 330 335

Gly Tyr Thr Val Lys Phe Asn Gly Ala Asp Tyr Glu Gln Leu Ile Ala
340 345 350

Ala Asp Gly Thr Val Asn His Pro Leu Val Asp Lys Thr Val Gln Val

355 360 365

Ala Tyr Val Val Thr Asp Thr Ala Thr Gly Asn Thr Lys Thr Thr Ser
370 375 380

Asp Ile Pro Tyr Val Val Lys Gly Thr Asn Gln Gln Gln Glu Gly Asn
385 390 395 400

Asn Ala Lys Pro Thr Ile Ile Pro Glu Ile Ala Glu Trp His Ser Thr
405 410 415

Ser Ala Ala Lys Leu Ala Ala Ser Ala Val Thr Lys Val Val Tyr Asp
420 425 430

Asp Asp Ser Leu Lys Ala Val Val Asp Glu Phe Val Ala Asp Tyr Lys
435 440 445

Asp Phe Thr Gly Ile Lys Leu Thr Ala Lys Lys Gly Ala Ala Glu Ala
450 455 460

Gly Ala Phe Asn Phe Val Lys Thr Asp Ser Thr Ala Ala Ile Ala Gln
465 470 475 480

Leu Gly Asp Glu Gly Tyr Thr Met Asp Ile Arg Ala Asp Arg Val Val
485 490 495

Ala Lys Ser Ser Ser Val Thr Gly Asn Met Tyr Ala Met Gln Thr Ile
500 505 510

Leu Gln Met Thr Lys Gln Asp Ala Asn Gly Phe Val Ile Gly Ser Met
515 520 525

Arg Asp Tyr Pro Arg Phe Thr Thr Arg Gly Leu Leu Leu Asp Val Ala
530 535 540

Arg Lys Pro Val Ser Leu Glu Met Met Arg Glu Ile Thr Arg Thr Met
545 550 555 560

Arg Tyr Tyr Lys Met Asn Asp Phe Gln Ala His Leu Ser Asp Asn Tyr
565 570 575

Ile Phe Leu Glu Asn Tyr Gly Lys Gly Asp Asn Glu Asp Glu Ala Phe
580 585 590

Lys Ala Tyr Asp Ala Phe Arg Leu Glu Ser Ser Leu Thr Asn Asp Lys
595 600 605

```
Gly Glu Ser Pro Thr Ala Glu Asp Tyr Ser Ile Ser Lys Lys Thr Phe
    610                 615                 620

Lys Gln Phe Ile Gln Asp Glu Arg Ala Leu Gly Met Asn Val Val Pro
625                 630                 635                 640

Glu Ile Asp Val Pro Ala His Ala Asn Ser Phe Thr Lys Ile Trp Pro
                645                 650                 655

Glu Leu Met Val Lys Gly Arg Val Ser Pro Ile Asn Ser Asn Arg Pro
            660                 665                 670

Leu Ile Asp His Leu Asp Val Ser Lys Pro Glu Thr Ile Ala Lys Ile
        675                 680                 685

Lys Glu Ile Phe Asp Asp Tyr Thr Lys Gly Asp Asp Pro Thr Phe Asp
    690                 695                 700

Ser Asp Thr Thr Val His Ile Gly Ala Gln Glu Phe Leu Tyr Asn Tyr
705                 710                 715                 720

Thr Ala Tyr Arg Lys Phe Ile Asn Glu Ile Val Pro Tyr Ile Lys Asp
                725                 730                 735

Thr Asn Thr Val Arg Met Trp Gly Gly Leu Thr Trp Ile Asn Asp His
            740                 745                 750

Lys Thr Glu Ile Thr Lys Asp Ala Ile Glu Asn Val Glu Met Asn Leu
        755                 760                 765

Trp Ser Lys Asp Trp Ala Asp Gly Leu Gln Met Tyr Asn Met Gly Tyr
    770                 775                 780

Lys Leu Ile Asn Thr Ile Asp Asp Tyr Gly Tyr Met Val Pro Asn Gly
785                 790                 795                 800

Ser Tyr Gly Arg Ala Asn Ala Tyr Gly Asp Leu Leu Asn Ile Ser Arg
                805                 810                 815

Val Phe Asp Ser Phe Glu Pro Asn Lys Val Arg Ser Ser Gly Gly Tyr
            820                 825                 830

Gln Ala Val Pro Ser Gly Asp Asp Gln Met Leu Gly Ala Ala Phe Ala
        835                 840                 845

Ile Trp Ser Asp Asn Ile Asp Lys Ser Ala Ser Gly Leu Thr Glu Ser
    850                 855                 860
```

```
Asp Leu Tyr Trp Arg Phe Phe Asp Ala Met Pro Phe Tyr Ala Glu Lys
865                 870                 875                 880

Thr Trp Ala Ala Thr Gly Lys Glu Lys Gly Thr Ala Ala Lys Leu Thr
                885                 890                 895

Ala Leu Ala Ala Lys Gln Gly Thr Gly Pro Arg Thr Asn Pro Tyr Tyr
            900                 905                 910

Gln Ala Thr Ser Lys Asn Ser Val Tyr Glu Ser Tyr Asp Phe Asn Asp
        915                 920                 925

Gly Leu Ala Asp Ala Ser Gly Asn Gly Arg Asp Leu Thr Ile Gly Asp
    930                 935                 940

Gly Ser Lys Ala Ala Val Lys Asp Gln Ser Leu Lys Leu Ala Gly Gly
945                 950                 955                 960

Ser Ser Tyr Ala Thr Ser Lys Leu Asp Lys Leu Gly Asn Gly Asn Glu
                965                 970                 975

Leu Thr Phe Asp Val Thr Leu Gln Gln Ala Ala Lys Pro Gly Asp Ile
            980                 985                 990

Leu Phe Glu Ala Asp Ala Pro Tyr  Gly Thr His Asp Ile  Arg Val Met
        995                 1000                 1005

Glu Asn  Gly Lys Leu Gly Phe  Thr Arg Glu Leu Tyr  Asn Tyr Tyr
    1010                 1015                 1020

Phe Asp  Tyr Glu Leu Pro Val  Gly Lys Thr Val Thr  Val Thr Ile
    1025                 1030                 1035

Lys Val  Asp Gln Gln Thr Thr  Lys Leu Tyr Val Asp  Gly Glu Phe
    1040                 1045                 1050

Val Ser  Asp Ala Thr Gly Lys  Tyr Ile Asp Lys Gly  Ile Glu Lys
    1055                 1060                 1065

Lys Thr  Gly Ile Thr Ala Ala  Thr Phe Ala Leu Pro  Leu Gln Arg
    1070                 1075                 1080

Ile Gly  Ser Lys Thr Ser Ala  Ile Asn Gly Val Ile  Asp Asn Val
    1085                 1090                 1095

Ile Val  Lys Lys Ser Glu Ala  Glu Thr Asp Gln Tyr  Asn Lys Ser
    1100                 1105                 1110
```

Cys Trp Thr Gly Thr Thr Asn Ser Glu Thr Gln Tyr Asn Asp Thr
1115 1120 1125

Glu Gly Leu Leu Arg Tyr Ala Phe Asp Asn Asn Pro Ser Thr Ile
1130 1135 1140

Trp His Ser Asn Trp Lys Gly Ala Thr Asp Lys Leu Thr Gly Ser
1145 1150 1155

Asn Ser Phe Tyr Ala Glu Ile Asp Met Cys Gln Lys Tyr Thr Ile
1160 1165 1170

Asn Gln Phe Ser Phe Thr Pro Arg Thr Ser Gln Asp Ser Gly Gln
1175 1180 1185

Val Thr Lys Ala Asp Leu Tyr Val Lys Ala Asn Ala Asn Asp Glu
1190 1195 1200

Trp Lys Gln Val Ala Thr Asp Gln Val Phe Glu Ala Ser Arg Ala
1205 1210 1215

Lys Lys Thr Phe Met Phe Asp Glu Gln Glu Val Arg Tyr Val Lys
1220 1225 1230

Phe Val Ala Lys Ser Ser Asn Asp Gly Trp Val Ala Val Ser Glu
1235 1240 1245

Phe Gly Val Ala Asn Lys Pro Ser Ser Thr Val Arg Val Phe Val
1250 1255 1260

Ala Ala Asp Pro Ala Glu Gly Gly Thr Val Ser Val Ala Ala Glu
1265 1270 1275

Gly Glu Thr Gly Thr Asp Thr Ala Val Asp Val Ala Ser Gly Ala
1280 1285 1290

Ser Val Thr Ala Lys Ala Val Ala Ala Asp Gly Tyr Arg Phe Ser
1295 1300 1305

Gly Trp Phe Thr Thr Ala Ser Glu Thr Ala Val Ser Thr Asp Ala
1310 1315 1320

Thr Tyr Thr Phe Ala Ala Asp Gly Asn Thr Ala Leu Thr Ala Lys
1325 1330 1335

Phe Thr Lys Asp Ser Thr Pro Asp Pro Gly Pro Lys Pro Thr Ile

1340 1345 1350

Ser Ser Ile Ala Val Thr Lys Pro Thr Val Thr Asp Tyr Lys Val
1355 1360 1365

Gly Asp Thr Phe Asp Ala Thr Gly Leu Ala Val Thr Ala Thr Met
1370 1375 1380

Ser Asp Gly Ser Thr Lys Thr Leu Thr Ala Gly Glu Tyr Thr Leu
1385 1390 1395

Ser Ala Thr Gln Asp Gly Ala Ala Val Ala Leu Asp Lys Ala Phe
1400 1405 1410

Ala Lys Ala Gly Lys Val Thr Val Thr Val Thr Ala Asn Gly Lys
1415 1420 1425

Thr Ala Thr Phe Asp Val Thr Val Thr Ala Lys Asp Pro Asp Pro
1430 1435 1440

Glu Pro Ala Thr Leu Lys Ser Ile Lys Val Thr Ser Lys Pro Asp
1445 1450 1455

Lys Thr Thr Tyr Thr Val Asp Glu Thr Phe Ala Lys Thr Gly Leu
1460 1465 1470

Ala Val Thr Gly Thr Trp Ser Asp Gly Lys Thr Ala Leu Leu Lys
1475 1480 1485

Asp Gly Glu Tyr Lys Leu Ser Ala Val Asp Ala Asp Gly Lys Thr
1490 1495 1500

Val Asp Leu Thr Lys Pro Phe Thr Ala Ala Gly Asp Val Thr Val
1505 1510 1515

Thr Val Thr Ser Gly Lys Leu Thr Asp Ser Phe Thr Ile Thr Val
1520 1525 1530

Lys Ala Lys Thr Val Thr Pro Ala Pro Gly Asp Asn Lys Pro Gly
1535 1540 1545

Glu Asn Lys Pro Gly Ala Asp Lys Pro Lys Pro Asn Thr Pro Asp
1550 1555 1560

Glu Val Ala Lys
1565

```
<210>  8
<211>  1567
<212>  PRT
<213>  Artificial Sequence

<220>
<223>  Artificial sequence

<400>  8

Ser Asp Asp Asn Leu Ala Leu Asn Gln Thr Val Thr Ala Ser Ser Tyr
1               5                   10                  15

Glu Val Ala Thr Thr Ala Pro Glu Lys Ala Val Asp Gly Asp Leu Gly
            20                  25                  30

Thr Arg Trp Gly Thr Ala Gln Asn Lys Ala Ala Asn Glu Trp Ile Glu
        35                  40                  45

Val Gly Leu Gly Gly Thr Lys Thr Val Lys Gln Ile Asn Ile Asp Phe
    50                  55                  60

Glu Arg Lys Asp Ala Asp Gln Asn Ile Thr Ser Phe Lys Val Glu Leu
65                  70                  75                  80

Lys Gln Gly Asp Thr Tyr Thr Lys Val Tyr Gln Lys Asp Thr Arg Ala
                85                  90                  95

Lys Gln Gln Glu Ile Ile Leu Leu Asp Gln Ala Gln Gln Ala Ser Ala
            100                 105                 110

Val Lys Val Thr Val Leu Ser Ala Asp Gly Gly Thr Met Asn Trp Val
        115                 120                 125

Asn Val Gly Ile Asn Glu Ile Ser Val Tyr Ser Ala Pro Lys Glu Thr
    130                 135                 140

Val Leu Asp Thr Ala Asp Thr Asn His Met Leu Gly Ala Thr Met Thr
145                 150                 155                 160

Ala Ser Ser Asn Glu Thr Ala Thr Leu Thr Pro Asp Lys Ala Ile Asp
                165                 170                 175

Gln Asn Arg Thr Gly Arg Asn Asn Arg Trp Ala Ser Gly Tyr Glu Thr
            180                 185                 190

Pro Ser Asn Ile Trp Leu Lys Ala Glu Phe Pro Arg Leu Thr Ala Val
        195                 200                 205

Lys Asp Ile Arg Ile Tyr Phe Phe Glu Arg Asp Val Asn Pro Lys Pro
```

```
        210                 215                 220

Thr Asn Val Gln Ser Phe Asp Leu Ser Tyr Thr Asp Ser Glu Gly Thr
225                 230                 235                 240

Glu His Thr Leu Lys Ser Gly Tyr Ala Met Thr Ala Ser Gly Ala Gly
                245                 250                 255

Tyr Val Ala Asp Val Val Ile Gln Leu Asp Gln Ala Val Asn Ala Arg
            260                 265                 270

Ser Leu Lys Leu Ser Asn Phe Ala Ile Lys Ser Ser Glu Tyr Asn Asn
        275                 280                 285

Val Ser Val Ala Glu Trp Glu Ala Tyr Ser Asn Asp Gln Ala Glu Pro
    290                 295                 300

Gly Ala Thr Leu Asp Ser Val Val Ser Asp Leu Glu Ser Asn His Leu
305                 310                 315                 320

Thr Ile Glu Thr Asp Thr Asp Thr Leu Ala Leu Pro Thr Val Pro Asp
                325                 330                 335

Gly Tyr Thr Val Lys Phe Asn Gly Ala Asp Tyr Glu Gln Leu Ile Ala
            340                 345                 350

Ala Asp Gly Thr Val Asn His Pro Leu Val Asp Lys Thr Val Gln Val
        355                 360                 365

Ala Tyr Val Val Thr Asp Thr Ala Thr Gly Asn Thr Lys Thr Thr Ser
    370                 375                 380

Asp Ile Pro Tyr Val Val Lys Gly Thr Asn Gln Gln Gln Glu Gly Asn
385                 390                 395                 400

Asn Ala Lys Pro Thr Ile Ile Pro Glu Ile Ala Glu Trp His Ser Thr
                405                 410                 415

Ser Ala Ala Lys Leu Ala Ala Ser Ala Val Thr Lys Val Val Tyr Asp
            420                 425                 430

Asp Asp Ser Leu Lys Ala Val Val Asp Glu Phe Val Ala Asp Tyr Lys
        435                 440                 445

Asp Phe Thr Gly Ile Lys Leu Thr Ala Lys Lys Gly Ala Ala Glu Ala
    450                 455                 460
```

```
Gly Ala Phe Asn Phe Val Lys Thr Asp Ser Thr Ala Ala Ile Ala Gln
465                 470                 475                 480

Leu Gly Asp Glu Gly Tyr Thr Met Asp Ile Arg Ala Asp Arg Val Val
                485                 490                 495

Ala Lys Ser Ser Ser Val Thr Gly Asn Met Tyr Ala Met Gln Thr Ile
            500                 505                 510

Leu Gln Met Thr Lys Gln Asp Ala Asn Gly Phe Val Ile Gly Ser Met
        515                 520                 525

Arg Asp Tyr Pro Arg Phe Thr Thr Arg Gly Leu Leu Leu Asp Val Ala
    530                 535                 540

Arg Lys Pro Val Ser Leu Glu Met Met Arg Glu Ile Thr Arg Thr Met
545                 550                 555                 560

Arg Tyr Tyr Lys Met Asn Asp Phe Gln Ala His Leu Ser Asp Asn Tyr
                565                 570                 575

Ile Phe Leu Glu Asn Tyr Gly Lys Gly Asp Asn Glu Asp Glu Ala Phe
            580                 585                 590

Lys Ala Tyr Asp Ala Phe Arg Leu Glu Ser Ser Leu Thr Asn Asp Lys
        595                 600                 605

Gly Glu Ser Pro Thr Ala Glu Asp Tyr Ser Ile Ser Lys Lys Thr Phe
    610                 615                 620

Lys Gln Phe Ile Gln Asp Glu Arg Ala Leu Gly Met Asn Val Val Pro
625                 630                 635                 640

Glu Ile Asp Val Pro Ala His Ala Asn Ser Phe Thr Lys Ile Trp Pro
                645                 650                 655

Glu Leu Met Val Lys Gly Arg Val Ser Pro Ile Asn Ser Asn Arg Pro
            660                 665                 670

Leu Ile Asp His Leu Asp Val Ser Lys Pro Glu Thr Ile Ala Lys Ile
        675                 680                 685

Lys Glu Ile Phe Asp Asp Tyr Thr Lys Gly Asp Asp Pro Thr Phe Asp
    690                 695                 700

Ser Asp Thr Thr Val His Ile Gly Ala Asp Glu Phe Leu Tyr Asn Tyr
705                 710                 715                 720
```

```
Thr Ala Tyr Arg Lys Phe Ile Asn Glu Ile Val Pro Tyr Ile Lys Asp
                725                 730                 735

Thr Asn Thr Val Arg Met Trp Gly Gly Leu Thr Trp Ile Asn Asp His
            740                 745                 750

Lys Thr Glu Ile Thr Lys Asp Ala Ile Glu Asn Val Glu Met Asn Leu
        755                 760                 765

Trp Ser Lys Asp Trp Ala Asp Gly Leu Gln Met Tyr Asn Met Gly Tyr
    770                 775                 780

Lys Leu Ile Asn Thr Ile Asp Asp Tyr Gly Phe Met Val Pro Asn Gly
785                 790                 795                 800

Ser Tyr Gly Arg Ala Asn Ala Tyr Gly Asp Leu Leu Asn Ile Ser Arg
                805                 810                 815

Val Phe Asp Ser Phe Glu Pro Asn Lys Val Arg Ser Ser Gly Gly Tyr
            820                 825                 830

Gln Ala Val Pro Ser Gly Asp Asp Gln Met Leu Gly Ala Ala Phe Ala
        835                 840                 845

Ile Trp Ser Asp Asn Ile Asp Lys Ser Ala Ser Gly Leu Thr Glu Ser
    850                 855                 860

Asp Leu Tyr Trp Arg Phe Phe Asp Ala Met Pro Phe Tyr Ala Glu Lys
865                 870                 875                 880

Thr Trp Ala Ala Thr Gly Lys Glu Lys Gly Thr Ala Ala Lys Leu Thr
                885                 890                 895

Ala Leu Ala Ala Lys Gln Gly Thr Gly Pro Arg Thr Asn Pro Tyr Tyr
            900                 905                 910

Gln Ala Thr Ser Lys Asn Ser Val Tyr Glu Ser Tyr Asp Phe Asn Asp
        915                 920                 925

Gly Leu Ala Asp Ala Ser Gly Asn Gly Arg Asp Leu Thr Ile Gly Asp
    930                 935                 940

Gly Ser Lys Ala Ala Val Lys Asp Gln Ser Leu Lys Leu Ala Gly Gly
945                 950                 955                 960

Ser Ser Tyr Ala Thr Ser Lys Leu Asp Lys Leu Gly Asn Gly Asn Glu
                965                 970                 975
```

```
Leu Thr Phe Asp Val Thr Leu Gln Gln Ala Ala Lys Pro Gly Asp Ile
            980                 985                 990

Leu Phe Glu Ala Asp Ala Pro Tyr  Gly Thr His Asp Ile  Arg Val Met
        995                 1000                 1005

Glu Asn  Gly Lys Leu Gly Phe  Thr Arg Glu Leu Tyr  Asn Tyr Tyr
    1010                 1015                 1020

Phe Asp  Tyr Glu Leu Pro Val  Gly Lys Thr Val Thr  Val Thr Ile
    1025                 1030                 1035

Lys Val  Asp Gln Gln Thr Thr  Lys Leu Tyr Val Asp  Gly Glu Phe
    1040                 1045                 1050

Val Ser  Asp Ala Thr Gly Lys  Tyr Ile Asp Lys Gly  Ile Glu Lys
    1055                 1060                 1065

Lys Thr  Gly Ile Thr Ala Ala  Thr Phe Ala Leu Pro  Leu Gln Arg
    1070                 1075                 1080

Ile Gly  Ser Lys Thr Ser Ala  Ile Asn Gly Val Ile  Asp Asn Val
    1085                 1090                 1095

Ile Val  Lys Lys Ser Glu Ala  Glu Thr Asp Gln Tyr  Asn Lys Ser
    1100                 1105                 1110

Cys Trp  Thr Gly Thr Thr Asn  Ser Glu Thr Gln Tyr  Asn Asp Thr
    1115                 1120                 1125

Glu Gly  Leu Leu Arg Tyr Ala  Phe Asp Asn Asn Pro  Ser Thr Ile
    1130                 1135                 1140

Trp His  Ser Asn Trp Lys Gly  Ala Thr Asp Lys Leu  Thr Gly Ser
    1145                 1150                 1155

Asn Ser  Phe Tyr Ala Glu Ile  Asp Met Cys Gln Lys  Tyr Thr Ile
    1160                 1165                 1170

Asn Gln  Phe Ser Phe Thr Pro  Arg Thr Ser Gln Asp  Ser Gly Gln
    1175                 1180                 1185

Val Thr  Lys Ala Asp Leu Tyr  Val Lys Ala Asn Ala  Asn Asp Glu
    1190                 1195                 1200

Trp Lys  Gln Val Ala Thr Asp  Gln Val Phe Glu Ala  Ser Arg Ala
```

1205 1210 1215

Lys Lys Thr Phe Met Phe Asp Glu Gln Glu Val Arg Tyr Val Lys
1220 1225 1230

Phe Val Ala Lys Ser Ser Asn Asp Gly Trp Val Ala Val Ser Glu
1235 1240 1245

Phe Gly Val Ala Asn Lys Pro Ser Ser Thr Val Arg Val Phe Val
1250 1255 1260

Ala Ala Asp Pro Ala Glu Gly Gly Thr Val Ser Val Ala Ala Glu
1265 1270 1275

Gly Glu Thr Gly Thr Asp Thr Ala Val Asp Val Ala Ser Gly Ala
1280 1285 1290

Ser Val Thr Ala Lys Ala Val Ala Ala Asp Gly Tyr Arg Phe Ser
1295 1300 1305

Gly Trp Phe Thr Thr Ala Ser Glu Thr Ala Val Ser Thr Asp Ala
1310 1315 1320

Thr Tyr Thr Phe Ala Ala Asp Gly Asn Thr Ala Leu Thr Ala Lys
1325 1330 1335

Phe Thr Lys Asp Ser Thr Pro Asp Pro Gly Pro Lys Pro Thr Ile
1340 1345 1350

Ser Ser Ile Ala Val Thr Lys Pro Thr Val Thr Asp Tyr Lys Val
1355 1360 1365

Gly Asp Thr Phe Asp Ala Thr Gly Leu Ala Val Thr Ala Thr Met
1370 1375 1380

Ser Asp Gly Ser Thr Lys Thr Leu Thr Ala Gly Glu Tyr Thr Leu
1385 1390 1395

Ser Ala Thr Gln Asp Gly Ala Ala Val Ala Leu Asp Lys Ala Phe
1400 1405 1410

Ala Lys Ala Gly Lys Val Thr Val Thr Val Thr Ala Asn Gly Lys
1415 1420 1425

Thr Ala Thr Phe Asp Val Thr Val Thr Ala Lys Asp Pro Asp Pro
1430 1435 1440

Glu Pro Ala Thr Leu Lys Ser Ile Lys Val Thr Ser Lys Pro Asp
1445 1450 1455

Lys Thr Thr Tyr Thr Val Asp Glu Thr Phe Ala Lys Thr Gly Leu
1460 1465 1470

Ala Val Thr Gly Thr Trp Ser Asp Gly Lys Thr Ala Leu Leu Lys
1475 1480 1485

Asp Gly Glu Tyr Lys Leu Ser Ala Val Asp Ala Asp Gly Lys Thr
1490 1495 1500

Val Asp Leu Thr Lys Pro Phe Thr Ala Ala Gly Asp Val Thr Val
1505 1510 1515

Thr Val Thr Ser Gly Lys Leu Thr Asp Ser Phe Thr Ile Thr Val
1520 1525 1530

Lys Ala Lys Thr Val Thr Pro Ala Pro Gly Asp Asn Lys Pro Gly
1535 1540 1545

Glu Asn Lys Pro Gly Ala Asp Lys Pro Lys Pro Asn Thr Pro Asp
1550 1555 1560

Glu Val Ala Lys
1565

<210> 9
<211> 1030
<212> PRT
<213> Artificial Sequence

<220>
<223> Artificial sequence

<400> 9

Ala Asp Asp Ser Ala Ala Gly Tyr Ser Ala Thr Ala Pro Val Asn Leu
1 5 10 15

Thr Arg Pro Ala Thr Val Pro Ser Met Asp Gly Trp Thr Asp Gly Thr
20 25 30

Gly Ala Trp Thr Leu Gly Glu Gly Thr Arg Val Val Ser Ser Asp Ala
35 40 45

Leu Ala Ala Arg Ala Gln Ser Leu Ala Ser Glu Leu Thr Lys Phe Thr
50 55 60

Asp Val Asp Ile Lys Ala Ala Thr Gly Ser Ala Thr Gly Lys Asp Ile

```
65                  70                  75                  80

Ser Leu Thr Leu Asp Ala Ser Lys Lys Ala Glu Leu Gly Asp Glu Gly
                85                  90                  95

Phe Lys Leu Asn Ile Gly Ser Lys Gly Leu Glu Val Ile Gly Ala Thr
            100                 105                 110

Asp Ile Gly Val Phe Tyr Gly Thr Arg Ser Val Ser Gln Met Leu Arg
        115                 120                 125

Gln Gly Gln Leu Thr Leu Pro Ala Gly Thr Val Ala Thr Lys Pro Lys
    130                 135                 140

Tyr Lys Glu Arg Gly Ala Thr Leu Cys Ala Cys Gln Ile Asn Ile Ser
145                 150                 155                 160

Thr Asp Trp Ile Asp Arg Phe Leu Ser Asp Met Ala Asp Leu Arg Leu
                165                 170                 175

Asn Tyr Val Leu Leu Glu Met Lys Leu Lys Pro Glu Glu Asp Asn Thr
            180                 185                 190

Lys Lys Ala Ala Thr Trp Ser Tyr Tyr Thr Arg Asp Asp Val Lys Lys
        195                 200                 205

Phe Val Lys Lys Ala Asn Asn Tyr Gly Ile Asp Val Ile Pro Glu Ile
    210                 215                 220

Asn Ser Pro Gly His Met Asn Val Trp Leu Glu Asn Tyr Pro Glu Tyr
225                 230                 235                 240

Gln Leu Ala Asp Asn Ser Gly Arg Lys Asp Pro Asn Lys Leu Asp Ile
                245                 250                 255

Ser Asn Pro Glu Ala Val Lys Phe Tyr Lys Thr Leu Ile Asp Glu Tyr
            260                 265                 270

Asp Gly Val Phe Thr Thr Lys Tyr Trp His Met Gly Ala Glu Glu Tyr
        275                 280                 285

Met Ile Gly Thr Ser Phe Asp Asn Tyr Ser Lys Leu Lys Thr Phe Ala
    290                 295                 300

Glu Lys Gln Tyr Gly Ala Gly Ala Thr Pro Asn Asp Ala Phe Thr Gly
305                 310                 315                 320
```

```
Phe Ile Asn Asp Ile Asp Lys Tyr Val Lys Ala Lys Gly Lys Gln Leu
                325                 330                 335

Arg Ile Trp Asn Asp Gly Ile Val Asn Thr Lys Asn Val Ser Leu Asn
            340                 345                 350

Lys Asp Ile Val Ile Glu Tyr Trp Tyr Gly Ala Gly Arg Lys Pro Gln
        355                 360                 365

Glu Leu Val Gln Asp Gly Tyr Thr Leu Met Asn Ala Thr Gln Ala Leu
    370                 375                 380

Tyr Trp Ser Arg Ser Ala Gln Val Tyr Lys Val Asn Ala Ala Arg Leu
385                 390                 395                 400

Tyr Asn Asn Asn Trp Asn Val Gly Thr Phe Asp Gly Gly Arg Gln Ile
                405                 410                 415

Asp Lys Asn Tyr Asp Lys Leu Thr Gly Ala Lys Val Ser Ile Trp Pro
            420                 425                 430

Asp Ser Ser Tyr Phe Gln Thr Glu Asn Glu Val Glu Lys Glu Ile Phe
        435                 440                 445

Asp Gly Met Arg Phe Ile Ser Gln Met Thr Trp Ser Asp Ser Arg Pro
    450                 455                 460

Trp Ala Thr Trp Asn Asp Met Lys Ala Asp Ile Asp Lys Ile Gly Tyr
465                 470                 475                 480

Pro Leu Asp Ile Arg Glu Tyr Asp Tyr Thr Pro Val Asp Ala Gly Ile
                485                 490                 495

Tyr Asp Ile Pro Gln Leu Lys Ser Ile Ser Lys Gly Pro Trp Glu Leu
            500                 505                 510

Ile Thr Thr Pro Asp Gly Tyr Tyr Gln Met Lys Asp Thr Val Ser Gly
        515                 520                 525

Lys Cys Leu Ala Leu Phe Thr Gly Ser Lys His Leu Asp Val Val Thr
    530                 535                 540

Gln Val Gly Ala Arg Pro Glu Leu Arg Asn Cys Ala Asp Val Ser Val
545                 550                 555                 560

Gly Gln Asp Gln Arg Asn Thr Ala Asn Glu Arg Asn Thr Gln Lys Trp
                565                 570                 575
```

```
Gln Ile Arg Ala Asp Lys Asp Gly Lys Tyr Thr Ile Ser Pro Ala Leu
            580                 585                 590

Thr Gln Gln Arg Leu Ala Ile Ala Thr Gly Asn Glu Gln Asn Ile Asp
        595                 600                 605

Leu Glu Thr His Arg Pro Ala Ala Gly Thr Val Ala Gln Phe Pro Ala
    610                 615                 620

Asp Leu Val Ser Asp Asn Ala Leu Phe Thr Leu Thr Gly His Met Gly
625                 630                 635                 640

Met Ser Ala Thr Val Asp Ser Lys Thr Val Asn Pro Ala Ser Pro Ser
                645                 650                 655

Lys Ile Thr Val Lys Val Arg Ala Ala Ser Asn Ala Asn Thr Gly Asp
            660                 665                 670

Val Thr Val Thr Pro Val Val Pro Glu Gly Trp Glu Ile Lys Pro Gly
        675                 680                 685

Ser Val Ser Leu Lys Ser Ile Pro Ala Gly Lys Ala Ala Ile Ala Tyr
    690                 695                 700

Phe Asn Val Val Asn Thr Thr Gly Thr Gly Asp Ala Thr Val Gln Phe
705                 710                 715                 720

Lys Leu Thr Asn Thr Lys Thr Gly Glu Glu Leu Gly Thr Thr Ser Val
                725                 730                 735

Ala Leu Thr Gly Ser Leu Thr Lys Asp Val Glu Ala Ser Asp Tyr Ala
            740                 745                 750

Ala Ser Ser Gln Glu Thr Thr Gly Glu His Ala Pro Val Gly Asn Ala
        755                 760                 765

Phe Asp Lys Asn Ala Asn Thr Phe Trp His Ser Lys Tyr Ser Asn Pro
    770                 775                 780

Ser Ala Asn Leu Pro His Trp Leu Ala Phe Lys Ala Ser Pro Gly Glu
785                 790                 795                 800

Gly Asn Lys Ile Ala Ala Ile Thr His Leu Tyr Arg Gln Asp Lys Leu
                805                 810                 815

Asn Gly Pro Ala Lys Asn Val Ala Val Tyr Val Val Ala Ala Ser Asp
            820                 825                 830
```

```
Ala Asn Ser Val Ala Asp Val Thr Asn Trp Gly Glu Pro Val Ala Thr
        835                 840                 845

Ala Glu Phe Pro Tyr Thr Lys Glu Leu Gln Thr Ile Ala Leu Pro Asn
    850                 855                 860

Thr Ile Pro Ser Gly Asp Val Tyr Val Lys Phe Gln Ile Asn Asp Ala
865                 870                 875                 880

Trp Gly Leu Thr Glu Thr Ser Ala Gly Val Thr Trp Ala Ala Val Ala
                885                 890                 895

Glu Leu Ala Ala Thr Ala Lys Ala Thr Pro Val Glu Leu Thr Glu Pro
            900                 905                 910

Glu Gln Pro Lys Asp Asn Pro Glu Val Thr Glu Thr Pro Glu Ala Thr
        915                 920                 925

Gly Val Thr Val Ser Gly Asp Gly Val Ala Asn Gly Ala Leu Ser Leu
    930                 935                 940

Lys Lys Gly Thr Thr Ala Gln Leu Thr Ala Lys Val Ala Pro Asp Asp
945                 950                 955                 960

Ala Asp Gln Ala Val Thr Trp Ala Ser Ser Asp Asp Lys Val Val Thr
                965                 970                 975

Val Asp Lys Thr Gly Lys Val Thr Ala Val Ala Lys Gly Val Ala Lys
            980                 985                 990

Val Thr Ala Thr Thr Ala Asn Gly  Lys Ser Ala Ser Val  Thr Val Thr
        995                 1000                 1005

Val Thr  Glu Asp Ser Glu Val  Pro Gly Pro Thr Gly  Pro Thr Glu
    1010                 1015                 1020

Pro Thr  Lys Pro Gly Thr Glu
    1025                 1030
```

```
<210>  10
<211>  1030
<212>  PRT
<213>  Artificial Sequence

<220>
<223>  Artificial sequence

<400>  10
```

```
Ala Asp Asp Ser Ala Ala Gly Tyr Ser Ala Thr Ala Pro Val Asn Leu
1               5                   10                  15

Thr Arg Pro Ala Thr Val Pro Ser Met Asp Gly Trp Thr Asp Gly Thr
            20                  25                  30

Gly Ala Trp Thr Leu Gly Glu Gly Thr Arg Val Val Ser Ser Asp Ala
        35                  40                  45

Leu Ala Ala Arg Ala Gln Ser Leu Ala Ser Glu Leu Thr Lys Phe Thr
    50                  55                  60

Asp Val Asp Ile Lys Ala Ala Thr Gly Ser Ala Thr Gly Lys Asp Ile
65                  70                  75                  80

Ser Leu Thr Leu Asp Ala Ser Lys Lys Ala Glu Leu Gly Asp Glu Gly
                85                  90                  95

Phe Lys Leu Asn Ile Gly Ser Lys Gly Leu Glu Val Ile Gly Ala Thr
            100                 105                 110

Asp Ile Gly Val Phe Tyr Gly Thr Arg Ser Val Ser Gln Met Leu Arg
        115                 120                 125

Gln Gly Gln Leu Thr Leu Pro Ala Gly Thr Val Ala Thr Lys Pro Lys
    130                 135                 140

Tyr Lys Glu Arg Gly Ala Thr Leu Cys Ala Cys Gln Ile Asn Ile Ser
145                 150                 155                 160

Thr Asp Trp Ile Asp Arg Phe Leu Ser Asp Met Ala Asp Leu Arg Leu
                165                 170                 175

Asn Tyr Val Leu Leu Glu Met Lys Leu Lys Pro Glu Glu Asp Asn Thr
            180                 185                 190

Lys Lys Ala Ala Thr Trp Ser Tyr Tyr Thr Arg Asp Asp Val Lys Lys
        195                 200                 205

Phe Val Lys Lys Ala Asn Asn Tyr Gly Ile Asp Val Ile Pro Glu Ile
    210                 215                 220

Asn Ser Pro Gly His Met Asn Val Trp Leu Glu Asn Tyr Pro Glu Tyr
225                 230                 235                 240

Gln Leu Ala Asp Asn Ser Gly Arg Lys Asp Pro Asn Lys Leu Asp Ile
                245                 250                 255
```

```
Ser Asn Pro Glu Ala Val Lys Phe Tyr Lys Thr Leu Ile Asp Glu Tyr
            260                 265                 270

Asp Gly Val Phe Thr Thr Lys Tyr Trp His Met Gly Ala Ala Glu Tyr
        275                 280                 285

Met Ile Gly Thr Ser Phe Asp Asn Tyr Ser Lys Leu Lys Thr Phe Ala
    290                 295                 300

Glu Lys Gln Tyr Gly Ala Gly Ala Thr Pro Asn Asp Ala Phe Thr Gly
305                 310                 315                 320

Phe Ile Asn Asp Ile Asp Lys Tyr Val Lys Ala Lys Gly Lys Gln Leu
                325                 330                 335

Arg Ile Trp Asn Asp Gly Ile Val Asn Thr Lys Asn Val Ser Leu Asn
            340                 345                 350

Lys Asp Ile Val Ile Glu Tyr Trp Tyr Gly Ala Gly Arg Lys Pro Gln
        355                 360                 365

Glu Leu Val Gln Asp Gly Tyr Thr Leu Met Asn Ala Thr Gln Ala Leu
    370                 375                 380

Tyr Trp Ser Arg Ser Ala Gln Val Tyr Lys Val Asn Ala Ala Arg Leu
385                 390                 395                 400

Tyr Asn Asn Asn Trp Asn Val Gly Thr Phe Asp Gly Gly Arg Gln Ile
                405                 410                 415

Asp Lys Asn Tyr Asp Lys Leu Thr Gly Ala Lys Val Ser Ile Trp Pro
            420                 425                 430

Asp Ser Ser Tyr Phe Gln Thr Glu Asn Glu Val Glu Lys Glu Ile Phe
        435                 440                 445

Asp Gly Met Arg Phe Ile Ser Gln Met Thr Trp Ser Asp Ser Arg Pro
    450                 455                 460

Trp Ala Thr Trp Asn Asp Met Lys Ala Asp Ile Asp Lys Ile Gly Tyr
465                 470                 475                 480

Pro Leu Asp Ile Arg Glu Tyr Asp Tyr Thr Pro Val Asp Ala Gly Ile
                485                 490                 495

Tyr Asp Ile Pro Gln Leu Lys Ser Ile Ser Lys Gly Pro Trp Glu Leu
```

500 505 510

Ile Thr Thr Pro Asp Gly Tyr Tyr Gln Met Lys Asp Thr Val Ser Gly
515 520 525

Lys Cys Leu Ala Leu Phe Thr Gly Ser Lys His Leu Asp Val Val Thr
530 535 540

Gln Val Gly Ala Arg Pro Glu Leu Arg Asn Cys Ala Asp Val Ser Val
545 550 555 560

Gly Gln Asp Gln Arg Asn Thr Ala Asn Glu Arg Asn Thr Gln Lys Trp
565 570 575

Gln Ile Arg Ala Asp Lys Asp Gly Lys Tyr Thr Ile Ser Pro Ala Leu
580 585 590

Thr Gln Gln Arg Leu Ala Ile Ala Thr Gly Asn Glu Gln Asn Ile Asp
595 600 605

Leu Glu Thr His Arg Pro Ala Ala Gly Thr Val Ala Gln Phe Pro Ala
610 615 620

Asp Leu Val Ser Asp Asn Ala Leu Phe Thr Leu Thr Gly His Met Gly
625 630 635 640

Met Ser Ala Thr Val Asp Ser Lys Thr Val Asn Pro Ala Ser Pro Ser
645 650 655

Lys Ile Thr Val Lys Val Arg Ala Ala Ser Asn Ala Asn Thr Gly Asp
660 665 670

Val Thr Val Thr Pro Val Val Pro Glu Gly Trp Glu Ile Lys Pro Gly
675 680 685

Ser Val Ser Leu Lys Ser Ile Pro Ala Gly Lys Ala Ala Ile Ala Tyr
690 695 700

Phe Asn Val Val Asn Thr Thr Gly Thr Gly Asp Ala Thr Val Gln Phe
705 710 715 720

Lys Leu Thr Asn Thr Lys Thr Gly Glu Glu Leu Gly Thr Thr Ser Val
725 730 735

Ala Leu Thr Gly Ser Leu Thr Lys Asp Val Glu Ala Ser Asp Tyr Ala
740 745 750

Ala Ser Ser Gln Glu Thr Thr Gly Glu His Ala Pro Val Gly Asn Ala
755 760 765

Phe Asp Lys Asn Ala Asn Thr Phe Trp His Ser Lys Tyr Ser Asn Pro
770 775 780

Ser Ala Asn Leu Pro His Trp Leu Ala Phe Lys Ala Ser Pro Gly Glu
785 790 795 800

Gly Asn Lys Ile Ala Ala Ile Thr His Leu Tyr Arg Gln Asp Lys Leu
805 810 815

Asn Gly Pro Ala Lys Asn Val Ala Val Tyr Val Val Ala Ala Ser Asp
820 825 830

Ala Asn Ser Val Ala Asp Val Thr Asn Trp Gly Glu Pro Val Ala Thr
835 840 845

Ala Glu Phe Pro Tyr Thr Lys Glu Leu Gln Thr Ile Ala Leu Pro Asn
850 855 860

Thr Ile Pro Ser Gly Asp Val Tyr Val Lys Phe Gln Ile Asn Asp Ala
865 870 875 880

Trp Gly Leu Thr Glu Thr Ser Ala Gly Val Thr Trp Ala Ala Val Ala
885 890 895

Glu Leu Ala Ala Thr Ala Lys Ala Thr Pro Val Glu Leu Thr Glu Pro
900 905 910

Glu Gln Pro Lys Asp Asn Pro Glu Val Thr Glu Thr Pro Glu Ala Thr
915 920 925

Gly Val Thr Val Ser Gly Asp Gly Val Ala Asn Gly Ala Leu Ser Leu
930 935 940

Lys Lys Gly Thr Thr Ala Gln Leu Thr Ala Lys Val Ala Pro Asp Asp
945 950 955 960

Ala Asp Gln Ala Val Thr Trp Ala Ser Ser Asp Asp Lys Val Val Thr
965 970 975

Val Asp Lys Thr Gly Lys Val Thr Ala Val Ala Lys Gly Val Ala Lys
980 985 990

Val Thr Ala Thr Thr Ala Asn Gly Lys Ser Ala Ser Val Thr Val Thr
995 1000 1005

```
Val Thr  Glu Asp Ser Glu Val  Pro Gly Pro Thr Gly  Pro Thr Glu
    1010                 1015                 1020

Pro Thr  Lys Pro Gly Thr Glu
    1025                 1030


<210>  11
<211>  1030
<212>  PRT
<213>  Artificial Sequence

<220>
<223>  Artificial sequence

<400>  11

Ala Asp Asp Ser Ala Ala Gly Tyr Ser Ala Thr Ala Pro Val Asn Leu
1               5                   10                  15

Thr Arg Pro Ala Thr Val Pro Ser Met Asp Gly Trp Thr Asp Gly Thr
            20                  25                  30

Gly Ala Trp Thr Leu Gly Glu Gly Thr Arg Val Val Ser Ser Asp Ala
        35                  40                  45

Leu Ala Ala Arg Ala Gln Ser Leu Ala Ser Glu Leu Thr Lys Phe Thr
    50                  55                  60

Asp Val Asp Ile Lys Ala Ala Thr Gly Ser Ala Thr Gly Lys Asp Ile
65                  70                  75                  80

Ser Leu Thr Leu Asp Ala Ser Lys Lys Ala Glu Leu Gly Asp Glu Gly
                85                  90                  95

Phe Lys Leu Asn Ile Gly Ser Lys Gly Leu Glu Val Ile Gly Ala Thr
            100                 105                 110

Asp Ile Gly Val Phe Tyr Gly Thr Arg Ser Val Ser Gln Met Leu Arg
        115                 120                 125

Gln Gly Gln Leu Thr Leu Pro Ala Gly Thr Val Ala Thr Lys Pro Lys
    130                 135                 140

Tyr Lys Glu Arg Gly Ala Thr Leu Cys Ala Cys Gln Ile Asn Ile Ser
145                 150                 155                 160

Thr Asp Trp Ile Asp Arg Phe Leu Ser Asp Met Ala Asp Leu Arg Leu
                165                 170                 175
```

Asn Tyr Val Leu Leu Glu Met Lys Leu Lys Pro Glu Glu Asp Asn Thr
180 185 190

Lys Lys Ala Ala Thr Trp Ser Tyr Tyr Thr Arg Asp Asp Val Lys Lys
195 200 205

Phe Val Lys Lys Ala Asn Asn Tyr Gly Ile Asp Val Ile Pro Glu Ile
210 215 220

Asn Ser Pro Gly His Met Asn Val Trp Leu Glu Asn Tyr Pro Glu Tyr
225 230 235 240

Gln Leu Ala Asp Asn Ser Gly Arg Lys Asp Pro Asn Lys Leu Asp Ile
245 250 255

Ser Asn Pro Glu Ala Val Lys Phe Tyr Lys Thr Leu Ile Asp Glu Tyr
260 265 270

Asp Gly Val Phe Thr Thr Lys Tyr Trp His Met Gly Ala Asp Glu Tyr
275 280 285

Met Ile Gly Thr Ser Phe Asp Asn Tyr Ser Lys Leu Lys Thr Phe Ala
290 295 300

Glu Lys Gln Tyr Gly Ala Gly Ala Thr Pro Asn Asp Ala Phe Thr Gly
305 310 315 320

Phe Ile Asn Asp Ile Asp Lys Tyr Val Lys Ala Lys Gly Lys Gln Leu
325 330 335

Arg Ile Trp Asn Asp Gly Ile Val Asn Thr Lys Asn Val Ser Leu Asn
340 345 350

Lys Asp Ile Val Ile Glu Tyr Trp Tyr Gly Ala Gly Arg Lys Pro Gln
355 360 365

Glu Leu Val Gln Asp Gly Tyr Thr Leu Met Asn Ala Thr Gln Ala Leu
370 375 380

Phe Trp Ser Arg Ser Ala Gln Val Tyr Lys Val Asn Ala Ala Arg Leu
385 390 395 400

Tyr Asn Asn Asn Trp Asn Val Gly Thr Phe Asp Gly Gly Arg Gln Ile
405 410 415

Asp Lys Asn Tyr Asp Lys Leu Thr Gly Ala Lys Val Ser Ile Trp Pro
420 425 430

Asp Ser Ser Tyr Phe Gln Thr Glu Asn Glu Val Glu Lys Glu Ile Phe
435 440 445

Asp Gly Met Arg Phe Ile Ser Gln Met Thr Trp Ser Asp Ser Arg Pro
450 455 460

Trp Ala Thr Trp Asn Asp Met Lys Ala Asp Ile Asp Lys Ile Gly Tyr
465 470 475 480

Pro Leu Asp Ile Arg Glu Tyr Asp Tyr Thr Pro Val Asp Ala Gly Ile
485 490 495

Tyr Asp Ile Pro Gln Leu Lys Ser Ile Ser Lys Gly Pro Trp Glu Leu
500 505 510

Ile Thr Thr Pro Asp Gly Tyr Tyr Gln Met Lys Asp Thr Val Ser Gly
515 520 525

Lys Cys Leu Ala Leu Phe Thr Gly Ser Lys His Leu Asp Val Val Thr
530 535 540

Gln Val Gly Ala Arg Pro Glu Leu Arg Asn Cys Ala Asp Val Ser Val
545 550 555 560

Gly Gln Asp Gln Arg Asn Thr Ala Asn Glu Arg Asn Thr Gln Lys Trp
565 570 575

Gln Ile Arg Ala Asp Lys Asp Gly Lys Tyr Thr Ile Ser Pro Ala Leu
580 585 590

Thr Gln Gln Arg Leu Ala Ile Ala Thr Gly Asn Glu Gln Asn Ile Asp
595 600 605

Leu Glu Thr His Arg Pro Ala Ala Gly Thr Val Ala Gln Phe Pro Ala
610 615 620

Asp Leu Val Ser Asp Asn Ala Leu Phe Thr Leu Thr Gly His Met Gly
625 630 635 640

Met Ser Ala Thr Val Asp Ser Lys Thr Val Asn Pro Ala Ser Pro Ser
645 650 655

Lys Ile Thr Val Lys Val Arg Ala Ala Ser Asn Ala Asn Thr Gly Asp
660 665 670

Val Thr Val Thr Pro Val Val Pro Glu Gly Trp Glu Ile Lys Pro Gly
675 680 685

Ser Val Ser Leu Lys Ser Ile Pro Ala Gly Lys Ala Ala Ile Ala Tyr
    690                 695                 700

Phe Asn Val Val Asn Thr Thr Gly Thr Gly Asp Ala Thr Val Gln Phe
705                 710                 715                 720

Lys Leu Thr Asn Thr Lys Thr Gly Glu Glu Leu Gly Thr Thr Ser Val
                725                 730                 735

Ala Leu Thr Gly Ser Leu Thr Lys Asp Val Glu Ala Ser Asp Tyr Ala
            740                 745                 750

Ala Ser Ser Gln Glu Thr Thr Gly Glu His Ala Pro Val Gly Asn Ala
        755                 760                 765

Phe Asp Lys Asn Ala Asn Thr Phe Trp His Ser Lys Tyr Ser Asn Pro
    770                 775                 780

Ser Ala Asn Leu Pro His Trp Leu Ala Phe Lys Ala Ser Pro Gly Glu
785                 790                 795                 800

Gly Asn Lys Ile Ala Ala Ile Thr His Leu Tyr Arg Gln Asp Lys Leu
                805                 810                 815

Asn Gly Pro Ala Lys Asn Val Ala Val Tyr Val Val Ala Ala Ser Asp
            820                 825                 830

Ala Asn Ser Val Ala Asp Val Thr Asn Trp Gly Glu Pro Val Ala Thr
        835                 840                 845

Ala Glu Phe Pro Tyr Thr Lys Glu Leu Gln Thr Ile Ala Leu Pro Asn
    850                 855                 860

Thr Ile Pro Ser Gly Asp Val Tyr Val Lys Phe Gln Ile Asn Asp Ala
865                 870                 875                 880

Trp Gly Leu Thr Glu Thr Ser Ala Gly Val Thr Trp Ala Ala Val Ala
                885                 890                 895

Glu Leu Ala Ala Thr Ala Lys Ala Thr Pro Val Glu Leu Thr Glu Pro
            900                 905                 910

Glu Gln Pro Lys Asp Asn Pro Glu Val Thr Glu Thr Pro Glu Ala Thr
        915                 920                 925

Gly Val Thr Val Ser Gly Asp Gly Val Ala Asn Gly Ala Leu Ser Leu

930 935 940

Lys Lys Gly Thr Thr Ala Gln Leu Thr Ala Lys Val Ala Pro Asp Asp
945 950 955 960

Ala Asp Gln Ala Val Thr Trp Ala Ser Ser Asp Asp Lys Val Val Thr
965 970 975

Val Asp Lys Thr Gly Lys Val Thr Ala Val Ala Lys Gly Val Ala Lys
980 985 990

Val Thr Ala Thr Thr Ala Asn Gly Lys Ser Ala Ser Val Thr Val Thr
995 1000 1005

Val Thr Glu Asp Ser Glu Val Pro Gly Pro Thr Gly Pro Thr Glu
1010 1015 1020

Pro Thr Lys Pro Gly Thr Glu
1025 1030

<210> 12
<211> 1597
<212> PRT
<213> Artificial Sequence

<220>
<223> Artificial sequence

<400> 12

Met Ala Ser Met Thr Gly Gly Gln Gln Met Gly Arg Asp Pro Asn Ser
1 5 10 15

Ser Ser Val Asp Lys Leu Ser Asp Asp Asn Leu Ala Leu Asn Gln Thr
20 25 30

Val Thr Ala Ser Ser Tyr Glu Val Ala Thr Thr Ala Pro Glu Lys Ala
35 40 45

Val Asp Gly Asp Leu Gly Thr Arg Trp Gly Thr Ala Gln Asn Lys Ala
50 55 60

Ala Asn Glu Trp Ile Glu Val Gly Leu Gly Gly Thr Lys Thr Val Lys
65 70 75 80

Gln Ile Asn Ile Asp Phe Glu Arg Lys Asp Ala Asp Gln Asn Ile Thr
85 90 95

Ser Phe Lys Val Glu Leu Lys Gln Gly Asp Thr Tyr Thr Lys Val Tyr
100 105 110

Gln Lys Asp Thr Arg Ala Lys Gln Gln Glu Ile Ile Leu Leu Asp Gln
        115                 120                 125

Ala Gln Gln Ala Ser Ala Val Lys Val Thr Val Leu Ser Ala Asp Gly
    130                 135                 140

Gly Thr Met Asn Trp Val Asn Val Gly Ile Asn Glu Ile Ser Val Tyr
145                 150                 155                 160

Ser Ala Pro Lys Glu Thr Val Leu Asp Thr Ala Asp Thr Asn His Met
                165                 170                 175

Leu Gly Ala Thr Met Thr Ala Ser Ser Asn Glu Thr Ala Thr Leu Thr
            180                 185                 190

Pro Asp Lys Ala Ile Asp Gln Asn Arg Thr Gly Arg Asn Asn Arg Trp
        195                 200                 205

Ala Ser Gly Tyr Glu Thr Pro Ser Asn Ile Trp Leu Lys Ala Glu Phe
    210                 215                 220

Pro Arg Leu Thr Ala Val Lys Asp Ile Arg Ile Tyr Phe Phe Glu Arg
225                 230                 235                 240

Asp Val Asn Pro Lys Pro Thr Asn Val Gln Ser Phe Asp Leu Ser Tyr
                245                 250                 255

Thr Asp Ser Glu Gly Thr Glu His Thr Leu Lys Ser Gly Tyr Ala Met
            260                 265                 270

Thr Ala Ser Gly Ala Gly Tyr Val Ala Asp Val Val Ile Gln Leu Asp
        275                 280                 285

Gln Ala Val Asn Ala Arg Ser Leu Lys Leu Ser Asn Phe Ala Ile Lys
    290                 295                 300

Ser Ser Glu Tyr Asn Asn Val Ser Val Ala Glu Trp Glu Ala Tyr Ser
305                 310                 315                 320

Asn Asp Gln Ala Glu Pro Gly Ala Thr Leu Asp Ser Val Val Ser Asp
                325                 330                 335

Leu Glu Ser Asn His Leu Thr Ile Glu Thr Asp Thr Asp Thr Leu Ala
            340                 345                 350

Leu Pro Thr Val Pro Asp Gly Tyr Thr Val Lys Phe Asn Gly Ala Asp

355 360 365

Tyr Glu Gln Leu Ile Ala Ala Asp Gly Thr Val Asn His Pro Leu Val
370 375 380

Asp Lys Thr Val Gln Val Ala Tyr Val Val Thr Asp Thr Ala Thr Gly
385 390 395 400

Asn Thr Lys Thr Thr Ser Asp Ile Pro Tyr Val Val Lys Gly Thr Asn
405 410 415

Gln Gln Gln Glu Gly Asn Asn Ala Lys Pro Thr Ile Ile Pro Glu Ile
420 425 430

Ala Glu Trp His Ser Thr Ser Ala Ala Lys Leu Ala Ala Ser Ala Val
435 440 445

Thr Lys Val Val Tyr Asp Asp Asp Ser Leu Lys Ala Val Val Asp Glu
450 455 460

Phe Val Ala Asp Tyr Lys Asp Phe Thr Gly Ile Lys Leu Thr Ala Lys
465 470 475 480

Lys Gly Ala Ala Glu Ala Gly Ala Phe Asn Phe Val Lys Thr Asp Ser
485 490 495

Thr Ala Ala Ile Ala Gln Leu Gly Asp Glu Gly Tyr Thr Met Asp Ile
500 505 510

Arg Ala Asp Arg Val Val Ala Lys Ser Ser Ser Val Thr Gly Asn Met
515 520 525

Tyr Ala Met Gln Thr Ile Leu Gln Met Thr Lys Gln Asp Ala Asn Gly
530 535 540

Phe Val Ile Gly Ser Met Arg Asp Tyr Pro Arg Phe Thr Thr Arg Gly
545 550 555 560

Leu Leu Leu Asp Val Ala Arg Lys Pro Val Ser Leu Glu Met Met Arg
565 570 575

Glu Ile Thr Arg Thr Met Arg Tyr Tyr Lys Met Asn Asp Phe Gln Ala
580 585 590

His Leu Ser Asp Asn Tyr Ile Phe Leu Glu Asn Tyr Gly Lys Gly Asp
595 600 605

```
Asn Glu Asp Glu Ala Phe Lys Ala Tyr Asp Ala Phe Arg Leu Glu Ser
    610                 615                 620

Ser Leu Thr Asn Asp Lys Gly Glu Ser Pro Thr Ala Glu Asp Tyr Ser
625                 630                 635                 640

Ile Ser Lys Lys Thr Phe Lys Gln Phe Ile Gln Asp Glu Arg Ala Leu
                645                 650                 655

Gly Met Asn Val Val Pro Glu Ile Asp Val Pro Ala His Ala Asn Ser
            660                 665                 670

Phe Thr Lys Ile Trp Pro Glu Leu Met Val Lys Gly Arg Val Ser Pro
        675                 680                 685

Ile Asn Ser Asn Arg Pro Leu Ile Asp His Leu Asp Val Ser Lys Pro
    690                 695                 700

Glu Thr Ile Ala Lys Ile Lys Glu Ile Phe Asp Asp Tyr Thr Lys Gly
705                 710                 715                 720

Asp Asp Pro Thr Phe Asp Ser Asp Thr Thr Val His Ile Gly Ala Asp
                725                 730                 735

Glu Phe Leu Tyr Asn Tyr Thr Ala Tyr Arg Lys Phe Ile Asn Glu Ile
            740                 745                 750

Val Pro Tyr Ile Lys Asp Thr Asn Thr Val Arg Met Trp Gly Gly Leu
        755                 760                 765

Thr Trp Ile Asn Asp His Lys Thr Glu Ile Thr Lys Asp Ala Ile Glu
    770                 775                 780

Asn Val Glu Met Asn Leu Trp Ser Lys Asp Trp Ala Asp Gly Leu Gln
785                 790                 795                 800

Met Tyr Asn Met Gly Tyr Lys Leu Ile Asn Thr Ile Asp Asp Tyr Gly
                805                 810                 815

Tyr Met Val Pro Asn Gly Ser Tyr Gly Arg Ala Asn Ala Tyr Gly Asp
            820                 825                 830

Leu Leu Asn Ile Ser Arg Val Phe Asp Ser Phe Glu Pro Asn Lys Val
        835                 840                 845

Arg Ser Ser Gly Gly Tyr Gln Ala Val Pro Ser Gly Asp Asp Gln Met
    850                 855                 860
```

Leu Gly Ala Ala Phe Ala Ile Trp Ser Asp Asn Ile Asp Lys Ser Ala
865 870 875 880

Ser Gly Leu Thr Glu Ser Asp Leu Tyr Trp Arg Phe Phe Asp Ala Met
885 890 895

Pro Phe Tyr Ala Glu Lys Thr Trp Ala Ala Thr Gly Lys Glu Lys Gly
900 905 910

Thr Ala Ala Lys Leu Thr Ala Leu Ala Ala Lys Gln Gly Thr Gly Pro
915 920 925

Arg Thr Asn Pro Tyr Tyr Gln Ala Thr Ser Lys Asn Ser Val Tyr Glu
930 935 940

Ser Tyr Asp Phe Asn Asp Gly Leu Ala Asp Ala Ser Gly Asn Gly Arg
945 950 955 960

Asp Leu Thr Ile Gly Asp Gly Ser Lys Ala Ala Val Lys Asp Gln Ser
965 970 975

Leu Lys Leu Ala Gly Gly Ser Ser Tyr Ala Thr Ser Lys Leu Asp Lys
980 985 990

Leu Gly Asn Gly Asn Glu Leu Thr Phe Asp Val Thr Leu Gln Gln Ala
995 1000 1005

Ala Lys Pro Gly Asp Ile Leu Phe Glu Ala Asp Ala Pro Tyr Gly
1010 1015 1020

Thr His Asp Ile Arg Val Met Glu Asn Gly Lys Leu Gly Phe Thr
1025 1030 1035

Arg Glu Leu Tyr Asn Tyr Tyr Phe Asp Tyr Glu Leu Pro Val Gly
1040 1045 1050

Lys Thr Val Thr Val Thr Ile Lys Val Asp Gln Gln Thr Thr Lys
1055 1060 1065

Leu Tyr Val Asp Gly Glu Phe Val Ser Asp Ala Thr Gly Lys Tyr
1070 1075 1080

Ile Asp Lys Gly Ile Glu Lys Lys Thr Gly Ile Thr Ala Ala Thr
1085 1090 1095

Phe Ala Leu Pro Leu Gln Arg Ile Gly Ser Lys Thr Ser Ala Ile
1100 1105 1110

Asn Gly Val Ile Asp Asn Val Ile Val Lys Lys Ser Glu Ala Glu
1115 1120 1125

Thr Asp Gln Tyr Asn Lys Ser Cys Trp Thr Gly Thr Thr Asn Ser
1130 1135 1140

Glu Thr Gln Tyr Asn Asp Thr Glu Gly Leu Leu Arg Tyr Ala Phe
1145 1150 1155

Asp Asn Asn Pro Ser Thr Ile Trp His Ser Asn Trp Lys Gly Ala
1160 1165 1170

Thr Asp Lys Leu Thr Gly Ser Asn Ser Phe Tyr Ala Glu Ile Asp
1175 1180 1185

Met Cys Gln Lys Tyr Thr Ile Asn Gln Phe Ser Phe Thr Pro Arg
1190 1195 1200

Thr Ser Gln Asp Ser Gly Gln Val Thr Lys Ala Asp Leu Tyr Val
1205 1210 1215

Lys Ala Asn Ala Asn Asp Glu Trp Lys Gln Val Ala Thr Asp Gln
1220 1225 1230

Val Phe Glu Ala Ser Arg Ala Lys Lys Thr Phe Met Phe Asp Glu
1235 1240 1245

Gln Glu Val Arg Tyr Val Lys Phe Val Ala Lys Ser Ser Asn Asp
1250 1255 1260

Gly Trp Val Ala Val Ser Glu Phe Gly Val Ala Asn Lys Pro Ser
1265 1270 1275

Ser Thr Val Arg Val Phe Val Ala Ala Asp Pro Ala Glu Gly Gly
1280 1285 1290

Thr Val Ser Val Ala Ala Glu Gly Glu Thr Gly Thr Asp Thr Ala
1295 1300 1305

Val Asp Val Ala Ser Gly Ala Ser Val Thr Ala Lys Ala Val Ala
1310 1315 1320

Ala Asp Gly Tyr Arg Phe Ser Gly Trp Phe Thr Thr Ala Ser Glu
1325 1330 1335

Thr Ala Val Ser Thr Asp Ala Thr Tyr Thr Phe Ala Ala Asp Gly

1340 1345 1350

Asn Thr Ala Leu Thr Ala Lys Phe Thr Lys Asp Ser Thr Pro Asp
1355 1360 1365

Pro Gly Pro Lys Pro Thr Ile Ser Ser Ile Ala Val Thr Lys Pro
1370 1375 1380

Thr Val Thr Asp Tyr Lys Val Gly Asp Thr Phe Asp Ala Thr Gly
1385 1390 1395

Leu Ala Val Thr Ala Thr Met Ser Asp Gly Ser Thr Lys Thr Leu
1400 1405 1410

Thr Ala Gly Glu Tyr Thr Leu Ser Ala Thr Gln Asp Gly Ala Ala
1415 1420 1425

Val Ala Leu Asp Lys Ala Phe Ala Lys Ala Gly Lys Val Thr Val
1430 1435 1440

Thr Val Thr Ala Asn Gly Lys Thr Ala Thr Phe Asp Val Thr Val
1445 1450 1455

Thr Ala Lys Asp Pro Asp Pro Glu Pro Ala Thr Leu Lys Ser Ile
1460 1465 1470

Lys Val Thr Ser Lys Pro Asp Lys Thr Thr Tyr Thr Val Asp Glu
1475 1480 1485

Thr Phe Ala Lys Thr Gly Leu Ala Val Thr Gly Thr Trp Ser Asp
1490 1495 1500

Gly Lys Thr Ala Leu Leu Lys Asp Gly Glu Tyr Lys Leu Ser Ala
1505 1510 1515

Val Asp Ala Asp Gly Lys Thr Val Asp Leu Thr Lys Pro Phe Thr
1520 1525 1530

Ala Ala Gly Asp Val Thr Val Thr Val Thr Ser Gly Lys Leu Thr
1535 1540 1545

Asp Ser Phe Thr Ile Thr Val Lys Ala Lys Thr Val Thr Pro Ala
1550 1555 1560

Pro Gly Asp Asn Lys Pro Gly Glu Asn Lys Pro Gly Ala Asp Lys
1565 1570 1575

Pro Lys Pro Asn Thr Pro Asp Glu Val Ala Lys Leu Glu His His
1580 1585 1590

His His His His
1595

<210> 13
<211> 1597
<212> PRT
<213> Artificial Sequence

<220>
<223> Artificial sequence

<400> 13

Met Ala Ser Met Thr Gly Gly Gln Gln Met Gly Arg Asp Pro Asn Ser
1 5 10 15

Ser Ser Val Asp Lys Leu Ser Asp Asp Asn Leu Ala Leu Asn Gln Thr
20 25 30

Val Thr Ala Ser Ser Tyr Glu Val Ala Thr Thr Ala Pro Glu Lys Ala
35 40 45

Val Asp Gly Asp Leu Gly Thr Arg Trp Gly Thr Ala Gln Asn Lys Ala
50 55 60

Ala Asn Glu Trp Ile Glu Val Gly Leu Gly Gly Thr Lys Thr Val Lys
65 70 75 80

Gln Ile Asn Ile Asp Phe Glu Arg Lys Asp Ala Asp Gln Asn Ile Thr
85 90 95

Ser Phe Lys Val Glu Leu Lys Gln Gly Asp Thr Tyr Thr Lys Val Tyr
100 105 110

Gln Lys Asp Thr Arg Ala Lys Gln Gln Glu Ile Ile Leu Leu Asp Gln
115 120 125

Ala Gln Gln Ala Ser Ala Val Lys Val Thr Val Leu Ser Ala Asp Gly
130 135 140

Gly Thr Met Asn Trp Val Asn Val Gly Ile Asn Glu Ile Ser Val Tyr
145 150 155 160

Ser Ala Pro Lys Glu Thr Val Leu Asp Thr Ala Asp Thr Asn His Met
165 170 175

Leu Gly Ala Thr Met Thr Ala Ser Ser Asn Glu Thr Ala Thr Leu Thr

180 185 190

Pro Asp Lys Ala Ile Asp Gln Asn Arg Thr Gly Arg Asn Asn Arg Trp
195 200 205

Ala Ser Gly Tyr Glu Thr Pro Ser Asn Ile Trp Leu Lys Ala Glu Phe
210 215 220

Pro Arg Leu Thr Ala Val Lys Asp Ile Arg Ile Tyr Phe Phe Glu Arg
225 230 235 240

Asp Val Asn Pro Lys Pro Thr Asn Val Gln Ser Phe Asp Leu Ser Tyr
245 250 255

Thr Asp Ser Glu Gly Thr Glu His Thr Leu Lys Ser Gly Tyr Ala Met
260 265 270

Thr Ala Ser Gly Ala Gly Tyr Val Ala Asp Val Val Ile Gln Leu Asp
275 280 285

Gln Ala Val Asn Ala Arg Ser Leu Lys Leu Ser Asn Phe Ala Ile Lys
290 295 300

Ser Ser Glu Tyr Asn Asn Val Ser Val Ala Glu Trp Glu Ala Tyr Ser
305 310 315 320

Asn Asp Gln Ala Glu Pro Gly Ala Thr Leu Asp Ser Val Val Ser Asp
325 330 335

Leu Glu Ser Asn His Leu Thr Ile Glu Thr Asp Thr Asp Thr Leu Ala
340 345 350

Leu Pro Thr Val Pro Asp Gly Tyr Thr Val Lys Phe Asn Gly Ala Asp
355 360 365

Tyr Glu Gln Leu Ile Ala Ala Asp Gly Thr Val Asn His Pro Leu Val
370 375 380

Asp Lys Thr Val Gln Val Ala Tyr Val Val Thr Asp Thr Ala Thr Gly
385 390 395 400

Asn Thr Lys Thr Thr Ser Asp Ile Pro Tyr Val Val Lys Gly Thr Asn
405 410 415

Gln Gln Gln Glu Gly Asn Asn Ala Lys Pro Thr Ile Ile Pro Glu Ile
420 425 430

```
Ala Glu Trp His Ser Thr Ser Ala Ala Lys Leu Ala Ala Ser Ala Val
        435                 440                 445

Thr Lys Val Val Tyr Asp Asp Asp Ser Leu Lys Ala Val Val Asp Glu
    450                 455                 460

Phe Val Ala Asp Tyr Lys Asp Phe Thr Gly Ile Lys Leu Thr Ala Lys
465                 470                 475                 480

Lys Gly Ala Ala Glu Ala Gly Ala Phe Asn Phe Val Lys Thr Asp Ser
                485                 490                 495

Thr Ala Ala Ile Ala Gln Leu Gly Asp Glu Gly Tyr Thr Met Asp Ile
            500                 505                 510

Arg Ala Asp Arg Val Val Ala Lys Ser Ser Ser Val Thr Gly Asn Met
        515                 520                 525

Tyr Ala Met Gln Thr Ile Leu Gln Met Thr Lys Gln Asp Ala Asn Gly
    530                 535                 540

Phe Val Ile Gly Ser Met Arg Asp Tyr Pro Arg Phe Thr Thr Arg Gly
545                 550                 555                 560

Leu Leu Leu Asp Val Ala Arg Lys Pro Val Ser Leu Glu Met Met Arg
                565                 570                 575

Glu Ile Thr Arg Thr Met Arg Tyr Tyr Lys Met Asn Asp Phe Gln Ala
            580                 585                 590

His Leu Ser Asp Asn Tyr Ile Phe Leu Glu Asn Tyr Gly Lys Gly Asp
        595                 600                 605

Asn Glu Asp Glu Ala Phe Lys Ala Tyr Asp Ala Phe Arg Leu Glu Ser
    610                 615                 620

Ser Leu Thr Asn Asp Lys Gly Glu Ser Pro Thr Ala Glu Asp Tyr Ser
625                 630                 635                 640

Ile Ser Lys Lys Thr Phe Lys Gln Phe Ile Gln Asp Glu Arg Ala Leu
                645                 650                 655

Gly Met Asn Val Val Pro Glu Ile Asp Val Pro Ala His Ala Asn Ser
            660                 665                 670

Phe Thr Lys Ile Trp Pro Glu Leu Met Val Lys Gly Arg Val Ser Pro
        675                 680                 685
```

```
Ile Asn Ser Asn Arg Pro Leu Ile Asp His Leu Asp Val Ser Lys Pro
    690                 695                 700

Glu Thr Ile Ala Lys Ile Lys Glu Ile Phe Asp Asp Tyr Thr Lys Gly
705                 710                 715                 720

Asp Asp Pro Thr Phe Asp Ser Asp Thr Thr Val His Ile Gly Ala Glu
                725                 730                 735

Glu Phe Leu Tyr Asn Tyr Thr Ala Tyr Arg Lys Phe Ile Asn Glu Ile
            740                 745                 750

Val Pro Tyr Ile Lys Asp Thr Asn Thr Val Arg Met Trp Gly Gly Leu
        755                 760                 765

Thr Trp Ile Asn Asp His Lys Thr Glu Ile Thr Lys Asp Ala Ile Glu
    770                 775                 780

Asn Val Glu Met Asn Leu Trp Ser Lys Asp Trp Ala Asp Gly Leu Gln
785                 790                 795                 800

Met Tyr Asn Met Gly Tyr Lys Leu Ile Asn Thr Ile Asp Asp Tyr Gly
                805                 810                 815

Tyr Met Val Pro Asn Gly Ser Tyr Gly Arg Ala Asn Ala Tyr Gly Asp
            820                 825                 830

Leu Leu Asn Ile Ser Arg Val Phe Asp Ser Phe Glu Pro Asn Lys Val
        835                 840                 845

Arg Ser Ser Gly Gly Tyr Gln Ala Val Pro Ser Gly Asp Asp Gln Met
    850                 855                 860

Leu Gly Ala Ala Phe Ala Ile Trp Ser Asp Asn Ile Asp Lys Ser Ala
865                 870                 875                 880

Ser Gly Leu Thr Glu Ser Asp Leu Tyr Trp Arg Phe Phe Asp Ala Met
                885                 890                 895

Pro Phe Tyr Ala Glu Lys Thr Trp Ala Ala Thr Gly Lys Glu Lys Gly
            900                 905                 910

Thr Ala Ala Lys Leu Thr Ala Leu Ala Ala Lys Gln Gly Thr Gly Pro
        915                 920                 925

Arg Thr Asn Pro Tyr Tyr Gln Ala Thr Ser Lys Asn Ser Val Tyr Glu
    930                 935                 940
```

```
Ser Tyr Asp Phe Asn Asp Gly Leu Ala Asp Ala Ser Gly Asn Gly Arg
945                 950                 955                 960

Asp Leu Thr Ile Gly Asp Gly Ser Lys Ala Ala Val Lys Asp Gln Ser
                965                 970                 975

Leu Lys Leu Ala Gly Gly Ser Ser Tyr Ala Thr Ser Lys Leu Asp Lys
            980                 985                 990

Leu Gly Asn Gly Asn Glu Leu Thr  Phe Asp Val Thr Leu  Gln Gln Ala
        995                 1000                1005

Ala Lys  Pro Gly Asp Ile Leu  Phe Glu Ala Asp Ala  Pro Tyr Gly
    1010                 1015                 1020

Thr His  Asp Ile Arg Val Met  Glu Asn Gly Lys Leu  Gly Phe Thr
    1025                 1030                 1035

Arg Glu  Leu Tyr Asn Tyr Tyr  Phe Asp Tyr Glu Leu  Pro Val Gly
    1040                 1045                 1050

Lys Thr  Val Thr Val Thr Ile  Lys Val Asp Gln Gln  Thr Thr Lys
    1055                 1060                 1065

Leu Tyr  Val Asp Gly Glu Phe  Val Ser Asp Ala Thr  Gly Lys Tyr
    1070                 1075                 1080

Ile Asp  Lys Gly Ile Glu Lys  Lys Thr Gly Ile Thr  Ala Ala Thr
    1085                 1090                 1095

Phe Ala  Leu Pro Leu Gln Arg  Ile Gly Ser Lys Thr  Ser Ala Ile
    1100                 1105                 1110

Asn Gly  Val Ile Asp Asn Val  Ile Val Lys Lys Ser  Glu Ala Glu
    1115                 1120                 1125

Thr Asp  Gln Tyr Asn Lys Ser  Cys Trp Thr Gly Thr  Thr Asn Ser
    1130                 1135                 1140

Glu Thr  Gln Tyr Asn Asp Thr  Glu Gly Leu Leu Arg  Tyr Ala Phe
    1145                 1150                 1155

Asp Asn  Asn Pro Ser Thr Ile  Trp His Ser Asn Trp  Lys Gly Ala
    1160                 1165                 1170

Thr Asp  Lys Leu Thr Gly Ser  Asn Ser Phe Tyr Ala  Glu Ile Asp
```

```
    1175                1180                1185

Met Cys Gln Lys Tyr Thr Ile Asn Gln Phe Ser Phe Thr Pro Arg
    1190                1195                1200

Thr Ser Gln Asp Ser Gly Gln Val Thr Lys Ala Asp Leu Tyr Val
    1205                1210                1215

Lys Ala Asn Ala Asn Asp Glu Trp Lys Gln Val Ala Thr Asp Gln
    1220                1225                1230

Val Phe Glu Ala Ser Arg Ala Lys Lys Thr Phe Met Phe Asp Glu
    1235                1240                1245

Gln Glu Val Arg Tyr Val Lys Phe Val Ala Lys Ser Ser Asn Asp
    1250                1255                1260

Gly Trp Val Ala Val Ser Glu Phe Gly Val Ala Asn Lys Pro Ser
    1265                1270                1275

Ser Thr Val Arg Val Phe Val Ala Ala Asp Pro Ala Glu Gly Gly
    1280                1285                1290

Thr Val Ser Val Ala Ala Glu Gly Glu Thr Gly Thr Asp Thr Ala
    1295                1300                1305

Val Asp Val Ala Ser Gly Ala Ser Val Thr Ala Lys Ala Val Ala
    1310                1315                1320

Ala Asp Gly Tyr Arg Phe Ser Gly Trp Phe Thr Thr Ala Ser Glu
    1325                1330                1335

Thr Ala Val Ser Thr Asp Ala Thr Tyr Thr Phe Ala Ala Asp Gly
    1340                1345                1350

Asn Thr Ala Leu Thr Ala Lys Phe Thr Lys Asp Ser Thr Pro Asp
    1355                1360                1365

Pro Gly Pro Lys Pro Thr Ile Ser Ser Ile Ala Val Thr Lys Pro
    1370                1375                1380

Thr Val Thr Asp Tyr Lys Val Gly Asp Thr Phe Asp Ala Thr Gly
    1385                1390                1395

Leu Ala Val Thr Ala Thr Met Ser Asp Gly Ser Thr Lys Thr Leu
    1400                1405                1410
```

Thr Ala Gly Glu Tyr Thr Leu Ser Ala Thr Gln Asp Gly Ala Ala
1415 1420 1425

Val Ala Leu Asp Lys Ala Phe Ala Lys Ala Gly Lys Val Thr Val
1430 1435 1440

Thr Val Thr Ala Asn Gly Lys Thr Ala Thr Phe Asp Val Thr Val
1445 1450 1455

Thr Ala Lys Asp Pro Asp Pro Glu Pro Ala Thr Leu Lys Ser Ile
1460 1465 1470

Lys Val Thr Ser Lys Pro Asp Lys Thr Thr Tyr Thr Val Asp Glu
1475 1480 1485

Thr Phe Ala Lys Thr Gly Leu Ala Val Thr Gly Thr Trp Ser Asp
1490 1495 1500

Gly Lys Thr Ala Leu Leu Lys Asp Gly Glu Tyr Lys Leu Ser Ala
1505 1510 1515

Val Asp Ala Asp Gly Lys Thr Val Asp Leu Thr Lys Pro Phe Thr
1520 1525 1530

Ala Ala Gly Asp Val Thr Val Thr Val Thr Ser Gly Lys Leu Thr
1535 1540 1545

Asp Ser Phe Thr Ile Thr Val Lys Ala Lys Thr Val Thr Pro Ala
1550 1555 1560

Pro Gly Asp Asn Lys Pro Gly Glu Asn Lys Pro Gly Ala Asp Lys
1565 1570 1575

Pro Lys Pro Asn Thr Pro Asp Glu Val Ala Lys Leu Glu His His
1580 1585 1590

His His His His
1595

<210> 14
<211> 1597
<212> PRT
<213> Artificial Sequence

<220>
<223> Artificial sequence

<400> 14

Met Ala Ser Met Thr Gly Gly Gln Gln Met Gly Arg Asp Pro Asn Ser

```
1               5                   10                  15

Ser Ser Val Asp Lys Leu Ser Asp Asp Asn Leu Ala Leu Asn Gln Thr
            20                  25                  30

Val Thr Ala Ser Ser Tyr Glu Val Ala Thr Thr Ala Pro Glu Lys Ala
        35                  40                  45

Val Asp Gly Asp Leu Gly Thr Arg Trp Gly Thr Ala Gln Asn Lys Ala
    50                  55                  60

Ala Asn Glu Trp Ile Glu Val Gly Leu Gly Gly Thr Lys Thr Val Lys
65                  70                  75                  80

Gln Ile Asn Ile Asp Phe Glu Arg Lys Asp Ala Asp Gln Asn Ile Thr
                85                  90                  95

Ser Phe Lys Val Glu Leu Lys Gln Gly Asp Thr Tyr Thr Lys Val Tyr
            100                 105                 110

Gln Lys Asp Thr Arg Ala Lys Gln Gln Glu Ile Ile Leu Leu Asp Gln
        115                 120                 125

Ala Gln Gln Ala Ser Ala Val Lys Val Thr Val Leu Ser Ala Asp Gly
    130                 135                 140

Gly Thr Met Asn Trp Val Asn Val Gly Ile Asn Glu Ile Ser Val Tyr
145                 150                 155                 160

Ser Ala Pro Lys Glu Thr Val Leu Asp Thr Ala Asp Thr Asn His Met
                165                 170                 175

Leu Gly Ala Thr Met Thr Ala Ser Ser Asn Glu Thr Ala Thr Leu Thr
            180                 185                 190

Pro Asp Lys Ala Ile Asp Gln Asn Arg Thr Gly Arg Asn Asn Arg Trp
        195                 200                 205

Ala Ser Gly Tyr Glu Thr Pro Ser Asn Ile Trp Leu Lys Ala Glu Phe
    210                 215                 220

Pro Arg Leu Thr Ala Val Lys Asp Ile Arg Ile Tyr Phe Phe Glu Arg
225                 230                 235                 240

Asp Val Asn Pro Lys Pro Thr Asn Val Gln Ser Phe Asp Leu Ser Tyr
                245                 250                 255
```

```
Thr Asp Ser Glu Gly Thr Glu His Thr Leu Lys Ser Gly Tyr Ala Met
            260                 265                 270

Thr Ala Ser Gly Ala Gly Tyr Val Ala Asp Val Val Ile Gln Leu Asp
        275                 280                 285

Gln Ala Val Asn Ala Arg Ser Leu Lys Leu Ser Asn Phe Ala Ile Lys
    290                 295                 300

Ser Ser Glu Tyr Asn Asn Val Ser Val Ala Glu Trp Glu Ala Tyr Ser
305                 310                 315                 320

Asn Asp Gln Ala Glu Pro Gly Ala Thr Leu Asp Ser Val Val Ser Asp
                325                 330                 335

Leu Glu Ser Asn His Leu Thr Ile Glu Thr Asp Thr Asp Thr Leu Ala
            340                 345                 350

Leu Pro Thr Val Pro Asp Gly Tyr Thr Val Lys Phe Asn Gly Ala Asp
        355                 360                 365

Tyr Glu Gln Leu Ile Ala Ala Asp Gly Thr Val Asn His Pro Leu Val
    370                 375                 380

Asp Lys Thr Val Gln Val Ala Tyr Val Val Thr Asp Thr Ala Thr Gly
385                 390                 395                 400

Asn Thr Lys Thr Thr Ser Asp Ile Pro Tyr Val Val Lys Gly Thr Asn
                405                 410                 415

Gln Gln Gln Glu Gly Asn Asn Ala Lys Pro Thr Ile Ile Pro Glu Ile
            420                 425                 430

Ala Glu Trp His Ser Thr Ser Ala Ala Lys Leu Ala Ala Ser Ala Val
        435                 440                 445

Thr Lys Val Val Tyr Asp Asp Asp Ser Leu Lys Ala Val Val Asp Glu
    450                 455                 460

Phe Val Ala Asp Tyr Lys Asp Phe Thr Gly Ile Lys Leu Thr Ala Lys
465                 470                 475                 480

Lys Gly Ala Ala Glu Ala Gly Ala Phe Asn Phe Val Lys Thr Asp Ser
                485                 490                 495

Thr Ala Ala Ile Ala Gln Leu Gly Asp Glu Gly Tyr Thr Met Asp Ile
            500                 505                 510
```

```
Arg Ala Asp Arg Val Val Ala Lys Ser Ser Ser Val Thr Gly Asn Met
        515                 520                 525

Tyr Ala Met Gln Thr Ile Leu Gln Met Thr Lys Gln Asp Ala Asn Gly
    530                 535                 540

Phe Val Ile Gly Ser Met Arg Asp Tyr Pro Arg Phe Thr Thr Arg Gly
545                 550                 555                 560

Leu Leu Leu Asp Val Ala Arg Lys Pro Val Ser Leu Glu Met Met Arg
                565                 570                 575

Glu Ile Thr Arg Thr Met Arg Tyr Tyr Lys Met Asn Asp Phe Gln Ala
            580                 585                 590

His Leu Ser Asp Asn Tyr Ile Phe Leu Glu Asn Tyr Gly Lys Gly Asp
        595                 600                 605

Asn Glu Asp Glu Ala Phe Lys Ala Tyr Asp Ala Phe Arg Leu Glu Ser
    610                 615                 620

Ser Leu Thr Asn Asp Lys Gly Glu Ser Pro Thr Ala Glu Asp Tyr Ser
625                 630                 635                 640

Ile Ser Lys Lys Thr Phe Lys Gln Phe Ile Gln Asp Glu Arg Ala Leu
                645                 650                 655

Gly Met Asn Val Val Pro Glu Ile Asp Val Pro Ala His Ala Asn Ser
            660                 665                 670

Phe Thr Lys Ile Trp Pro Glu Leu Met Val Lys Gly Arg Val Ser Pro
        675                 680                 685

Ile Asn Ser Asn Arg Pro Leu Ile Asp His Leu Asp Val Ser Lys Pro
    690                 695                 700

Glu Thr Ile Ala Lys Ile Lys Glu Ile Phe Asp Asp Tyr Thr Lys Gly
705                 710                 715                 720

Asp Asp Pro Thr Phe Asp Ser Asp Thr Thr Val His Ile Gly Ala Ala
                725                 730                 735

Glu Phe Leu Tyr Asn Tyr Thr Ala Tyr Arg Lys Phe Ile Asn Glu Ile
            740                 745                 750

Val Pro Tyr Ile Lys Asp Thr Asn Thr Val Arg Met Trp Gly Gly Leu
        755                 760                 765
```

```
Thr Trp Ile Asn Asp His Lys Thr Glu Ile Thr Lys Asp Ala Ile Glu
    770                 775                 780

Asn Val Glu Met Asn Leu Trp Ser Lys Asp Trp Ala Asp Gly Leu Gln
785                 790                 795                 800

Met Tyr Asn Met Gly Tyr Lys Leu Ile Asn Thr Ile Asp Asp Tyr Gly
                805                 810                 815

Tyr Met Val Pro Asn Gly Ser Tyr Gly Arg Ala Asn Ala Tyr Gly Asp
            820                 825                 830

Leu Leu Asn Ile Ser Arg Val Phe Asp Ser Phe Glu Pro Asn Lys Val
        835                 840                 845

Arg Ser Ser Gly Gly Tyr Gln Ala Val Pro Ser Gly Asp Asp Gln Met
    850                 855                 860

Leu Gly Ala Ala Phe Ala Ile Trp Ser Asp Asn Ile Asp Lys Ser Ala
865                 870                 875                 880

Ser Gly Leu Thr Glu Ser Asp Leu Tyr Trp Arg Phe Phe Asp Ala Met
                885                 890                 895

Pro Phe Tyr Ala Glu Lys Thr Trp Ala Ala Thr Gly Lys Glu Lys Gly
            900                 905                 910

Thr Ala Ala Lys Leu Thr Ala Leu Ala Ala Lys Gln Gly Thr Gly Pro
        915                 920                 925

Arg Thr Asn Pro Tyr Tyr Gln Ala Thr Ser Lys Asn Ser Val Tyr Glu
    930                 935                 940

Ser Tyr Asp Phe Asn Asp Gly Leu Ala Asp Ala Ser Gly Asn Gly Arg
945                 950                 955                 960

Asp Leu Thr Ile Gly Asp Gly Ser Lys Ala Ala Val Lys Asp Gln Ser
                965                 970                 975

Leu Lys Leu Ala Gly Gly Ser Ser Tyr Ala Thr Ser Lys Leu Asp Lys
            980                 985                 990

Leu Gly Asn Gly Asn Glu Leu Thr  Phe Asp Val Thr Leu  Gln Gln Ala
        995                 1000                1005

Ala Lys  Pro Gly Asp Ile Leu  Phe Glu Ala Asp Ala  Pro Tyr Gly
```

1010 1015 1020

Thr His Asp Ile Arg Val Met Glu Asn Gly Lys Leu Gly Phe Thr
1025 1030 1035

Arg Glu Leu Tyr Asn Tyr Tyr Phe Asp Tyr Glu Leu Pro Val Gly
1040 1045 1050

Lys Thr Val Thr Val Thr Ile Lys Val Asp Gln Gln Thr Thr Lys
1055 1060 1065

Leu Tyr Val Asp Gly Glu Phe Val Ser Asp Ala Thr Gly Lys Tyr
1070 1075 1080

Ile Asp Lys Gly Ile Glu Lys Lys Thr Gly Ile Thr Ala Ala Thr
1085 1090 1095

Phe Ala Leu Pro Leu Gln Arg Ile Gly Ser Lys Thr Ser Ala Ile
1100 1105 1110

Asn Gly Val Ile Asp Asn Val Ile Val Lys Lys Ser Glu Ala Glu
1115 1120 1125

Thr Asp Gln Tyr Asn Lys Ser Cys Trp Thr Gly Thr Thr Asn Ser
1130 1135 1140

Glu Thr Gln Tyr Asn Asp Thr Glu Gly Leu Leu Arg Tyr Ala Phe
1145 1150 1155

Asp Asn Asn Pro Ser Thr Ile Trp His Ser Asn Trp Lys Gly Ala
1160 1165 1170

Thr Asp Lys Leu Thr Gly Ser Asn Ser Phe Tyr Ala Glu Ile Asp
1175 1180 1185

Met Cys Gln Lys Tyr Thr Ile Asn Gln Phe Ser Phe Thr Pro Arg
1190 1195 1200

Thr Ser Gln Asp Ser Gly Gln Val Thr Lys Ala Asp Leu Tyr Val
1205 1210 1215

Lys Ala Asn Ala Asn Asp Glu Trp Lys Gln Val Ala Thr Asp Gln
1220 1225 1230

Val Phe Glu Ala Ser Arg Ala Lys Lys Thr Phe Met Phe Asp Glu
1235 1240 1245

Gln Glu Val Arg Tyr Val Lys Phe Val Ala Lys Ser Ser Asn Asp
1250 1255 1260

Gly Trp Val Ala Val Ser Glu Phe Gly Val Ala Asn Lys Pro Ser
1265 1270 1275

Ser Thr Val Arg Val Phe Val Ala Ala Asp Pro Ala Glu Gly Gly
1280 1285 1290

Thr Val Ser Val Ala Ala Glu Gly Glu Thr Gly Thr Asp Thr Ala
1295 1300 1305

Val Asp Val Ala Ser Gly Ala Ser Val Thr Ala Lys Ala Val Ala
1310 1315 1320

Ala Asp Gly Tyr Arg Phe Ser Gly Trp Phe Thr Thr Ala Ser Glu
1325 1330 1335

Thr Ala Val Ser Thr Asp Ala Thr Tyr Thr Phe Ala Ala Asp Gly
1340 1345 1350

Asn Thr Ala Leu Thr Ala Lys Phe Thr Lys Asp Ser Thr Pro Asp
1355 1360 1365

Pro Gly Pro Lys Pro Thr Ile Ser Ser Ile Ala Val Thr Lys Pro
1370 1375 1380

Thr Val Thr Asp Tyr Lys Val Gly Asp Thr Phe Asp Ala Thr Gly
1385 1390 1395

Leu Ala Val Thr Ala Thr Met Ser Asp Gly Ser Thr Lys Thr Leu
1400 1405 1410

Thr Ala Gly Glu Tyr Thr Leu Ser Ala Thr Gln Asp Gly Ala Ala
1415 1420 1425

Val Ala Leu Asp Lys Ala Phe Ala Lys Ala Gly Lys Val Thr Val
1430 1435 1440

Thr Val Thr Ala Asn Gly Lys Thr Ala Thr Phe Asp Val Thr Val
1445 1450 1455

Thr Ala Lys Asp Pro Asp Pro Glu Pro Ala Thr Leu Lys Ser Ile
1460 1465 1470

Lys Val Thr Ser Lys Pro Asp Lys Thr Thr Tyr Thr Val Asp Glu
1475 1480 1485

Thr Phe Ala Lys Thr Gly Leu Ala Val Thr Gly Thr Trp Ser Asp
1490 1495 1500

Gly Lys Thr Ala Leu Leu Lys Asp Gly Glu Tyr Lys Leu Ser Ala
1505 1510 1515

Val Asp Ala Asp Gly Lys Thr Val Asp Leu Thr Lys Pro Phe Thr
1520 1525 1530

Ala Ala Gly Asp Val Thr Val Thr Val Thr Ser Gly Lys Leu Thr
1535 1540 1545

Asp Ser Phe Thr Ile Thr Val Lys Ala Lys Thr Val Thr Pro Ala
1550 1555 1560

Pro Gly Asp Asn Lys Pro Gly Glu Asn Lys Pro Gly Ala Asp Lys
1565 1570 1575

Pro Lys Pro Asn Thr Pro Asp Glu Val Ala Lys Leu Glu His His
1580 1585 1590

His His His His
1595

<210> 15
<211> 1597
<212> PRT
<213> Artificial Sequence

<220>
<223> Artificial sequence

<400> 15

Met Ala Ser Met Thr Gly Gly Gln Gln Met Gly Arg Asp Pro Asn Ser
1 5 10 15

Ser Ser Val Asp Lys Leu Ser Asp Asp Asn Leu Ala Leu Asn Gln Thr
20 25 30

Val Thr Ala Ser Ser Tyr Glu Val Ala Thr Thr Ala Pro Glu Lys Ala
35 40 45

Val Asp Gly Asp Leu Gly Thr Arg Trp Gly Thr Ala Gln Asn Lys Ala
50 55 60

Ala Asn Glu Trp Ile Glu Val Gly Leu Gly Gly Thr Lys Thr Val Lys
65 70 75 80

```
Gln Ile Asn Ile Asp Phe Glu Arg Lys Asp Ala Asp Gln Asn Ile Thr
                85                  90                  95

Ser Phe Lys Val Glu Leu Lys Gln Gly Asp Thr Tyr Thr Lys Val Tyr
            100                 105                 110

Gln Lys Asp Thr Arg Ala Lys Gln Gln Glu Ile Ile Leu Leu Asp Gln
        115                 120                 125

Ala Gln Gln Ala Ser Ala Val Lys Val Thr Val Leu Ser Ala Asp Gly
    130                 135                 140

Gly Thr Met Asn Trp Val Asn Val Gly Ile Asn Glu Ile Ser Val Tyr
145                 150                 155                 160

Ser Ala Pro Lys Glu Thr Val Leu Asp Thr Ala Asp Thr Asn His Met
                165                 170                 175

Leu Gly Ala Thr Met Thr Ala Ser Ser Asn Glu Thr Ala Thr Leu Thr
            180                 185                 190

Pro Asp Lys Ala Ile Asp Gln Asn Arg Thr Gly Arg Asn Asn Arg Trp
        195                 200                 205

Ala Ser Gly Tyr Glu Thr Pro Ser Asn Ile Trp Leu Lys Ala Glu Phe
    210                 215                 220

Pro Arg Leu Thr Ala Val Lys Asp Ile Arg Ile Tyr Phe Phe Glu Arg
225                 230                 235                 240

Asp Val Asn Pro Lys Pro Thr Asn Val Gln Ser Phe Asp Leu Ser Tyr
                245                 250                 255

Thr Asp Ser Glu Gly Thr Glu His Thr Leu Lys Ser Gly Tyr Ala Met
            260                 265                 270

Thr Ala Ser Gly Ala Gly Tyr Val Ala Asp Val Val Ile Gln Leu Asp
        275                 280                 285

Gln Ala Val Asn Ala Arg Ser Leu Lys Leu Ser Asn Phe Ala Ile Lys
    290                 295                 300

Ser Ser Glu Tyr Asn Asn Val Ser Val Ala Glu Trp Glu Ala Tyr Ser
305                 310                 315                 320

Asn Asp Gln Ala Glu Pro Gly Ala Thr Leu Asp Ser Val Val Ser Asp
                325                 330                 335
```

```
Leu Glu Ser Asn His Leu Thr Ile Glu Thr Asp Thr Asp Thr Leu Ala
            340                 345                 350

Leu Pro Thr Val Pro Asp Gly Tyr Thr Val Lys Phe Asn Gly Ala Asp
        355                 360                 365

Tyr Glu Gln Leu Ile Ala Ala Asp Gly Thr Val Asn His Pro Leu Val
    370                 375                 380

Asp Lys Thr Val Gln Val Ala Tyr Val Val Thr Asp Thr Ala Thr Gly
385                 390                 395                 400

Asn Thr Lys Thr Thr Ser Asp Ile Pro Tyr Val Val Lys Gly Thr Asn
                405                 410                 415

Gln Gln Gln Glu Gly Asn Asn Ala Lys Pro Thr Ile Ile Pro Glu Ile
            420                 425                 430

Ala Glu Trp His Ser Thr Ser Ala Ala Lys Leu Ala Ala Ser Ala Val
        435                 440                 445

Thr Lys Val Val Tyr Asp Asp Asp Ser Leu Lys Ala Val Val Asp Glu
    450                 455                 460

Phe Val Ala Asp Tyr Lys Asp Phe Thr Gly Ile Lys Leu Thr Ala Lys
465                 470                 475                 480

Lys Gly Ala Ala Glu Ala Gly Ala Phe Asn Phe Val Lys Thr Asp Ser
                485                 490                 495

Thr Ala Ala Ile Ala Gln Leu Gly Asp Glu Gly Tyr Thr Met Asp Ile
            500                 505                 510

Arg Ala Asp Arg Val Val Ala Lys Ser Ser Ser Val Thr Gly Asn Met
        515                 520                 525

Tyr Ala Met Gln Thr Ile Leu Gln Met Thr Lys Gln Asp Ala Asn Gly
    530                 535                 540

Phe Val Ile Gly Ser Met Arg Asp Tyr Pro Arg Phe Thr Thr Arg Gly
545                 550                 555                 560

Leu Leu Leu Asp Val Ala Arg Lys Pro Val Ser Leu Glu Met Met Arg
                565                 570                 575

Glu Ile Thr Arg Thr Met Arg Tyr Tyr Lys Met Asn Asp Phe Gln Ala
            580                 585                 590
```

His Leu Ser Asp Asn Tyr Ile Phe Leu Glu Asn Tyr Gly Lys Gly Asp
595 600 605

Asn Glu Asp Glu Ala Phe Lys Ala Tyr Asp Ala Phe Arg Leu Glu Ser
610 615 620

Ser Leu Thr Asn Asp Lys Gly Glu Ser Pro Thr Ala Glu Asp Tyr Ser
625 630 635 640

Ile Ser Lys Lys Thr Phe Lys Gln Phe Ile Gln Asp Glu Arg Ala Leu
645 650 655

Gly Met Asn Val Val Pro Glu Ile Asp Val Pro Ala His Ala Asn Ser
660 665 670

Phe Thr Lys Ile Trp Pro Glu Leu Met Val Lys Gly Arg Val Ser Pro
675 680 685

Ile Asn Ser Asn Arg Pro Leu Ile Asp His Leu Asp Val Ser Lys Pro
690 695 700

Glu Thr Ile Ala Lys Ile Lys Glu Ile Phe Asp Asp Tyr Thr Lys Gly
705 710 715 720

Asp Asp Pro Thr Phe Asp Ser Asp Thr Thr Val His Ile Gly Ala Gln
725 730 735

Glu Phe Leu Tyr Asn Tyr Thr Ala Tyr Arg Lys Phe Ile Asn Glu Ile
740 745 750

Val Pro Tyr Ile Lys Asp Thr Asn Thr Val Arg Met Trp Gly Gly Leu
755 760 765

Thr Trp Ile Asn Asp His Lys Thr Glu Ile Thr Lys Asp Ala Ile Glu
770 775 780

Asn Val Glu Met Asn Leu Trp Ser Lys Asp Trp Ala Asp Gly Leu Gln
785 790 795 800

Met Tyr Asn Met Gly Tyr Lys Leu Ile Asn Thr Ile Asp Asp Tyr Gly
805 810 815

Tyr Met Val Pro Asn Gly Ser Tyr Gly Arg Ala Asn Ala Tyr Gly Asp
820 825 830

Leu Leu Asn Ile Ser Arg Val Phe Asp Ser Phe Glu Pro Asn Lys Val

835 840 845

Arg Ser Ser Gly Gly Tyr Gln Ala Val Pro Ser Gly Asp Asp Gln Met
850 855 860

Leu Gly Ala Ala Phe Ala Ile Trp Ser Asp Asn Ile Asp Lys Ser Ala
865 870 875 880

Ser Gly Leu Thr Glu Ser Asp Leu Tyr Trp Arg Phe Phe Asp Ala Met
885 890 895

Pro Phe Tyr Ala Glu Lys Thr Trp Ala Ala Thr Gly Lys Glu Lys Gly
900 905 910

Thr Ala Ala Lys Leu Thr Ala Leu Ala Ala Lys Gln Gly Thr Gly Pro
915 920 925

Arg Thr Asn Pro Tyr Tyr Gln Ala Thr Ser Lys Asn Ser Val Tyr Glu
930 935 940

Ser Tyr Asp Phe Asn Asp Gly Leu Ala Asp Ala Ser Gly Asn Gly Arg
945 950 955 960

Asp Leu Thr Ile Gly Asp Gly Ser Lys Ala Ala Val Lys Asp Gln Ser
965 970 975

Leu Lys Leu Ala Gly Gly Ser Ser Tyr Ala Thr Ser Lys Leu Asp Lys
980 985 990

Leu Gly Asn Gly Asn Glu Leu Thr Phe Asp Val Thr Leu Gln Gln Ala
995 1000 1005

Ala Lys Pro Gly Asp Ile Leu Phe Glu Ala Asp Ala Pro Tyr Gly
1010 1015 1020

Thr His Asp Ile Arg Val Met Glu Asn Gly Lys Leu Gly Phe Thr
1025 1030 1035

Arg Glu Leu Tyr Asn Tyr Tyr Phe Asp Tyr Glu Leu Pro Val Gly
1040 1045 1050

Lys Thr Val Thr Val Thr Ile Lys Val Asp Gln Gln Thr Thr Lys
1055 1060 1065

Leu Tyr Val Asp Gly Glu Phe Val Ser Asp Ala Thr Gly Lys Tyr
1070 1075 1080

```
Ile Asp  Lys Gly Ile Glu Lys  Lys Thr Gly Ile Thr  Ala Ala Thr
    1085                 1090                 1095

Phe Ala  Leu Pro Leu Gln Arg  Ile Gly Ser Lys Thr  Ser Ala Ile
    1100                 1105                 1110

Asn Gly  Val Ile Asp Asn Val  Ile Val Lys Lys Ser  Glu Ala Glu
    1115                 1120                 1125

Thr Asp  Gln Tyr Asn Lys Ser  Cys Trp Thr Gly Thr  Thr Asn Ser
    1130                 1135                 1140

Glu Thr  Gln Tyr Asn Asp Thr  Glu Gly Leu Leu Arg  Tyr Ala Phe
    1145                 1150                 1155

Asp Asn  Asn Pro Ser Thr Ile  Trp His Ser Asn Trp  Lys Gly Ala
    1160                 1165                 1170

Thr Asp  Lys Leu Thr Gly Ser  Asn Ser Phe Tyr Ala  Glu Ile Asp
    1175                 1180                 1185

Met Cys  Gln Lys Tyr Thr Ile  Asn Gln Phe Ser Phe  Thr Pro Arg
    1190                 1195                 1200

Thr Ser  Gln Asp Ser Gly Gln  Val Thr Lys Ala Asp  Leu Tyr Val
    1205                 1210                 1215

Lys Ala  Asn Ala Asn Asp Glu  Trp Lys Gln Val Ala  Thr Asp Gln
    1220                 1225                 1230

Val Phe  Glu Ala Ser Arg Ala  Lys Lys Thr Phe Met  Phe Asp Glu
    1235                 1240                 1245

Gln Glu  Val Arg Tyr Val Lys  Phe Val Ala Lys Ser  Ser Asn Asp
    1250                 1255                 1260

Gly Trp  Val Ala Val Ser Glu  Phe Gly Val Ala Asn  Lys Pro Ser
    1265                 1270                 1275

Ser Thr  Val Arg Val Phe Val  Ala Ala Asp Pro Ala  Glu Gly Gly
    1280                 1285                 1290

Thr Val  Ser Val Ala Ala Glu  Gly Glu Thr Gly Thr  Asp Thr Ala
    1295                 1300                 1305

Val Asp  Val Ala Ser Gly Ala  Ser Val Thr Ala Lys  Ala Val Ala
    1310                 1315                 1320
```

```
Ala Asp  Gly Tyr Arg Phe Ser  Gly Trp Phe Thr Thr  Ala Ser Glu
    1325                 1330                 1335

Thr Ala  Val Ser Thr Asp Ala  Thr Tyr Thr Phe Ala  Ala Asp Gly
    1340                 1345                 1350

Asn Thr  Ala Leu Thr Ala Lys  Phe Thr Lys Asp Ser  Thr Pro Asp
    1355                 1360                 1365

Pro Gly  Pro Lys Pro Thr Ile  Ser Ser Ile Ala Val  Thr Lys Pro
    1370                 1375                 1380

Thr Val  Thr Asp Tyr Lys Val  Gly Asp Thr Phe Asp  Ala Thr Gly
    1385                 1390                 1395

Leu Ala  Val Thr Ala Thr Met  Ser Asp Gly Ser Thr  Lys Thr Leu
    1400                 1405                 1410

Thr Ala  Gly Glu Tyr Thr Leu  Ser Ala Thr Gln Asp  Gly Ala Ala
    1415                 1420                 1425

Val Ala  Leu Asp Lys Ala Phe  Ala Lys Ala Gly Lys  Val Thr Val
    1430                 1435                 1440

Thr Val  Thr Ala Asn Gly Lys  Thr Ala Thr Phe Asp  Val Thr Val
    1445                 1450                 1455

Thr Ala  Lys Asp Pro Asp Pro  Glu Pro Ala Thr Leu  Lys Ser Ile
    1460                 1465                 1470

Lys Val  Thr Ser Lys Pro Asp  Lys Thr Thr Tyr Thr  Val Asp Glu
    1475                 1480                 1485

Thr Phe  Ala Lys Thr Gly Leu  Ala Val Thr Gly Thr  Trp Ser Asp
    1490                 1495                 1500

Gly Lys  Thr Ala Leu Leu Lys  Asp Gly Glu Tyr Lys  Leu Ser Ala
    1505                 1510                 1515

Val Asp  Ala Asp Gly Lys Thr  Val Asp Leu Thr Lys  Pro Phe Thr
    1520                 1525                 1530

Ala Ala  Gly Asp Val Thr Val  Thr Val Thr Ser Gly  Lys Leu Thr
    1535                 1540                 1545

Asp Ser  Phe Thr Ile Thr Val  Lys Ala Lys Thr Val  Thr Pro Ala
    1550                 1555                 1560
```

```
Pro Gly  Asp Asn Lys Pro Gly  Glu Asn Lys Pro Gly  Ala Asp Lys
    1565                 1570                 1575

Pro Lys  Pro Asn Thr Pro Asp  Glu Val Ala Lys Leu  Glu His His
    1580                 1585                 1590

His His  His His
    1595

<210>  16
<211>  1597
<212>  PRT
<213>  Artificial Sequence

<220>
<223>  Artificial sequence

<400>  16

Met Ala Ser Met Thr Gly Gly Gln Gln Met Gly Arg Asp Pro Asn Ser
1               5                   10                  15

Ser Ser Val Asp Lys Leu Ser Asp Asp Asn Leu Ala Leu Asn Gln Thr
            20                  25                  30

Val Thr Ala Ser Ser Tyr Glu Val Ala Thr Thr Ala Pro Glu Lys Ala
        35                  40                  45

Val Asp Gly Asp Leu Gly Thr Arg Trp Gly Thr Ala Gln Asn Lys Ala
    50                  55                  60

Ala Asn Glu Trp Ile Glu Val Gly Leu Gly Gly Thr Lys Thr Val Lys
65                  70                  75                  80

Gln Ile Asn Ile Asp Phe Glu Arg Lys Asp Ala Asp Gln Asn Ile Thr
                85                  90                  95

Ser Phe Lys Val Glu Leu Lys Gln Gly Asp Thr Tyr Thr Lys Val Tyr
            100                 105                 110

Gln Lys Asp Thr Arg Ala Lys Gln Gln Glu Ile Ile Leu Leu Asp Gln
        115                 120                 125

Ala Gln Gln Ala Ser Ala Val Lys Val Thr Val Leu Ser Ala Asp Gly
    130                 135                 140

Gly Thr Met Asn Trp Val Asn Val Gly Ile Asn Glu Ile Ser Val Tyr
145                 150                 155                 160
```

Ser Ala Pro Lys Glu Thr Val Leu Asp Thr Ala Asp Thr Asn His Met
                165                 170                 175

Leu Gly Ala Thr Met Thr Ala Ser Ser Asn Glu Thr Ala Thr Leu Thr
            180                 185                 190

Pro Asp Lys Ala Ile Asp Gln Asn Arg Thr Gly Arg Asn Asn Arg Trp
        195                 200                 205

Ala Ser Gly Tyr Glu Thr Pro Ser Asn Ile Trp Leu Lys Ala Glu Phe
    210                 215                 220

Pro Arg Leu Thr Ala Val Lys Asp Ile Arg Ile Tyr Phe Phe Glu Arg
225                 230                 235                 240

Asp Val Asn Pro Lys Pro Thr Asn Val Gln Ser Phe Asp Leu Ser Tyr
                245                 250                 255

Thr Asp Ser Glu Gly Thr Glu His Thr Leu Lys Ser Gly Tyr Ala Met
            260                 265                 270

Thr Ala Ser Gly Ala Gly Tyr Val Ala Asp Val Val Ile Gln Leu Asp
        275                 280                 285

Gln Ala Val Asn Ala Arg Ser Leu Lys Leu Ser Asn Phe Ala Ile Lys
    290                 295                 300

Ser Ser Glu Tyr Asn Asn Val Ser Val Ala Glu Trp Glu Ala Tyr Ser
305                 310                 315                 320

Asn Asp Gln Ala Glu Pro Gly Ala Thr Leu Asp Ser Val Val Ser Asp
                325                 330                 335

Leu Glu Ser Asn His Leu Thr Ile Glu Thr Asp Thr Asp Thr Leu Ala
            340                 345                 350

Leu Pro Thr Val Pro Asp Gly Tyr Thr Val Lys Phe Asn Gly Ala Asp
        355                 360                 365

Tyr Glu Gln Leu Ile Ala Ala Asp Gly Thr Val Asn His Pro Leu Val
    370                 375                 380

Asp Lys Thr Val Gln Val Ala Tyr Val Val Thr Asp Thr Ala Thr Gly
385                 390                 395                 400

Asn Thr Lys Thr Thr Ser Asp Ile Pro Tyr Val Val Lys Gly Thr Asn
                405                 410                 415

Gln Gln Gln Glu Gly Asn Asn Ala Lys Pro Thr Ile Ile Pro Glu Ile
420 425 430

Ala Glu Trp His Ser Thr Ser Ala Ala Lys Leu Ala Ala Ser Ala Val
435 440 445

Thr Lys Val Val Tyr Asp Asp Asp Ser Leu Lys Ala Val Val Asp Glu
450 455 460

Phe Val Ala Asp Tyr Lys Asp Phe Thr Gly Ile Lys Leu Thr Ala Lys
465 470 475 480

Lys Gly Ala Ala Glu Ala Gly Ala Phe Asn Phe Val Lys Thr Asp Ser
485 490 495

Thr Ala Ala Ile Ala Gln Leu Gly Asp Glu Gly Tyr Thr Met Asp Ile
500 505 510

Arg Ala Asp Arg Val Val Ala Lys Ser Ser Ser Val Thr Gly Asn Met
515 520 525

Tyr Ala Met Gln Thr Ile Leu Gln Met Thr Lys Gln Asp Ala Asn Gly
530 535 540

Phe Val Ile Gly Ser Met Arg Asp Tyr Pro Arg Phe Thr Thr Arg Gly
545 550 555 560

Leu Leu Leu Asp Val Ala Arg Lys Pro Val Ser Leu Glu Met Met Arg
565 570 575

Glu Ile Thr Arg Thr Met Arg Tyr Tyr Lys Met Asn Asp Phe Gln Ala
580 585 590

His Leu Ser Asp Asn Tyr Ile Phe Leu Glu Asn Tyr Gly Lys Gly Asp
595 600 605

Asn Glu Asp Glu Ala Phe Lys Ala Tyr Asp Ala Phe Arg Leu Glu Ser
610 615 620

Ser Leu Thr Asn Asp Lys Gly Glu Ser Pro Thr Ala Glu Asp Tyr Ser
625 630 635 640

Ile Ser Lys Lys Thr Phe Lys Gln Phe Ile Gln Asp Glu Arg Ala Leu
645 650 655

Gly Met Asn Val Val Pro Glu Ile Asp Val Pro Ala His Ala Asn Ser

660 665 670

Phe Thr Lys Ile Trp Pro Glu Leu Met Val Lys Gly Arg Val Ser Pro
675 680 685

Ile Asn Ser Asn Arg Pro Leu Ile Asp His Leu Asp Val Ser Lys Pro
690 695 700

Glu Thr Ile Ala Lys Ile Lys Glu Ile Phe Asp Asp Tyr Thr Lys Gly
705 710 715 720

Asp Asp Pro Thr Phe Asp Ser Asp Thr Thr Val His Ile Gly Ala Asp
725 730 735

Glu Phe Leu Tyr Asn Tyr Thr Ala Tyr Arg Lys Phe Ile Asn Glu Ile
740 745 750

Val Pro Tyr Ile Lys Asp Thr Asn Thr Val Arg Met Trp Gly Gly Leu
755 760 765

Thr Trp Ile Asn Asp His Lys Thr Glu Ile Thr Lys Asp Ala Ile Glu
770 775 780

Asn Val Glu Met Asn Leu Trp Ser Lys Asp Trp Ala Asp Gly Leu Gln
785 790 795 800

Met Tyr Asn Met Gly Tyr Lys Leu Ile Asn Thr Ile Asp Asp Tyr Gly
805 810 815

Phe Met Val Pro Asn Gly Ser Tyr Gly Arg Ala Asn Ala Tyr Gly Asp
820 825 830

Leu Leu Asn Ile Ser Arg Val Phe Asp Ser Phe Glu Pro Asn Lys Val
835 840 845

Arg Ser Ser Gly Gly Tyr Gln Ala Val Pro Ser Gly Asp Asp Gln Met
850 855 860

Leu Gly Ala Ala Phe Ala Ile Trp Ser Asp Asn Ile Asp Lys Ser Ala
865 870 875 880

Ser Gly Leu Thr Glu Ser Asp Leu Tyr Trp Arg Phe Phe Asp Ala Met
885 890 895

Pro Phe Tyr Ala Glu Lys Thr Trp Ala Ala Thr Gly Lys Glu Lys Gly
900 905 910

```
Thr Ala Ala Lys Leu Thr Ala Leu Ala Ala Lys Gln Gly Thr Gly Pro
        915                 920                 925

Arg Thr Asn Pro Tyr Tyr Gln Ala Thr Ser Lys Asn Ser Val Tyr Glu
    930                 935                 940

Ser Tyr Asp Phe Asn Asp Gly Leu Ala Asp Ala Ser Gly Asn Gly Arg
945                 950                 955                 960

Asp Leu Thr Ile Gly Asp Gly Ser Lys Ala Ala Val Lys Asp Gln Ser
                965                 970                 975

Leu Lys Leu Ala Gly Gly Ser Ser Tyr Ala Thr Ser Lys Leu Asp Lys
            980                 985                 990

Leu Gly Asn Gly Asn Glu Leu Thr  Phe Asp Val Thr Leu  Gln Gln Ala
        995                 1000                 1005

Ala Lys  Pro Gly Asp Ile Leu  Phe Glu Ala Asp Ala  Pro Tyr Gly
    1010                 1015                 1020

Thr His  Asp Ile Arg Val Met  Glu Asn Gly Lys Leu  Gly Phe Thr
    1025                 1030                 1035

Arg Glu  Leu Tyr Asn Tyr Tyr  Phe Asp Tyr Glu Leu  Pro Val Gly
    1040                 1045                 1050

Lys Thr  Val Thr Val Thr Ile  Lys Val Asp Gln Gln  Thr Thr Lys
    1055                 1060                 1065

Leu Tyr  Val Asp Gly Glu Phe  Val Ser Asp Ala Thr  Gly Lys Tyr
    1070                 1075                 1080

Ile Asp  Lys Gly Ile Glu Lys  Lys Thr Gly Ile Thr  Ala Ala Thr
    1085                 1090                 1095

Phe Ala  Leu Pro Leu Gln Arg  Ile Gly Ser Lys Thr  Ser Ala Ile
    1100                 1105                 1110

Asn Gly  Val Ile Asp Asn Val  Ile Val Lys Lys Ser  Glu Ala Glu
    1115                 1120                 1125

Thr Asp  Gln Tyr Asn Lys Ser  Cys Trp Thr Gly Thr  Thr Asn Ser
    1130                 1135                 1140

Glu Thr  Gln Tyr Asn Asp Thr  Glu Gly Leu Leu Arg  Tyr Ala Phe
    1145                 1150                 1155
```

Asp Asn Asn Pro Ser Thr Ile Trp His Ser Asn Trp Lys Gly Ala
1160 1165 1170

Thr Asp Lys Leu Thr Gly Ser Asn Ser Phe Tyr Ala Glu Ile Asp
1175 1180 1185

Met Cys Gln Lys Tyr Thr Ile Asn Gln Phe Ser Phe Thr Pro Arg
1190 1195 1200

Thr Ser Gln Asp Ser Gly Gln Val Thr Lys Ala Asp Leu Tyr Val
1205 1210 1215

Lys Ala Asn Ala Asn Asp Glu Trp Lys Gln Val Ala Thr Asp Gln
1220 1225 1230

Val Phe Glu Ala Ser Arg Ala Lys Lys Thr Phe Met Phe Asp Glu
1235 1240 1245

Gln Glu Val Arg Tyr Val Lys Phe Val Ala Lys Ser Ser Asn Asp
1250 1255 1260

Gly Trp Val Ala Val Ser Glu Phe Gly Val Ala Asn Lys Pro Ser
1265 1270 1275

Ser Thr Val Arg Val Phe Val Ala Ala Asp Pro Ala Glu Gly Gly
1280 1285 1290

Thr Val Ser Val Ala Ala Glu Gly Glu Thr Gly Thr Asp Thr Ala
1295 1300 1305

Val Asp Val Ala Ser Gly Ala Ser Val Thr Ala Lys Ala Val Ala
1310 1315 1320

Ala Asp Gly Tyr Arg Phe Ser Gly Trp Phe Thr Thr Ala Ser Glu
1325 1330 1335

Thr Ala Val Ser Thr Asp Ala Thr Tyr Thr Phe Ala Ala Asp Gly
1340 1345 1350

Asn Thr Ala Leu Thr Ala Lys Phe Thr Lys Asp Ser Thr Pro Asp
1355 1360 1365

Pro Gly Pro Lys Pro Thr Ile Ser Ser Ile Ala Val Thr Lys Pro
1370 1375 1380

Thr Val Thr Asp Tyr Lys Val Gly Asp Thr Phe Asp Ala Thr Gly
1385 1390 1395

Leu Ala Val Thr Ala Thr Met Ser Asp Gly Ser Thr Lys Thr Leu
1400 1405 1410

Thr Ala Gly Glu Tyr Thr Leu Ser Ala Thr Gln Asp Gly Ala Ala
1415 1420 1425

Val Ala Leu Asp Lys Ala Phe Ala Lys Ala Gly Lys Val Thr Val
1430 1435 1440

Thr Val Thr Ala Asn Gly Lys Thr Ala Thr Phe Asp Val Thr Val
1445 1450 1455

Thr Ala Lys Asp Pro Asp Pro Glu Pro Ala Thr Leu Lys Ser Ile
1460 1465 1470

Lys Val Thr Ser Lys Pro Asp Lys Thr Thr Tyr Thr Val Asp Glu
1475 1480 1485

Thr Phe Ala Lys Thr Gly Leu Ala Val Thr Gly Thr Trp Ser Asp
1490 1495 1500

Gly Lys Thr Ala Leu Leu Lys Asp Gly Glu Tyr Lys Leu Ser Ala
1505 1510 1515

Val Asp Ala Asp Gly Lys Thr Val Asp Leu Thr Lys Pro Phe Thr
1520 1525 1530

Ala Ala Gly Asp Val Thr Val Thr Val Thr Ser Gly Lys Leu Thr
1535 1540 1545

Asp Ser Phe Thr Ile Thr Val Lys Ala Lys Thr Val Thr Pro Ala
1550 1555 1560

Pro Gly Asp Asn Lys Pro Gly Glu Asn Lys Pro Gly Ala Asp Lys
1565 1570 1575

Pro Lys Pro Asn Thr Pro Asp Glu Val Ala Lys Leu Glu His His
1580 1585 1590

His His His His
1595

<210> 17
<211> 1039
<212> PRT
<213> Artificial Sequence

<220>
<223> Artificial sequence

<400> 17

Met Ala Asp Asp Ser Ala Ala Gly Tyr Ser Ala Thr Ala Pro Val Asn
1 5 10 15

Leu Thr Arg Pro Ala Thr Val Pro Ser Met Asp Gly Trp Thr Asp Gly
20 25 30

Thr Gly Ala Trp Thr Leu Gly Glu Gly Thr Arg Val Val Ser Ser Asp
35 40 45

Ala Leu Ala Ala Arg Ala Gln Ser Leu Ala Ser Glu Leu Thr Lys Phe
50 55 60

Thr Asp Val Asp Ile Lys Ala Ala Thr Gly Ser Ala Thr Gly Lys Asp
65 70 75 80

Ile Ser Leu Thr Leu Asp Ala Ser Lys Lys Ala Glu Leu Gly Asp Glu
85 90 95

Gly Phe Lys Leu Asn Ile Gly Ser Lys Gly Leu Glu Val Ile Gly Ala
100 105 110

Thr Asp Ile Gly Val Phe Tyr Gly Thr Arg Ser Val Ser Gln Met Leu
115 120 125

Arg Gln Gly Gln Leu Thr Leu Pro Ala Gly Thr Val Ala Thr Lys Pro
130 135 140

Lys Tyr Lys Glu Arg Gly Ala Thr Leu Cys Ala Cys Gln Ile Asn Ile
145 150 155 160

Ser Thr Asp Trp Ile Asp Arg Phe Leu Ser Asp Met Ala Asp Leu Arg
165 170 175

Leu Asn Tyr Val Leu Leu Glu Met Lys Leu Lys Pro Glu Glu Asp Asn
180 185 190

Thr Lys Lys Ala Ala Thr Trp Ser Tyr Tyr Thr Arg Asp Asp Val Lys
195 200 205

Lys Phe Val Lys Lys Ala Asn Asn Tyr Gly Ile Asp Val Ile Pro Glu
210 215 220

Ile Asn Ser Pro Gly His Met Asn Val Trp Leu Glu Asn Tyr Pro Glu
225 230 235 240

Tyr Gln Leu Ala Asp Asn Ser Gly Arg Lys Asp Pro Asn Lys Leu Asp
245 250 255

Ile Ser Asn Pro Glu Ala Val Lys Phe Tyr Lys Thr Leu Ile Asp Glu
260 265 270

Tyr Asp Gly Val Phe Thr Thr Lys Tyr Trp His Met Gly Ala Asp Glu
275 280 285

Tyr Met Ile Gly Thr Ser Phe Asp Asn Tyr Ser Lys Leu Lys Thr Phe
290 295 300

Ala Glu Lys Gln Tyr Gly Ala Gly Ala Thr Pro Asn Asp Ala Phe Thr
305 310 315 320

Gly Phe Ile Asn Asp Ile Asp Lys Tyr Val Lys Ala Lys Gly Lys Gln
325 330 335

Leu Arg Ile Trp Asn Asp Gly Ile Val Asn Thr Lys Asn Val Ser Leu
340 345 350

Asn Lys Asp Ile Val Ile Glu Tyr Trp Tyr Gly Ala Gly Arg Lys Pro
355 360 365

Gln Glu Leu Val Gln Asp Gly Tyr Thr Leu Met Asn Ala Thr Gln Ala
370 375 380

Leu Tyr Trp Ser Arg Ser Ala Gln Val Tyr Lys Val Asn Ala Ala Arg
385 390 395 400

Leu Tyr Asn Asn Asn Trp Asn Val Gly Thr Phe Asp Gly Gly Arg Gln
405 410 415

Ile Asp Lys Asn Tyr Asp Lys Leu Thr Gly Ala Lys Val Ser Ile Trp
420 425 430

Pro Asp Ser Ser Tyr Phe Gln Thr Glu Asn Glu Val Glu Lys Glu Ile
435 440 445

Phe Asp Gly Met Arg Phe Ile Ser Gln Met Thr Trp Ser Asp Ser Arg
450 455 460

Pro Trp Ala Thr Trp Asn Asp Met Lys Ala Asp Ile Asp Lys Ile Gly
465 470 475 480

Tyr Pro Leu Asp Ile Arg Glu Tyr Asp Tyr Thr Pro Val Asp Ala Gly

```
                485                 490                 495

Ile Tyr Asp Ile Pro Gln Leu Lys Ser Ile Ser Lys Gly Pro Trp Glu
            500                 505                 510

Leu Ile Thr Thr Pro Asp Gly Tyr Tyr Gln Met Lys Asp Thr Val Ser
        515                 520                 525

Gly Lys Cys Leu Ala Leu Phe Thr Gly Ser Lys His Leu Asp Val Val
    530                 535                 540

Thr Gln Val Gly Ala Arg Pro Glu Leu Arg Asn Cys Ala Asp Val Ser
545                 550                 555                 560

Val Gly Gln Asp Gln Arg Asn Thr Ala Asn Glu Arg Asn Thr Gln Lys
                565                 570                 575

Trp Gln Ile Arg Ala Asp Lys Asp Gly Lys Tyr Thr Ile Ser Pro Ala
            580                 585                 590

Leu Thr Gln Gln Arg Leu Ala Ile Ala Thr Gly Asn Glu Gln Asn Ile
        595                 600                 605

Asp Leu Glu Thr His Arg Pro Ala Ala Gly Thr Val Ala Gln Phe Pro
    610                 615                 620

Ala Asp Leu Val Ser Asp Asn Ala Leu Phe Thr Leu Thr Gly His Met
625                 630                 635                 640

Gly Met Ser Ala Thr Val Asp Ser Lys Thr Val Asn Pro Ala Ser Pro
                645                 650                 655

Ser Lys Ile Thr Val Lys Val Arg Ala Ala Ser Asn Ala Asn Thr Gly
            660                 665                 670

Asp Val Thr Val Thr Pro Val Val Pro Glu Gly Trp Glu Ile Lys Pro
        675                 680                 685

Gly Ser Val Ser Leu Lys Ser Ile Pro Ala Gly Lys Ala Ala Ile Ala
    690                 695                 700

Tyr Phe Asn Val Val Asn Thr Thr Gly Thr Gly Asp Ala Thr Val Gln
705                 710                 715                 720

Phe Lys Leu Thr Asn Thr Lys Thr Gly Glu Glu Leu Gly Thr Thr Ser
                725                 730                 735
```

Val Ala Leu Thr Gly Ser Leu Thr Lys Asp Val Glu Ala Ser Asp Tyr
740 745 750

Ala Ala Ser Ser Gln Glu Thr Thr Gly Glu His Ala Pro Val Gly Asn
755 760 765

Ala Phe Asp Lys Asn Ala Asn Thr Phe Trp His Ser Lys Tyr Ser Asn
770 775 780

Pro Ser Ala Asn Leu Pro His Trp Leu Ala Phe Lys Ala Ser Pro Gly
785 790 795 800

Glu Gly Asn Lys Ile Ala Ala Ile Thr His Leu Tyr Arg Gln Asp Lys
805 810 815

Leu Asn Gly Pro Ala Lys Asn Val Ala Val Tyr Val Val Ala Ala Ser
820 825 830

Asp Ala Asn Ser Val Ala Asp Val Thr Asn Trp Gly Glu Pro Val Ala
835 840 845

Thr Ala Glu Phe Pro Tyr Thr Lys Glu Leu Gln Thr Ile Ala Leu Pro
850 855 860

Asn Thr Ile Pro Ser Gly Asp Val Tyr Val Lys Phe Gln Ile Asn Asp
865 870 875 880

Ala Trp Gly Leu Thr Glu Thr Ser Ala Gly Val Thr Trp Ala Ala Val
885 890 895

Ala Glu Leu Ala Ala Thr Ala Lys Ala Thr Pro Val Glu Leu Thr Glu
900 905 910

Pro Glu Gln Pro Lys Asp Asn Pro Glu Val Thr Glu Thr Pro Glu Ala
915 920 925

Thr Gly Val Thr Val Ser Gly Asp Gly Val Ala Asn Gly Ala Leu Ser
930 935 940

Leu Lys Lys Gly Thr Thr Ala Gln Leu Thr Ala Lys Val Ala Pro Asp
945 950 955 960

Asp Ala Asp Gln Ala Val Thr Trp Ala Ser Ser Asp Asp Lys Val Val
965 970 975

Thr Val Asp Lys Thr Gly Lys Val Thr Ala Val Ala Lys Gly Val Ala
980 985 990

Lys Val Thr Ala Thr Thr Ala Asn Gly Lys Ser Ala Ser Val Thr Val
995 1000 1005

Thr Val Thr Glu Asp Ser Glu Val Pro Gly Pro Thr Gly Pro Thr
1010 1015 1020

Glu Pro Thr Lys Pro Gly Thr Glu Leu Glu His His His His His
1025 1030 1035

His

<210> 18
<211> 1039
<212> PRT
<213> Artificial Sequence

<220>
<223> Artificial sequence

<400> 18

Met Ala Asp Asp Ser Ala Ala Gly Tyr Ser Ala Thr Ala Pro Val Asn
1 5 10 15

Leu Thr Arg Pro Ala Thr Val Pro Ser Met Asp Gly Trp Thr Asp Gly
20 25 30

Thr Gly Ala Trp Thr Leu Gly Glu Gly Thr Arg Val Val Ser Ser Asp
35 40 45

Ala Leu Ala Ala Arg Ala Gln Ser Leu Ala Ser Glu Leu Thr Lys Phe
50 55 60

Thr Asp Val Asp Ile Lys Ala Ala Thr Gly Ser Ala Thr Gly Lys Asp
65 70 75 80

Ile Ser Leu Thr Leu Asp Ala Ser Lys Lys Ala Glu Leu Gly Asp Glu
85 90 95

Gly Phe Lys Leu Asn Ile Gly Ser Lys Gly Leu Glu Val Ile Gly Ala
100 105 110

Thr Asp Ile Gly Val Phe Tyr Gly Thr Arg Ser Val Ser Gln Met Leu
115 120 125

Arg Gln Gly Gln Leu Thr Leu Pro Ala Gly Thr Val Ala Thr Lys Pro
130 135 140

Lys Tyr Lys Glu Arg Gly Ala Thr Leu Cys Ala Cys Gln Ile Asn Ile
145                 150                 155                 160

Ser Thr Asp Trp Ile Asp Arg Phe Leu Ser Asp Met Ala Asp Leu Arg
                165                 170                 175

Leu Asn Tyr Val Leu Leu Glu Met Lys Leu Lys Pro Glu Glu Asp Asn
            180                 185                 190

Thr Lys Lys Ala Ala Thr Trp Ser Tyr Tyr Thr Arg Asp Asp Val Lys
        195                 200                 205

Lys Phe Val Lys Lys Ala Asn Asn Tyr Gly Ile Asp Val Ile Pro Glu
    210                 215                 220

Ile Asn Ser Pro Gly His Met Asn Val Trp Leu Glu Asn Tyr Pro Glu
225                 230                 235                 240

Tyr Gln Leu Ala Asp Asn Ser Gly Arg Lys Asp Pro Asn Lys Leu Asp
                245                 250                 255

Ile Ser Asn Pro Glu Ala Val Lys Phe Tyr Lys Thr Leu Ile Asp Glu
            260                 265                 270

Tyr Asp Gly Val Phe Thr Thr Lys Tyr Trp His Met Gly Ala Glu Glu
        275                 280                 285

Tyr Met Ile Gly Thr Ser Phe Asp Asn Tyr Ser Lys Leu Lys Thr Phe
    290                 295                 300

Ala Glu Lys Gln Tyr Gly Ala Gly Ala Thr Pro Asn Asp Ala Phe Thr
305                 310                 315                 320

Gly Phe Ile Asn Asp Ile Asp Lys Tyr Val Lys Ala Lys Gly Lys Gln
                325                 330                 335

Leu Arg Ile Trp Asn Asp Gly Ile Val Asn Thr Lys Asn Val Ser Leu
            340                 345                 350

Asn Lys Asp Ile Val Ile Glu Tyr Trp Tyr Gly Ala Gly Arg Lys Pro
        355                 360                 365

Gln Glu Leu Val Gln Asp Gly Tyr Thr Leu Met Asn Ala Thr Gln Ala
    370                 375                 380

Leu Tyr Trp Ser Arg Ser Ala Gln Val Tyr Lys Val Asn Ala Ala Arg
385                 390                 395                 400

```
Leu Tyr Asn Asn Asn Trp Asn Val Gly Thr Phe Asp Gly Gly Arg Gln
                405                 410                 415

Ile Asp Lys Asn Tyr Asp Lys Leu Thr Gly Ala Lys Val Ser Ile Trp
            420                 425                 430

Pro Asp Ser Ser Tyr Phe Gln Thr Glu Asn Glu Val Glu Lys Glu Ile
        435                 440                 445

Phe Asp Gly Met Arg Phe Ile Ser Gln Met Thr Trp Ser Asp Ser Arg
    450                 455                 460

Pro Trp Ala Thr Trp Asn Asp Met Lys Ala Asp Ile Asp Lys Ile Gly
465                 470                 475                 480

Tyr Pro Leu Asp Ile Arg Glu Tyr Asp Tyr Thr Pro Val Asp Ala Gly
                485                 490                 495

Ile Tyr Asp Ile Pro Gln Leu Lys Ser Ile Ser Lys Gly Pro Trp Glu
            500                 505                 510

Leu Ile Thr Thr Pro Asp Gly Tyr Tyr Gln Met Lys Asp Thr Val Ser
        515                 520                 525

Gly Lys Cys Leu Ala Leu Phe Thr Gly Ser Lys His Leu Asp Val Val
    530                 535                 540

Thr Gln Val Gly Ala Arg Pro Glu Leu Arg Asn Cys Ala Asp Val Ser
545                 550                 555                 560

Val Gly Gln Asp Gln Arg Asn Thr Ala Asn Glu Arg Asn Thr Gln Lys
                565                 570                 575

Trp Gln Ile Arg Ala Asp Lys Asp Gly Lys Tyr Thr Ile Ser Pro Ala
            580                 585                 590

Leu Thr Gln Gln Arg Leu Ala Ile Ala Thr Gly Asn Glu Gln Asn Ile
        595                 600                 605

Asp Leu Glu Thr His Arg Pro Ala Ala Gly Thr Val Ala Gln Phe Pro
    610                 615                 620

Ala Asp Leu Val Ser Asp Asn Ala Leu Phe Thr Leu Thr Gly His Met
625                 630                 635                 640

Gly Met Ser Ala Thr Val Asp Ser Lys Thr Val Asn Pro Ala Ser Pro
                645                 650                 655
```

Ser Lys Ile Thr Val Lys Val Arg Ala Ala Ser Asn Ala Asn Thr Gly
660 665 670

Asp Val Thr Val Thr Pro Val Val Pro Glu Gly Trp Glu Ile Lys Pro
675 680 685

Gly Ser Val Ser Leu Lys Ser Ile Pro Ala Gly Lys Ala Ala Ile Ala
690 695 700

Tyr Phe Asn Val Val Asn Thr Thr Gly Thr Gly Asp Ala Thr Val Gln
705 710 715 720

Phe Lys Leu Thr Asn Thr Lys Thr Gly Glu Glu Leu Gly Thr Thr Ser
725 730 735

Val Ala Leu Thr Gly Ser Leu Thr Lys Asp Val Glu Ala Ser Asp Tyr
740 745 750

Ala Ala Ser Ser Gln Glu Thr Thr Gly Glu His Ala Pro Val Gly Asn
755 760 765

Ala Phe Asp Lys Asn Ala Asn Thr Phe Trp His Ser Lys Tyr Ser Asn
770 775 780

Pro Ser Ala Asn Leu Pro His Trp Leu Ala Phe Lys Ala Ser Pro Gly
785 790 795 800

Glu Gly Asn Lys Ile Ala Ala Ile Thr His Leu Tyr Arg Gln Asp Lys
805 810 815

Leu Asn Gly Pro Ala Lys Asn Val Ala Val Tyr Val Val Ala Ala Ser
820 825 830

Asp Ala Asn Ser Val Ala Asp Val Thr Asn Trp Gly Glu Pro Val Ala
835 840 845

Thr Ala Glu Phe Pro Tyr Thr Lys Glu Leu Gln Thr Ile Ala Leu Pro
850 855 860

Asn Thr Ile Pro Ser Gly Asp Val Tyr Val Lys Phe Gln Ile Asn Asp
865 870 875 880

Ala Trp Gly Leu Thr Glu Thr Ser Ala Gly Val Thr Trp Ala Ala Val
885 890 895

Ala Glu Leu Ala Ala Thr Ala Lys Ala Thr Pro Val Glu Leu Thr Glu

900 905 910

Pro Glu Gln Pro Lys Asp Asn Pro Glu Val Thr Glu Thr Pro Glu Ala
915 920 925

Thr Gly Val Thr Val Ser Gly Asp Gly Val Ala Asn Gly Ala Leu Ser
930 935 940

Leu Lys Lys Gly Thr Thr Ala Gln Leu Thr Ala Lys Val Ala Pro Asp
945 950 955 960

Asp Ala Asp Gln Ala Val Thr Trp Ala Ser Ser Asp Asp Lys Val Val
965 970 975

Thr Val Asp Lys Thr Gly Lys Val Thr Ala Val Ala Lys Gly Val Ala
980 985 990

Lys Val Thr Ala Thr Thr Ala Asn Gly Lys Ser Ala Ser Val Thr Val
995 1000 1005

Thr Val Thr Glu Asp Ser Glu Val Pro Gly Pro Thr Gly Pro Thr
1010 1015 1020

Glu Pro Thr Lys Pro Gly Thr Glu Leu Glu His His His His His
1025 1030 1035

His

<210> 19
<211> 1039
<212> PRT
<213> Artificial Sequence

<220>
<223> Artificial sequence

<400> 19

Met Ala Asp Asp Ser Ala Ala Gly Tyr Ser Ala Thr Ala Pro Val Asn
1 5 10 15

Leu Thr Arg Pro Ala Thr Val Pro Ser Met Asp Gly Trp Thr Asp Gly
20 25 30

Thr Gly Ala Trp Thr Leu Gly Glu Gly Thr Arg Val Val Ser Ser Asp
35 40 45

Ala Leu Ala Ala Arg Ala Gln Ser Leu Ala Ser Glu Leu Thr Lys Phe
50 55 60

Thr Asp Val Asp Ile Lys Ala Ala Thr Gly Ser Ala Thr Gly Lys Asp
65                  70                  75                  80

Ile Ser Leu Thr Leu Asp Ala Ser Lys Lys Ala Glu Leu Gly Asp Glu
                85                  90                  95

Gly Phe Lys Leu Asn Ile Gly Ser Lys Gly Leu Glu Val Ile Gly Ala
            100                 105                 110

Thr Asp Ile Gly Val Phe Tyr Gly Thr Arg Ser Val Ser Gln Met Leu
        115                 120                 125

Arg Gln Gly Gln Leu Thr Leu Pro Ala Gly Thr Val Ala Thr Lys Pro
    130                 135                 140

Lys Tyr Lys Glu Arg Gly Ala Thr Leu Cys Ala Cys Gln Ile Asn Ile
145                 150                 155                 160

Ser Thr Asp Trp Ile Asp Arg Phe Leu Ser Asp Met Ala Asp Leu Arg
                165                 170                 175

Leu Asn Tyr Val Leu Leu Glu Met Lys Leu Lys Pro Glu Glu Asp Asn
            180                 185                 190

Thr Lys Lys Ala Ala Thr Trp Ser Tyr Tyr Thr Arg Asp Asp Val Lys
        195                 200                 205

Lys Phe Val Lys Lys Ala Asn Asn Tyr Gly Ile Asp Val Ile Pro Glu
    210                 215                 220

Ile Asn Ser Pro Gly His Met Asn Val Trp Leu Glu Asn Tyr Pro Glu
225                 230                 235                 240

Tyr Gln Leu Ala Asp Asn Ser Gly Arg Lys Asp Pro Asn Lys Leu Asp
                245                 250                 255

Ile Ser Asn Pro Glu Ala Val Lys Phe Tyr Lys Thr Leu Ile Asp Glu
            260                 265                 270

Tyr Asp Gly Val Phe Thr Thr Lys Tyr Trp His Met Gly Ala Ala Glu
        275                 280                 285

Tyr Met Ile Gly Thr Ser Phe Asp Asn Tyr Ser Lys Leu Lys Thr Phe
    290                 295                 300

Ala Glu Lys Gln Tyr Gly Ala Gly Ala Thr Pro Asn Asp Ala Phe Thr

305 310 315 320

Gly Phe Ile Asn Asp Ile Asp Lys Tyr Val Lys Ala Lys Gly Lys Gln
325 330 335

Leu Arg Ile Trp Asn Asp Gly Ile Val Asn Thr Lys Asn Val Ser Leu
340 345 350

Asn Lys Asp Ile Val Ile Glu Tyr Trp Tyr Gly Ala Gly Arg Lys Pro
355 360 365

Gln Glu Leu Val Gln Asp Gly Tyr Thr Leu Met Asn Ala Thr Gln Ala
370 375 380

Leu Tyr Trp Ser Arg Ser Ala Gln Val Tyr Lys Val Asn Ala Ala Arg
385 390 395 400

Leu Tyr Asn Asn Asn Trp Asn Val Gly Thr Phe Asp Gly Gly Arg Gln
405 410 415

Ile Asp Lys Asn Tyr Asp Lys Leu Thr Gly Ala Lys Val Ser Ile Trp
420 425 430

Pro Asp Ser Ser Tyr Phe Gln Thr Glu Asn Glu Val Glu Lys Glu Ile
435 440 445

Phe Asp Gly Met Arg Phe Ile Ser Gln Met Thr Trp Ser Asp Ser Arg
450 455 460

Pro Trp Ala Thr Trp Asn Asp Met Lys Ala Asp Ile Asp Lys Ile Gly
465 470 475 480

Tyr Pro Leu Asp Ile Arg Glu Tyr Asp Tyr Thr Pro Val Asp Ala Gly
485 490 495

Ile Tyr Asp Ile Pro Gln Leu Lys Ser Ile Ser Lys Gly Pro Trp Glu
500 505 510

Leu Ile Thr Thr Pro Asp Gly Tyr Tyr Gln Met Lys Asp Thr Val Ser
515 520 525

Gly Lys Cys Leu Ala Leu Phe Thr Gly Ser Lys His Leu Asp Val Val
530 535 540

Thr Gln Val Gly Ala Arg Pro Glu Leu Arg Asn Cys Ala Asp Val Ser
545 550 555 560

Val Gly Gln Asp Gln Arg Asn Thr Ala Asn Glu Arg Asn Thr Gln Lys
565 570 575

Trp Gln Ile Arg Ala Asp Lys Asp Gly Lys Tyr Thr Ile Ser Pro Ala
580 585 590

Leu Thr Gln Gln Arg Leu Ala Ile Ala Thr Gly Asn Glu Gln Asn Ile
595 600 605

Asp Leu Glu Thr His Arg Pro Ala Ala Gly Thr Val Ala Gln Phe Pro
610 615 620

Ala Asp Leu Val Ser Asp Asn Ala Leu Phe Thr Leu Thr Gly His Met
625 630 635 640

Gly Met Ser Ala Thr Val Asp Ser Lys Thr Val Asn Pro Ala Ser Pro
645 650 655

Ser Lys Ile Thr Val Lys Val Arg Ala Ala Ser Asn Ala Asn Thr Gly
660 665 670

Asp Val Thr Val Thr Pro Val Val Pro Glu Gly Trp Glu Ile Lys Pro
675 680 685

Gly Ser Val Ser Leu Lys Ser Ile Pro Ala Gly Lys Ala Ala Ile Ala
690 695 700

Tyr Phe Asn Val Val Asn Thr Thr Gly Thr Gly Asp Ala Thr Val Gln
705 710 715 720

Phe Lys Leu Thr Asn Thr Lys Thr Gly Glu Glu Leu Gly Thr Thr Ser
725 730 735

Val Ala Leu Thr Gly Ser Leu Thr Lys Asp Val Glu Ala Ser Asp Tyr
740 745 750

Ala Ala Ser Ser Gln Glu Thr Thr Gly Glu His Ala Pro Val Gly Asn
755 760 765

Ala Phe Asp Lys Asn Ala Asn Thr Phe Trp His Ser Lys Tyr Ser Asn
770 775 780

Pro Ser Ala Asn Leu Pro His Trp Leu Ala Phe Lys Ala Ser Pro Gly
785 790 795 800

Glu Gly Asn Lys Ile Ala Ala Ile Thr His Leu Tyr Arg Gln Asp Lys
805 810 815

```
Leu Asn Gly Pro Ala Lys Asn Val Ala Val Tyr Val Val Ala Ala Ser
            820                 825                 830

Asp Ala Asn Ser Val Ala Asp Val Thr Asn Trp Gly Glu Pro Val Ala
        835                 840                 845

Thr Ala Glu Phe Pro Tyr Thr Lys Glu Leu Gln Thr Ile Ala Leu Pro
    850                 855                 860

Asn Thr Ile Pro Ser Gly Asp Val Tyr Val Lys Phe Gln Ile Asn Asp
865                 870                 875                 880

Ala Trp Gly Leu Thr Glu Thr Ser Ala Gly Val Thr Trp Ala Ala Val
                885                 890                 895

Ala Glu Leu Ala Ala Thr Ala Lys Ala Thr Pro Val Glu Leu Thr Glu
            900                 905                 910

Pro Glu Gln Pro Lys Asp Asn Pro Glu Val Thr Glu Thr Pro Glu Ala
        915                 920                 925

Thr Gly Val Thr Val Ser Gly Asp Gly Val Ala Asn Gly Ala Leu Ser
    930                 935                 940

Leu Lys Lys Gly Thr Thr Ala Gln Leu Thr Ala Lys Val Ala Pro Asp
945                 950                 955                 960

Asp Ala Asp Gln Ala Val Thr Trp Ala Ser Ser Asp Asp Lys Val Val
                965                 970                 975

Thr Val Asp Lys Thr Gly Lys Val Thr Ala Val Ala Lys Gly Val Ala
            980                 985                 990

Lys Val Thr Ala Thr Thr Ala Asn  Gly Lys Ser Ala Ser  Val Thr Val
        995                 1000                1005

Thr Val  Thr Glu Asp Ser Glu  Val Pro Gly Pro Thr  Gly Pro Thr
    1010                1015                1020

Glu Pro  Thr Lys Pro Gly Thr  Glu Leu Glu His His  His His His
    1025                1030                1035

His


<210>  20
<211>  1039
```

<212> PRT
<213> Artificial Sequence

<220>
<223> Artificial sequence

<400> 20

```
Met Ala Asp Asp Ser Ala Ala Gly Tyr Ser Ala Thr Ala Pro Val Asn
1               5                   10                  15

Leu Thr Arg Pro Ala Thr Val Pro Ser Met Asp Gly Trp Thr Asp Gly
            20                  25                  30

Thr Gly Ala Trp Thr Leu Gly Glu Gly Thr Arg Val Val Ser Ser Asp
        35                  40                  45

Ala Leu Ala Ala Arg Ala Gln Ser Leu Ala Ser Glu Leu Thr Lys Phe
    50                  55                  60

Thr Asp Val Asp Ile Lys Ala Ala Thr Gly Ser Ala Thr Gly Lys Asp
65                  70                  75                  80

Ile Ser Leu Thr Leu Asp Ala Ser Lys Lys Ala Glu Leu Gly Asp Glu
                85                  90                  95

Gly Phe Lys Leu Asn Ile Gly Ser Lys Gly Leu Glu Val Ile Gly Ala
            100                 105                 110

Thr Asp Ile Gly Val Phe Tyr Gly Thr Arg Ser Val Ser Gln Met Leu
        115                 120                 125

Arg Gln Gly Gln Leu Thr Leu Pro Ala Gly Thr Val Ala Thr Lys Pro
    130                 135                 140

Lys Tyr Lys Glu Arg Gly Ala Thr Leu Cys Ala Cys Gln Ile Asn Ile
145                 150                 155                 160

Ser Thr Asp Trp Ile Asp Arg Phe Leu Ser Asp Met Ala Asp Leu Arg
                165                 170                 175

Leu Asn Tyr Val Leu Leu Glu Met Lys Leu Lys Pro Glu Glu Asp Asn
            180                 185                 190

Thr Lys Lys Ala Ala Thr Trp Ser Tyr Tyr Thr Arg Asp Asp Val Lys
        195                 200                 205

Lys Phe Val Lys Lys Ala Asn Asn Tyr Gly Ile Asp Val Ile Pro Glu
    210                 215                 220
```

```
Ile Asn Ser Pro Gly His Met Asn Val Trp Leu Glu Asn Tyr Pro Glu
225                 230                 235                 240

Tyr Gln Leu Ala Asp Asn Ser Gly Arg Lys Asp Pro Asn Lys Leu Asp
                245                 250                 255

Ile Ser Asn Pro Glu Ala Val Lys Phe Tyr Lys Thr Leu Ile Asp Glu
            260                 265                 270

Tyr Asp Gly Val Phe Thr Thr Lys Tyr Trp His Met Gly Ala Asp Glu
        275                 280                 285

Tyr Met Ile Gly Thr Ser Phe Asp Asn Tyr Ser Lys Leu Lys Thr Phe
    290                 295                 300

Ala Glu Lys Gln Tyr Gly Ala Gly Ala Thr Pro Asn Asp Ala Phe Thr
305                 310                 315                 320

Gly Phe Ile Asn Asp Ile Asp Lys Tyr Val Lys Ala Lys Gly Lys Gln
                325                 330                 335

Leu Arg Ile Trp Asn Asp Gly Ile Val Asn Thr Lys Asn Val Ser Leu
            340                 345                 350

Asn Lys Asp Ile Val Ile Glu Tyr Trp Tyr Gly Ala Gly Arg Lys Pro
        355                 360                 365

Gln Glu Leu Val Gln Asp Gly Tyr Thr Leu Met Asn Ala Thr Gln Ala
    370                 375                 380

Leu Phe Trp Ser Arg Ser Ala Gln Val Tyr Lys Val Asn Ala Ala Arg
385                 390                 395                 400

Leu Tyr Asn Asn Asn Trp Asn Val Gly Thr Phe Asp Gly Gly Arg Gln
                405                 410                 415

Ile Asp Lys Asn Tyr Asp Lys Leu Thr Gly Ala Lys Val Ser Ile Trp
            420                 425                 430

Pro Asp Ser Ser Tyr Phe Gln Thr Glu Asn Glu Val Glu Lys Glu Ile
        435                 440                 445

Phe Asp Gly Met Arg Phe Ile Ser Gln Met Thr Trp Ser Asp Ser Arg
    450                 455                 460

Pro Trp Ala Thr Trp Asn Asp Met Lys Ala Asp Ile Asp Lys Ile Gly
465                 470                 475                 480
```

```
Tyr Pro Leu Asp Ile Arg Glu Tyr Asp Tyr Thr Pro Val Asp Ala Gly
                485                 490                 495

Ile Tyr Asp Ile Pro Gln Leu Lys Ser Ile Ser Lys Gly Pro Trp Glu
            500                 505                 510

Leu Ile Thr Thr Pro Asp Gly Tyr Tyr Gln Met Lys Asp Thr Val Ser
        515                 520                 525

Gly Lys Cys Leu Ala Leu Phe Thr Gly Ser Lys His Leu Asp Val Val
    530                 535                 540

Thr Gln Val Gly Ala Arg Pro Glu Leu Arg Asn Cys Ala Asp Val Ser
545                 550                 555                 560

Val Gly Gln Asp Gln Arg Asn Thr Ala Asn Glu Arg Asn Thr Gln Lys
                565                 570                 575

Trp Gln Ile Arg Ala Asp Lys Asp Gly Lys Tyr Thr Ile Ser Pro Ala
            580                 585                 590

Leu Thr Gln Gln Arg Leu Ala Ile Ala Thr Gly Asn Glu Gln Asn Ile
        595                 600                 605

Asp Leu Glu Thr His Arg Pro Ala Ala Gly Thr Val Ala Gln Phe Pro
    610                 615                 620

Ala Asp Leu Val Ser Asp Asn Ala Leu Phe Thr Leu Thr Gly His Met
625                 630                 635                 640

Gly Met Ser Ala Thr Val Asp Ser Lys Thr Val Asn Pro Ala Ser Pro
                645                 650                 655

Ser Lys Ile Thr Val Lys Val Arg Ala Ala Ser Asn Ala Asn Thr Gly
            660                 665                 670

Asp Val Thr Val Thr Pro Val Val Pro Glu Gly Trp Glu Ile Lys Pro
        675                 680                 685

Gly Ser Val Ser Leu Lys Ser Ile Pro Ala Gly Lys Ala Ala Ile Ala
    690                 695                 700

Tyr Phe Asn Val Val Asn Thr Thr Gly Thr Gly Asp Ala Thr Val Gln
705                 710                 715                 720

Phe Lys Leu Thr Asn Thr Lys Thr Gly Glu Glu Leu Gly Thr Thr Ser
```

725 730 735

Val Ala Leu Thr Gly Ser Leu Thr Lys Asp Val Glu Ala Ser Asp Tyr
740 745 750

Ala Ala Ser Ser Gln Glu Thr Thr Gly Glu His Ala Pro Val Gly Asn
755 760 765

Ala Phe Asp Lys Asn Ala Asn Thr Phe Trp His Ser Lys Tyr Ser Asn
770 775 780

Pro Ser Ala Asn Leu Pro His Trp Leu Ala Phe Lys Ala Ser Pro Gly
785 790 795 800

Glu Gly Asn Lys Ile Ala Ala Ile Thr His Leu Tyr Arg Gln Asp Lys
805 810 815

Leu Asn Gly Pro Ala Lys Asn Val Ala Val Tyr Val Val Ala Ala Ser
820 825 830

Asp Ala Asn Ser Val Ala Asp Val Thr Asn Trp Gly Glu Pro Val Ala
835 840 845

Thr Ala Glu Phe Pro Tyr Thr Lys Glu Leu Gln Thr Ile Ala Leu Pro
850 855 860

Asn Thr Ile Pro Ser Gly Asp Val Tyr Val Lys Phe Gln Ile Asn Asp
865 870 875 880

Ala Trp Gly Leu Thr Glu Thr Ser Ala Gly Val Thr Trp Ala Ala Val
885 890 895

Ala Glu Leu Ala Ala Thr Ala Lys Ala Thr Pro Val Glu Leu Thr Glu
900 905 910

Pro Glu Gln Pro Lys Asp Asn Pro Glu Val Thr Glu Thr Pro Glu Ala
915 920 925

Thr Gly Val Thr Val Ser Gly Asp Gly Val Ala Asn Gly Ala Leu Ser
930 935 940

Leu Lys Lys Gly Thr Thr Ala Gln Leu Thr Ala Lys Val Ala Pro Asp
945 950 955 960

Asp Ala Asp Gln Ala Val Thr Trp Ala Ser Ser Asp Asp Lys Val Val
965 970 975

Thr Val Asp Lys Thr Gly Lys Val Thr Ala Val Ala Lys Gly Val Ala
980 985 990

Lys Val Thr Ala Thr Thr Ala Asn Gly Lys Ser Ala Ser Val Thr Val
995 1000 1005

Thr Val Thr Glu Asp Ser Glu Val Pro Gly Pro Thr Gly Pro Thr
1010 1015 1020

Glu Pro Thr Lys Pro Gly Thr Glu Leu Glu His His His His His
1025 1030 1035

His

<210> 21
<211> 494
<212> PRT
<213> Actinomycetales bacterium

<400> 21

Met Ser Pro Val Met Asn Thr Val Ile Pro Arg Pro Ser Ser Val Leu
1 5 10 15

Gly Thr Gly Arg Ser Phe Glu Leu Pro Ala Asp Ala Arg Ile Leu Val
20 25 30

Gln Pro Asn Ala Pro Glu Val Ala Gln Ile Gly Glu Phe Leu Ala Ala
35 40 45

Ala Leu Arg Pro Ala Thr Gly Leu Ala Leu Pro Val Thr Ala Gly Ala
50 55 60

Ala Gln Glu Arg Gly Thr Ile His Leu Thr Thr Ala Ala Ala Asp Pro
65 70 75 80

Ala Leu Gly Ala Glu Gly Tyr Glu Leu Leu Val Ala Pro His Gly Val
85 90 95

Leu Leu Ala Ala Pro Arg Pro Ala Gly Leu Phe Arg Gly Val Gln Thr
100 105 110

Ile Arg Gln Leu Leu Pro Ala Ala Val Glu Arg Arg Thr Val Ala Pro
115 120 125

Gly Pro Trp Thr Leu Pro Thr Gly Val Leu Arg Asp Ala Pro Arg Phe
130 135 140

Ala Trp Arg Gly Ala Met Leu Asp Val Ala Arg His Phe Phe Pro Val
145 150 155 160

Ala Asp Val Lys Arg Tyr Leu Asp Leu Leu Ala Tyr Tyr Lys Phe Asn
165 170 175

Val Leu His Leu His Leu Thr Asp Asp Gln Gly Trp Arg Ile Glu Ile
180 185 190

Lys Ser Trp Pro Arg Leu Ala Glu Tyr Gly Gly Ser Thr Ala Val Asp
195 200 205

Gly Ala Pro Gly Gly Tyr Tyr Thr Gln Glu Gln Tyr Ala Asp Leu Ala
210 215 220

Arg Tyr Ala Ala Glu Arg Tyr Ile Thr Ile Val Pro Glu Ile Asp Met
225 230 235 240

Pro Ser His Thr Asn Ala Ala Leu Ala Ser Tyr Ala Leu Leu Asn Cys
245 250 255

Glu Gly Glu Ala Pro Ala Leu Tyr Thr Gly Thr Ala Val Gly Phe Ser
260 265 270

Thr Leu Cys Met Gly Lys Glu Thr Thr Phe Arg Phe Met Asp Asp Val
275 280 285

Leu Gly Glu Leu Ala Ala Leu Thr Pro Gly Pro Tyr Leu His Ile Gly
290 295 300

Gly Asp Glu Ala Asp Ser Thr Ser Pro Glu Asp Tyr Gln Thr Phe Met
305 310 315 320

Arg Gln Val Gln Ala Ile Val Gln Ser His Gly Lys Thr Val Val Gly
325 330 335

Trp Gly Glu Ile Ala Gln Ala Pro Leu Thr Pro Gly Thr Met Val Gln
340 345 350

His Trp Arg Gln Asp Leu Thr Pro Gln Ala Ala Ala Ser Gly His Lys
355 360 365

Val Ile Leu Ser Pro Ala Gly Lys Thr Tyr Leu Asp Met Lys Tyr Asp
370 375 380

Glu Ala Thr Pro Phe Gly Leu Ser Trp Ala Gly Tyr Val Thr Val Gln
385 390 395 400

Asp Ala Phe Ala Trp Asn Pro Gly Ala Tyr Leu Ala Gly Val Gly Glu
405 410 415

Asp Ala Val Ala Gly Val Glu Ala Pro Leu Trp Thr Glu Thr Ile Pro
420 425 430

Thr Phe Ala Glu Ala Glu Phe Met Leu Phe Pro Arg Leu Leu Ala Val
435 440 445

Ala Glu Leu Gly Trp Ser Pro Ala Ala Gly Arg Thr Trp Asp Glu Phe
450 455 460

Arg Ser Arg Leu Ala His His Gly Pro Arg Leu Glu Gly Leu Gly Val
465 470 475 480

Asn Phe Phe Arg Ala Pro Glu Ile Asp Trp Pro Glu Thr Leu
485 490

<210> 22
<211> 537
<212> PRT
<213> Bacteroidetes bacterium

<400> 22

Met Lys Thr Ile Ala Ser Leu Leu Ile Ala Gly Ser Leu Ala Thr Ser
1 5 10 15

Ala Ala Glu Pro Pro Ala Leu Ile Pro Gln Pro Val Ser Val Gln Pro
20 25 30

Gly Glu Gly Ser Phe Lys Phe Ser Ala Gly Thr Gly Ile Arg His Asp
35 40 45

Arg Ala Leu Glu Ser Glu Ala Lys Leu Leu Ala Ser Asp Leu Ala Lys
50 55 60

Leu Thr Asp Ser Gln Pro Lys Thr Val Val Lys Glu Leu Thr Asn Ala
65 70 75 80

Leu Pro Ser Glu Ile Leu Leu Asp Phe Ser Glu Lys Thr Asp Leu Pro
85 90 95

Pro Ser Gly Tyr Glu Leu Lys Val Gln Pro Asn Gly Val Val Ile Arg
100 105 110

Gly Lys Asp Ala Ala Gly Val Phe Leu Gly Thr Arg Thr Leu Leu Gln
115 120 125

```
Leu Leu Pro Ala Lys Leu Ala Glu Gly Ser Ala Ala Ile Pro Ala Val
    130                 135                 140

Thr Ile Thr Asp Tyr Pro Arg Phe Ala Trp Arg Gly Met Met Leu Asp
145                 150                 155                 160

Val Gly Arg His Phe His Pro Val Pro Asp Ile Lys Arg Phe Ile Asp
                165                 170                 175

Trp Met Ala Phe His Lys Leu Asn Ser Phe His Trp His Leu Thr Glu
            180                 185                 190

Asp Gln Gly Trp Arg Ile Glu Ile Lys Lys Tyr Pro Lys Leu Thr Glu
        195                 200                 205

Val Gly Ala Phe Arg Glu Ser Ser Pro Pro Tyr Gly Asn Arg Asn Ser
    210                 215                 220

Asp Asp Gly Val Arg Tyr Gly Gly Phe Tyr Thr Gln Glu Gln Leu Lys
225                 230                 235                 240

Asp Val Val Ala Tyr Ala Ala Ala Arg His Ile Thr Val Val Pro Glu
                245                 250                 255

Ile Glu Met Pro Gly His Ala Ala Ala Ala Ile Ala Ala Tyr Pro Glu
            260                 265                 270

Leu Gly Asn Thr Asp Ile Pro Gly Tyr Ser Pro Lys Val Met Thr Arg
        275                 280                 285

Trp Gly Val His Pro Tyr Ile Phe Ser Pro Lys Glu Glu Thr Phe Thr
    290                 295                 300

Phe Leu Glu Asp Val Leu Ser Glu Val Cys Asp Leu Phe Pro Ser Lys
305                 310                 315                 320

Tyr Ile His Ile Gly Gly Asp Glu Ala Pro Lys Asp Gln Trp Lys Gln
                325                 330                 335

Ser Lys Phe Ala Gln Glu Val Ile Lys Arg Glu Gly Leu Lys Asn Glu
            340                 345                 350

Glu Glu Leu Gln Ser Trp Phe Ile Arg Arg Ile Gly Arg Phe Leu Glu
        355                 360                 365

Ser Lys Asn Arg Asn Leu Ile Gly Trp Asp Glu Ile Gln Glu Gly Gly
    370                 375                 380
```

Leu Pro Lys Thr Ala Thr Met Met Val Trp Arg Asp Ala Lys Trp Ala
385 390 395 400

Lys His Ala Leu Ser Leu Gly Asn Asn Val Val Met Ala Thr Thr Ser
405 410 415

His Thr Tyr Leu Asp Tyr Tyr Gln Asn Pro Ala Ala Thr Glu Leu Ala
420 425 430

Lys Gly Val Glu Tyr Glu Ala Ile Gly Gly His Leu Pro Leu Glu Lys
435 440 445

Val Tyr Ser Tyr Asn Pro Thr Phe Val Ala Glu Asn Pro Gln Gln Glu
450 455 460

Lys Gln Ile Leu Gly Thr Gln Ala Gln Leu Trp Ser Glu Tyr Phe Lys
465 470 475 480

Asp Met Lys Lys Val Glu Tyr His Ala Phe Pro Arg Ile Ala Ala Leu
485 490 495

Ala Glu Val Ala Trp Thr Pro Leu Ala Leu Lys Asn Phe Asp Gly Phe
500 505 510

Ser Lys Arg Leu Glu Gly Ile Met Gln His Tyr Glu Ala Gly Asn Leu
515 520 525

His Tyr Cys Lys Pro Ser Thr Glu Lys
530 535

<210> 23
<211> 885
<212> PRT
<213> Serratia marcescens

<400> 23

Met Asn Ala Phe Lys Leu Ser Ala Leu Ala Arg Leu Thr Ala Thr Met
1 5 10 15

Gly Phe Leu Gly Gly Met Gly Ser Ala Met Ala Asp Gln Gln Leu Val
20 25 30

Asp Gln Leu Ser Gln Leu Lys Leu Asn Val Lys Met Leu Asp Asn Arg
35 40 45

Ala Gly Glu Asn Gly Val Asp Cys Ala Ala Leu Gly Ala Asp Trp Ala
50 55 60

```
Ser Cys Asn Arg Val Leu Phe Thr Leu Ser Asn Asp Gly Gln Ala Ile
65                  70                  75                  80

Asp Gly Lys Asp Trp Val Ile Tyr Phe His Ser Pro Arg Gln Thr Leu
                85                  90                  95

Arg Val Asp Asn Asp Gln Phe Lys Ile Ala His Leu Thr Gly Asp Leu
            100                 105                 110

Tyr Lys Leu Glu Pro Thr Ala Lys Phe Ser Gly Phe Pro Ala Gly Lys
        115                 120                 125

Ala Val Glu Ile Pro Val Val Ala Glu Tyr Trp Gln Leu Phe Arg Asn
    130                 135                 140

Asp Phe Leu Pro Arg Trp Tyr Ala Thr Ser Gly Asp Ala Lys Pro Lys
145                 150                 155                 160

Met Leu Ala Asn Thr Asp Thr Glu Asn Leu Asp Gln Phe Val Ala Pro
                165                 170                 175

Phe Thr Gly Asp Gln Trp Lys Arg Thr Lys Asp Asp Lys Asn Ile Leu
            180                 185                 190

Met Thr Pro Ala Ser Arg Phe Val Ser Asn Ala Asp Leu Gln Thr Leu
        195                 200                 205

Pro Ala Gly Ala Leu Arg Gly Lys Ile Val Pro Thr Pro Met Gln Val
    210                 215                 220

Lys Val His Ala Gln Asp Ala Asp Leu Arg Lys Gly Val Ala Leu Asp
225                 230                 235                 240

Leu Ser Thr Leu Val Lys Pro Ala Ala Asp Val Val Ser Gln Arg Phe
                245                 250                 255

Ala Leu Leu Gly Val Pro Val Gln Thr Asn Gly Tyr Pro Ile Lys Thr
            260                 265                 270

Asp Ile Gln Pro Gly Lys Phe Lys Gly Ala Met Ala Val Ser Gly Ala
        275                 280                 285

Tyr Glu Leu Lys Ile Gly Lys Lys Glu Ala Gln Val Ile Gly Phe Asp
    290                 295                 300

Gln Ala Gly Val Phe Tyr Gly Leu Gln Ser Ile Leu Ser Leu Val Pro
```

305 310 315 320

Ser Asp Gly Ser Gly Lys Ile Ala Thr Leu Asp Ala Ser Asp Ala Pro
325 330 335

Arg Phe Pro Tyr Arg Gly Ile Phe Leu Asp Val Ala Arg Asn Phe His
340 345 350

Lys Lys Asp Ala Val Leu Arg Leu Leu Asp Gln Met Ala Ala Tyr Lys
355 360 365

Leu Asn Lys Phe His Phe His Leu Ser Asp Asp Glu Gly Trp Arg Ile
370 375 380

Glu Ile Pro Gly Leu Pro Glu Leu Thr Glu Val Gly Gly Gln Arg Cys
385 390 395 400

His Asp Leu Ser Glu Thr Thr Cys Leu Leu Pro Gln Tyr Gly Gln Gly
405 410 415

Pro Asp Val Tyr Gly Gly Phe Phe Ser Arg Gln Asp Tyr Ile Asp Ile
420 425 430

Ile Lys Tyr Ala Gln Ala Arg Gln Ile Glu Val Ile Pro Glu Ile Asp
435 440 445

Met Pro Ala His Ala Arg Ala Ala Val Val Ser Met Glu Ala Arg Tyr
450 455 460

Lys Lys Leu His Ala Ala Gly Lys Glu Gln Glu Ala Asn Glu Phe Arg
465 470 475 480

Leu Val Asp Pro Thr Asp Thr Ser Asn Thr Thr Ser Val Gln Phe Phe
485 490 495

Asn Arg Gln Ser Tyr Leu Asn Pro Cys Leu Asp Ser Ser Gln Arg Phe
500 505 510

Val Asp Lys Val Ile Gly Glu Ile Ala Gln Met His Lys Glu Ala Gly
515 520 525

Gln Pro Ile Lys Thr Trp His Phe Gly Gly Asp Glu Ala Lys Asn Ile
530 535 540

Arg Leu Gly Ala Gly Tyr Thr Asp Lys Ala Lys Pro Glu Pro Gly Lys
545 550 555 560

Gly Ile Ile Asp Gln Ser Asn Glu Asp Lys Pro Trp Ala Lys Ser Gln
565 570 575

Val Cys Gln Thr Met Ile Lys Glu Gly Lys Val Ala Asp Met Glu His
580 585 590

Leu Pro Ser Tyr Phe Gly Gln Glu Val Ser Lys Leu Val Lys Ala His
595 600 605

Gly Ile Asp Arg Met Gln Ala Trp Gln Asp Gly Leu Lys Asp Ala Glu
610 615 620

Ser Ser Lys Ala Phe Ala Thr Ser Arg Val Gly Val Asn Phe Trp Asp
625 630 635 640

Thr Leu Tyr Trp Gly Gly Phe Asp Ser Val Asn Asp Trp Ala Asn Lys
645 650 655

Gly Tyr Glu Val Val Val Ser Asn Pro Asp Tyr Val Tyr Met Asp Phe
660 665 670

Pro Tyr Glu Val Asn Pro Asp Glu Arg Gly Tyr Tyr Trp Gly Thr Arg
675 680 685

Phe Ser Asp Glu Arg Lys Val Phe Ser Phe Ala Pro Asp Asn Met Pro
690 695 700

Gln Asn Ala Glu Thr Ser Val Asp Arg Asp Gly Asn His Phe Asn Ala
705 710 715 720

Lys Ser Asp Lys Pro Trp Pro Gly Ala Tyr Gly Leu Ser Ala Gln Leu
725 730 735

Trp Ser Glu Thr Gln Arg Thr Asp Pro Gln Met Glu Tyr Met Ile Phe
740 745 750

Pro Arg Ala Leu Ser Val Ala Glu Arg Ser Trp His Arg Ala Gly Trp
755 760 765

Glu Gln Asp Tyr Arg Ala Gly Arg Glu Tyr Lys Gly Gly Glu Thr His
770 775 780

Phe Val Asp Thr Gln Ala Leu Glu Lys Asp Trp Leu Arg Phe Ala Asn
785 790 795 800

Ile Leu Gly Gln Arg Glu Leu Ala Lys Leu Asp Lys Gly Gly Val Ala
805 810 815

Tyr Arg Leu Pro Val Pro Gly Ala Arg Val Ala Ala Gly Lys Leu Glu
820 825 830

Ala Asn Ile Ala Leu Pro Gly Leu Gly Ile Glu Tyr Ser Thr Asp Gly
835 840 845

Gly Lys Gln Trp Gln Arg Tyr Asp Ala Lys Ala Lys Pro Ala Val Ser
850 855 860

Gly Glu Val Gln Val Arg Ser Val Ser Pro Asp Gly Lys Arg Tyr Ser
865 870 875 880

Arg Ala Glu Lys Val
885

<210> 24
<211> 506
<212> PRT
<213> Streptomyces plicatus

<400> 24

Met Thr Thr Gly Ala Ala Pro Asp Arg Lys Ala Pro Val Arg Pro Thr
1 5 10 15

Pro Leu Asp Arg Val Ile Pro Ala Pro Ala Ser Val Asp Pro Gly Gly
20 25 30

Ala Pro Tyr Arg Ile Thr Arg Gly Thr His Ile Arg Val Asp Asp Ser
35 40 45

Arg Glu Ala Arg Arg Val Gly Asp Tyr Leu Ala Asp Leu Leu Arg Pro
50 55 60

Ala Thr Gly Tyr Arg Leu Pro Val Thr Ala His Gly His Gly Gly Ile
65 70 75 80

Arg Leu Arg Leu Ala Gly Gly Pro Tyr Gly Asp Glu Gly Tyr Arg Leu
85 90 95

Asp Ser Gly Pro Ala Gly Val Thr Ile Thr Ala Arg Lys Ala Ala Gly
100 105 110

Leu Phe His Gly Val Gln Thr Leu Arg Gln Leu Leu Pro Pro Ala Val
115 120 125

Glu Lys Asp Ser Ala Gln Pro Gly Pro Trp Leu Val Ala Gly Gly Thr
130 135 140

Ile Glu Asp Thr Pro Arg Tyr Ala Trp Arg Ser Ala Met Leu Asp Val
145 150 155 160

Ser Arg His Phe Phe Gly Val Asp Glu Val Lys Arg Tyr Ile Asp Arg
165 170 175

Val Ala Arg Tyr Lys Tyr Asn Lys Leu His Leu His Leu Ser Asp Asp
180 185 190

Gln Gly Trp Arg Ile Ala Ile Asp Ser Trp Pro Arg Leu Ala Thr Tyr
195 200 205

Gly Gly Ser Thr Glu Val Gly Gly Gly Pro Gly Gly Tyr Tyr Thr Lys
210 215 220

Ala Glu Tyr Lys Glu Ile Val Arg Tyr Ala Ala Ser Arg His Leu Glu
225 230 235 240

Val Val Pro Glu Ile Asp Met Pro Gly His Thr Asn Ala Ala Leu Ala
245 250 255

Ser Tyr Ala Glu Leu Asn Cys Asp Gly Val Ala Pro Pro Leu Tyr Thr
260 265 270

Gly Thr Lys Val Gly Phe Ser Ser Leu Cys Val Asp Lys Asp Val Thr
275 280 285

Tyr Asp Phe Val Asp Asp Val Ile Gly Glu Leu Ala Ala Leu Thr Pro
290 295 300

Gly Arg Tyr Leu His Ile Gly Gly Asp Glu Ala His Ser Thr Pro Lys
305 310 315 320

Ala Asp Phe Val Ala Phe Met Lys Arg Val Gln Pro Ile Val Ala Lys
325 330 335

Tyr Gly Lys Thr Val Val Gly Trp His Gln Leu Ala Gly Ala Glu Pro
340 345 350

Val Glu Gly Ala Leu Val Gln Tyr Trp Gly Leu Asp Arg Thr Gly Asp
355 360 365

Ala Glu Lys Ala Glu Val Ala Glu Ala Ala Arg Asn Gly Thr Gly Leu
370 375 380

Ile Leu Ser Pro Ala Asp Arg Thr Tyr Leu Asp Met Lys Tyr Thr Lys
385 390 395 400

```
Asp Thr Pro Leu Gly Leu Ser Trp Ala Gly Tyr Val Glu Val Gln Arg
                405                 410                 415

Ser Tyr Asp Trp Asp Pro Ala Gly Tyr Leu Pro Gly Ala Pro Ala Asp
            420                 425                 430

Ala Val Arg Gly Val Glu Ala Pro Leu Trp Thr Glu Thr Leu Ser Asp
        435                 440                 445

Pro Asp Gln Leu Asp Tyr Met Ala Phe Pro Arg Leu Pro Gly Val Ala
    450                 455                 460

Glu Leu Gly Trp Ser Pro Ala Ser Thr His Asp Trp Asp Thr Tyr Lys
465                 470                 475                 480

Val Arg Leu Ala Ala Gln Ala Pro Tyr Trp Glu Ala Ala Gly Ile Asp
                485                 490                 495

Phe Tyr Arg Ser Pro Gln Val Pro Trp Thr
            500                 505
```

<210>  25
<211>  737
<212>  PRT
<213>  Artificial Sequence

<220>
<223>  Artificial sequence

<220>
<221>  misc_feature
<222>  (7)..(7)
<223>  Xaa can be any naturally occurring amino acid

<220>
<221>  misc_feature
<222>  (11)..(13)
<223>  Xaa can be any naturally occurring amino acid

<220>
<221>  misc_feature
<222>  (21)..(22)
<223>  Xaa can be any naturally occurring amino acid

<220>
<221>  misc_feature
<222>  (34)..(34)
<223>  Xaa can be any naturally occurring amino acid

<220>
<221>  misc_feature
<222>  (40)..(40)
<223>  Xaa can be any naturally occurring amino acid

<220>
<221> misc_feature
<222> (42)..(42)
<223> Xaa can be any naturally occurring amino acid

<220>
<221> misc_feature
<222> (45)..(45)
<223> Xaa can be any naturally occurring amino acid

<220>
<221> misc_feature
<222> (51)..(51)
<223> Xaa can be any naturally occurring amino acid

<220>
<221> misc_feature
<222> (53)..(53)
<223> Xaa can be any naturally occurring amino acid

<220>
<221> misc_feature
<222> (55)..(55)
<223> Xaa can be any naturally occurring amino acid

<220>
<221> misc_feature
<222> (65)..(65)
<223> Xaa can be any naturally occurring amino acid

<220>
<221> misc_feature
<222> (72)..(72)
<223> Xaa can be any naturally occurring amino acid

<220>
<221> misc_feature
<222> (74)..(74)
<223> Xaa can be any naturally occurring amino acid

<220>
<221> misc_feature
<222> (79)..(79)
<223> Xaa can be any naturally occurring amino acid

<220>
<221> misc_feature
<222> (84)..(84)
<223> Xaa can be any naturally occurring amino acid

<220>
<221> misc_feature
<222> (86)..(86)
<223> Xaa can be any naturally occurring amino acid

<220>
<221> misc_feature
<222> (107)..(107)
<223> Xaa can be any naturally occurring amino acid

<220>
<221> misc_feature

<222> (111)..(112)
<223> Xaa can be any naturally occurring amino acid

<220>
<221> misc_feature
<222> (123)..(123)
<223> Xaa can be any naturally occurring amino acid

<220>
<221> misc_feature
<222> (137)..(137)
<223> Xaa can be any naturally occurring amino acid

<220>
<221> misc_feature
<222> (139)..(139)
<223> Xaa can be any naturally occurring amino acid

<220>
<221> misc_feature
<222> (165)..(165)
<223> Xaa can be any naturally occurring amino acid

<220>
<221> misc_feature
<222> (168)..(168)
<223> Xaa can be any naturally occurring amino acid

<220>
<221> misc_feature
<222> (210)..(210)
<223> Xaa can be any naturally occurring amino acid

<220>
<221> misc_feature
<222> (215)..(216)
<223> Xaa can be any naturally occurring amino acid

<220>
<221> misc_feature
<222> (233)..(233)
<223> Xaa can be any naturally occurring amino acid

<220>
<221> misc_feature
<222> (247)..(247)
<223> Xaa can be any naturally occurring amino acid

<220>
<221> misc_feature
<222> (252)..(252)
<223> Xaa can be any naturally occurring amino acid

<220>
<221> misc_feature
<222> (287)..(287)
<223> Xaa can be any naturally occurring amino acid

<220>
<221> misc_feature
<222> (299)..(299)
<223> Xaa can be any naturally occurring amino acid

<220>
<221> misc_feature
<222> (317)..(318)
<223> Xaa can be any naturally occurring amino acid

<220>
<221> misc_feature
<222> (337)..(337)
<223> Xaa can be any naturally occurring amino acid

<220>
<221> misc_feature
<222> (346)..(346)
<223> Xaa can be any naturally occurring amino acid

<220>
<221> misc_feature
<222> (350)..(350)
<223> Xaa can be any naturally occurring amino acid

<220>
<221> misc_feature
<222> (368)..(368)
<223> Xaa can be any naturally occurring amino acid

<220>
<221> misc_feature
<222> (385)..(385)
<223> Xaa can be any naturally occurring amino acid

<220>
<221> misc_feature
<222> (398)..(398)
<223> Xaa can be any naturally occurring amino acid

<220>
<221> misc_feature
<222> (426)..(426)
<223> Xaa can be any naturally occurring amino acid

<220>
<221> misc_feature
<222> (429)..(429)
<223> Xaa can be any naturally occurring amino acid

<220>
<221> misc_feature
<222> (435)..(435)
<223> Xaa can be any naturally occurring amino acid

<220>
<221> misc_feature
<222> (549)..(549)
<223> Xaa can be any naturally occurring amino acid

<220>
<221> misc_feature
<222> (557)..(557)
<223> Xaa can be any naturally occurring amino acid

<220>
<221> misc_feature
<222> (608)..(608)

<223> Xaa can be any naturally occurring amino acid

<220>
<221> misc_feature
<222> (610)..(611)
<223> Xaa can be any naturally occurring amino acid

<220>
<221> misc_feature
<222> (639)..(639)
<223> Xaa can be any naturally occurring amino acid

<220>
<221> misc_feature
<222> (642)..(642)
<223> Xaa can be any naturally occurring amino acid

<220>
<221> misc_feature
<222> (645)..(645)
<223> Xaa can be any naturally occurring amino acid

<220>
<221> misc_feature
<222> (647)..(647)
<223> Xaa can be any naturally occurring amino acid

<220>
<221> misc_feature
<222> (693)..(693)
<223> Xaa can be any naturally occurring amino acid

<220>
<221> misc_feature
<222> (711)..(711)
<223> Xaa can be any naturally occurring amino acid

<220>
<221> misc_feature
<222> (729)..(729)
<223> Xaa can be any naturally occurring amino acid

<400> 25

```
Met Ala Ser Met Thr Gly Xaa Gly Gln Met Xaa Xaa Xaa Pro Val Asn
1               5                   10                  15

Val Pro Arg Pro Xaa Xaa Val Gln Ser Phe Asp Ser Gly Tyr Ala Ser
            20                  25                  30

Gly Xaa Gly Tyr Glu Trp Thr Xaa Pro Xaa Gly Tyr Xaa Val Val Asp
        35                  40                  45

Asp Asp Xaa Leu Xaa Ala Xaa Val Asp Glu Phe Val Ala Asp Leu Lys
    50                  55                  60

Xaa Phe Thr Gly Ile Lys Leu Xaa Ala Xaa Lys Gly Ala Ala Xaa Ala
65                  70                  75                  80
```

```
Gly Ala Phe Xaa Val Xaa Leu Asp Ser Ser Ala Ala Ala Gln Leu Gly
                85                  90                  95

Asp Glu Gly Tyr Thr Leu Asp Ile Gly Ala Xaa Gly Val Val Xaa Xaa
            100                 105                 110

Gly Ser Ser Val Thr Gly Val Phe Tyr Gly Xaa Gln Thr Ile Leu Gln
        115                 120                 125

Met Leu Lys Gln Asp Ala Asn Gly Xaa Pro Xaa Gly Thr Met Arg Asp
    130                 135                 140

Tyr Pro Arg Phe Thr Thr Arg Gly Ala Leu Leu Asp Val Ala Arg Lys
145                 150                 155                 160

Pro Val Ser Glu Xaa Met Arg Xaa Ile Thr Arg Met Ala Tyr Tyr Lys
                165                 170                 175

Leu Asn Asp Phe Gln Ala His Leu Ser Asp Asn Glu Trp Leu Glu Asn
            180                 185                 190

Tyr Gly Lys Gly Asp Asn Glu Asp Glu Ala Ala Tyr Phe Tyr Thr Lys
        195                 200                 205

Lys Xaa Phe Lys Gln Phe Xaa Xaa Asp Ala Arg Ala Leu Gly Ile Asn
    210                 215                 220

Val Val Pro Glu Ile Asp Val Pro Xaa His Ala Asn Ser Phe Leu Lys
225                 230                 235                 240

Ile Tyr Pro Glu Leu Met Xaa Asp Gly Arg Val Xaa Pro Pro Leu Ile
                245                 250                 255

Asp His Leu Asp Val Ser Lys Pro Glu Thr Ile Ala Phe Ile Lys Glu
            260                 265                 270

Ile Ile Asp Glu Tyr Asp Pro Thr Phe Thr Ser Asp Lys Tyr Xaa His
        275                 280                 285

Ile Gly Ala Asp Glu Phe Leu Tyr Asn Tyr Xaa Ala Thr Pro Asp Tyr
    290                 295                 300

Arg Lys Phe Ile Asn Glu Ile Val Pro Tyr Val Lys Xaa Xaa Gly Val
305                 310                 315                 320

Arg Met Val Ile Trp Gly Gly Leu Thr Trp Ile Asn Asp Glu Ile Thr
                325                 330                 335
```

Xaa Asp Gly Ile Glu Asn Val Glu Met Xaa Leu Trp Ser Xaa Asp Trp
340 345 350

Ala Asp Gln Met Tyr Asn Met Gly Tyr Lys Leu Ile Asn Thr Ile Xaa
355 360 365

Asp Tyr Gly Tyr Met Val Pro Asp Gly Ser Tyr Gly Arg Ala Asn Pro
370 375 380

Xaa Gly Trp Asp Leu Leu Asn Ile Ser Arg Val Phe Asp Xaa Trp Glu
385 390 395 400

Pro Gly Gly Tyr Gln Ala Val Pro Gly Asp Asp Gln Val Leu Gly Ala
405 410 415

Ala Phe Ala Leu Trp Ser Asp Asn Ile Xaa Thr Asp Xaa Asp Leu Tyr
420 425 430

Tyr Arg Xaa Phe Pro Ala Leu Pro Phe Val Ala Glu Lys Gly Trp Ala
435 440 445

Ala Lys Pro Gly Asp Ile Leu Phe Glu Ala Ala Pro Ala Gly Thr His
450 455 460

Asp Ile Arg Tyr Phe Asp Val Ser Asp Ala Thr Gly Thr Gly Ile Thr
465 470 475 480

Ala Ala Thr Phe Ala Leu Pro Leu Gln Arg Ile Gly Ser Lys Thr Ser
485 490 495

Ala Ile Asn Gly Val Ile Asp Asn Val Ile Val Lys Lys Ser Glu Ala
500 505 510

Thr Gly Thr Thr Asn Ser Glu Thr Gln Tyr Asn Asp Thr Glu Gly Leu
515 520 525

Leu Arg Tyr Ala Phe Asp Asn Asn Pro Ser Thr Ile Trp His Ser Asn
530 535 540

Tyr Lys Gly Ala Xaa Asp Lys Leu Thr Gly Ser Asn Xaa His Tyr Tyr
545 550 555 560

Thr Ile Asn Gln Phe Ser Phe Thr Pro Arg Thr Ser Gln Asp Ser Gly
565 570 575

Gln Val Thr Lys Ala Asp Leu Tyr Val Lys Ala Asn Ala Asn Asp Glu

```
            580                 585                 590

Trp Lys Gln Val Ala Thr Ser Ser Asn Asp Gly Trp Val Ala Val Xaa
        595                 600                 605

Glu Xaa Xaa Val Ala Asn Lys Pro Ser Ser Ala Lys Phe Thr Lys Asp
    610                 615                 620

Ser Thr Pro Asp Pro Gly Pro Lys Ala Val Thr Lys Pro Glu Xaa Thr
625                 630                 635                 640

Gly Xaa Thr Phe Xaa Ala Xaa Gly Leu Ala Val Thr Ala Thr Met Ser
                645                 650                 655

Asp Gly Ser Thr Lys Thr Leu Thr Ala Gly Glu Ala Pro Gln Asp Pro
            660                 665                 670

Asp Lys Thr Thr Thr Trp Ser Asp Gly Lys Thr Ala Leu Leu Lys Asp
        675                 680                 685

Gly Glu Tyr Lys Xaa Ser Ala Val Asp Ala Asp Gly Lys Thr Val Asp
    690                 695                 700

Leu Thr Lys Pro Phe Thr Xaa Ala Gly Asp Val Pro Gly Asp Asn Lys
705                 710                 715                 720

Pro Gly Glu Asn Lys Pro Gly Thr Xaa Leu Glu His His His His His
                725                 730                 735

His


<210>  26
<211>  43
<212>  PRT
<213>  Artificial Sequence

<220>
<223>  Artificial sequence

<400>  26

Thr Thr Thr Thr Thr Thr Ala Ala Ala Ala Ala Ala Ala Ala Cys Cys
1               5                   10                  15

Cys Cys His His His Cys Cys Cys Cys Ala Ala Ala Arg Val His His
            20                  25                  30

His His His Thr Thr Thr Thr Thr Thr Thr Thr
        35                  40
```

```
<210>  27
<211>  36
<212>  PRT
<213>  Artificial Sequence

<220>
<223>  Artificial sequence

<400>  27

Thr Thr Ala Ala Ala Ala Ala Ala Ala Ala Cys Cys Cys Cys His His
1               5                   10                  15

Cys Cys Cys Cys Ala Ala Ala Asp Leu Ser Ser His His His His His
            20                  25                  30

Thr Thr Thr Thr
        35
```